(19) Europäisches Patentamt
European Patent Office
Office européen des brevets

(11) **EP 4 026 906 A1**

(12) **EUROPEAN PATENT APPLICATION**
published in accordance with Art. 153(4) EPC

(43) Date of publication:
**13.07.2022 Bulletin 2022/28**

(21) Application number: **20906472.4**

(22) Date of filing: **25.12.2020**

(51) International Patent Classification (IPC):
***C12N 15/11** (2006.01)* ***C12P 21/02** (2006.01)*

(52) Cooperative Patent Classification (CPC):
**C12N 15/11; C12P 21/02**

(86) International application number:
**PCT/JP2020/048652**

(87) International publication number:
**WO 2021/132546 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2019 PCT/JP2019/051241**
**25.06.2020 JP 2020109292**

(71) Applicant: **CHUGAI SEIYAKU KABUSHIKI KAISHA**
**Tokyo 115-8543 (JP)**

(72) Inventors:
- **KAGOTANI, Mana**
**Kamakura-shi, Kanagawa 247-8530 (JP)**
- **YAMASHITA, Hiroko**
**Kamakura-shi, Kanagawa 247-8530 (JP)**

(74) Representative: **Vossius & Partner**
**Patentanwälte Rechtsanwälte mbB**
**Siebertstraße 3**
**81675 München (DE)**

(54) **COMPOSITION FOR TRANSLATION, AND METHOD FOR PRODUCING PEPTIDE**

(57) An objective of the present invention is to provide novel means that allow reducing the rate of mistranslation into unintended amino acids due to misreading of codons. In a number of embodiments, the present disclosure provides compositions for translation which contain a tRNA having a specific combination of bases at positions 32, 33, 37, and 38 (tRNA numbering rule) in the tRNA. According to a specific embodiment of the present disclosure, the rate of introduction of unintended amino acids due to codon misreading can be reduced.

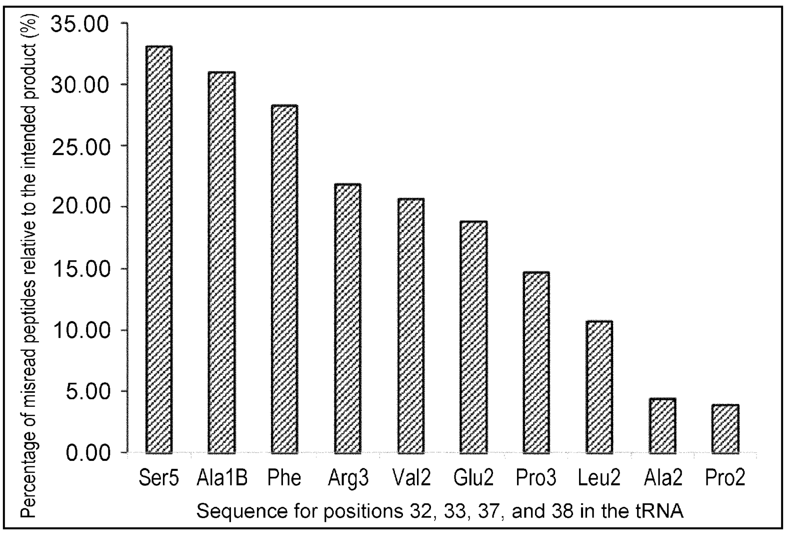


FIG. 1

EP 4 026 906 A1

## Description

[Technical Field]

The present disclosure relates to compositions for translation and methods for producing peptides.

[Background Art]

Cell-free translation systems artificially reconstituted by mixing together only the factors involved in protein translation (Non-Patent Literature (NPL) 1) are being used in a wide range of fields from elucidation of life phenomena to development of new drugs. Since cell-free translation systems do not use microorganisms and cells, they can be used to synthesize highly toxic proteins. Furthermore, since components such as amino acids, tRNAs, and aminoacyl-tRNA synthetases can be removed or added depending on the purpose, correspondence between codons and amino acids can be changed (reprogramming of the genetic code). Because of such features, cell-free translation systems are being applied to synthesis of proteins containing unnatural amino acids, and to construction of display libraries introduced with various building blocks. Cell-free translation systems are thus very useful techniques, but sometimes generate unintended peptides through codon misreading. For example, it has been reported that when RF1 was inactivated and an unnatural amino acid was assigned to the amber codon (UAG), misincorporation of tyrosine (UAU, UAC), lysine (AAG), and glutamine (GAG) occurred at this codon at a certain rate (NPL 2).

Examples of reprogramming the genetic code by artificially dividing codon boxes have been reported. In NPL 3, assignment of one unnatural amino acid and one natural amino acid to C-G-C/G, G-U-C/G, and G-G-C/G was attempted. This literature shows, as a measure for reducing misreading of the GGC codon by $tRNA^{Gly}{}_{ccc}$ attached with Gly, stopping the translation reaction before depletion of a tRNA that carries the anticodon complementary to GGC, *i.e.*, GCC, and that is precharged with an amino acid; however, it does not show what kind of composition for translation can reduce the codon misreading. Artificial division of codon boxes has also been undertaken in NPL 4 to 7 and in Patent Literature (PTL) 1 and 2, but these documents are silent on methods for reducing codon misreading.

tRNAs are known to have specific conserved bases at positions 32 and 38 in the anticodon loop depending on the anticodon triplets. According to a report using tRNA(Ala)GGC carrying the GGC anticodon, the use of the bases conserved in natural $tRNA(Ala)_{GGC}$ at positions 32 and 38 caused less misreading of the GUC codon; on the other hand, alteration of the bases at positions 32 and 38 was shown to cause this tRNA to misread the GUC codon, leading to misincorporation of alanine (GCC) in translation (NPL 8). This literature only studied misreading of the GUC codon by tRNA(Ala)GGC using the natural genetic code table, and does not mention codon misreading that may take place due to reprogramming of the genetic code table.

[Citation List]

[Patent Literature]

[PL 1] WO2019/139126
[PL 2] WO2012/026566

[Non-patent Literature]

[NPL 1] Shimizu et al., Nat. Biotechnol. 2001 Aug; 19(8): 751-755
[NPL 2] Gan et al., Biochem. Biophys. Acta, 2017 Dec; 1861; 3047-3052.
[NPL 3] Iwane et al., Method Mol. Biol., 2018, 1728, 17-47.
[NPL 4] Passioura et al., J Am. Chem. Soc., 2018, 140, 11551-11555.
[NPL 5] Passioura et al., Cell Chem. Biol., 2018, 25, 906-915.
[NPL 6] Iwane et al., Nat. Chem., 2016, 8, 317-325.
[NPL 7] Azusa et al., Peptide Science 2009, 2010 Mar; 17-18.
[NPL 8] urakami et al., Nat. Struct. Mol. Biol., 2009 Mar; 16; 353-358.

[Summary of Invention]

[Technical Problem]

The present invention was achieved in view of the above circumstances. An objective of the present invention is to provide compositions for translation that enable reducing the rate of mistranslation into unintended amino acids attributable to misreading of codons by tRNAs, and methods for producing peptides using the compositions for translation.

[Solution to Problem]

The present inventors discovered that the above problem can be solved by selecting specific bases in the anticodon loop of the tRNA used for translation. The present disclosure is based on such findings, and specifically encompasses the embodiments exemplified below:

[1] a composition for translation, comprising a first tRNA to which a first amino acid is attached and a second tRNA to which a second amino acid is attached, wherein

a combination of bases at positions 32, 33, 37, and 38 (tRNA numbering rule) in the first tRNA is:

(1) 32U, 33U, 37G, and 38A;
(2) 32A, 33U, 37G, and 38U;
(3) 32A, 33U, 37A, and 38U;
(4) 32U, 33U, 37G, and 38U;
(5) 32U, 33U, 37A, and 38U; or
(6) 32C, 33U, 37G, and 38A;

bases at the first letters of the anticodons in the first tRNA and the second tRNA are different from each other,
bases at the second letters of the anticodons in the first tRNA and the second tRNA are the same,
bases at the third letters of the anticodons in the first tRNA and the second tRNA are the same, and
at least one of the first amino acid and the second amino acid is an unnatural amino acid, and wherein A is adenine, C is cytosine, G is guanine, and U is uracil;

[2] the composition of [1], wherein the anticodon in the first tRNA is $N_{11}N_{12}N_{13}$ (herein, $N_{11}$, $N_{12}$, and $N_{13}$ are each independently A, C, G, or U);
[3] the composition of [1] or [2], wherein the anticodon in the second tRNA is $N_{21}N_{22}N_{23}$ (herein, $N_{21}$, $N_{22}$, and $N_{23}$ are each independently A, C, G, or U);
[4] the composition of any one of [1] to [3], wherein a tRNA body of the first tRNA is a chimeric tRNA body;
[5] the composition of any one of [1] to [4], wherein the base at the first letter of the anticodon in the first tRNA is A or G, and the base at the first letter of the anticodon in the second tRNA is C or U; or the base at the first letter of the anticodon in the first tRNA is C or U, and the base at the first letter of the anticodon in the second tRNA is A or G;
[6] the composition of any one of [1] to [5], wherein the base at the first letter of the anticodon in the first tRNA is A or G, and the base at the first letter of the anticodon in the second tRNA is C or U;
[7] the composition of any one of [1] to [6], wherein the base at the first letter of the anticodon in the first tRNA is A, and the base at the first letter of the anticodon in the second tRNA is C;

the base at the first letter of the anticodon in the first tRNA is C, and the base at the first letter of the anticodon in the second tRNA is A;
the base at the first letter of the anticodon in the first tRNA is G, and the base at the first letter of the anticodon in the second tRNA is C;
the base at the first letter of the anticodon in the first tRNA is C, and the base at the first letter of the anticodon in the second tRNA is G;
the base at the first letter of the anticodon in the first tRNA is A, and the base at the first letter of the anticodon in the second tRNA is U;
the base at the first letter of the anticodon in the first tRNA is U, and the base at the first letter of the anticodon in the second tRNA is A;
the base at the first letter of the anticodon in the first tRNA is G, and the base at the first letter of the anticodon in the second tRNA is U; or
the base at the first letter of the anticodon in the first tRNA is U, and the base at the first letter of the anticodon

in the second tRNA is G;

[8] the composition of any one of [1] to [7], wherein the base at the first letter of the anticodon in the first tRNA is A, and the base at the first letter of the anticodon in the second tRNA is C;

the base at the first letter of the anticodon in the first tRNA is C, and the base at the first letter of the anticodon in the second tRNA is A;
the base at the first letter of the anticodon in the first tRNA is A, and the base at the first letter of the anticodon in the second tRNA is U;
the base at the first letter of the anticodon in the first tRNA is U, and the base at the first letter of the anticodon in the second tRNA is A;
the base at the first letter of the anticodon in the first tRNA is G, and the base at the first letter of the anticodon in the second tRNA is U; or
the base at the first letter of the anticodon in the first tRNA is U, and the base at the first letter of the anticodon in the second tRNA is G;

[9] the composition of any one of [1] to [7], wherein the base at the first letter of the anticodon in the first tRNA is A, and the base at the first letter of the anticodon in the second tRNA is C;

the base at the first letter of the anticodon in the first tRNA is C, and the base at the first letter of the anticodon in the second tRNA is A;
the base at the first letter of the anticodon in the first tRNA is A, and the base at the first letter of the anticodon in the second tRNA is U;
the base at the first letter of the anticodon in the first tRNA is G, and the base at the first letter of the anticodon in the second tRNA is U; or
the base at the first letter of the anticodon in the first tRNA is G, and the base at the first letter of the anticodon in the second tRNA is C;

[10] the composition of any one of [1] to [7], wherein the base at the first letter of the anticodon in the first tRNA is A, and the base at the first letter of the anticodon in the second tRNA is C;

the base at the first letter of the anticodon in the first tRNA is C, and the base at the first letter of the anticodon in the second tRNA is A;
the base at the first letter of the anticodon in the first tRNA is G, and the base at the first letter of the anticodon in the second tRNA is C; or
the base at the first letter of the anticodon in the first tRNA is C, and the base at the first letter of the anticodon in the second tRNA is G;

[11] the composition of any one of [1] to [10], wherein the base at the first letter of the anticodon in the first tRNA is A, and the base at the first letter of the anticodon in the second tRNA is C; or the base at the first letter of the anticodon in the first tRNA is C, and the base at the first letter of the anticodon in the second tRNA is A;
[12] the composition of any one of [1] to [7] and [10], wherein the base at the first letter of the anticodon in the first tRNA is G, and the base at the first letter of the anticodon in the second tRNA is C; or
the base at the first letter of the anticodon in the first tRNA is C, and the base at the first letter of the anticodon in the second tRNA is G;
[13] the composition of any one of [1] to [8], wherein the base at the first letter of the anticodon in the first tRNA is A, and the base at the first letter of the anticodon in the second tRNA is U; or the base at the first letter of the anticodon in the first tRNA is U, and the base at the first letter of the anticodon in the second tRNA is A;
[14] the composition of any one of [1] to [8], wherein the base at the first letter of the anticodon in the first tRNA is G, and the base at the first letter of the anticodon in the second tRNA is U; or the base at the first letter of the anticodon in the first tRNA is U, and the base at the first letter of the anticodon in the second tRNA is G;
[15] the composition of any one of [1] to [11], wherein the base at the first letter of the anticodon in the first tRNA is A, and the base at the first letter of the anticodon in the second tRNA is C;
[16] the composition of any one of [1] to [15], wherein a combination of bases at the second and third letters of the anticodons in the first and the second tRNAs is:

(i) G at the second letter and G at the third letter;
(ii) A at the second letter and G at the third letter;

(iii) C at the second letter and C at the third letter;
(iv) G at the second letter and C at the third letter;
(v) A at the second letter and C at the third letter;
(vi) G at the second letter and U at the third letter;
(vii) G at the second letter and A at the third letter; or
(viii) C at the second letter and G at the third letter;

[17] the composition of any one of [1] to [16], wherein a combination of bases at the second and third letters of the anticodons in the first and the second tRNAs is

(i) G at the second letter and G at the third letter;
(ii) A at the second letter and G at the third letter;
(iii) C at the second letter and C at the third letter;
(iv) G at the second letter and C at the third letter;
(v) A at the second letter and C at the third letter;
(vi) G at the second letter and U at the third letter; or
(vii) G at the second letter and A at the third letter;

[18] the composition of any one of [1] to [17], wherein a combination of bases at the second and third letters of the anticodons in the first and the second tRNAs is:

(i) G at the second letter and G at the third letter;
(ii) A at the second letter and G at the third letter; or
(iii) C at the second letter and C at the third letter;

[19] the composition of any one of [1] to [18], wherein the base is G at the second letter and the base is G at the third letter of the anticodons in the first and the second tRNAs;
[20] the composition of any one of [1] to [18], wherein the base is A at the second letter and the base is G at the third letter of the anticodons in the first and the second tRNAs;
[21] the composition of any one of [1] to [18], wherein the base is C at the second letter and the base is C at the third letter of the anticodons in the first and the second tRNAs;
[22] the composition of any one of [1] to [21], wherein the base sequences of positions 1 to 31 and positions 39 to 74 (tRNA numbering rule) in the first tRNA are not derived from any of the base sequences of positions 1 to 31 and positions 39 to 74 (tRNA numbering rule) in a tRNA having at least one base sequence selected from the group consisting of SEQ ID NOs: 275, 294, 295, 296, 302, 303, and 304;
[23] the composition of any one of [1] to [22], wherein the base sequences of positions 1 to 31 and positions 39 to 74 (tRNA numbering rule) in the first tRNA are derived from the base sequences of positions 1 to 31 and positions 39 to 74 (tRNA numbering rule) in a tRNA having at least one base sequence selected from the group consisting of (a) SEQ ID NO: 253, (b) SEQ ID NO: 255, and (c) SEQ ID NO: 254;
[24] the composition of any one of [1] to [23], wherein the base sequences of positions 1 to 31 and positions 39 to 74 (tRNA numbering rule) in the first tRNA are the base sequences of positions 1 to 31 and positions 39 to 74 (tRNA numbering rule) in a tRNA having a base sequence set forth in at least one selected from the group consisting of (a) SEQ ID NO: 253, (b) SEQ ID NO: 255, and (c) SEQ ID NO: 254;
[25] the composition of any one of [1] to [24], wherein the base sequences of positions 1 to 31 and positions 39 to 74 (tRNA numbering rule) in the first tRNA have sequence identity of 80% or more, preferably 90% or more, and more preferably 95% or more to the base sequences of positions 1 to 31 and positions 39 to 74 (tRNA numbering rule) in a tRNA having the base sequence of any one of (a) to (c) of [24];
[26] the composition of any one of [1] to [25], wherein the base sequences of positions 1 to 31 and positions 39 to 74 (tRNA numbering rule) in the second tRNA are not derived from any of the base sequences of positions 1 to 31 and positions 39 to 74 (tRNA numbering rule) in a tRNA having at least one base sequence selected from the group consisting of SEQ ID NOs: 275, 294, 295, 296, 302, 303, and 304;
[27] the composition of any one of [1] to [26], wherein the base sequences of positions 1 to 31 and positions 39 to 74 (tRNA numbering rule) in the second tRNA are derived from the base sequences of positions 1 to 31 and positions 39 to 74 (tRNA numbering rule) in a tRNA having at least one base sequence selected from the group consisting of (a) SEQ ID NO: 253, (b) SEQ ID NO: 255, and (c) SEQ ID NO: 254;
[28] the composition of any one of [1] to [27], wherein the base sequences of positions 1 to 31 and positions 39 to 74 (tRNA numbering rule) in the second tRNA are the base sequences of positions 1 to 31 and positions 39 to 74 (tRNA numbering rule) in a tRNA having at least one base sequence selected from the group consisting of (a) SEQ

ID NO: 253, (b) SEQ ID NO: 255, and (c) SEQ ID NO: 254;
[29] the composition of any one of [1] to [28], wherein the base sequences of positions 1 to 31 and positions 39 to 74 (tRNA numbering rule) in the second tRNA have sequence identity of 80% or more, preferably 90% or more, and more preferably 95% or more to the base sequences of positions 1 to 31 and positions 39 to 74 (tRNA numbering rule) in a tRNA having the base sequence of any one of (a) to (c) of [28];
[30] the composition of any one of [1] to [29], wherein the base sequences of positions 1 to 31 and positions 39 to 74 (tRNA numbering rule) in the first tRNA and the second tRNA are identical or have sequence identity of 80% or more, preferably 90% or more, and more preferably 95% or more;
[31] the composition of any one of [1] to [30], wherein the base sequences of positions 1 to 31 and positions 39 to 74 (tRNA numbering rule) in the first tRNA and the second tRNA are identical;
[32] the composition of any one of [4] to [31], wherein the chimeric tRNA body is a chimeric tRNA body in which the combination of bases at positions 32, 33, 37, and 38 (tRNA numbering rule) and the base sequences of the other portions have different origins;
[33] the composition of any one of [1] to [32], wherein the base sequence of positions 32 to 38 (tRNA numbering rule) in the first tRNA is different from the base sequence of positions 32 to 38 (tRNA numbering rule) in a wild-type tRNA from *Escherichia coli*;
[34] the composition of any one of [1] to [33], wherein the first tRNA is a tRNA having a chimeric anticodon loop;
[35] the composition of any one of [1] to [34], wherein the second amino acid is an unnatural amino acid;
[36] the composition of any one of [1] to [35], wherein the first amino acid is an unnatural amino acid;
[37] the composition of any one of [1] to [36], wherein either one or both of the first amino acid and the second amino acid are N-substituted amino acids;
[38] the composition of any one of [1] to [37], wherein the first tRNA is an artificial tRNA;
[39] the composition of any one of [1] to [38], wherein the second tRNA is an artificial tRNA;
[40] the composition of any one of [1] to [39], wherein the base sequence of positions 75 and 76 (tRNA numbering rule) in the first tRNA is CA;
[41] the composition of any one of [1] to [40], wherein the base sequence of positions 75 and 76 (tRNA numbering rule) in the second tRNA is CA;
[42] the composition of any one of [1] to [41], wherein the first tRNA is a tRNA not containing a modified base;
[43] the composition of any one of [1] to [42], wherein the second tRNA is a tRNA not containing a modified base;
[44] the composition of any one of [1] to [43], wherein the first amino acid is attached to the 3' end of the first tRNA;
[45] the composition of any one of [1] to [44], wherein the second amino acid is attached to the 3' end of the second tRNA;
[46] the composition of any one of [1] to [45], wherein the first tRNA is a tRNA having a base sequence consisting of A, U, G, and C;
[47] the composition of any one of [1] to [46], wherein the second tRNA is a tRNA having a base sequence consisting of A, U, G, and C;
[48] the composition of any one of [1] to [47], wherein the first tRNA is a tRNA to which an amino acid is attached outside a translation system;
[49] the composition of any one of [1] to [48], wherein the second tRNA is a tRNA to which an amino acid is attached outside a translation system;
[50] the composition of any one of [1] to [49], wherein the first tRNA and the second tRNA are E. coli-derived tRNAs;
[51] the composition of any one of [1] to [50], wherein the combination of bases at positions 32, 33, 37, and 38 (tRNA numbering rule) in the first tRNA is:

(1) 32U, 33U, 37G, and 38A;
(2) 32A, 33U, 37G, and 38U;
(3) 32A, 33U, 37A, and 38U; or
(4) 32U, 33U, 37G, and 38U;

[52] the composition of any one of [1] to [51],
wherein the combination of bases at positions 32, 33, 37, and 38 (tRNA numbering rule) in the first tRNA is:

(1) 32U, 33U, 37G, and 38A;
(2) 32A, 33U, 37G, and 38U; or
(3) 32A, 33U, 37A, and 38U;

[53] the composition of any one of [1] to [52],
wherein the combination of bases at positions 32, 33, 37, and 38 (tRNA numbering rule) in the first tRNA is:

(1) 32U, 33U, 37G, and 38A;
(3) 32A, 33U, 37A, and 38U; or
(4) 32U, 33U, 37G, and 38U;

[54] the composition of any one of [1] to [53], wherein the combination of bases at positions 32, 33, 37, and 38 (tRNA numbering rule) in the first tRNA is either 32U, 33U, 37G, and 38A, or 32U, 33U, 37G, and 38U;
[55] the composition of any one of [1] to [54], wherein the combination of bases at positions 32, 33, 37, and 38 (tRNA numbering rule) in the first tRNA is 32U, 33U, 37G, and 38A;
[56] the composition of any one of [1] to [52], wherein the combination of bases at positions 32, 33, 37, and 38 (tRNA numbering rule) in the first tRNA is 32A, 33U, 37G, and 38U;
[57] the composition of any one of [1] to [53], wherein the combination of bases at positions 32, 33, 37, and 38 (tRNA numbering rule) in the first tRNA is 32A, 33U, 37A, and 38U;
[58] the composition of any one of [1] to [51], wherein the combination of bases at positions 32, 33, 37, and 38 (tRNA numbering rule) in the first tRNA is 32U, 33U, 37G, and 38U;
[59] the composition of any one of [1] to [50], wherein the combination of bases at positions 32, 33, 37, and 38 (tRNA numbering rule) in the first tRNA is 32U, 33U, 37A, and 38U;
[60] the composition of any one of [1] to [50], wherein the combination of bases at positions 32, 33, 37, and 38 (tRNA numbering rule) in the first tRNA is 32C, 33U, 37G, and 38A;
[61] the composition of any one of [1] to [60], wherein the combination of bases at positions 32, 33, 37, and 38 (tRNA numbering rule) in the second tRNA is:

(1) 32U, 33U, 37G, and 38A;
(2) 32A, 33U, 37G, and 38U;
(3) 32A, 33U, 37A, and 38U;
(4) 32U, 33U, 37G, and 38U;
(5) 32U, 33U, 37A, and 38U; or
(6) 32C, 33U, 37G, and 38A;

[62] the composition of any one of [1] to [61], wherein the combination of bases at positions 32, 33, 37, and 38 (tRNA numbering rule) in the second tRNA is:

(1) 32U, 33U, 37G, and 38A;
(2) 32A, 33U, 37G, and 38U;
(3) 32A, 33U, 37A, and 38U; or
(4) 32U, 33U, 37G, and 38U;

[63] the composition of any one of [1] to [62], wherein a tRNA body of the second tRNA is a chimeric tRNA body;
[64] the composition of any one of [1] to [63], which is a composition for cell-free translation;
[65] the composition of any one of [1] to [64], which is a composition for reconstituted cell-free translation;
[66] the composition of any one of [1] to [65], which is a composition for cell-free translation that has been reconstituted by an E. coli-derived factor;
[67] the composition of any one of [1] to [66], comprising an E. coli-derived ribosome;
[68] the composition of any one of [1] to [67], comprising an mRNA that has a codon complementary to an anticodon in the second tRNA;
[69] the composition of any one of [1] to [68], comprising an mRNA that has a codon complementary to an anticodon in the first tRNA and a codon complementary to an anticodon in the second tRNA, wherein these complementary codons may optionally be included in the same or different mRNAs;
[70] the composition of any one of [1] to [69], which does not comprise an aminoacyl-tRNA synthetase (aaRS) that can attach an amino acid to the first tRNA or the second tRNA;
[71] the composition of any one of [1] to [70] (provided that, when the first tRNA and the second tRNA have a chimeric tRNA body which has the combination of bases specified in (4) of the above [1] at positions 32, 33, 37, and 38 (tRNA numbering rule), and which at positions 1 to 31 and positions 39 to 74 (tRNA numbering rule) has the base sequences of positions 1 to 31 and positions 39 to 74 (tRNA numbering rule) of a tRNA having the base sequence of SEQ ID NO: 255, a composition in which the combination of the anticodons in the first tRNA and the second tRNA is GCG and CCG, and a composition in which the combination of the anticodons in the first tRNA and the second tRNA is CCG and GCG, are excluded);
[72] the composition of any one of [1] to [71] (provided that, when the first tRNA and the second tRNA have a chimeric tRNA body which has the combination of bases specified in (4) of the above [1] at positions 32, 33, 37, and 38 (tRNA

numbering rule), a composition in which the combination of the anticodons in the first tRNA and the second tRNA is GCG and CCG, and a composition in which the combination of the anticodons in the first tRNA and the second tRNA is CCG and GCG, are excluded);

[73] the composition of any one of [1] to [72] (provided that, when the first tRNA and the second tRNA have a chimeric tRNA body which has the combination of bases specified in (4) of the above [1] at positions 32, 33, 37, and 38 (tRNA numbering rule), a composition in which the combinations of the anticodons in the first tRNA and the second tRNA is AAG and UAG, a composition in which the combination of the anticodons in the first tRNA and the second tRNA is AAG and CAG, and a composition in which the combination of the anticodons in the first tRNA and the second tRNA is UAG and CAG, are excluded);

[74] the composition of any one of [1] to [73] (provided that, when the first tRNA and the second tRNA have a chimeric tRNA body which has the combination of bases specified in (4) of the above [1] at positions 32, 33, 37, and 38 (tRNA numbering rule), a composition in which both bases at the second letter of the anticodons in the first tRNA and the second tRNA are C and both bases at the third letter of the anticodons in the first tRNA and the second tRNA are G, is excluded);

[75] the composition of any one of [1] to [74] (provided that, when the first tRNA and the second tRNA have a chimeric tRNA body which has the combination of bases specified in (4) of the above [1] at positions 32, 33, 37, and 38 (tRNA numbering rule), a composition in which both bases at the second letter of the anticodons in the first tRNA and the second tRNA are A and both bases at the third letter of the anticodons in the first tRNA and the second tRNA are G, is excluded);

[76] the composition of any one of [1] to [75] (provided that a composition in which both bases at the second letter of the anticodons in the first tRNA and the second tRNA are C and both bases at the third letter of the anticodons in the first tRNA and the second tRNA are G, is excluded);

[77] the composition of any one of [1] to [76] (provided that, when the first tRNA has the combination of bases specified in (4) of the above [1] at positions 32, 33, 37, and 38 (tRNA numbering rule), a composition in which the base at the second letter of the anticodon in the first tRNA is A and the base at the third letter of the anticodon in the first tRNA is G, is excluded);

[78] the composition of any one of [1] to [77] (provided that, when the first tRNA has the combination of bases specified in (1) of the above [1] at positions 32, 33, 37, and 38 (tRNA numbering rule), compositions in which the anticodons in the first tRNA are UGG and CGG, are excluded);

[79] the composition of any one of [1] to [78] (provided that, when the first tRNA has the combination of bases specified in (2) of the above [1] at positions 32, 33, 37, and 38 (tRNA numbering rule), compositions in which the anticodons in the first tRNA are AGG and GGG, are excluded);

[80] the composition of any one of [1] to [79] (provided that, when the first tRNA has a chimeric tRNA body which has the combination of bases specified in (6) of the above [1] at positions 32, 33, 37, and 38 (tRNA numbering rule), compositions in which the anticodons in the first tRNA are GCG and CCG, are excluded);

[81] the composition of any one of [1] to [80] (provided that, when the first tRNA has a chimeric tRNA body which has the combination of bases specified in (3) of the above [1] at positions 32, 33, 37, and 38 (tRNA numbering rule), compositions in which the anticodons in the first tRNA are AGC and GGC, are excluded);

[82] the composition of any one of [1] to [81], wherein the first tRNA and the second tRNA are elongator tRNAs;

[83] the composition of any one of [37] to [82], wherein the N-substituted amino acid is an N-methyl amino acid;

[84] the composition of any one of [1] to [83], wherein the amino acid is a translatable amino acid;

[85] the composition of any one of [1] to [84], wherein the second tRNA is a tRNA having a chimeric anticodon loop;

[86] a method for producing the composition for translation of any one of [1] to [85], which comprises preparing the first tRNA by attaching the first amino acid to a tRNA outside a translation system, and/or preparing the second tRNA by attaching the second amino acid to a tRNA outside a translation system;

[87] the method of [86], wherein the first tRNA and/or the second tRNA are prepared by at least one method selected from the group consisting of the pCpA method, the pdCpA method, a method using a flexizyme, and a method involving aaRS;

[88] the method of [86] or [87], comprising preparing the first tRNA by *in vitro* transcription; and

[89] the method of any one of [86] to [88], comprising preparing the second tRNA by *in vitro* transcription.

[A1] a method for producing a peptide, comprising translating a nucleic acid using the composition for translation of any one of [1] to [85] or a composition for translation produced by the method of any one of [86] to [89];

[A2] a method for reducing misreading of a codon complementary to the anticodon in the second tRNA by the first tRNA, comprising translating a nucleic acid using the composition for translation of any one of [1] to [85] or a composition for translation produced by the method of any one of [86] to [89];

[A3] the method of [A1] or [A2], wherein the nucleic acid is an mRNA carrying a codon complementary to an anticodon in the second tRNA;
[A4] the method of [A3], wherein the nucleic acid is an mRNA carrying a codon complementary to an anticodon in the first tRNA, and optionally, the codon complementary to an anticodon in the first tRNA and the codon complementary to an anticodon in the second tRNA may be included in the same or different mRNAs;
[A5] the method of any one of [A1] to [A4], comprising obtaining a peptide library by translating a nucleic acid library containing the above nucleic acids;
[A6] the method of any one of [A1] to [A5], wherein the codon complementary to an anticodon in the first tRNA is a codon that forms Watson-Crick base pairs at all three bases with the anticodon;
[A7] the method of any one of [A1] to [A6], wherein the codon complementary to an anticodon in the second tRNA is a codon that forms Watson-Crick base pairs at all three bases with the anticodon;
[A8] the method of any one of [A1] to [A5], wherein the third letter of the codon complementary to an anticodon in the first tRNA forms a wobble base pair with the first letter of the anticodon; [A9] the method of any one of [A1] to [A6] or [A8], wherein the third letter of the codon complementary to the anticodon in the second tRNA forms a wobble base pair with the first letter of the anticodon;
[A10] the method of any one of [A1] to [A9], wherein the nucleic acid is mRNA;
[A11] a peptide or a peptide library produced by the method of any one of [A1] to [A10];
[A12] a peptide produced by using the composition for translation of any one of [1] to [85] or the composition for translation produced by the method of any one of [86] to [89];
[A13] a peptide library produced by using the composition for translation of any one of [1] to [85] or the composition for translation produced by the method of any one of [86] to [89]; and
[A14] a method for identifying a peptide that binds to a target molecule comprising: producing a peptide library using the method of any one of [A1] to [A10]; and contacting the target molecule with the peptide library.

[B1] a method for reducing misreading of a second codon by a tRNA carrying an anticodon complementary to a first codon, comprising substituting at least one base selected from the group consisting of bases at positions 32, 33, 37, and 38 (tRNA numbering rule) in the tRNA , wherein the bases at the first letters of the first codon and the second codon are the same, the bases at the second letters of the first codon and the second codon are the same, and the bases at the third letters of the first codon and the second codon are different from each other;
[B2] the method of [B1], wherein, a combination of bases at positions 32, 33, 37, and 38 (tRNA numbering rule) after the substitution is:

(1) 32U, 33U, 37G, and 38A;
(2) 32A, 33U, 37G, and 38U;
(3) 32A, 33U, 37A, and 38U;
(4) 32U, 33U, 37G, and 38U;
(5) 32U, 33U, 37A, and 38U; or
(6) 32C, 33U, 37G, and 38A;
and wherein, A is adenine, C is cytosine, G is guanine, and U is uracil;

[B3] the method of [B1] or [B2], comprising substituting at least one base selected from the group consisting of bases at positions 32, 33, 37, and 38 (tRNA numbering rule) in a tRNA carrying an anticodon complementary to the first codon;
[B4] the method of any one of [B1] to [B3], wherein the first codon is $M_1M_2X$ and the second codon is $M_1M_2Y$,

the above $M_1$ and $M_2$ are each independently A, C, G, or U, and
the above X and Y are each selected from A, C, G, and U, and are different from each other;

[B5] the method of [B4], wherein the combination of the bases of the above X and Y is any one selected from the following group:
(a1) U and G; (a2) G and U; (a3) U and A; (a4) A and U; (a5) C and A; (a6) A and C; (a7) C and G; and (a8) G and C;
[B6] the method of [B4] or [B5], wherein the combination of the bases of the above X and Y is any one selected from the following group:
(a1) U and G; (a2) G and U; (a3) U and A; (a5) C and A; and (a7) C and G;
[B7] the method of [B5], wherein the combination of bases of the above X and Y is (a1) U and G or (a2) G and U;
[B8] the method of any one of [B5] to [B7], wherein the combination of bases of the above X and Y is (a1) U and G;

[B9] the method of any one of [B5] to [B7], wherein the combination of bases of the above X and Y is (a2) G and U;
[B10] the method of [B5] or [B6], wherein the combination of bases of the above X and Y is (a3) U and A;
[B11] the method of [B5] or [B6], wherein the combination of bases of the above X and Y is (a5) C and A;
[B12] the method of [B5] or [B6], wherein the combination of bases of the above X and Y is (a7) C and G;
[B13] the method of any one of [B4] to [B12], wherein the above $M_1M_2$ is a base sequence of any one selected from the following group:
(b1) CC; (b2) CU; (b3) GG; (b4) GU; (b5) GC; (b6) UC; (b7) CG; and (b8) AC;
[B14] the method of [B13], wherein the above $M_1M_2$ is any one base sequence selected from the following group: (b1) CC; (b2) CU; and (b3) GG;
[B15] the method of [B13] or [B14], wherein the base sequence of the above $M_1M_2$ is (b1) CC;
[B16] the method of [B13] or [B14], wherein the base sequence of the above $M_1M_2$ is (b2) CU;
[B17] the method of [B13] or [B14], wherein the base sequence of the above $M_1M_2$ is (b3) GG;
[B18] the method of any one of [B1] to [B17], wherein the first codon and the anticodon in the tRNA form Watson-Crick base pairs at all three bases;
[B19] the method of any one of [B1] to [B17], wherein the base at the third letter of the first codon and the base at the first letter of the anticodon in the tRNA form a wobble base pair;
[B20] the method of any one of [B1] to [B19], wherein the base sequences of positions 1 to 31 and positions 39 to 74 (tRNA numbering rule) in the tRNA are derived from the base sequences of positions 1 to 31 and positions 39 to 74 (tRNA numbering rule) in a tRNA having the base sequence of at least one selected from the group consisting of (a) SEQ ID NO: 253, (b) SEQ ID NO: 255, and (c) SEQ ID NO: 254;
[B21] the method of any one of [B1] to [B20], wherein the base sequences of positions 1 to 31 and positions 39 to 74 (tRNA numbering rule) in the tRNA are the base sequences of positions 1 to 31 and positions 39 to 74 (tRNA numbering rule) in a tRNA having the base sequence of at least one selected from the group consisting of (a) SEQ ID NO: 253, (b) SEQ ID NO: 255, and (c) SEQ ID NO: 254;
[B22] the method of any one of [B1] to [B21], wherein the base sequences of positions 1 to 31 and positions 39 to 74 (tRNA numbering rule) in the tRNA have sequence identity of 80% or more, preferably 90% or more, and more preferably 95% or more to the base sequences of positions 1 to 31 and positions 39 to 74 (tRNA numbering rule) in a tRNA having the base sequence of any one of (a) to (c) of [B20];
[B23] the method of any one of [B1] to [B22], comprising attaching an amino acid to the tRNA outside a translation system;
[B24] the method of [B23], wherein attaching the amino acid to the tRNA outside a translation system is performed by at least one method selected from the group consisting of the pCpA method, the pdCpA method, a method using a flexizyme, and a method involving aaRS;
[B25] the method of [B23] or [B24], wherein the amino acid is an unnatural amino acid;
[B26] the method of [B23] to [B25], wherein the unnatural amino acid is an N-substituted amino acid;
[B27] the method of any one of [B1] to [B26], further comprising synthesizing the tRNA from a template nucleic acid by *in vitro* transcription;
[B28] the method of any one of [B1] to [B27], wherein the base at position 37 (tRNA numbering rule) before the engineering of the tRNA is A;
[B29] the method of any one of [B1] to [B28], wherein the base at position 33 (tRNA numbering rule) before the engineering of the tRNA is U;
[B30] the method of any one of [B1] to [B29], wherein the base at position 32 (tRNA numbering rule) before the engineering of the tRNA is C or U;
[B31] the method of any one of [B1] to [B30], wherein the base at position 32 (tRNA numbering rule) before the engineering of the tRNA is C;
[B32] the method of any one of [B1] to [B30], wherein the base at position 32 (tRNA numbering rule) before the engineering of the tRNA is U;
[B33] the method of any one of [B1] to [B32], wherein the base at position 38 (tRNA numbering rule) before the engineering of the tRNA is A or C;
[B34] the method of any one of [B1] to [B33], wherein the base at position 38 (tRNA numbering rule) before the engineering of the tRNA is A;
[B35] the method of any one of [B1] to [B33], wherein the base at position 38 (tRNA numbering rule) before the engineering of the tRNA is C;
[B36] the method of any one of [B1] to [B35], wherein the combination of bases at positions 32, 33, 37, and 38 (tRNA numbering rule) after the substitution is:

(1) 32U, 33U, 37G, and 38A;
(2) 32A, 33U, 37G, and 38U;

(3) 32A, 33U, 37A, and 38U; or
(4) 32U, 33U, 37G, and 38U;

[B37] the method of any one of [B1] to [B36], wherein the combination of bases at positions 32, 33, 37, and 38 (tRNA numbering rule) after the substitution is:

(1) 32U, 33U, 37G, and 38A;
(2) 32A, 33U, 37G, and 38U; or
(3) 32A, 33U, 37A, and 38U;

[B38] the method of any one of [B1] to [B37] (provided that, when the combination of bases at positions 32, 33, 37, and 38 (tRNA numbering rule) after the substitution is the combination specified in (4) of [B2], and the base sequences of positions 1 to 31 and positions 39 to 74 (tRNA numbering rule) of the tRNA are the base sequences of positions 1 to 31 and positions 39 to 74 (tRNA numbering rule) of a tRNA having the base sequence of SEQ ID NO: 255, a method in which the combination of the first codon and the second codon is CGC and CGG, and a method in which the combination of the first codon and the second codon is CGG and CGC, are excluded);
[B39] the method of any one of [B1] to [B38], further comprising translating a nucleic acid in a translation system comprising a tRNA subjected to base substitution;
[B40] the method of any one of [B26] to [B39], wherein the N-substituted amino acid is an N-methylamino acid; and
[B41] the method of any one of [B23] to [B40], wherein the amino acid is a translatable amino acid.

[Effects of the Invention]

Compositions for translation containing a tRNA that has specific bases at positions 32, 33, 37, and 38 (tRNA numbering rule), methods for producing a peptide comprising translating a nucleic acid using this tRNA, and such were provided in the present disclosure. Using the compositions, methods, and such of the present disclosure enables reducing the rate of mistranslation into unintended amino acids attributable to misreading of codons by tRNAs during peptide synthesis.

[Brief Description of Drawings]

Fig. 1 shows the rate of misreading of the CCG codon according to differences in the combination of bases at positions 32, 33, 37, and 38 of tRNA(Glu2). The values in the figure are based on the results of translating an mRNA carrying the CCG codon (see Table 6 for specific amount of translation) in the coexistence of tRNAs carrying cgg and agg as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Gly:Phe:SPh2Cl:Ile:Ile:Ala:Ile:Gly (SEQ ID NO: 242) (μM)/ amount of translated peptide corresponding to the sequence BdpF:Thr:Phe:Ile:Ile:Gly:Phe:MeHph:Ile:Ile:Ala:Ile:Gly (SEQ ID NO: 241) (μM) x 100

Fig. 2 shows the rate of misreading of the CCU codon according to differences in the combination of bases at positions 32, 33, 37, and 38 of tRNA(Glu2). The values in the figure are based on the results of translating an mRNA carrying the CCU codon (see Table 7 for specific amount of translation) in the coexistence of tRNAs carrying agg and cgg as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Gly:Phe:SPh2Cl:Ile:Ile:Ala:Ile:Gly (SEQ ID NO: 242) (μM)/ amount of translated peptide corresponding to the sequence BdpF:Thr:Phe:Ile:Ile:Gly:Phe:MeHph:Ile:Ile:Ala:Ile:Gly (SEQ ID NO: 241) (μM) x 100

Fig. 3 shows the rate of misreading of the GGG codon according to differences in the combination of bases at positions 32, 33, 37, and 38 of tRNA(Glu2). The values in the figure are based on the results of translating an mRNA carrying the GGG codon (see Table 8 for specific amount of translation) in the coexistence of tRNAs carrying ccc and acc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:SPh2Cl:Ile:Ile:Pro:Ile:Leu (SEQ ID NO: 244) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 4 shows the rate of misreading of the GGU codon according to differences in the combination of bases at positions 32, 33, 37, and 38 of tRNA(Glu2). The values in the figure are based on the results of translating an mRNA carrying the GGU codon (see Table 9 for specific amount of translation) in the coexistence of tRNAs carrying acc and ccc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:SPh2Cl:Ile:Ile:Pro:Ile:Leu (SEQ ID NO: 244) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 5 shows the rate of misreading of the CUG codon according to differences in the combination of bases at positions 32, 33, 37, and 38 of tRNA(Glu2). The values in the figure are based on the results of translating an mRNA carrying the CUG codon (see Table 10 for specific amount of translation) in the coexistence of tRNAs carrying cag and aag as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Gly:Phe:SPh2Cl:Ile:Ile:Pro:Ile:Gly (SEQ ID NO: 240) (μM)/ amount of translated peptide corresponding to the sequence BdpF:Thr:Phe:Ile:Ile:Gly:Phe:MeHph:Ile:Ile:Pro:Ile:Gly (SEQ ID NO: 239) (μM) x 100

Fig. 6 shows the rate of misreading of the CUU codon according to differences in the combination of bases at positions 32, 33, 37, and 38 of tRNA(Glu2). The values in the figure are based on the results of translating an mRNA carrying the CUU codon (see Table 11 for specific amount of translation) in the coexistence of tRNAs carrying aag and cag as anticodons, and the percentage (%) of misread peptides relative to an intended product, which is on the vertical axis, was calculated from the following equation:

BdpF:Thr:Phe:Ile:Ile:Gly:Phe:MeHph:Ile:Ile:Pro:Ile:Gly (SEQ ID NO: 239) (μM) x 100

Fig. 7 shows the rate of misreading of the CUG codon according to differences in the combination of bases at positions 32, 33, 37, and 38 of tRNA(AsnE2). The values in the figure are based on the results of translating an mRNA carrying the CUG codon (see Table 12 for

Fig. 7 shows the rate of misreading of the CUG codon according to differences in the combination of bases at positions 32, 33, 37, and 38 of tRNA(AsnE2). The values in the figure are based on the results of translating an mRNA carrying the CUG codon (see Table 12 for specific amount of translation) in the coexistence of tRNAs carrying cag and aag as anticodons, and the percentage (%) of misread peptides relative to an intended product, which is on the vertical axis, was calculated from the following equation:

BdpF:Thr:Phe:Ile:Ile:Gly:Phe:MeHph:Ile:Ile:Pro:Ile:Gly (SEQ ID NO: 239) (μM) x 100

Fig. 8 shows the rate of misreading of the CUU codon according to differences in the combination of bases at positions 32, 33, 37, and 38 of tRNA(AsnE2). The values in the figure are based on the results of translating an mRNA carrying the CUU codon (see Table 13 for

Fig. 8 shows the rate of misreading of the CUU codon according to differences in the combination of bases at positions 32, 33, 37, and 38 of tRNA(AsnE2). The values in the figure are based on the results of translating an mRNA carrying the CUU codon (see Table 13 for specific amount of translation) in the coexistence of tRNAs carrying aag and cag as anticodons, and the percentage (%) of misread peptides relative to an intended product, which is on the vertical axis, was calculated from the following equation:

BdpF:Thr:Phe:Ile:Ile:Gly:Phe:MeHph:Ile:Ile:Pro:Ile:Gly (SEQ ID NO: 239) (μM) x 100

Fig. 9 shows the rate of misreading of the CUG codon according to differences in the combination of bases at positions 32, 33, 37, and 38 of tRNA(Asp1). The values in the figure are based on the results of translating an mRNA carrying the CUG codon (see Table 14 for

Fig. 9 shows the rate of misreading of the CUG codon according to differences in the combination of bases at positions 32, 33, 37, and 38 of tRNA(Asp1). The values in the figure are based on the results of translating an mRNA carrying the CUG codon (see Table 14 for specific amount of translation) in the coexistence of tRNAs carrying cag and aag as anticodons, and the percentage (%) of misread peptides relative to an intended product, which is on the vertical axis, was calculated from the following equation:

BdpF:Thr:Phe:Ile:Ile:Gly:Phe:MeHph:Ile:Ile:Pro:Ile:Gly (SEQ ID NO: 239) (μM) x 100

Fig. 10 shows the rate of misreading of the CUU codon according to differences in the combination of bases at positions 32, 33, 37, and 38 of tRNA(Asp1). The values in the figure are based on the results of translating an mRNA carrying the CUU codon (see Table 15 for specific amount of translation) in the coexistence of tRNAs carrying aag and cag as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Gly:Phe:SPh2Cl:Ile:Ile:Pro:Ile:Gly (SEQ ID NO: 240) (μM)/ amount of translated peptide corresponding to the sequence BdpF:Thr:Phe:Ile:Ile:Gly:Phe:MeHph:Ile:Ile:Pro:Ile:Gly (SEQ ID NO: 239) (μM) x 100

Fig. 11 shows the rate of misreading of the GGA codon according to differences in the combination of bases at positions 32, 33, 37, and 38 of tRNA(Glu2). The values in the figure are based on the results of translating an mRNA carrying the GGA codon (see Table 16 for specific amount of translation) in the coexistence of tRNAs carrying ucc and acc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:SPh2Cl:Ile:Ile:Pro:Ile:Leu (SEQ ID NO: 244) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 12 shows the rate of misreading of the GGA codon according to differences in the combination of bases at positions 32, 33, 37, and 38 of tRNA(Glu2). The values in the figure are based on the results of translating an mRNA carrying the GGA codon (see Table 17 for specific amount of translation) in the coexistence of tRNAs carrying ucc and gcc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:SPh2Cl:Ile:Ile:Pro:Ile:Leu (SEQ ID NO: 244) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 13 shows the rate of misreading of the GGG codon according to differences in the combination of bases at positions 32, 33, 37, and 38 of tRNA(Glu2). The values in the figure are based on the results of translating an mRNA carrying the GGG codon (see Table 18 for specific amount of translation) in the coexistence of tRNAs carrying ccc and gcc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:SPh2Cl:Ile:Ile:Pro:Ile:Leu (SEQ ID NO: 244) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 14 shows the rate of misreading of the GGG codon according to differences in the combination of bases at positions 32, 33, 37, and 38 of tRNA(Glu2). The values in the figure are based on the results of translating an mRNA carrying the GGG codon (see Table 21 for specific amount of translation) in the coexistence of tRNAs carrying acc and ccc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:Pic2:Ile:Ile:Pro:Ile:Leu (SEQ ID NO: 245) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 15 shows the rate of misreading of the GGG codon according to differences in the combination of bases at positions 32, 33, 37, and 38 of tRNA(Glu2). The values in the figure are based on the results of translating an mRNA carrying the GGG codon (see Table 22 for specific amount of translation) in the coexistence of tRNAs carrying acc and ccc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:MeG:Ile:Ile:Pro:Ile:Leu (SEQ ID NO: 246) (μM)/ amount of translated peptide corresponding to the sequence BdpF:Thr:Phe:Ile:Ile:Leu:Phe:MeHph:Ile:Ile:Pro:Ile:Leu (SEQ ID NO: 243) (μM) x 100

Fig. 16 shows the rate of misreading of the GGG codon according to differences in the combination of bases at positions 32, 33, 37, and 38 of tRNA(Glu2). The values in the figure are based on the results of translating an mRNA carrying the GGG codon (see Table 23 for specific amount of translation) in the coexistence of tRNAs carrying acc and ccc as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Leu:Phe:dA:Ile:Ile:Pro:Ile:Leu (SEQ ID NO: 248) (μM)/ amount of translated peptide corresponding to the sequence BdpF:Thr:Phe:Ile:Ile:Leu:Phe:nBuG:Ile:Ile:Pro:Ile:Leu (SEQ ID NO: 247) (μM) x 100

Fig. 17 shows the rate of misreading of the CCG codon according to differences in the combination of bases at positions 32, 33, 37, and 38 of tRNA(Glu2). The values in the figure are based on the results of translating an mRNA carrying the CCG codon (see Table 24 for specific amount of translation) in the coexistence of tRNAs carrying cgg and agg as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Gly:Phe:SPh2Cl:Ile:Ile:Ala:Ile:Gly (SEQ ID NO: 242) (μM)/ amount of translated peptide corresponding to the sequence BdpF:Thr:Phe:Ile:Ile:Gly:Phe:MeG:Ile:Ile:Ala:Ile:Gly (SEQ ID NO: 249) (μM) x 100

Fig. 18 shows the rate of misreading of the CCU codon according to differences in the combination of bases at positions 32, 33, 37, and 38 of tRNA(Glu2). The values in the figure are based on the results of translating an mRNA carrying the CCU codon (see Table 25 for specific amount of translation) in the coexistence of tRNAs carrying agg and cgg as anticodons, and the percentage (%) of misread peptides relative to an intended product, which is on the vertical axis, was calculated from the following equation:

percentage of misread peptides relative to the intended product (%) = amount of translated peptide corresponding to the sequence BdpF:Thr:Phe:Ile:Ile:Gly:Phe:SPh2Cl:Ile:Ile:Ala:Ile:Gly (SEQ ID NO: 242) (μM)/ amount of translated peptide corresponding to the sequence BdpF:Thr:Phe:Ile:Ile:Gly:Phe:MeG:Ile:Ile:Ala:Ile:Gly (SEQ ID NO: 249) (μM) x 100

[Description of Embodiments]

For the purpose of interpreting this specification, the following definitions will apply and whenever applicable, terms used in the singular will also include the plural, and vice versa. It is to be understood that the terminology used herein is for the purpose of describing particular embodiments only and is not intended to be limiting. If any of the following definitions conflict with any document incorporated herein by reference, the following definitions shall control.

"Codon" refers to a set of three bases (triplet) that corresponds to each amino acid, when genetic information in a living body is translated to a protein. For DNA, four bases, adenine (A), guanine (G), cytosine (C), and thymine (T), are used. For mRNA, four bases, adenine (A), guanine (G), cytosine (C) and uracil (U), are used. The table showing the correspondence between each codon and amino acid is called the genetic code table or codon table, and 20 amino acids are assigned to 61 codons excluding the stop codon (Table 1). The genetic code table shown in Table 1 is used commonly for almost all eukaryote and prokaryote (eubacteria and archaea); therefore, it is called the standard genetic code table or the universal genetic code table. In the present disclosure, a genetic code table used for naturally-occurring organisms is referred to as the natural genetic code table, and it is distinguished from an artificially reprogrammed genetic code table (the correspondence between codons and amino acids is engineered). In the genetic code table, generally, four codons which are the same in the first and second letters and which differ only in the third letter are grouped into one box, and this group is called a codon box. In the present disclosure, a specific codon box may be represented by positioning "M" referring to any base selected from A, C, G, and U, after the bases at the first and second letters of the codon. For example, the codon box assigned to Ser in the natural genetic code table, in which U is at the first letter and C is at the second letter of the codons, is denoted as "UCM", and the codon box assigned to Pro is denoted as "CCM".

[Table 1]

| | U | | C | | A | | G | | |
|---|---|---|---|---|---|---|---|---|---|
| U | UUU | Phe | UCU | Ser | UAU UAC | Tyr | UGU | Cys | U C A G |
| | UUC | | UCC | | | | UGC | | |
| | UUA | Leu | UCA | | UAA | Stop | UGA | Stop | |
| | UUG | | UCG | | UAG | | UGG | Trp | |
| C | CUU | Leu | CCU | Pro | CAU CAC | His | CGU | Arg | U C A G |
| | CUC | | CCC | | | | CGC | | |
| | CUA | | CCA | | CAA | Gln | CGA | | |
| | CUG | | CCG | | CAG | | CGG | | |
| A | AUU | Ile | ACU | Thr | AAU | Asn | AGU | Ser | U C A G |
| | AUC | | ACC | | AAC | | AGC | | |
| | AUA | | ACA | | AAA | Lys | AGA AGG | Arg | |
| | AUG | Met | ACG | | AAG | | | | |
| G | GUU | Val | GCU | Ala | GAU | Asp | GGU | Gly | U C A G |
| | GUC | | GCC | | GAC | | GGC | | |
| | GUA | | GCA | | GAA | Glu | GGA | | |
| | GUG | | GCG | | GAG | | GGG | | |

In the present disclosure, a codon in mRNA may be expressed as "$M_1M_2M_3$". Here, $M_1$, $M_2$, and $M_3$ represent the bases for the first letter, the second letter, and the third letter of the codon, respectively.

"Anticodon" refers to three consecutive bases on tRNA that correspond to a codon on the mRNA. Similar to mRNA, four bases, adenine (A), guanine (G), cytosine (C), and uracil (U), are used for the anticodon. Furthermore, modified bases obtained by modifying these bases may be used. When the codon is specifically recognized by the anticodon, the genetic information on the mRNA is read and translated into a protein. The codon sequence on the mRNA in the 5' to 3' direction and the anticodon sequence on the tRNA in the 5' to 3' direction bind complementarily; therefore, complementary base pairs are formed between the bases for the first, second, and third letters of the codon, and the bases for the third, second, and first letters of the anticodon, respectively. In the present disclosure, "modified bases" refer to bases having structures partially different from those of A, C, G, and U.

In the present disclosure, an anticodon in the first tRNA may be represented by "$N_{11}N_{12}N_{13}$" and an anticodon in the second tRNA may be represented by "$N_{21}N_{22}N_{23}$". Here, $N_{11}$, $N_{12}$, and $N_{13}$, and $N_{21}$, $N_{22}$, and $N_{23}$ represent the bases at the first letter, second letter, and third letter of the anticodons, respectively. According to the tRNA numbering rule described below, $N_{11}$, $N_{12}$, and $N_{13}$, and $N_{21}$, $N_{22}$, and $N_{23}$ are numbered as positions 34, 35, and 36 of the tRNAs, respectively.

In the present disclosure, bases A, C, G, U, and T may be denoted by lowercase letters, but the uppercase letters and lowercase letters are used synonymously; for example, GGG and ggg are used synonymously.

In the present disclosure, thermodynamically stable base pairs are referred to as being "complementary" to each other. In addition to Watson-Crick base pairs such as adenine and uracil (A-U) and guanine and cytosine (G-C), a non-Watson-Crick-type wobble base pair formed between guanine and uracil (G-U) is also included in the "complementary" base pairs of the present disclosure. In particular, since there is some spatial fluctuation (wobble) between the third letter of the codon and the first letter of the anticodon, formation of a non-Watson-Crick base pair, as described above, may be permitted (wobble hypothesis).

In the present disclosure, a constant relationship between a codon and an anticodon may be referred to as "complementary". A codon-anticodon relationship where Watson-Crick base pairs are formed between the first letter of the codon and the third letter of the anticodon and between the second letter of the codon and the second letter of the anticodon, and a Watson-Crick-type or where a wobble base pair is formed between the third letter of the codon and the first letter of the anticodon is referred to as "complementary". For example, anticodons complementary to the UCU codon are AGA and GGA, and codons complementary to the GCG anticodon are CGC and CGU.

"Messenger RNA (mRNA)" refers to an RNA that carries genetic information that can be translated into a protein. Genetic information is coded on mRNA as codons, and each of these codons corresponds to one among all 20 different amino acids. Protein translation begins at the initiation codon and ends at the stop codon. In principle, the initiation codon in eukaryotes is AUG, but in prokaryotes (eubacteria and archaea), GUG and UUG may also be used as initiation codons in addition to AUG. AUG is a codon that encodes methionine (Met), and in eukaryotes and archaea, translation is initiated directly from methionine. On the other hand, in eubacteria, only the initiation codon AUG corresponds to N-formylmethionine (fMet); therefore, translation is initiated from formylmethionine. There are three stop codons: UAA (ochre), UAG (amber), and UGA (opal). When the stop codon is recognized by a protein called a translation termination factor (release factor (RF)), the peptide chain synthesized up to that point is dissociated from the tRNA, and the translation process ends.

"Transfer RNA (tRNA)" refers to a short RNA of 100 bases or less that mediates peptide synthesis using mRNA as a template. In terms of secondary structure, it has a cloverleaf-like structure consisting of three stem loops (the D arm, the anticodon arm, and the T arm) and one stem (the acceptor stem). Depending on the tRNA, an additional variable loop may be included. The anticodon arm has a region consisting of three consecutive bases called an anticodon, and the codon is recognized when the anticodon forms a base pair with the codon on the mRNA. Meanwhile, a nucleic acid sequence (CCA sequence) consisting of cytidine-cytidine-adenosine exists at the 3' end of tRNA, and an amino acid is added to the adenosine residue at the end (specifically, the hydroxyl group at position 2 or position 3 of the ribose of the adenosine residue and the carboxyl group of the amino acid form an ester bond). A tRNA to which an amino acid is bound is called "an aminoacyl tRNA". In the present disclosure, aminoacyl tRNA is also included in the definition of tRNA. Further, as described later, a method is known in which two terminal residues (C and A) are removed from the CCA sequence of tRNA and then this is used for the synthesis of aminoacyl-tRNA. Such a tRNA from which the CA sequence at the 3' end has been removed is also included in the definition of tRNA in the present disclosure. Addition of amino acids to tRNA is carried out by an enzyme called aminoacyl-tRNA synthetase (aaRS or ARS), *in vivo.* Usually, there is one aminoacyl-tRNA synthetase for each amino acid, and each aminoacyl-tRNA synthetase specifically recognizes only a specific tRNA as a substrate from multiple tRNAs; accordingly, correspondence between tRNAs and amino acids is strictly controlled.

Each base in a tRNA is numbered according to the tRNA numbering rule (Sprinzl et al., Nucleic Acids Res (1998) 26: 148-153). In the present disclosure, bases in the tRNAs are numbered according to this numbering rule. For example, the anticodon is numbered as positions 34 to 36 and the CCA sequence at the 3' end is numbered as positions 74 to 76, respectively.

In the present disclosure, to indicate a base at a specific position in a tRNA, the tRNA numbering rule (Sprinzl et al., Nucleic Acids Res (1998) 26: 148-153) and the base abbreviations (A, C, G, or U) are used. For example, "32U"

means that the base at position 32 according to the tRNA numbering rule is U (uracil). Furthermore, these are used to indicate substitutions of bases at specific positions in tRNA. For example, "C32U" means substitution from C (cytosine) to U (uracil) at position 32 according to the tRNA numbering rule.

"Anticodon loop" refers to the bases at positions 32 to 38 in tRNA, or more specifically seven consecutive bases containing three consecutive bases of the anticodon and two bases each on the 5' side and 3' side of the anticodon. Four types of bases, adenine (A), guanine (G), cytosine (C), and uracil (U), are used in the anticodon loop. Modified bases obtained by modifying them may also be used.

A "tRNA body" in the present disclosure refers to the main part (the main structural portion composed of nucleic acids) of the tRNA excluding the anticodon (positions 34 to 36). In some embodiments, the tRNA body of the present disclosure refers to positions 1 to 33 and positions 37 to 76 in a tRNA. In another embodiment, the tRNA body of the present disclosure refers to positions 1 to 33 and positions 37 to 74 in a tRNA. A "chimeric tRNA body" refers to a tRNA body in which a portion of the tRNA body is derived from a specific source or a specific type of tRNA, while the remaining portion is derived from a different source or a different type of tRNA. Chimeric tRNA bodies do not include tRNA bodies derived from only a single type of tRNA. A chimeric tRNA body may be a tRNA body derived from two or more types of tRNAs, or it may be derived from 3 or more types of tRNAs. A chimeric tRNA body of the present disclosure may be a chimeric tRNA body whose combination of bases at positions 32, 33, 37, and 38 and the base sequences of the other portions have different origins. An example of a chimeric tRNA body is a tRNA body whose combination of bases at positions 32, 33, 37, and 38 is derived from tRNA Pro2, and the remaining portions of the nucleic acid sequence are derived from tRNA Glu2. In the present disclosure, to determine the origin of the combination of bases at positions 32, 33, 37, and 38 of a certain tRNA, one can refer to the combination of bases at positions 32, 33, 37, and 38 in the base sequences (SEQ ID NOs: 274 to 319) of tRNAs derived from *Escherichia coli*, and determine that it is derived from a tRNA having the same combination of bases at positions 32, 33, 37, and 38. For example, the base combination CUxxxAC of positions 32, 33, 37, and 38, can be determined to be derived from tRNA Glu2 or tRNA Asp1 by referring to SEQ ID NOs: 274 to 319. In this case, tRNA bodies whose base sequences of the portions other than positions 32, 33, 37, and 38 are derived from tRNA Glu2 or tRNA Asp1, are not included in the chimeric tRNA bodies, since these tRNA bodies are tRNA bodies derived from only a single type of tRNA. Based on the base sequence information for positions 32 to 38 in a tRNA, the tRNA origin can be determined according to the following criteria: UUxxxAC = Ala1B; AUxxxAU = Ala2; CUxxxGA = Arg3; CUxxxAC = Glu2 or Asp1; UUxxxGU = Leu2, Leu1, or Leu3; UUxxxAA = Phe, Cys, Gly1, Gly3, Leu4, Leu5, Thr1, Thr2, or Thr3; AUxxxGU = Pro2; UUxxxGA = Pro3 or Pro1; CUxxxAA = Ser5, Arg2, Arg4, Arg5, Asn, Gly2, Ile1, Ile2, Lys, Met, Ser1, Ser2, Ser3, Thr4, Trp, Tyr1, Tyr2, or Val1; and UUxxxAU = Val2 (collective term for Val2A and Val2B), Gln1, Gln2, or His (here, xxx refers to the three bases of the anticodon). For example, the base combination at positions 32, 33, 37, and 38 in tRNAs whose base sequence is UUxxxGU for positions 32 to 38 can be determined to be derived from tRNA Leu2, tRNA Leu1, or tRNA Leu3.

A base, combination of bases, or base sequence is "derived from" a certain origin refers to the base, combination of bases, or base sequence or a sequence highly similar to that base, combination of bases, or base sequence being isolated from a certain origin. For example, when a base, combination of bases, or base sequence constituting a tRNA is isolated from a specific type of tRNA, the base, combination of bases, or base sequence is described as being "derived from" the specific type of tRNA.

In the present disclosure, a tRNA may be described as follows:

- "tRNA Xxx" or "tRNA(Xxx)": refers to a tRNA body having a specific base sequence distinguished by Xxx. Examples include tRNA Glu2 or tRNA(Glu2), tRNA Ser5 or tRNA(Ser5), tRNA AsnE2 or tRNA(AsnE2), and tRNA Asp1 or tRNA(Asp1).
- "tRNA(Xxx)nnn": refers to a full length tRNA whose anticodon sequence is nnn, which is a tRNA carrying a specific tRNA body distinguished by Xxx. Examples include tRNA(Glu2)uga and tRNA(AsnE2)uga.
- "tRNA(Xxx+Yyy)": refers to a chimeric tRNA body which has a specific base sequence distinguished by Xxx at positions other than positions 32, 33, 37, and 38, and has a specific combination of bases distinguished by Yyy at positions 32, 33, 37, and 38. Examples include tRNA(Glu2+Ser5) and tRNA(AsnE2+Phe).
- "tRNA(Xxx+Yyy)nnn": refers to a full length tRNA whose anticodon sequence is nnn, which is a tRNA carrying a chimeric tRNA body which has a specific base sequence distinguished by Xxx at positions other than positions 32, 33, 37, and 38, and has a combination of specific bases distinguished by Yyy at positions 32, 33, 37, and 38. Examples include tRNA(Glu2+Ser5)uga and tRNA(AsnE2+Phe)uga.
- "-CA" may be added at the end of the name of the above tRNA to indicate that the CA sequence at the 3' end of the tRNA has been removed. Examples include tRNA(Glu2)-CA, tRNA(Glu2)uga-CA, tRNA(Glu2+Ser5)-CA, and tRNA(Glu2+Ser5)uga-CA.

In the present disclosure, a specific combination of bases at positions 32, 33, 37, and 38 in a tRNA may be denoted by the name of the tRNA from which it is derived. In the present disclosure, the base sequence of positions 32

to 38 is denoted XXxxxXX, and the combination of bases at positions 32, 33, 37, and 38 in a tRNA is named as follows:

AlaIB sequence = UUxxxAC; Ala2 sequence = AUxxxAU; Arg3 sequence = CUxxxGA; Glu2 sequence = CUxxxAC; Leu2 sequence = UUxxxGU; Phe sequence = UUxxxAA; Pro2 sequence = AUxxxGU; Pro3 sequence = UUxxxGA; Ser5 sequence = CUxxxAA; and Val2 sequence = UUxxxAU.

"Initiator tRNA" is a specific tRNA used at the start of mRNA translation. The initiator tRNA attached to the initiator amino acid is catalyzed by a translation initiation factor (IF), introduced into the ribosome, and binds to the initiation codon on the mRNA, thereby translation is initiated. Since AUG, which is a methionine codon, is generally used as an initiation codon, the initiator tRNA has an anticodon corresponding to AUG, and has methionine (formylmethyonine for prokaryotes) attached to it as the initiator amino acid. Examples of the initiator tRNA include tRNA fMet (SEQ ID NOs: 283 and 284).

"Elongator tRNA" is a tRNA used in the elongation reaction of a peptide chain in the translation step. In peptide synthesis, amino-acid-attached elongator tRNA is sequentially transported to the ribosome by the GTP bound translation elongation factor (EF) EF-Tu/eEF-1, and this promotes the peptide chain elongation reaction. Examples of the elongator tRNA include tRNAs corresponding to various amino acids (SEQ ID NOs: 274 to 282 and 285 to 319).

"Translation system" in the present disclosure is defined as a composition for translating a peptide (it may be called a "composition for translation" in the present disclosure). A typical translation system contains as constituent components, ribosomes, translation factors, tRNAs, amino acids, aminoacyl-tRNA synthetase (aaRS), and factors necessary for peptide translation reactions such as ATP and GTP, but is not limited thereto. The main types of translation systems include translation systems that utilize living cells and translation systems that utilize cell extract solutions (cell-free translation systems (used synonymously to "compositions for cell-free translation" in the present disclosure)). As the translation system utilizing living cells, a known example is a system in which a desired aminoacyl-tRNA and mRNA are introduced into living cells such as Xenopus oocytes and mammalian cells by the microinjection method or the lipofection method to perform peptide translation (Nowak et al., Science (1995) 268: 439-442). Known examples of cell-free translation systems include translation systems that utilize extract solutions from *E. coli* (Chen et al., Methods Enzymol (1983) 101: 674-690), yeast (Gasior et al., J Biol Chem (1979) 254: 3965-3969), wheat germ (Erickson et al., Methods Enzymol (1983) 96: 38-50), rabbit reticulocytes (Jackson et al., Methods Enzymol (1983)96: 50-74), HeLa cells (Barton et al., Methods Enzymol (1996) 275: 35-57), insect cells (Swerdel et al., Comp Biochem Physiol B (1989) 93: 803-806), and such. Such a translation system can be appropriately prepared by a method known to those skilled in the art or a similar method. The cell-free translation system also includes a translation system constructed by isolating and purifying each of the factors required for peptide translation and reconstituting them (reconstituted cell-free translation system) (Shimizu et al., Nat Biotech (2001) 19: 751-755). Reconstituted cell-free translation systems may usually include ribosomes, amino acids, tRNAs, aminoacyl-tRNA synthetases (aaRS), translation initiation factors (for example, IF1, IF2, and IF3), translation elongation factors (for example, EF-Tu, EF-Ts, and EF-G), translation termination factors (for example, RF1, RF2, and RF3), ribosome recycling factors (RRF), NTPs as energy sources, energy regeneration systems, and other factors required for translation, but are not limited thereto. When the transcription reaction from DNA is also performed, RNA polymerases and the like may be further included. Various factors contained in the cell-free translation system can be isolated and purified by methods well known to those skilled in the art, and a reconstituted cell-free translation system can be appropriately constructed using them. Alternatively, a commercially available reconstituted cell-free translation system such as PUREfrex® from Gene Frontier or PURExpress® from New England BioLabs can be used. For a reconstituted cell-free translation system, a desired translation system can be constructed by reconstituting only the necessary components from the constituent components of the translation system.

An aminoacyl-tRNA, synthesized in a translation system by including a specific combination of amino acid, tRNA, and aminoacyl-tRNA synthetase in the translation system, is used for peptide translation. Instead of the above, aminoacyl-tRNA prepared outside the translation system can be directly used as a constituent component of the translation system (this is sometimes called a "pre-charge method" in the present disclosure). Examples of the pre-charge method include the method of attaching an amino acid to a tRNA using aaRS outside a translation system, the pdCpA method, the pCpA method, and the method using an artificial RNA catalyst (flexizyme). In particular, when amino acids that are difficult to aminoacylate with an aaRS, such as some unnatural amino acids, are used for translation, it is desirable to use the pre-charge method which uses as a constituent component a tRNA aminoacylated in advance with an unnatural amino acid by the pdCpA method, the pCpA method, or the method using a flexizyme.

The translation is started by adding an mRNA to the translation system. An mRNA usually contains a sequence that encodes the target peptide, and may further include a sequence for increasing the efficiency of the translation reaction (for example, a Shine-Dalgarno (SD) sequence in prokaryotes, or a Kozac sequence in eukaryotes). Pre-transcribed mRNA may be added directly to the system, or instead of mRNA, a template DNA containing a promoter and an RNA polymerase appropriate for the DNA (for example, T7 promoter and T7 RNA polymerase) can be added to the system so that mRNA will be transcribed from the template DNA.

"Misreading of a codon" refers to introduction of an unintended amino acid by translation, due to the recognition of a codon not complementary to a specific anticodon by an aminoacyl tRNA carrying the specific anticodon. An example is unintentional introduction of the amino acid acylated on the tRNA by translation, due to the erroneous recognition of the CCG codon by an aminoacyl tRNA carrying the AGG anticodon complementary to the CCU codon. In the natural genetic code table, Pro is assigned to both the CCG and CCU codons; therefore, the amino acid introduced by translation is not different whether or not such misreading takes place. However, when the genetic code table is reprogrammed such that different amino acids are assigned to the CCG and CCU codons, this codon misreading may become a problem.

In the present disclosure, "alkyl" is a monovalent group derived from an aliphatic hydrocarbon by removing one arbitrary hydrogen atom; it does not contain a hetero atom or an unsaturated carbon-carbon bond in the skeleton; and it has a subset of hydrocarbyl or hydrocarbon-group structures containing hydrogen and carbon atoms. The length of the carbon chain length, n, is in the range of 1 to 20. The examples of alkyl include $C_2$-$C_{10}$ alkyl, $C_1$-$C_6$ alkyl, and $C_1$-$C_3$ alkyl, and specific examples include methyl, ethyl, propyl, butyl, pentyl, hexyl, isopropyl, t-butyl, sec-butyl, 1-methylpropyl, 1,1-dimethylpropyl, 2,2-dimethylpropyl, 1,2-dimethylpropyl, 1,1,2-trimethylpropyl, 1,2,2-trimethylpropyl, 1,1,2,2-tetramethylpropyl, 1-methylbutyl, 2-methylbutyl, 3-methylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, isopentyl, and neopentyl.

In the present disclosure, "cycloalkyl" means a saturated or partially saturated cyclic monovalent aliphatic hydrocarbon group, and includes a monocyclic ring, a bicyclic ring, and a spiro ring. Examples of cycloalkyl include $C_3$-$C_{10}$ cycloalkyl, and specific examples include cyclopropyl, cyclobutyl, cyclopentyl, cyclohexyl, cycloheptyl, cyclooctyl, and bicyclo[2.2.1]heptyl.

In the present disclosure, "alkenyl" is a monovalent group having at least one double bond (two adjacent SP2 carbon atoms). Depending on the arrangement of double bonds and substituents (if present), the geometric configuration of the double bond can be entgegen (E) or zusammen (Z), and cis or trans configurations. It can be a straight chain or branched chain alkenyl, and includes a straight chain alkenyl containing an internal olefin. Examples of the alkenyl include $C_2$-$C_{10}$ alkenyl and $C_2$-$C_6$ alkenyl, and specific examples include vinyl, allyl, 1-propenyl, 2-propenyl, 1-butenyl, 2-butenyl (including cis and trans), 3-butenyl, pentenyl, and hexenyl.

In the present disclosure, "alkynyl" is a monovalent group having at least one triple bond (two adjacent SP carbon atoms). It can be a straight or branched chain alkynyl, and includes an internal alkylene. Examples of the alkynyl include $C_2$-$C_{10}$ alkynyl and $C_2$-$C_6$ alkynyl, and specific examples include ethynyl, 1-propynyl, propargyl, 3-butynyl, pentynyl, hexynyl, 3-phenyl-2-propinyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

In the present disclosure, "aryl" means a monovalent aromatic hydrocarbon ring. Examples of the aryl include $C_6$-$C_{10}$ aryl, and specific examples include phenyl and naphthyl (such as 1-naphthyl and 2-naphthyl).

In the present disclosure, "heteroaryl" means a monovalent aromatic ring group containing a hetero atom in the atoms constituting the ring, and may be partially saturated. The ring may be a monocyclic ring or a fused bicyclic ring (for example, a bicyclic heteroaryl formed by fusing with benzene or a monocyclic heteroaryl). The number of atoms constituting the ring is, for example, five to ten (5- to 10-membered heteroaryl). The number of heteroatoms contained in the ring-constituting atoms is, for example, one to five. Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzooxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

In the present disclosure, "arylalkyl (aralkyl)" is a group containing both aryl and alkyl, and means, for example, a group in which at least one hydrogen atom of the above-mentioned alkyl is substituted with aryl. Examples of the aralkyl include $C_5$-$C_{10}$ aryl $C_1$-$C_6$ alkyl, and specific examples include benzyl.

In the present disclosure, "alkylene" means a divalent group derived by further removing one arbitrary hydrogen atom from the above-mentioned "alkyl", and may be linear or branched. Examples of the straight chain alkylene include $C_2$-$C_6$ straight chain alkylene, $C_4$-$C_5$ straight chain alkylene and the like. Specific examples include $-CH_2-$, $-(CH_2)_2-$, $-(CH_2)_3-$, - $(CH_2)_4-$, $-(CH_2)_5-$, and $-(CH_2)_6-$. Examples of the branched alkylene include $C_2$-$C_6$ branched alkylene and $C_4$-$C_5$ branched alkylene. Specific examples include $-CH(CH_3)CH_2-$, $-C(CH_3)_2-$, - $CH(CH_3)CH_2CH_2-$, $-C(CH_3)_2CH_2-$, $-CH_2CH(CH_3)CH_2-$, $CH_2C(CH_3)_2-$, and $-CH_2CH_2CH(CH_3)-$.

In the present disclosure, "alkenylene" means a divalent group derived by further removing one arbitrary hydrogen atom from the above-mentioned "alkenyl", and may be linear or branched. Depending on the arrangement of double bonds and substituents (if present), it can take the form of entgegen (E) or zusammen (Z), and cis or trans configurations. Examples of the straight chain alkenylene include $C_2$-$C_6$ straight chain alkenylene and $C_4$-$C_5$ straight chain alkenylene. Specific examples include -CH=CH-, $-CH=CHCH_2-$, $-CH_2CH=CH-$, - $CH=CHCH_2CH_2-$, $-CH_2CH=CHCH_2-$, $-CH_2CH_2CH=CH-$, $-CH=CHCH_2CH_2CH_2-$, - $CH_2CH=CHCH_2CH_2-$, $-CH_2CH_2CH=CHCH_2-$, and $-CH_2CH_2CH_2CH=CH-$.

In the present disclosure, "arylene" means a divalent group derived by further removing one arbitrary hydrogen

atom from the above-mentioned aryl. The ring may be a monocyclic ring or a fused ring. The number of atoms constituting the ring is not particularly limited, but is, for example, six to ten ($C_6$-$C_{10}$ arylene). Specific examples of arylene include phenylene and naphthylene.

In the present disclosure, "heteroarylene" means a divalent group derived by further removing one arbitrary hydrogen atom from the above-mentioned heteroaryl. The ring may be a monocyclic ring or a fused ring. The number of atoms constituting the ring is not particularly limited, but is, for example, five to ten (5- to 10-membered heteroarylene). As the heteroarylene, specific examples include pyrrolediyl, imidazoldiyl, pyrazolediyl, pyridinediyl, pyridazinediyl, pyrimidinediyl, pyrazinediyl, triazolediyl, triazinediyl, isoxazolediyl, oxazolediyl, oxadiazolediyl, isothiazolediyl, thiazolediyl, thiadiazolediyl, furandiyl, and thiophenediyl.

<Compositions, kits, and methods>

In one aspect, the present disclosure relates to compositions for translation and kits for translation, comprising a first tRNA to which a first amino acid is attached and a second tRNA to which a second amino acid is attached. In one aspect, the present disclosure relates to methods for translating a nucleic acid using a first tRNA to which a first amino acid is attached and a second tRNA to which a second amino acid is attached. Using these compositions, kits, and methods can reduce or suppress mistranslation into unintended amino acids attributable to misreading of codons by tRNAs. Therefore, in one aspect, the present disclosure relates to methods for producing peptides while reducing or suppressing misreading of codons by tRNAs, and compositions, kits, and such to be used for such methods.

Compositions in the present disclosure may contain in addition to tRNAs of the present disclosure, buffer, substances, and such generally used for nucleic acid translation. Furthermore, in one aspect, tRNAs of the present disclosure may be provided with various substances generally used for peptide translation by packaging them in advance as kits. In a further aspect, various substances included in the kits of the present disclosure may be in powder form or liquid form depending on the manner of use. Furthermore, these may be stored in appropriate containers, and used when appropriate.

<tRNA and tRNA body>

In some embodiments, a combination of bases at positions 32, 33, 37, and 38 of the tRNAs of the present disclosure may be

(1) 32U, 33U, 37G, and 38A;
(2) 32A, 33U, 37G, and 38U;
(3) 32A, 33U, 37A, and 38U;
(4) 32U, 33U, 37G, and 38U;
(5) 32U, 33U, 37A, and 38U; or
(6) 32C, 33U, 37G, and 38A.

In some embodiments, a tRNA of the present disclosure may be a tRNA whose positions 32, 33, 37, and 38 are a combination of bases selected from the group consisting of: the above (1) to (4); the above (1) to (3); or the above (1), (3), and (4); or the combination of bases of the above (1); the above (2); the above (3); the above (4); the above (5); or the above (6). In several embodiments, none of the bases at positions 32, 33, 37, and 38 in a tRNA of the present disclosure are modified bases.

In the present disclosure, the anticodon in the first tRNA of the present disclosure may be represented by $N_{11}N_{12}N_{13}$, and the anticodon in the second tRNA of the present disclosure may be represented by $N_{21}N_{22}N_{23}$. The above $N_{11}$, $N_{12}$, and $N_{13}$, and $N_{21}N_{22}N_{23}$ may be each independently A, C, G, or U. The first, second, and third letters of the anticodon in a tRNA of the present disclosure may be each independently A, C, G, or U. The base sequence of the second and third letters of the anticodon in a tRNA of the present disclosure may be CC, GC, AC, GU, CG, GG, AG, or GA; alternatively, it may be GG, AG, or CC. In some embodiments, the base sequence of the second and third letters of the above-mentioned anticodon is not CG. In some embodiments, a tRNA of the present disclosure may not have a modified base in the anticodon, or may have a modified base at the first letter of the anticodon, or may have the later-described modification on a nucleoside at this first letter.

In some embodiments, the tRNA of the present disclosure is a prokaryote-derived tRNA or a eukaryote-derived tRNA. A tRNA may be produced by engineering a prokaryote-derived tRNA or a eukaryote-derived tRNA, and the tRNA produced by the engineering may have the highest base sequence identity with the prokaryote-derived tRNA or the eukaryote-derived tRNA. Eukaryotes are further classified into animals, plants, fungi, and protists. The tRNA of the present disclosure may be, for example, a human-derived tRNA. Prokaryotes are further classified into eubacteria and archaea. Examples of eubacteria include *E. coli*, *Bacillus subtilis*, lactic acid bacteria, and *Desulfitobacterium hafniense.*

Examples of archaea include extreme halophile, thermophile, or methane bacteria (for example, *Methanosarcina mazei*, *Methanosarcina barkeri*, and *Methanocaldococcus jannaschii*). The tRNA of the present disclosure may be, for example, tRNA derived from *E. coli*, *Desulfitobacterium hafniense*, or *Methanosarcina mazei*.

In some embodiments, a tRNA of the present disclosure may differ from the base sequence of the reference tRNA in one or more bases; in 2 or more, 3 or more, 4 or more, 5 or more, 6 or more, 7 or more, 8 or more, 9 or more, 10 or more, 11 or more, or 12 or more bases; in 1, 2, 3, 4, 5, 6, 7, 8, 9, 10, 11, or 12 bases; or in 15 or fewer, 14 or fewer, 13 or fewer, 12 or fewer, 11 or fewer, 10 or fewer, 9 or fewer, 8 or fewer, 7 or fewer, 6 or fewer, 5 or fewer, 4 or fewer, 3 or fewer, or 2 or fewer bases. In some embodiments, a tRNA of the present disclosure may have a sequence identity of 80% or higher, 85% or higher, 90% or higher, 91% or higher, 92% or higher, 93% or higher, 94% or higher, 95% or higher, 96% or higher, 97% or higher, or 98% or higher relative to the base sequence of the reference tRNA. In the present disclosure, the "percent (%) sequence identity" relative to a certain base sequence is defined as the percentage of bases in a candidate sequence that are identical with the bases in the reference base sequence, after aligning the sequences and introducing gaps, if necessary, to achieve the maximum percent sequence identity, and not considering any conservative substitutions as part of the sequence identity. Alignment for purposes of determining percent base sequence identity can be achieved in various ways that are within the skill in the art, for instance, using publicly available computer software such as BLAST, BLAST-2, ALIGN, Megalign (DNASTAR) software, or GENETYX® (GENETYX CORPORATION). Those skilled in the art can determine appropriate parameters for achieving sequence alignment, including any algorithms needed to achieve maximal alignment over the full length of the sequences being compared.

In some embodiments, a tRNA of the present disclosure may be different in the base sequence of the anticodon loop (positions 32-38) from the base sequence of a reference tRNA. In a certain embodiment, a tRNA of the present disclosure may have a chimeric anticodon loop. "Chimeric anticodon loop" in the present disclosure means an anticodon loop in which the bases of positions 32, 33, 37, and 38 and the base sequence of the anticodon are derived from different tRNAs. For example, whether an anticodon loop containing the UGA anticodon is a chimeric anticodon loop can be determined as follows. First, since tRNA Ser1 (SEQ ID NO: 306) derived from *E. coli* is identified as a tRNA carrying the UGA anticodon, the UGA anticodon can be determined to be derived from tRNA Ser 1 (when determining the origin of an anticodon, one can refer to the base sequences of tRNAs set forth in SEQ ID NOs: 274 to 319). Here, the base sequence of the anticodon loop of the above-mentioned tRNA Ser1 is CUugaAA. Therefore, when the bases at positions 32, 33, 37, and 38 in the anticodon loop have a combination of bases other than that in the Ser5 sequence (32C, 33U, 37A, and 38A), this anticodon loop is determined to be a chimeric anticodon loop. In a certain embodiment, the anticodon loop in a tRNA of the present disclosure may be different from the anticodon loop (positions 32 to 38 according to the tRNA numbering rule) included in a tRNA having the base sequence of any one of SEQ ID NOs: 274 to 282, 285 to 304, and 306 to 319. In some embodiments, a tRNA body of the present disclosure may be different from the base sequence of a reference tRNA body in one or more bases; in 2 or more, 3 or more, 4 or more, 5 or more, 6 or more, 7 or more, 8 or more, 9 or more, 10 or more, 11 or more, or 12 or more bases; in 1, 2, 3, 4, 5, 6, 7, 8, 9, 10, 11, or 12 bases; or in 15 or fewer, 14 or fewer, 13 or fewer, 12 or fewer, 11 or fewer, 10 or fewer, 9 or fewer, 8 or fewer, 7 or fewer, 6 or fewer, 5 or fewer, 4 or fewer, 3 or fewer, or 2 or fewer bases. In some embodiments, a tRNA body of the present disclosure may have a sequence identity of 80% or higher, 85% or higher, 90% or higher, 91% or higher, 92% or higher, 93% or higher, 94% or higher, 95% or higher, 96% or higher, 97% or higher, or 98% or higher relative to the base sequence of the reference tRNA body. In some embodiments, a tRNA of the present disclosure may be different from a reference tRNA at least in the base of one position selected from the group consisting of positions 32, 33, 37, and 38. In some embodiments, a tRNA body of the present disclosure may be different from a reference tRNA body at least in the base of one position selected from the group consisting of positions 32, 33, 37, and 38. In some embodiments, a tRNA of the present disclosure is characterized in that it is not a naturally occurring tRNA. In an embodiment, a tRNA body of the present disclosure may be a tRNA body not derived from a tRNA having the base sequence of SEQ ID NO: 275, and/or a tRNA body not derived from any of the tRNAs having the base sequences of SEQ ID NOs: 294, 295, and 296, and/or a tRNA body not derived from any of the tRNAs having the base sequences of SEQ ID NOs: 302, 303, and 304. In an embodiment, when positions 32, 33, 37, and 38 in a tRNA body of the present disclosure have the Leu2 sequence, the tRNA body may not be derived from any of the tRNAs having the base sequences of SEQ ID NOs: 294, 295, and 296; when these positions have the Ala2 sequence, the tRNA body may not be derived from a tRNA having the base sequence of SEQ ID NO: 275; when these positions have the Pro2 sequence or the Pro3 sequence, the tRNA body may not be derived from any of the tRNAs having the base sequences of SEQ ID NOs: 302, 303, and 304.

A reference tRNA and a reference tRNA body of the present disclosure may each be a natural tRNA derived from any organism (for example, *E. coli*) or a body thereof; or an unnatural tRNA formed by artificially synthesizing a sequence different from that of a natural tRNA, or a body thereof; or a tRNA formed by artificially synthesizing the sequence of a natural tRNA or such (artificial tRNA), or a body thereof; or a tRNA chimera formed by artificially combining tRNAs of different origins, or a body thereof.

A reference tRNA or a reference tRNA body of the present disclosure may each be selected appropriately from tRNAs or tRNA bodies carrying any base sequences. In some embodiments, the reference tRNA or reference tRNA

body may be at least one tRNA selected from the group consisting of tRNA Ala, tRNA Arg, tRNA Asn, tRNA Asp, tRNA Cys, tRNA Gln, tRNA Glu, tRNA Gly, tRNA His, tRNA Ile, tRNA Leu, tRNA Lys, tRNA Met, tRNA Phe, tRNA Pro, tRNA Ser, tRNA Thr, tRNA Trp, tRNA Tyr, tRNA Val, and tRNA Sec (selenocysteine) (SEQ ID NOs: 274 to 282, and 285 to 319), and tRNA Glu2, tRNA AsnE2, and tRNA Asp1 (SEQ ID NOs: 322 to 324), or a body thereof. tRNA fMet (SEQ ID NOs: 283 and 284), tRNA Pyl (pyrrolysine), tRNA AsnE2 (see, Ohta, A.; Murakami, H.; Higashimura, E.; Suga, H. Chem. Biol. 2007, 14, 1315-1322) and bodies thereof may also be used as references. Furthermore, tRNA Pro1E2 (see, WO2019/077887), which is a tRNA chimera formed by transferring the T stem of tRNA Glu2 to tRNA Pro1 and further mutating it, and a body thereof, may also be used as references. In a certain embodiment, the tRNA or tRNA body of the present disclosure may be at least one tRNA selected from the group consisting of tRNA Glu2, tRNA Asp1, and tRNA AsnE2, or a body thereof. Exemplary base sequences for positions 1 to 74 of some tRNA bodies are shown in SEQ ID NOs: 253 to 255, 320, and 321. The reference tRNA bodies presented as examples herein may also be used as sequences from which the portions other than the anticodon loop in a chimeric tRNA body of the present disclosure originate.

In some embodiments, the difference of the tRNA or tRNA body of the present disclosure from the reference tRNA or the reference tRNA body may be generated by engineering a portion of the sequence of the reference tRNA or the reference tRNA body based on the sequence information thereof (for example, the base sequence information). In this case, once the sequence information of the tRNA or the tRNA body of the present disclosure is obtained, the tRNA or the tRNA body of the present disclosure can be prepared without requiring the reference sequence information thereafter. In the present disclosure, "engineer" means introducing to the base sequence of an existing tRNA or a tRNA body (existing sequence), at least 1, or 2, 3, 4, 5, 6, 7, 8, 9, 10, 11, or 12 or more of at least one type of alteration selected from the following group: (i) addition (adding any new base to the existing sequence); (ii) deletion (removing any nucleotide from the existing sequence); (iii) substitution (replacing any base in the existing sequence with any other base); (iv) insertion (adding any new nucleotide between any two nucleotides in the existing sequence); and (v) modification (changing a part of the structure of any nucleoside (for example, the base portion or the sugar portion) in the existing sequence to another structure). Engineering can be performed on any structure (for example, D arm, anticodon arm, T arm, acceptor stem, and variable loop) of the tRNA or tRNA body. In a certain embodiment, engineering of the tRNA or tRNA body of the present disclosure is performed on at least 1, 2 or more, 3 or more, or 4 bases selected from the group consisting of positions 32, 33, 37, and 38, or the group consisting of positions 32, 37, and 38 in the tRNA. In some embodiments, the tRNA and tRNA body of the present disclosure may not be those actually engineered based on a reference tRNA or a reference tRNA body. tRNAs or tRNA bodies carrying a base sequence that would be obtained if a reference tRNA or a reference tRNA body were engineered as mentioned above, are also included in the tRNAs and tRNA bodies of the present disclosure.

Engineering in the present disclosure includes, for example, substituting bases such that the combination of bases at positions 32, 33, 37, and 38 in a tRNA becomes the same as the combination of bases at the corresponding positions in the tRNA of the present disclosure. The base prior to substitution is not particularly limited; however, for example, the base at position 32 before substitution may be C or U, the base at position 37 before substitution may be A, the base at position 38 before substitution may be A or C, and/or the base at position 33 before substitution may be U. In some embodiments, when the base at position 32, 33, 37, or 38 is already the desired base, substitution is unnecessary. For example, when the base at position 33 of tRNA before substitution is U, and the desired base at position 33 is also U, substitution of the base at position 33 is unnecessary.

In some embodiments, tRNAs of the present disclosure may carry a chimeric tRNA body. Chimeric tRNA bodies include, for example, those whose anticodon loop portion (*i.e.*, positions 32, 33, 37, and 38) and the other portions of the tRNA body are derived from different tRNA bodies. The base sequences of positions 1 to 74 in the chimeric tRNA bodies are exemplified below:

[Table 2]

| Name of chimeric tRNA body | SEQ ID NO: | Origin of the parts other than the anticodon loop in the tRNA body | Origin of the combination of bases at positions 34,33,37,and 38 |
|---|---|---|---|
| tRNA(Glu2+ Leu2)-CA | 256 | tRNA Glu2 | tRNA Leu2 |
| tRNA(Glu2+Pro3)-CA | 257 | | tRNA Pro3 |
| tRNA(Glu2+Pro2)-CA | 258 | | tRNA Pro2 |
| tRNA(Glu2+Ala2)-CA | 259 | | tRNA Ala2 |
| tRNA(Glu2+Val2)-CA | 260 | | tRNA Val2 |
| tRNA(Glu2+Arg3)-CA | 261 | | tRNA Arg3 |

(continued)

| Name of chimeric tRNA body | SEQ ID NO: | Origin of the parts other than the anticodon loop in the tRNA body | Origin of the combination of bases at positions 34,33,37,and 38 |
|---|---|---|---|
| tRNA(Asp 1+Leu2) -CA | 262 | tRNAAsp1 | tRNA Leu2 |
| tRNA(Asp1+Pro3)-CA | 263 | | tRNA Pro3 |
| tRNA(Asp 1÷Pro2) - CA | 264 | | tRNA Pro2 |
| tRNA(Asp1+Ala2)-CA | 265 | | tRNA Ala2 |
| tRNA(Asp1+Val2)-CA | 266 | | tRNA Val2 |
| tRNA(Asp1+Arg3)-CA | 267 | | tRNA Arg3 |
| tRNA (AsnE2+Leu2)-CA | 268 | tRNAAsnE2 | tRNA Leu2 |
| tRNA(AsnE2+Pro3) - CA | 269 | | tRNA Pro3 |
| tRNA(AsnE2+Pro2) - CA | 270 | | tRNA Pro2 |
| tRNA (AsnE2+Ala2)-CA | 271 | | tRNA Ala2 |
| tRNA (AsnE2+Val2)-CA | 272 | | tRNA Val2 |
| tRNA (AsnE2+Arg3)-CA | 273 | | tRNA Arg3 |

In some embodiments, the base sequence of positions 32 to 38 in a tRNA of the present disclosure may be different from the base sequence of positions 32 to 38 in a wildtype tRNA of *E. coli* or a naturally occurring tRNA. Without limitation, in some embodiments, when tRNAs of the present disclosure carry a chimeric tRNA body which has the combination of bases 32U, 33U, 37G, and 38A, tRNAs whose anticodon(s) is/are UGG and/or CGG may be excluded from the tRNAs of the present disclosure. In some embodiments, when tRNAs of the present disclosure carry a chimeric tRNA body which has the combination of bases 32A, 33U, 37G, and 38U, tRNAs whose anticodon(s) is/are AGG and/or GGG may be excluded from the tRNAs of the present disclosure. In some embodiments, when tRNAs of the present disclosure carry a chimeric tRNA body which has the combination of bases 32A, 33U, 37A, and 38U, tRNAs whose anticodon(s) is/are AGC and/or GGC may be excluded from the tRNAs of the present disclosure. In some embodiments, when tRNAs of the present disclosure carry a chimeric tRNA body which has the combination of bases 32C, 33U, 37G, and 38A, tRNAs whose anticodon(s) is/are UCG and/or CCG may be excluded from the tRNAs of the present disclosure. In some embodiments, when tRNAs of the present disclosure carry a chimeric tRNA body which has the combination of bases 32U, 33U, 37G, and 38U, tRNAs whose anticodon has A as the base at the second letter and G as the base at the third letter may be excluded from the tRNAs of the present disclosure.

In some embodiments, although the base sequences other than those of the anticodon loops are not particularly limited in the tRNAs of the present disclosure, they may be selected from sequences other than those of tRNA Ala, tRNA Pro, and tRNA Leu. Here, the base sequences other than those of the anticodon loops may be base sequences at positions 1 to 31 and positions 39 to 76 of tRNA.

In some embodiments, the base sequences of positions 1 to 31 and positions 39 to 74 in a tRNA of the present disclosure are not particularly limited, but they may be derived from the base sequences of positions 1 to 31 and positions 39 to 74 in a tRNA having the base sequence of at least one selected from the group consisting of (a) SEQ ID NO: 253, (b) SEQ ID NO: 255, and (c) SEQ ID NO: 254. In a particular embodiment, the base sequences of positions 1 to 31 and positions 39 to 74 in a tRNA of the present disclosure may be the base sequences of positions 1 to 31 and positions 39 to 74 in a tRNA having the base sequence of at least one selected from the group consisting of the above (a) to (c). In some embodiments, the base sequences of positions 1 to 31 and positions 39 to 74 in a tRNA of the present disclosure may have a sequence identity of 80% or more, 85% or more, 90% or more, 91% or more, 92% or more, 93% or more, 94% or more, 95% or more, 96% or more, 97% or more, or 98% or more to the base sequences of positions 1 to 31 and

positions 39 to 74 in a tRNA having the base sequence of any one of the above (a) to (c).

In some embodiments, a tRNA of the present disclosure may have the base C at position 75 and the base A at position 76. Positions 75 and 76 in a tRNA of the present disclosure may be pCpA (a dinucleotide containing cytidine and adenosine) or pdCpA (a dinucleotide containing deoxycytidine and adenosine). In some embodiments, a tRNA of the present disclosure may be a tRNA which has an amino acid attached to its 3' end, or more specifically, to the adenosine residue at its 3' end, or even more specifically, to the adenosine residue at position 76 which is its 3' end.

Modification in the present disclosure includes, for example, a modification performed on the base or nucleoside of the first letter of the anticodon in tRNA (for example, replacement with lysidine, a lysidine derivative, agmatidine, or an agmatidine derivative). Here, a lysidine derivative is a molecule produced by modifying a part of the structure of lysidine (for example, the base portion), and which has the same codon discrimination ability (ability to form complementary base pairs) as that of lysidine when used as a part of an anticodon. Furthermore, an agmatidine derivative is a molecule produced by modifying a part of the structure of agmatidine (for example, the base portion) and which has the same codon discrimination ability (ability to form complementary base pairs) as that of agmatidine when used as a part of an anticodon. In the present disclosure, bases that have undergone modifications presented as examples herein or other modifications may be called "modified bases". In the present disclosure, "L" in the base sequence or the nucleic acid sequence means lysidine.

Lysidine in natural tRNA is synthesized by the action of an enzyme called tRNA Ile-lysidine synthetase (TilS). TilS has the activity of specifically recognizing tRNA corresponding to isoleucine (tRNA Ile2) as a substrate, and altering (converting) cytidine (C) at the first letter ($N_1$) of its anticodon to lysidine (k2C). The lysidine in a tRNA of the present disclosure may be synthesized with or without the mediation of TilS.

In some embodiments, tRNAs of the present disclosure may not contain modified bases. In the present disclosure, a tRNA prepared by *in vitro* transcription may be called "transcribed tRNA". The tRNAs of the present disclosure may be transcribed tRNAs, and they may be transcribed tRNAs not containing modified bases. In the present disclosure, the term "artificial tRNA" may be used to distinguish these from naturally-occurring tRNAs. The tRNAs of the present disclosure may be artificial tRNAs, and they may be artificial tRNAs not containing modified bases or modified nucleosides. Methods for producing tRNAs not containing modified bases, tRNAs not containing modified nucleosides, transcribed tRNAs, and artificial tRNAs are not particularly limited; however, they may be prepared, for example, by synthesizing tRNAs from template DNAs by *in vitro* transcription reaction using RNA polymerases such as T7 RNA polymerase, and purifying the RNAs when necessary. RNeasy kit (Qiagen) and such can be used for RNA purification. In some embodiments, the bases of the tRNAs of the present disclosure are composed of A, C, G, and U. In some embodiments, tRNAs of the present disclosure may have bases consisting of A, C, G, and U.

In some embodiments, an amino acid may be attached to a tRNA of the present disclosure. The amino acid is normally attached to the 3' end of a tRNA, or more specifically to the adenosine residue of the CCA sequence at the 3' end. In a certain embodiment, the above 3' end adenosine residue may be at position 76 according to the tRNA numbering rule. The specific types of amino acids attached to tRNA can each be appropriately selected from the amino acids described below, and examples include unnatural amino acids.

The amino acids in the present disclosure include $\alpha$-amino acids, $\beta$-amino acids, and $\gamma$-amino acids. Regarding three-dimensional structures, both L-type amino acids and D-type amino acids are included. Furthermore, amino acids in the present disclosure include natural and unnatural amino acids. The natural amino acids consist of the following 20$\alpha$-amino acids: glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro). Natural amino acids are usually L-type amino acids.

In the present disclosure, unnatural amino acids refer to all amino acids excluding the above-mentioned natural amino acids consisting of 20$\alpha$-amino acids. Examples of unnatural amino acids include $\beta$-amino acids, $\gamma$-amino acids, D-type amino acids, $\alpha$-amino acids whose side chains differ from natural amino acids, $\alpha,\alpha$-disubstituted amino acids, amino acids whose main chain amino group has a substituent (also referred to as "N-substituted amino acids" in this disclosure), and hydroxycarboxylic acid (hydroxy acid). Examples of N-substituted amino acids include, N-methyl amino acid, N-ethyl amino acid, N-propyl amino acid, N-butyl amino acid, but is not limited thereto. The side chain of the unnatural amino acid is not particularly limited, but may have, for example, alkyl, alkenyl, alkynyl, aryl, heteroaryl, aralkyl, and cycloalkyl, in addition to the hydrogen atom. Further, in the case of an $\alpha,\alpha$-disubstituted amino acid, two side chains may form a ring. Furthermore, these side chains may have one or more substituents. In a particular embodiment, the substituents can be selected from any functional group containing a halogen atom, O atom, S atom, N atom, B atom, Si atom, or P atom. For example, in the present disclosure, "$C_1$-$C_6$ alkyl having halogen as a substituent" means a "$C_1$-$C_6$ alkyl" in which at least one hydrogen atom in an alkyl is substituted with a halogen atom, and specific examples include, trifluoromethyl, difluoromethyl, fluoromethyl, pentafluoroethyl, tetrafluoroethyl, trifluoroethyl, difluoroethyl, fluoroethyl, trichloromethyl, dichloromethyl, chloromethyl, pentachloroethyl, tetrachloroethyl, trichloroethyl, dichloroethyl, and chloroethyl. In addition, for example, "$C_5$-$C_{10}$ aryl $C_1$-$C_6$ alkyl having a substituent" means "$C_5$-$C_{10}$ aryl $C_1$-$C_6$ alkyl" in

which at least one hydrogen atom in aryl and/or alkyl is substituted with a substituent. Furthermore, the meaning of the phrase "having two or more substituents" includes having a certain functional group (for example, a functional group containing an S atom) as a substituent, and the functional group has another substituent (for example, a substituent such as amino or halogen). For specific examples of unnatural amino acids, one can refer to WO2013/100132, WO2018/143145, and such.

The amino group of the main chain of the unnatural amino acid may be an unsubstituted amino group ($-NH_2$ group) or a substituted amino group (-NHR group). Here, R indicates an alkyl, alkenyl, alkynyl, aryl, heteroaryl, aralkyl, or cycloalkyl which optionally has a substituent. Further, like proline, the carbon chain attached to the N atom of the main chain amino group and the $\alpha$-position carbon atom may form a ring. The substituent can be selected from any functional group containing a halogen atom, O atom, S atom, N atom, B atom, Si atom, or P atom. Examples of alkyl substitution of an amino group include N-methylation, N-ethylation, N-propylation, and N-butylation, and example of aralkyl substitution of an amino group include N-benzylation. Specific examples of an N-methylamino acid include N-methylalanine, N-methylglycine, N-methylphenylalanine, N-methyltyrosine, N-methyl-3-chlorophenylalanine, N-methyl-4-chlorophenylalanine, N-methyl-4-methoxyphenylalanine, N-methyl-4-thiazolealanine, N-methylhistidine, N-methylserine and N-methylaspartic acid.

Examples of a substituent containing a halogen atom include fluoro (-F), chloro (-Cl), bromo (-Br), and iodo (-I).

Examples of a substituent containing an O atom include hydroxyl (-OH), oxy (-OR), carbonyl (-C=O-R), carboxyl ($-CO_2H$), oxycarbonyl (-C=O-OR), carbonyloxy (-O-C=O-R), thiocarbonyl (-C=O-SR), carbonylthio (-S-C=O-R), aminocarbonyl (-C=O-NHR), carbonyl amino (-NH-C=O-R), oxycarbonyl amino (-NH-C=O-OR), sulfonyl amino ($-NH-SO_2-R$), aminosulfonyl ($-SO_2-NHR$), sulfamoyl amino ($-NH-SO_2-NHR$), thiocarboxyl (-C(=O)-SH), carboxyl carbonyl ($-C(=O)-CO_2H$).

Examples of oxy (-OR) include alkoxy, cycloalkoxy, alkenyloxy, alkynyloxy, aryloxy, heteroaryloxy, and aralkyloxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Furthermore, the H atom attached to the N atom in -C=O-NHR may be substituted with a substituent selected from the group consisting of alkyl, cycloalkyl, alkenyl, alkynyl, aryl, heteroaryl, and aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Furthermore, the H atom attached to the N atom in -NH-C=O-R may be substituted with a substituent selected from the group consisting of alkyl, cycloalkyl, alkenyl, alkynyl, aryl, heteroaryl, and aralkyl.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Furthermore, the H atom attached to the N atom in -NH-C=O-OR may be substituted with a substituent selected from the group consisting of alkyl, cycloalkyl, alkenyl, alkynyl, aryl, heteroaryl, and aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. Furthermore, the H atom attached to the N atom in $-NH-SO_2-R$ may be substituted with a substituent selected from the group consisting of alkyl, cycloalkyl, alkenyl, alkynyl, aryl, heteroaryl, and aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Furthermore, the H atom attached to the N atom in $-SO_2-NHR$ may be substituted with a substituent selected from the group consisting of alkyl, cycloalkyl, alkenyl, alkynyl, aryl, heteroaryl, and aralkyl.

Examples of sulfamoylamino ($-NH-SO_2-NHR$) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Furthermore, at least one of the two H atoms attached to the N atoms in $-NH-SO_2-NHR$ may be substituted with a substituent selected from the group consisting of alkyl, cycloalkyl, alkenyl, alkynyl, aryl, heteroaryl, and aralkyl. When the two H atoms are both substituted, a substituent may each be independently selected, or these two substituents may

form a ring.

Examples of a substituent containing an S atom include thiol (-SH), thio (-S-R), sulfinyl (-S=O-R), sulfonyl ($-S(O)_2-R$), and sulfo ($-SO_3H$).

Examples of thio (-S-R) include alkylthio, cycloalkylthio, alkenylthio, alkynylthio, arylthiol, heteroarylthio, and aralkylthio.

Examples of sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl ($-S(O)_2-R$) include alkylsulfonyl, cycloalkylsulfonyl, alkenylsulfonyl, alkynylsulfonyl, arylsulfonyl, heteroarylsulfonyl, and aralkylsulfonyl.

Examples of a substituent containing an N atom include azide ($-N_3$), cyano (-CN), primary amino ($-NH_2$), secondary amino (-NH-R), tertiary amino (-NR(R')), amidino ($-C(=NH)-NH_2$), substituted amidino (-C(=NR)-NR'R"), guanidino ($-NH=C(=NH)-NH_2$), substituted guanidino (-NR-C(=NR'")-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

The two substituents R and R' on the N atom in the tertiary amino (-NR(R')) can each be independently selected from the group consisting of alkyl, cycloalkyl, alkenyl, alkynyl, aryl, heteroaryl, and aralkyl. Examples of the tertiary amino include, for example, alkyl(aralkyl)amino. These two substituents may form a ring.

The three substituents R, R', and R" on the N atom in the substituted amidino (-C(=NR)-NR'R") can each be independently selected from the group consisting of a hydrogen atom, alkyl, cycloalkyl, alkenyl, alkynyl, aryl, heteroaryl, and aralkyl. Examples of the substituted amidino include alkyl(aralkyl)(aryl)amidino. These substituents may together form a ring.

The four substituents R, R', R", and R'" on the N atom in the substituted guanidino (-NR-C(=NR'")-NR'R") can each be independently selected from the group consisting of a hydrogen atom, alkyl, cycloalkyl, alkenyl, alkynyl, aryl, heteroaryl, and aralkyl. These substituents may together form a ring.

The three substituents R, R', and R" on the N atom in the aminocarbonylamino (-NR-CO-NR'R") can each be independently selected from the group consisting of a hydrogen atom, alkyl, cycloalkyl, alkenyl, alkynyl, aryl, heteroaryl, and aralkyl. These substituents may together form a ring.

Examples of a substituent containing a B atom include boryl (-BR(R')) and dioxyboryl (-B(OR)(OR')). The two substituents R and R' on the B atom can each be independently selected from the group consisting of a hydrogen atom, alkyl, cycloalkyl, alkenyl, alkynyl, aryl, heteroaryl, and aralkyl. These substituents may together form a ring.

The hydroxycarboxylic acid in the present disclosure includes α-hydroxycarboxylic acid, β-hydroxycarboxylic acid, and γ-hydroxycarboxylic acid. A side chain other than a hydrogen atom may be attached to the carbon at the α-position in the hydroxycarboxylic acid, as with amino acids. Regarding three-dimensional structures, both the L-type and D-type can be included. The structure of the side chain can be defined similarly to the side chain of the above-mentioned natural amino acid or unnatural amino acid. Examples of hydroxycarboxylic acids include hydroxyacetic acid, lactic acid, and phenyllactic acid.

The amino acid in the present disclosure may be a translatable amino acid. As used in the present disclosure, a "translatable" amino acid means amino acids that can be incorporated into a peptide by translational synthesis (for example, using the translation system described in this disclosure). Whether a certain amino acid is translatable can be confirmed by a translation synthesis experiment using a tRNA to which the amino acid is attached. A reconstituted cell-free translation system may be used in the translation synthesis experiment (see for example, WO2013100132).

In some embodiments, examples of the amino acid in the present disclosure include Pic2 ((2S)-piperidine-2-carboxylic acid), dA ((2R)-2-aminopropanoic acid), MeHph ((2S)-2-(methylamino)-4-phenyl-butanoic acid), SPh2Cl ((2S)-2-amino-3-(2-chlorophenoxy)propanoic acid), MeG (2-(methylamino)acetic acid), nBuG (2-(butylamino)acetic acid), and the like.

The unnatural amino acid according to the present disclosure can be prepared by a conventionally known chemical synthesis method, a synthesis method described in the later-discussed Examples, or a synthesis method similar thereto.

<Preparation of tRNA>

A tRNA can be synthesized, for example, by preparing a DNA encoding a desired tRNA gene, then placing an appropriate promoter such as T7, T3, or SP6 upstream of the DNA, and performing a transcription reaction with the DNA as a template using an RNA polymerase adapted to each promoter. Furthermore, tRNA can also be prepared by purification from biological materials. For example, tRNA can be recovered by preparing an extract solution from a material containing tRNA such as cells, and adding thereto a probe containing a sequence complementary to the base sequence of tRNA. In this case, the material for the preparation may be cells transformed with an expression vector capable of expressing a desired tRNA. Usually, tRNAs synthesized by *in vitro* transcription only contain four typical

bases: adenine, guanine, cytosine, and uracil. On the other hand, tRNAs synthesized in cells may contain modified bases resulting from modification of the typical nucleosides. It is considered that a modified base (for example, lysidine) in a natural tRNA is specifically introduced into that tRNA by the action of an enzyme for that modification (for example, TilS) after the tRNA is synthesized by transcription.

Aminoacyl-tRNAs can also be prepared by chemical and/or biological synthesis methods. For example, an aminoacyl-tRNA can be synthesized using an aminoacyl-tRNA synthetase (ARS) to attach an amino acid to a tRNA. The amino acid may be either natural amino acid or unnatural amino acid as long as it can serve as a substrate for ARS. Alternatively, a natural amino acid may be attached to a tRNA and then chemically modified. Furthermore, as there are many reports that introducing an amino acid mutation into ARSs enhanced their action on unnatural amino acids (see for example, WO2006/135096, WO2007/061136, WO2007/103307, WO2008/001947, WO2010/141851, and WO2015/120287), such mutated ARSs may be used to attach an amino acid to tRNA. In addition to the method using ARSs, aminoacyl-tRNAs can be synthesized by, for example, removing the CA sequence from the 3' end of tRNA, and ligating an aminoacylated pdCpA (a dinucleotide comprising as nucleosides deoxycytidine and adenosine) to it using RNA ligase (pdCpA method; Hecht et al., J Biol Chem (1978) 253: 4517-4520). A method using pCpA (a dinucleotide comprising as nucleosides cytidine and adenosine) instead of pdCpA is also known (pCpA method; Wang et al., ACS Chem Biol (2015)10: 2187-2192). Furthermore, aminoacyl-tRNAs can also be synthesized by attaching an unnatural amino acid previously activated by esterification to a tRNA, using flexizyme, an artificial RNA catalyst (WO2007/066627, WO2012/026566, H. Murakami et al., Chemistry & Biology, Vol. 10, 2003, 655-662; H. Murakami et al., Chemistry & Biology, Vol. 10, 2003, 1077-1084; H. Murakami et al., Nature Methods 3, 2006, 357-359; N. Niwa et al., Bioorganic & Medicinal Chemistry Letters 19, 2009, 3892-3894). A flexizyme is an artificial RNA catalyst that can conjugate an amino acid or a hydroxyl acid to a tRNA. Flexizymes in the present disclosure include flexizyme (Fx) in its original form, and dinitrobenzyl flexizyme (dFx), enhanced flexizyme (eFx), and aminoflexizyme (aFx) that are engineered therefrom.

In one aspect, the present disclosure provides sets of tRNAs suitable for peptide translation. A set of tRNAs contains a plurality of different types of tRNAs, and a plurality of different types of amino acids can be translated from those tRNAs. In one aspect, the present disclosure provides a composition for translation, which contains a plurality of different types of tRNAs suitable for peptide translation. In another aspect, the present disclosure provides a method for producing the above composition for translation. In another aspect, the present disclosure provides a method for producing peptides, comprising providing a plurality of different types of tRNAs suitable for peptide translation. In one aspect, tRNAs of the present disclosure are included in the plurality of different types of tRNAs described above. The following description relates to such tRNAs suitable for peptide translation, compositions for translation, methods for producing compositions for translation, methods for reducing codon misreading, and methods for producing peptides.

In some embodiments, a set of tRNAs in the present disclosure may include the tRNA of the present disclosure described above (it may be referred to as "first tRNA" in the present disclosure) and a second tRNA. While any tRNA can be used as the second tRNA, it may be the above tRNA of the present disclosure, independently of the first tRNA.

In some embodiments, the bases at the first letter of the anticodons in the first tRNA and the second tRNA included in a set of tRNAs in the present disclosure may be different from each other. The bases at the second letter of the anticodons in the above-mentioned first tRNA and second tRNA may be the same, and the bases at the third letter of the anticodons in the above-mentioned first tRNA and second tRNA may be the same. In some embodiments, a codon complementary to the anticodon in the first tRNA and a codon complementary to the anticodon in the second tRNA may be present in the same codon box. In some embodiments, at least two types of amino acids can be translated from a single codon box by using the first tRNA of the present disclosure and the second tRNA of the present disclosure. In some embodiments, the base at the first letter of the anticodon in the first tRNA may be A or G, and the base at the first letter of the anticodon in the second tRNA may be C or U. In some embodiments, the respective anticodons in the first tRNA and the second tRNA may have the following combinations for the bases at the first letter: (A, C); (C, A); (G, C); (C, G); (A, U); (U, A); (G, U); and (U, G). Here, (A, C) indicates that the base at the first letter of the anticodon in the first tRNA is A, and the base at the first letter of the anticodon in the second tRNA is C.

In some embodiments, each tRNA bodies of the first tRNA and the second tRNA included in a set of tRNAs of the present disclosure, may be different from each other or the same. In some embodiments, the base sequences of positions 1 to 31 and positions 39 to 74 in the first tRNA and in the second tRNA may be the same, or they may have sequence identity of 80% or higher, 85% or higher, 90% or higher, 91% or higher, 92% or higher, 93% or higher, 94% or higher, 95% or higher, 96% or higher, 97% or higher, or 98% or higher.

In some embodiments, mutually different amino acids may be attached to the first tRNA and the second tRNA included in the sets of tRNAs in the present disclosure. In the present disclosure, sometimes the amino acid attached to the first tRNA is called the first amino acid, and the amino acid attached to the second tRNA is called the second amino acid. In a certain embodiment, at least one selected from the first amino acid and second amino acid in the present disclosure may be an unnatural amino acid. More specifically, at least one or both of the set of tRNAs in the present disclosure may have an unnatural amino acid attached thereto. In some embodiments, either one or both of the first tRNA and the second tRNA may be a tRNA to which an unnatural amino acid is attached outside a translation system.

Without being limiting, in some embodiments, when either one or both of the first tRNA the second tRNA have chimeric tRNA bodies carrying the combination of bases 32U, 33U, 37G, and 38U, sets of tRNAs containing the first tRNA and the second tRNA which have at least one anticodon combination selected from the group consisting of (L1) to (L4) below may be excluded from the sets of tRNAs of the present disclosure:

(L1) GCG and CCG, and CCG and GCG;
(L2) AAG and UAG, AAG and CAG, and UAG and CAG;
(L3) bases at the second letter are both C, and bases at the third letter are both G in the anticodons; and
(L4) bases at the second letter are both A, and bases at the third letter are both G in the anticodons.

The compositions for translation in the present disclosure are not limited as long as they contain tRNAs of the present disclosure, and they may contain constituent components necessary for translation, and contain the same constituent components as the translation systems of the present disclosure. Compositions for translation in the present disclosure may be cell-free translation systems or reconstituted cell-free translation systems. Without intending to be limiting, in some embodiments, a composition for translation of the present disclosure may be a cell-free translation system reconstituted by an E. coli-derived factor, and may contain ribosomes, translation initiation factors, translation termination factors, translation elongation factors, amino acids, aminoacyl-tRNA synthetase (aaRS), and such. In some embodiments, compositions for translation of the present disclosure may comprise E. coli-derived ribosomes. In some embodiments, tRNAs of the present disclosure may be E. coli-derived tRNAs.

In some embodiments, compositions for translation of the present disclosure may contain sets of tRNAs of the present disclosure. A composition for translation in the present disclosure may contain the following number of sets of tRNAs of the present disclosure: 1 set, 2 sets, 3 sets, 4 sets, 5 sets, 6 sets, 7 sets, or 8 sets; or 1 or more sets, 2 or more sets, 3 or more sets, 4 or more sets, 5 or more sets, or 6 or more sets; or not more than 8 sets, not more than 7 sets, not more than 6 sets, not more than 5 sets, not more than 4 sets, not more than 3 sets, or not more than 2 sets. In further embodiments, compositions for translation of the present disclosure may contain tRNAs other than those mentioned above.

In some embodiments, when a composition for translation of the present disclosure contains an aaRS, the set of tRNAs of the present disclosure contained in the composition for translation may be tRNAs having orthogonal relationship to the aaRS. The tRNAs having orthogonal relationship to aaRS means tRNAs that are not aminoacylated by the aaRS present in the composition for translation, but can be taken up into a ribosome for translational incorporation of an amino acid. Examples of such tRNAs include tRNA Glu2, tRNA AsnE2, tRNA Asp1, and tRNA Pro1E2, or tRNAs derived from them. When necessary, the aaRSs that recognize these tRNAs are removed from the compositions for translation. In some embodiments, a composition for translation of the present disclosure may not contain an aaRS that can attach an amino acid to either one of the set of tRNAs.

In some embodiments, compositions for translation and kits for translation of the present disclosure can reduce misreading of codons. Compositions for translation and kits for translation of the present disclosure can reduce the rate of translational incorporation of unintended amino acids caused by codon misreading. Compositions for translation and kits for translation of the present disclosure can reduce the rate of translation of a codon contained in a template mRNA by a tRNA carrying an anticodon not complementary to this codon. In some embodiments, compositions for translation and kits for translation of the present disclosure can reduce misreading of a codon by a tRNA in which the second and third letters of its anticodon are complementary to the second and first letters of the codon, respectively, the anticodon and the codon are in a relationship where Watson-Crick base pairs may be formed, and the first letter of the anticodon is not complementary to the third letter of the anticodon. In some embodiments, because of this reduction in misreading, use of a composition for translation and a kit for translation of the present disclosure will enable two or more amino acids to be assigned within the same codon box. In a specific embodiment, two or more unnatural amino acids may be assigned within the same codon box. For example, reducing misreading of the CCG codon by a tRNA carrying the AGG anticodon enables, while assigning an amino acid to the CCG codon, assigning a different amino acid to a codon complementary to the AGG anticodon (for example, CCU) , or more specifically, it enables assigning two different amino acids to the same codon box (the CCM box in this case).

This way, reducing the frequency of translational incorporation of a wrong amino acid caused by codon misreading, which may take place when two or more amino acids are assigned to the same codon box, enables increasing the number of amino acids assigned in the genetic code table and enables accurate peptide translation.

In some embodiments, since the properties of the tRNA itself can be changed according to the present disclosure, the compositions and kits are more convenient than methods involving adjusting the amount of aminoacyl tRNA contained in the composition for translation or constituting the kit for translation, or interrupting the translation reaction. Although not intended to be bound by specific theory, for example, when the amount of aminoacyl tRNA in a translation system is increased to prevent misreading of codons due to depletion of aminoacyl tRNAs, misreading may take place from the opposite direction. The compositions for translation and kits for translation of the present disclosure are also useful in

that they can accomplish accurate peptide translation without increasing the amount of aminoacyl tRNA in the translation system. In certain embodiments, use of a composition for translation and a kit for translation of the present disclosure in combination with the above-mentioned methods for adjusting the amount of aminoacyl tRNA and interrupting the translation reaction may result in better effects of reducing codon misreading. In one embodiment, in compositions for translation and kits for translation of the present disclosure, multiple types of tRNAs attached with different amino acids assigned to different codons in the same codon box, can be said to be in an independent relationship in which they do not misread each other, or more specifically, in an orthogonal relationship. The naturally occurring biological translation system essentially has a strict correspondence established between a codon and an amino acid; therefore, addition of a tRNA having no orthogonality may cause the correspondence to collapse, and lead to a catastrophic effect on the function of the translation system. Therefore, in one embodiment of the present disclosure, establishment of orthogonality among the multiple types of tRNAs may be one of the important features.

In some embodiments, compositions for translation and kits for translation of the present disclosure reduces codon misreading, while enabling assigning multiple types of amino acids, particularly multiple types of amino acids including unnatural amino acids, to each of codon boxes, such as UCM, CUM, CCM, CGM, ACM, GUM, GCM, and GGM, to which only one type of amino acid is assigned in the natural genetic code table. How to assign the amino acids is not particularly limited, and for example, the first amino acid may be assigned to the $M_1M_2U$ codon or the $M_1M_2C$ codon, and the second amino acid may be assigned to the $M_1M_2A$ codon or the $M_1M_2G$ codon; or the first amino acid may be assigned to the $M_1M_2U$ codon, and the second amino acid may be assigned to the $M_1M_2G$ codon.

In some embodiments, compositions for translation and kits for translation of the present disclosure may contain at least one type of mRNA carrying a codon complementary to the anticodon in the second tRNA, and/or at least one type of mRNA carrying a codon complementary to the anticodon in the first tRNA. In a certain embodiment, the two codons may exist on the same mRNA or on different mRNAs. In some embodiments, a composition for translation of the present disclosure may contain multiple types of mRNAs having sequences different from each other, and may contain an mRNA library.

In some embodiments, a method for producing a composition for translation and a kit for translation of the present disclosure may comprise a step of preparing the first and/or second tRNA by *in vitro* transcription. In some embodiments, a method for producing a composition for translation and a kit for translation of the present disclosure may comprise preparing a tRNA of the present disclosure by attaching an amino acid to the tRNA outside a translation system. The method may also comprise preparing a first tRNA and a second tRNA by attaching an amino acid to the tRNAs outside a translation system. In some embodiments, a method for producing a composition for translation and a kit for translation of the present disclosure may comprise the above method for preparing tRNAs of the present disclosure. In some embodiments, the above-mentioned amino acid may be an unnatural amino acid.

In some embodiments, a method for producing peptides of the present disclosure may comprise using a composition for translation or a kit for translation of the present disclosure to translate a nucleic acid that serves as a template. While the method for translation is not limited, examples include cell-free translation *(in vitro* translation), such as translation using a reconstituted cell-free translation system, or more specifically, translation using an *E. coli*-derived reconstituted cell-free translation system.

In some embodiments, methods for producing peptides of the present disclosure may comprise using a composition for translation or a kit for translation of the present disclosure to translate an mRNA carrying a codon complementary to an anticodon in a second tRNA and/or an mRNA carrying a codon complementary to an anticodon in a first tRNA. In a certain embodiment, the two codons may be included in the same mRNA or in different mRNAs. In some embodiments, methods for producing peptides of the present disclosure may comprise using a composition for translation of the present disclosure to translate multiple types of nucleic acids with sequences different from each other, translate a nucleic acid library, or translate an mRNA library. The mRNAs may encode peptides having the desired or random amino acid sequences. Adding the mRNAs to a translation system of the present disclosure will allow translation of the mRNAs into peptides. On the other hand, when RNA polymerases for transcribing DNAs into mRNAs are contained in the translation system, adding DNAs to the translation system of the present disclosure will allow transcription of the DNAs into mRNAs and translation of the mRNAs to peptides to be performed together.

In some embodiments, a codon complementary to the above-mentioned anticodon may be a codon that forms Watson-Crick base pairs with all three bases of the anticodon, or a codon whose third letter forms a wobble base pair with the first letter of the anticodon.

In some embodiments, a method for producing peptides of the present disclosure may comprise assigning multiple types of amino acids to at least 1, 2 or more, 3 or more, 4 or more, 5 or more, or 6 or more codon boxes selected from the group consisting of UCM, CUM, CCM, CGM, ACM, GUM, GCM, and GGM. Even in such cases, accurate peptide translation can be possible.

In some embodiments, according to a method for producing peptides of the present disclosure, codon misreading may be reduced, while assigning different types of amino acids to codons which are in the same codon box and in which the third letters are at least one combination selected from the group consisting of the following (i) to (iv): (i) U and G,

(ii) C and G, (iii) U and A, and (iv) C and A. In some embodiments, according to a translation method of the present disclosure, codon misreading may be reduced, while assigning different types of amino acids to the $UCM_3$, $CUM_3$, $CCM_3$, $CGM_3$, $ACM_3$, $GUM_3$, $GCM_3$, or $GGM_3$ codons in which the $M_3$'s are at least one combination selected from the group consisting of the above (i) to (iv).

In the present disclosure, reduction in codon misreading by tRNAs may be evaluated by using a plurality of tRNAs carrying different anticodons, to which different amino acids have been attached, to translate a single type of template mRNA. For the plurality of tRNAs, one can select tRNAs carrying an anticodon complementary to a particular codon that is to be translated, and tRNAs carrying an anticodon that is different from the above-mentioned anticodon and is complementary to a codon present in the same codon box as the above-mentioned codon.

For example, to evaluate misreading of the CCG codon by a tRNA carrying the AGG anticodon, a translation system containing a tRNA carrying the AGG anticodon to which an amino acid $AA_1$ is attached, and a tRNA carrying the CGG anticodon to which an amino acid $AA_2$ is attached, is used to translate a template mRNA containing the CCG codon. In this case, the targeted translated amino acid is $AA_2$ which is introduced through translation by the tRNA carrying the CGG anticodon complementary to the CCG codon, and the translated amino acid that occurred through misreading is $AA_1$ which is introduced through translation by the tRNA carrying the AGG anticodon not complementary to the CCG codon. Percentages of such translated products can be used as indices to evaluate reduction in misreading.

As the template mRNA for use in the above-mentioned evaluation, an mRNA selected from MR-1 to MR-7 described in the Examples may be used, or other mRNAs may be used according to the codon to be evaluated. As the translation system for translating the template mRNA, a prokaryote-derived reconstituted cell-free protein synthesis system (for example, the PURE system) may be used. "Translation condition 1" of the present disclosure may be used as the condition for translation. Translation methods and evaluation methods are described in more detail in the Examples.

In the present disclosure, reduction of codon misreading by tRNA can be evaluated by the percentage (%) of misread peptides relative to an intended product. This percentage is calculated using the following equation. When the percentage is low in comparison to control tRNA in which the combination of bases at positions 32, 33, 37 and 38 is un-engineered, the evaluated tRNA is determined to have effects of reducing codon misreading. The rate of reduction in percentage is not particularly limited, but a tRNA of the present disclosure may show 5% or higher, 10% or higher, 20% or higher, 30% or higher, 40% or higher, or 50% or higher reduction in the percentage as compared to the control. In the present disclosure, the peptide obtained when the codon is correctly read may be referred to as the correctly read translation product or intended product.

[Equation 1]

$$\text{Percentage of misread peptides relative to the intended product (\%)} = \frac{\text{Amount of translated peptide obtained when misreading takes place } (\mu M)}{\text{Amount of translated peptide obtained when correctly read } (\mu M)} \times 100$$

The amount of translated peptide may be determined by the following method. More specifically, the translation product solution obtained after completion of the translation reaction is diluted and analyzed using a LC-FLR-MS setup. An exemplary degree of dilution is 10-fold. By determining the retention time of the translated peptide of interest from the obtained MS data, and quantifying the fluorescence peak at the retention time, the amount of translated peptide is evaluated. The quantification is carried out by producing a calibration curve using a standard, and calculating the content by relative quantification. As the standard, LCT-67 or LCT-12 may be used.

The sequence of LCT-67 is BdpF:Thr:Phe:Ile:Ile:Gly:Phe:Ile:Ile:Ile:Pro:Ile:Gly (SEQ ID NO: 237), and the sequence of LCT-12 is
BdpF:Thr:Ile:Phe:Pro:Gly:Phe:Ile:Ile:Thr:Thr:Gly:Thr:Gly:Thr:Gly:Thr:Gly:Ala (SEQ ID NO: 238).

In one aspect, the present disclosure provides peptides and peptide libraries produced by using the compositions for translation of the present disclosure. The peptides of the present disclosure include peptides obtained by performing chemical modification or such after translation, and peptide-nucleic acid complexes formed by linking nucleic acids.

Examples of post-translational modification include cyclization of a linear peptide. As the bond for forming the cyclic portion, for example, a peptide bond formed from an amino group and a carboxyl group can be used. In addition, an amide bond, disulfide bond, ether bond, thioether bond, ester bond, thioester bond, carbon-carbon bond, alkyl bond, alkenyl bond, phosphonate ether bond, azo bond, amine bond, C=N-C bond, lactam bridge, carbamoyl bond, urea bond, thiourea bond, thioamide bond, sulfinyl bond, sulfonyl bond, triazole bond, benzoxazole bond, and such formed from a combination of appropriate functional groups can be used. The carbon-carbon bond can be formed by a transition metal-catalyzed reaction such as a Suzuki reaction, a Heck reaction, and a Sonogashira reaction. In one embodiment, the

peptides of the present disclosure contain at least one set of functional groups capable of forming the above-mentioned bond in the molecule. The formation of the cyclic portion may be performed by producing a linear peptide using the translation system of the present disclosure and then separately performing a reaction for linking the above-mentioned functional groups with each other. Regarding the synthesis of the peptide having a cyclic portion, one can refer to WO2013/100132, WO2012/026566, WO2012/033154, WO2012/074130, WO2015/030014, WO2018/052002, Comb. Chem. High Throughput Screen (2010)13: 75-87, Nat. Chem. Biol. (2009) 5: 502-507, Nat. Chem. Biol. (2009) 5: 888-90, Bioconjug. Chem. (2007) 18: 469-476, ChemBioChem (2009) 10: 787-798, Chem. Commun. (Camb) (2011) 47: 9946-9958, and such.

There are known methods for identifying peptides that may bond to a targeted molecule through a peptide library (display library) that uses peptide-nucleic acid complexes. A display library is a library in which a phenotype and a genotype are associated with each other as a result of formation of a single complex by linking a peptide to a nucleic acid encoding that peptide. Examples of major display libraries include libraries prepared by the mRNA display method (Roberts and Szostak, Proc. Natl. Acad. Sci. USA (1997) 94: 12297-12302), *in vitro* virus method (Nemoto et al., FEBS Lett. (1997) 414: 405-408), cDNA display method (Yamaguchi et al., Nucleic Acids Res. (2009) 37: e108), ribosome display method (Mattheakis et al, Proc. Natl. Acad. Sci. USA (1994) 91: 9022-9026), covalent display method (Reiersen et.al., Nucleic Acids Res. (2005) 33: e10), CIS display method (Odegrip et.al., Proc. Natl. Acad. Sci. USA (2004) 101: 2806-2810), and such. Alternatively, a library prepared by using the *in vitro* compartmentalization method (Tawfik and Griffiths, Nat. Biotechnol. (1998) 16: 652-656) can be mentioned as one embodiment of the display library.

In one aspect, the present disclosure provides a method for identifying a peptide having binding activity to a target molecule, which comprises contacting the target molecule with a peptide library described in the present disclosure. The target molecule is not particularly limited and can be appropriately selected from, for example, low molecular weight compounds, high molecular weight compounds, nucleic acids, peptides, proteins, sugars, and lipids. The target molecule may be a molecule existing outside the cell or a molecule existing inside the cell. Alternatively, it may be a molecule existing in the cell membrane, in which case any of the extracellular domain, the transmembrane domain, and the intracellular domain may be the target. In the step of contacting the target molecule with the peptide library, the target molecule is usually immobilized on some kind of solid-phase carrier (for example, a microtiter plate or microbeads). Then, by removing the peptides not binding to the target molecule and recovering only the peptides binding to the target molecule, the peptides having binding activity to the target molecule can be selectively concentrated (panning method). When the peptide library used is a nucleic acid display library, the recovered peptides have the nucleic acid encoding their respective genetic information attached to them; therefore, the nucleic acid sequence encoding the recovered peptide and the amino acid sequence can be readily identified by isolating and analyzing them. Furthermore, based on the obtained nucleic acid sequence or amino acid sequence, the identified peptides can be individually produced by chemical synthesis or gene recombination techniques.

In one aspect, the present disclosure provides methods for reducing misreading of codons by tRNAs, and compositions and kits for reducing misreading of codons by tRNAs. Such compositions and kits may contain tRNAs of the present disclosure. Furthermore, such a method may involve obtaining tRNAs of the present disclosure by engineering tRNAs. Specifically, it is a method for reducing misreading of a second codon by a tRNA carrying an anticodon complementary to a first codon, comprising substituting at least one base at a position selected from the group consisting of positions 32, 33, 37, and 38 in the tRNA; wherein, the substituted tRNA is a tRNA of the present disclosure, the bases at the first letters of the first codon and the second codon are the same, the bases at the second letters of the first codon and the second codon are the same, and the bases at the third letters of the first codon and the second codon are different from each other.

In some embodiments, in the method for reducing codon misreading of the present disclosure, the first codon is $M_1M_2X$ and the second codon is $M_1M_2Y$, wherein the above $M_1$ and $M_2$ are each independently A, C, G, or U, and the above X and Y are bases different from each other, each selected from A, C, G, and U. The combination of the bases for the above X and Y may be any one selected from the group consisting of (a1) to (a8) below: (a1) U and G; (a2) G and U; (a3) U and A; (a4) A and U; (a5) C and A; (a6) A and C; (a7) C and G; and (a8) G and C. In the present disclosure, the combination of the bases for the above X and Y is $M_{31}$ and $M_{32}$ means that the above X is base $M_{31}$ and the above Y is base $M_{32}$. In some embodiments, the combination of the bases for the above X and Y may be any one selected from the group consisting of the above (a1) to (a3), (a5), and (a7), or the group consisting of the above (a1) and (a2). In some embodiments, the above $M_1M_2$ may be a base sequence of any one selected from the group consisting of (b1) to (b8) below: (b1) CC; (b2) CU; (b3) GG; (b4) GU; (b5) GC; (b6) UC; (b7) CG; and (b8) AC. In certain embodiments, the above $M_1M_2$ may be any one base sequence selected from the group consisting of the above (b1) to (b3).

In some embodiments, in a method for reducing codon misreading of the present disclosure, the first codon and the anticodon in the tRNA may form Watson-Crick base pairs at all three bases, or the base at the third letter of the first codon and the base at the first letter of the anticodon in the tRNA may form a wobble base pair.

In some embodiments, methods for reducing codon misreading of the present disclosure may be methods for reducing misreading of a codon complementary to the anticodon in the second tRNA of the present disclosure by the

first tRNA of the present disclosure. In some embodiments, methods for reducing codon misreading of the present disclosure may include the above methods for preparing tRNAs of the present disclosure.

[Examples]

The present invention is further illustrated by the following examples, but is not limited thereto.
The following abbreviations were used in the Examples.

AA: ammonium acetate
$CH_2CN$: cyanomethyl group
DBU: 1,8-diazabicyclo[5.4.0]-7-undecene
DCM: dichloromethane
DIC: N,N-diisopropylcarbodiimide
DIPEA: N,N-diisopropylethylamine
DMF: dimethylformamide
DMSO: dimethyl sulfoxide
FA: formic acid
Fmoc: 9-fluorenylmethyloxycarbonyl group
F-Pnaz: 4-(2-(4-fluorophenyl)acetamido)benzyloxycarbonyl group

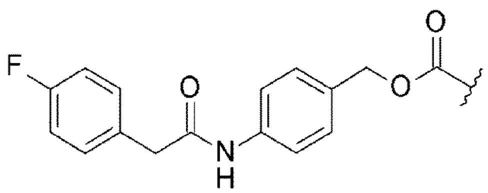

HFIP: 1,1,1,3,3,3-hexafluoro-2-propanol
MeCN: acetonitrile
NMP: N-methyl-2-pyrrolidone
TEA: triethylamine
TFA: trifluoroacetic acid
TFE: 2,2,2-trifluoroethanol
THF: tetrahydrofuran

The following abbreviations were used in this Example: Gly or G (glycine), Ile or I (isoleucine), Leu or L (leucine), Phe or F (phenylalanine), Pro or P (proline), Thr or T (threonine). In addition to these, the abbreviations shown in Table 3 were used in the present disclosure. For the amino acid sequences in the present disclosure, BdpFL-Phe may be written as "BdpF".

[Table 3]

| Abbreviation | Structural formula | | Abbreviation | Structural formula |
|---|---|---|---|---|
| BdpFL -Phe | 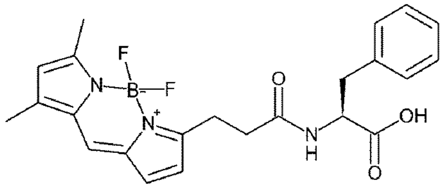 | | Pic2 | 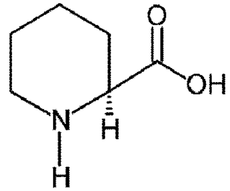 |
| dA | 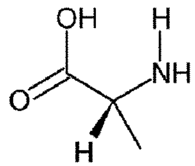 | | MeHph | 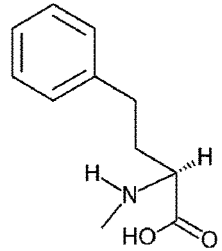 |
| SPh2Cl | 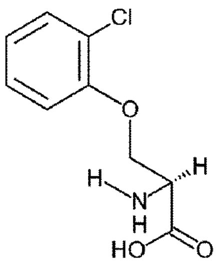 | | MeG | 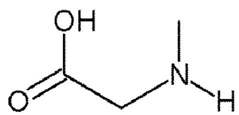 |
| nBuG | 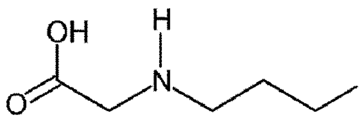 | | Thr (THP) | 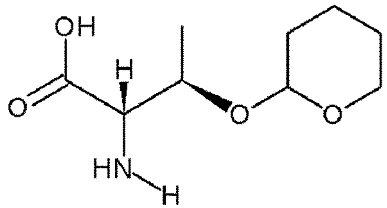 |

The LCMS analysis conditions are shown below in Table 4.

[Table 4]

| Preparative condition | System | Column (I.D. x length (mm)) | Mobile phase | Gradient (A/B) | Flow rate (ml/min) | Column temperature (°C) | Wavelength |
|---|---|---|---|---|---|---|---|
| SQD FA05_01 | Acquity UPLC/SQD | Aldrich Ascentis Express C18 1.7μm (2.1 x 50) | A) 0.1% FA, H2O B) 0.1% FA CH3CN | 95/5 => 0/100 (1.0min) => 0/100 (0.4min) | 1.0 | 35 | 210-400nm PDA total |
| SQD FA05_02 | Acquity UPLC/SQD2 | Aldrich Ascentis Express C18 2.7μm (2.1 x 50) | A) 0.1% FA, H2O B) 0.1% FA CH3CN | 95/5 => 0/100 (1.0min) => 0/100 (0.4min) | 0.9 | 35 | 210-400nm PDA total |
| SMD method 1 | Shimadzu LCMS-2020 LC-20AD | Shim-Pack XR-ODS 1.7μm (2.1 x 50) | A) 0.1% FA, H2O B) 0.1% FA CH3CN | 90/10 => 0/100 (1.1 min) => 0/100 (0.6 min) | 1.2 | 40 | 190-400nm PDA total |
| SMD method 2 | Shimadzu LCMS-2020 LC-20ADXR | CORTECS C18 2.7μm (2.1 x 50) | A) 0.1% FA, H2O B) 0.1% FA CH3CN | 90/10 => 0/100 (1.2 min) => 0/100 (0.5 min) | 1.0 | 40 | 190-400nm PDA total |
| SMD method 3 | Shimadzu LCMS-2020 LC-20ADXR | CORTECS C18 2.7μm (2.1 x 50) | A) 0.1% FA, H2O B) 0.1% FA CH3CN | 95/5 => 0/100 (1.0min) => 0/100 (0.5min) | 1.0 | 40 | 190-400nm PDA total |
| SMD method 4 | Shimadzu LCMS-2020 LC-30AD | Halo C18 2.0μm (2.1 x 30) | A) 0.05% TFA, H2O B) 0.05% TFA CH3CN | 95/5 => 0/100 (0.7min) => 0/100 (0.25 min) | 0.8 | 40 | 190-400nm PDA total |
| SQD AA50long | Acquity UPLC/SQD2 | Aldrich Ascentis Express C18 5.0μm (2.1 x 50) | A) 10mM AcONH4, H2O B) MeOH | 50/50 => 0/100 (4.5 min) => 0/100 (0.5 min) | 0.9 | 35 | 210-400nm PDA total |
| LTQ TEA/HFIP05_01 | Acquity UPLC/LTQ Orbitrap XL | Waters ACQUITY UPLC BEH C18 1.7μm (2.1 x 50) | A) 15mM TEA, 400mM HFIP H2O B) 15mM TEA, 400mM HFIP MeOH | 95/5 => 10/90 (9.0 min) => 10/90 (1.0 min) | 0.2 | 30 | 190-400nm PDA total |
| LTQ TEA/HFIP05_02 | Acquity UPLC/LTQ Orbitrap XL | Waters ACQUITY UPLC BEH C18 1.7μm (2.1 x 50) | A) 15mM TEA, 400mM HFIP H2O B) 15mM TEA, 400mM HFIP MeOH | 95/5 => 95/5 (8.0 min) => 10/90 (2.0 min) | 0.2 | 30 | 190-400nm PDA total |
| LTQ TEA/HFIP05_03 | Acquity UPLC/LTQ Orbitrap XL | Waters ACQUITY UPLC BEH C18 1.7μm (2.1 x 50) | A) 15mM TEA, 400mM HFIP H2O B) 15mM TEA, 400mM HFIP MeOH | 95/5 => 70/30 (9.0min) => 10/90 (1.0 min) | 0.2 | 30 | 190-400nm PDA total |

Example 1. Synthesis of aminoacyl pCpAs

Aminoacyl pCpAs (SS14, SS15, SS16, and SS45) were synthesized according to the following scheme.

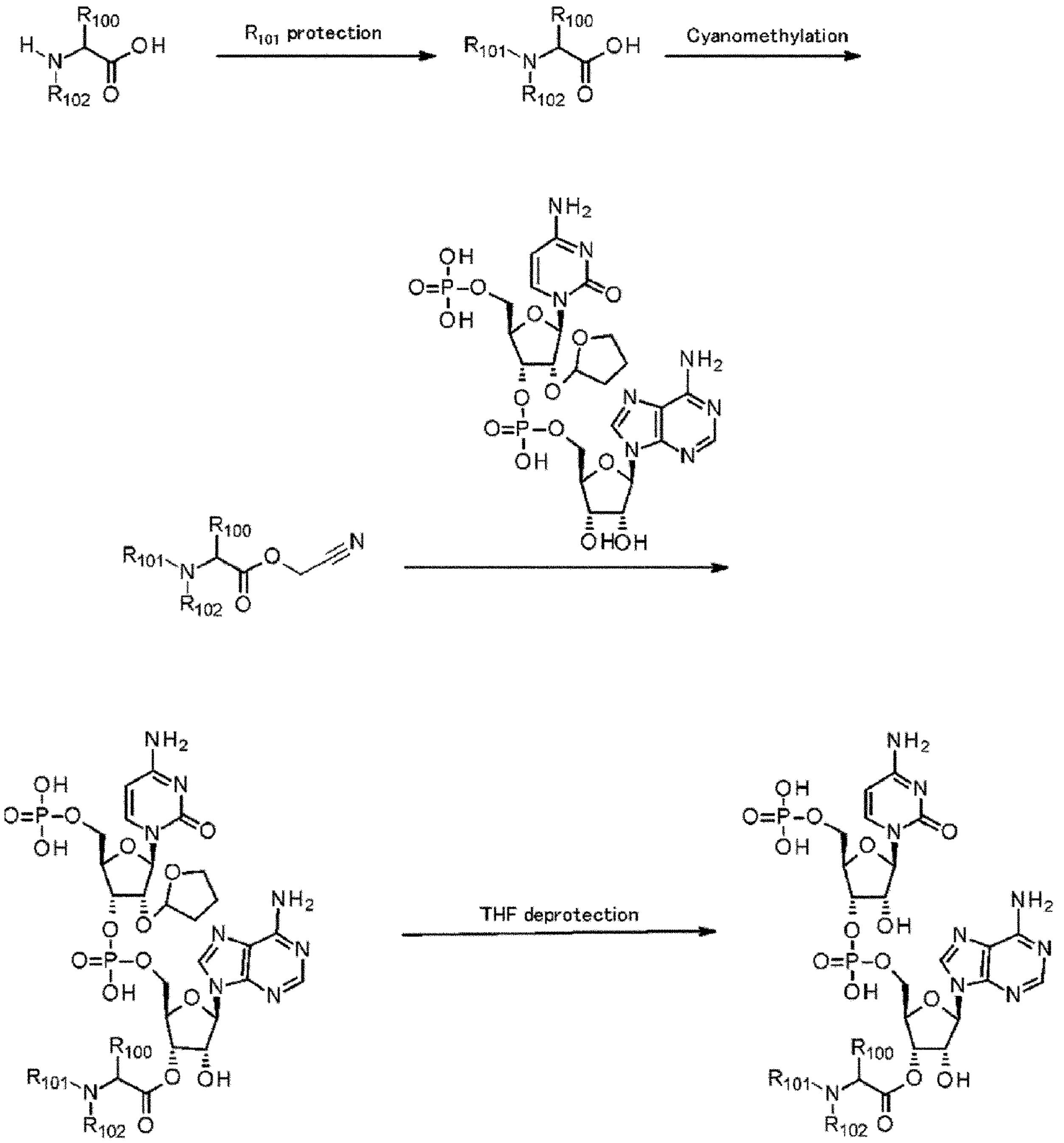


Synthesis of (S)-1-(((4-(2-(4-fluorophenyl)acetamido)benzyl)oxy)carbonyl)piperidine-2-carboxylic acid (Compound SS17, F-Pnaz-Pic2-OH)

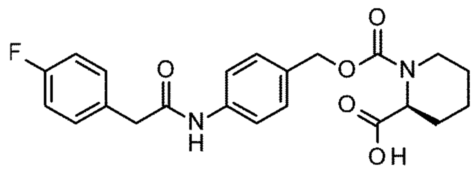

Under nitrogen atmosphere, DMF (330 μL) was added to a mixture of (S)-piperidine-2-carboxylic acid (42.6 mg, 0.33 mmol) and (4-nitrophenyl)-4-(2-(4-fluorophenyl)acetamido)benzyl carbonate (Compound ts11) (140 mg, 0.44 mmol) synthesized by the method of a patent literature (WO2018143145A1) at room temperature. After stirring this mixture at room temperature for 5minutes, triethylamine (105.6 μL, 2.25 mmol) was added at 0°C. The reaction mixture was stirred at room temperature for 30 minutes, and then purified by reverse-phase silica gel column chromatography (0.1% aqueous formic acid solution/0.1% formic acid-acetonitrile solution) to obtain (S)-1-(((4-(2-(4-fluorophenyl)acetamido)benzyl)oxy)carbonyl)piperidine-2-carboxylic acid (Compound SS17, F-Pnaz-Pic2-OH) (92 mg, 67%).

LCMS (ESI) m/z = 413 (M-H)$^-$

Retention time: 0.70 minutes (analysis condition SQDFA05_01)

Synthesis of 1-(4-(2-(4-fluorophenyl)acetamido)benzyl) 2-(cyanomethyl) (S)-piperidine-1,2-dicarboxylate (Compound SS18, F-Pnaz-Pic2-$OCH_2CN$)

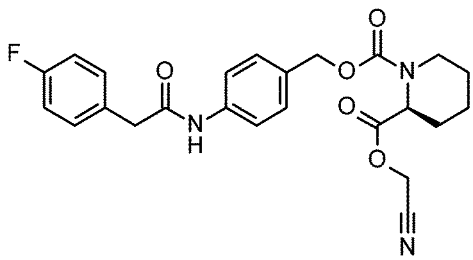

Under nitrogen atmosphere, (S)-1-(((4-(2-(4-fluorophenyl)acetamido)benzyl)oxy)carbonyl)piperidine-2-carboxylic acid (Compound SS17, F-Pnaz-Pic2-OH) (30 mg, 0.072 mmol) and N-ethyl-isopropylpropan-2-amine (DIPEA) (20.23 μL, 0.116 mmol) were dissolved in acetonitrile (90 μL), added with 2-bromoacetonitrile (5.34 μL, 0.080 mmol) at 0°C, and the mixture was stirred at room temperature for 2hours. The reaction solution was concentrated to obtain a crude product, 1-(4-(2-(4-fluorophenyl)acetamido)benzyl) 2-(cyanomethyl) (S)-piperidine-1,2-dicarboxylate (Compound SS18, F-Pnaz-Pic2-$OCH_2CN$). The obtained crude product was dissolved in acetonitrile (2.00 mL), and was directly used in the next step.

LCMS (ESI) m/z = 452 (M-H)$^-$

Retention time: 0.79 minutes (analysis condition SQDFA05_01)

Synthesis of 1-(4-(2-(4-fluorophenyl)acetamido)benzyl) 2-((2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl) (2S)-piperidine-1,2-dicarboxylate (Compound SS14, F-Pnaz-Pic2-pCpA)

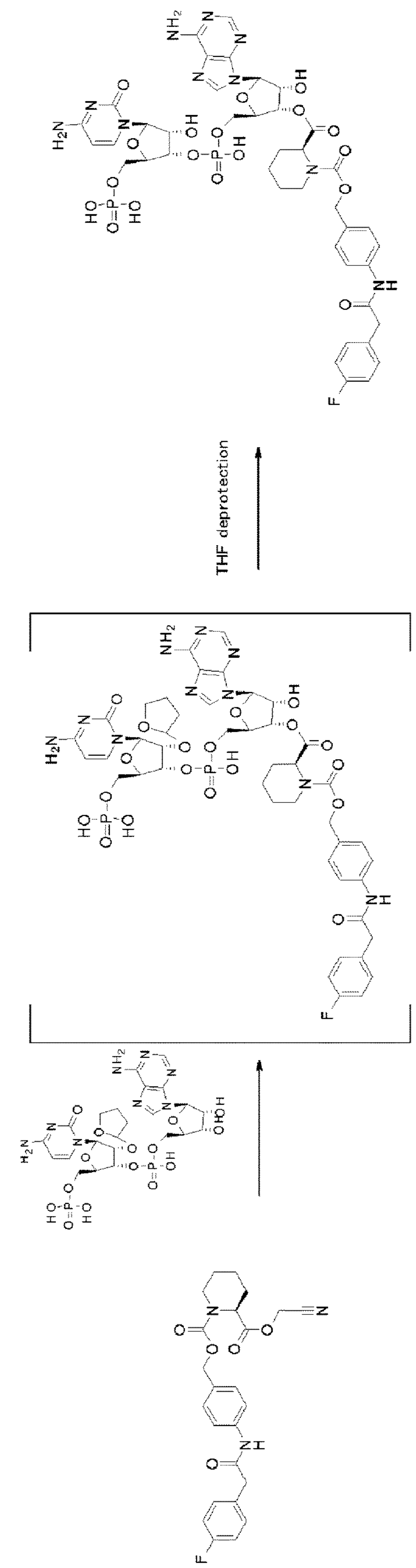


((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (113 mg, 0.156 mmol) synthesized by a method described in a literature (Helv. Chim. Acta, 90, 297-310) was dissolved in Buffer A (40 mL), a solution of 1-(4-(2-(4-fluorophenyl)acetamido)benzyl) 2-(cyanomethyl) (S)-piperidine-1,2-dicarboxylate (Compound SS18, F-Pnaz-Pic2-$OCH_2CN$) (35.4 mg, 0.078 mmol) in acetonitrile (2.00 mL) was added, and the mixture was stirred at room temperature for 150 minutes. The reaction solution was cooled to 0°C, and then trifluoroacetic acid (2.00 mL) was added. The reaction solution was stirred at 0°C for 45 minutes, and then purified by reverse-phase silica gel column chromatography (0.05% aqueous trifluoroacetic acid solution/ 0.05% trifluoroacetic acid-acetonitrile) to obtain the title compound (Compound SS14, F-Pnaz-Pic2-pCpA) (6.0 mg, 7.3%).

LCMS (ESI) m/z = 1047.5 (M-H)-

Retention time: 0.50 minutes (analysis condition SQDFA05_01)

Buffer A was prepared as follows.

Acetic acid was added to an aqueous solution of N,N,N-trimethylhexadecan-1-aminium chloride (6.40 g, 20 mmol) and imidazole (6.81 g, 100 mmol) to give Buffer A (1L) of 20 mM N,N,N-trimethylhexadecan-1-aminium and 100 mM imidazole at pH 8.

Synthesis of O-(2-chlorophenyl)-N-(((4-(2-(4-fluorophenyl)acetamido)benzyl)oxy)carbonyl)-L-serine (Compound SS19, F-Pnaz-SPh2Cl-OH)

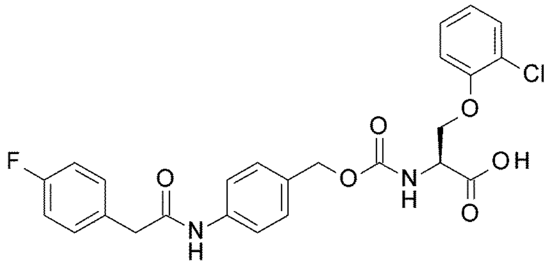

Under nitrogen atmosphere, DMSO (15 mL) and triethylamine (0.95 g, 9.42 mmol) were added to a mixture of O-(2-chlorophenyl)-L-serine (Compound aa63) (1.25 g, 5.80 mmol) synthesized by a method described in a patent literature (WO2018225864) and (4-nitrophenyl)-4-(2-(4-fluorophenyl)acetamido)benzyl carbonate (Compound ts11) (2 g, 4.71 mmol) synthesized by a method described in a patent literature (WO2018143145A1) at room temperature. The reaction mixture was stirred at room temperature for 16 hours and then purified by reverse-phase silica gel column chromatography (0.1% aqueous formic acid solution/0.1% formic acid-acetonitrile solution) to obtain O-(2-chlorophenyl)-N-(((4-(2-(4-fluorophenyl)acetamido)benzyl)oxy)carbonyl)-L-serine (Compound SS19, F-Pnaz-SPh2Cl-OH) (1.8 g, 73%).

LCMS (ESI) m/z = 523 (M+Na)+

Retention time: 1.26 minutes (analysis condition SMD method 1)

Synthesis of cyanomethyl O-(2-chlorophenyl)-N-(((4-(2-(4-fluorophenyl)acetamido)benzyl)oxy)carbonyl)-L-seiinate (Compound SS20, F-Pnaz-SPh2Cl-$OCH_2CN$)

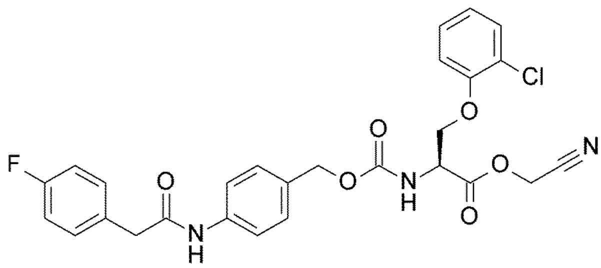

Under nitrogen atmosphere, O-(2-chlorophenyl)-N-(((4-(2-(4-fluorophenyl)acetamido)benzyl)oxy)carbonyl)-L-serine (Compound SS19, F-Pnaz-SPh2Cl-OH) (800 mg, 1.60 mmol) and N-ethyl-isopropylpropan-2-amine (DIPEA) (0.412 g, 3.19 mmol) were dissolved in DCM (15 mL), 2-bromoacetonitrile (760 mg, 6.34 mmol) was added at room temperature, and the mixture was stirred at room temperature for 16 hours. The reaction solution was concentrated and purified by reverse-phase silica gel column chromatography (0.1% aqueous formic acid solution/0.1% formic acid-acetonitrile solution) to obtain cyanomethyl O-(2-chlorophenyl)-N-(((4-(2-(4-fluorophenyl)acetamido)benzyl)oxy)carbonyl)-L-serinate (Compound SS20, F-Pnaz-SPh2Cl-$OCH_2CN$) (220 mg, 26%). The obtained product was dissolved in acetonitrile (5 mL), and used in the next step.

LCMS (ESI) m/z = 562 (M+Na)+

Retention time: 1.15 minutes (analysis condition SMD method 2)

Synthesis of (2R,3S,4R.5R)-2-((((((2R,3S,4K,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-chlorophenyl)-N-(((4-(2-(4-fluorophenyl)acetamido)benzyl)oxy)carbonyl)-L-serinate (Compound SS15, F-Pnaz-SPh2Cl-pCpA)

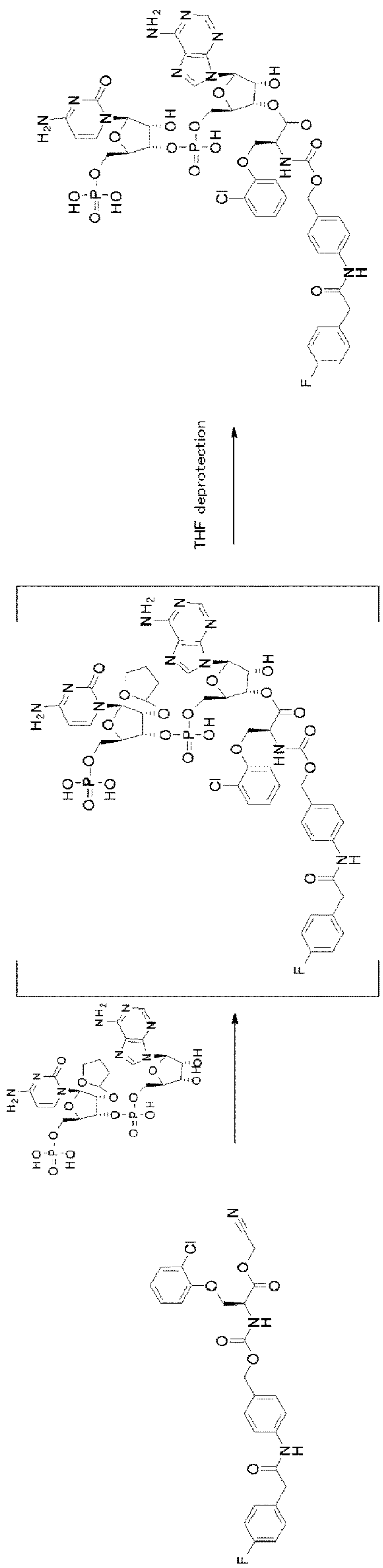


((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) was dissolved in Buffer A (100 mL), a solution of cyanomethyl O-(2-chlorophenyl)-N-(((4-(2-(4-fluorophenyl)acetamido)benzyl)oxy)carbonyl)-L-serinate (Compound SS20, F-Pnaz-SPh2Cl-$OCH_2CN$) (220 mg, 0.41 mmol) in acetonitrile (5 mL) was added to it dropwise over 15 minutes or longer using a syringe pump, and the mixture was stirred at room temperature for 5minutes. Next, trifluoroacetic acid (2.3 mL) was added to the reaction solution. The reaction solution was freeze-dried, and then purified by reverse-phase silica gel column chromatography (0.05% aqueous trifluoroacetic acid solution/ 0.05% trifluoroacetic acid-acetonitrile) to obtain the title compound (Compound SS15, F-Pnaz-SPh2Cl-pCpA) (20.7 mg, 2%).

LCMS (ESI) m/z = 1133.4 (M-H)-

Retention time: 0.55 minutes (analysis condition SQDFA05_01)

Synthesis of ((S)-2-(methylamino)-4-phenylbutanoic acid (Compound SS21, MeHph-OH)

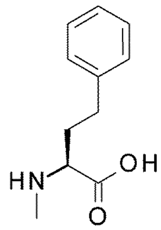

DCM (903 μL), water (903 μL), and piperidine (178 μL, 1.805 mmol) were added to (S)-2-((((9H-fluoren-9-yl)methoxy)carbonyl)(methyl)amino)-4-phenylbutanoic acid (Compound aa11) (150 mg, 0.361 mmol) synthesized by a method described in a patent literature (WO2018225864) at room temperature. The reaction mixture was stirred at room temperature for 30 minutes and then purified by reverse-phase silica gel column chromatography (0.1% aqueous formic acid solution/ 0.1% formic acid-acetonitrile solution) to obtain ((S)-2-(methylamino)-4-phenylbutanoic acid (Compound SS21, MeHph-OH) (55 mg, 79%).

LCMS (ESI) m/z = 192 (M-H)-

Retention time: 0.15 minutes (analysis condition SQDFA05_02)

Synthesis of (S)-2-((((4-(2-(4-fluorophenyl)acetamido)benzyl)oxy)carbonyl)(methyl)amino)-4-phenylbutanoic acid (Compound SS22, F-Pnaz-MeHph-OH)

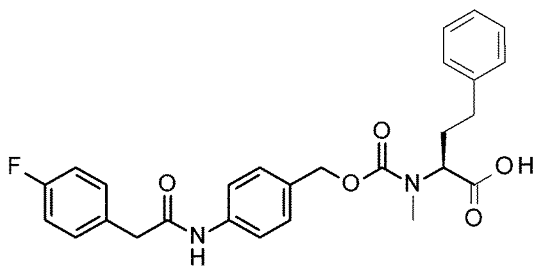

Under nitrogen atmosphere, DMSO (727 μL) was added to a mixture of ((S)-2-(methylamino)-4-phenylbutanoic acid (Compound SS21, MeHph-OH) (35.1 mg, 0.182 mmol) and (4-nitrophenyl)-4-(2-(4-fluorophenyl)acetamido)benzyl carbonate (Compound ts11) (85 mg, 0.20 mmol) synthesized a method described in by a patent literature (WO2018143145A1) at room temperature. Triethylamine (76 μL, 0.545 mmol) was added at 50°C. The reaction mixture was stirred at 40°C for 16 hours, and then purified by reverse-phase silica gel column chromatography (0.1% aqueous formic acid solution/ 0.1% formic acid-acetonitrile solution) to obtain (S)-2-((((4-(2-(4-fluorophenyl)acetamido)benzyl)oxy)carbonyl)(methyl)amino)-4-phenylbutanoic acid (Compound SS22, F-Pnaz-MeHph-OH) (80 mg, 92%).

LCMS (ESI) m/z = 477 (M-H)-

Retention time: 0.85 minutes (analysis condition SQDFA05_02)

Synthesis of cyanomethyl(S)-2-((((4-(2-(4-fluorophenyl)acetamido)benzyl)oxy)carbonyl)(methyl)amino)-4-phenylbutanoate (Compound SS23, F-Pnaz-MeHph-$OCH_2CN$)

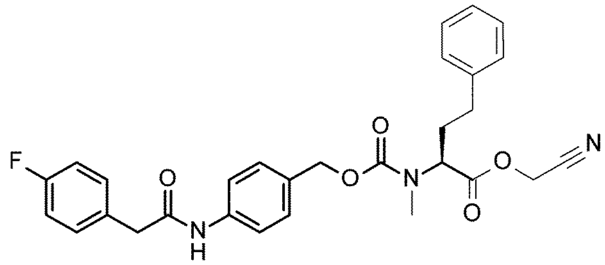

Under nitrogen atmosphere, acetonitrile (533 μL) was added to a mixture of (S)-2-((((4-(2-(4-fluorophenyl)acetamido)benzyl)oxy)carbonyl)(methyl)amino)-4-phenylbutanoic acid (Compound SS22, F-Pnaz-MeHph-OH) (77 mg, 0.16 mmol) and N-ethyl-isopropylpropan-2-amine (DIPEA) (31 μL, 0.176 mmol) at room temperature. Then, 2-bromoacetonitrile (86 μL, 1.280 mmol) was added at room temperature, and the reaction mixture was stirred at 40°C for 1hour. The reaction solution was concentrated to obtain a crude product, cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-$OCH_2CN$). The obtained crude product was dissolved in acetonitrile (5.00 mL) and was directly used in the next step.

LCMS (ESI) m/z = 516 (M-H)-

Retention time: 0.92 minutes (analysis condition SQDFA05_02)

Synthesis of (2R,3S,4R.5R)-2-((((((2R,3S,4K,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-((((4-(2-(4-fluorophenyl)acetamido)benzyl)oxy)carbonyl)(methyl)amino)-4-phenylbutanoate (Compound SS16, F-Pnaz-MeHph-pCpA)

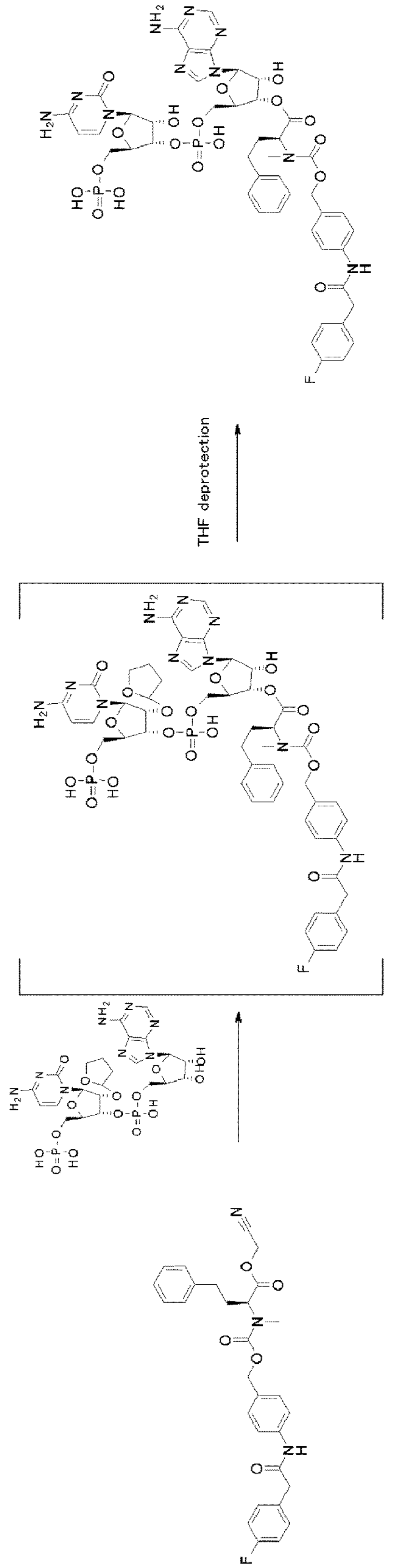


((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)hydroxy)phosphoryl)oxy)-4-((tetrahydrofuran-2-yl)oxy)tetrahydrofuran-2-yl)methyl dihydrogen phosphate (Compound pc01) (127 mg, 0.176 mmol) was dissolved in Buffer A (100 mL), a solution of cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-$OCH_2CN$) (83 mg, 0.16 mmol) in acetonitrile (5.00 mL) was added, and the mixture was stirred at room temperature for 1hour. The reaction solution was cooled to 0°C, and then trifluoroacetic acid (5.00 mL) was added. The reaction solution was stirred at 0°C for one hour, and then purified by reverse-phase silica gel column chromatography (0.05% aqueous trifluoroacetic acid solution/ 0.05% trifluoroacetic acid-acetonitrile), and then further purified by reverse-phase silica gel column chromatography (0.1% aqueous formic acid solution/ 0.1% formic acid acetonitrile solution) to obtain the title compound (Compound SS16, F-Pnaz-MeHph-pCpA) (26 mg, 14.6%).

LCMS (ESI) m/z = 1111.5 (M-H)-

Retention time: 0.64 minutes (analysis condition SQDFA05_02)

Synthesis of N-(((4-(2-(4-fluorophenyl)acetamide)benzyl)oxy)carbonyl)-N-methylglycine (Compound SS46, F-Pnaz-MeG-OH)

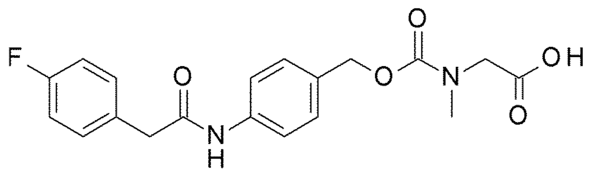

Under a nitrogen atmosphere, DMSO (15 mL) and triethylamine (953.4 mg, 9.42 mmol) were added at room temperature to a mixture of sarcosine (483 mg, 5.42 mmol) and (4-nitrophenyl)-4-(2-(4-fluorophenyl)acetamide)benzyl carbonate (Compound ts11) (2.0 g, 4.71 mmol) synthesized by a method described in a patent literature (WO2018143145A1). The reaction mixture was stirred at room temperature for 16 hours, and then purified by reverse-phase silica gel column chromatography (0.1% aqueous formic acid solution/0.1% formic acid-acetonitrile solution), to obtain N-(((4-(2-(4-fluorophenyl)acetamide)benzyl)oxy)carbonyl)-N-methylglycine (Compound SS46, F-Pnaz-MeG-OH) (1.4 g, 79%).

LCMS (ESI) m/z=397 (M+Na)+

Retention time: 0.88 minutes (analysis condition SMD method 3)

Synthesis of cyanomethyl N-(((4-(2-(4-fluorophenyl)acetamide)benzyl)oxy)carbonyt)-N-methylglycinate (Compound SS47, F-Pnaz-MeG-$OCH_2CN$)

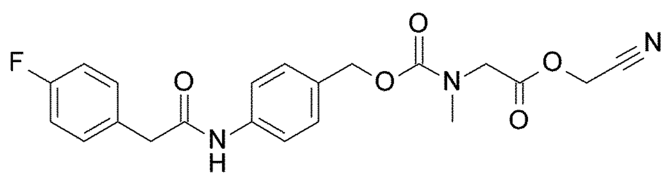

Under a nitrogen atmosphere, N-(((4-(2-(4-fluorophenyl)acetamide)benzyl)oxy)carbonyl)-N-methylglycine (Compound SS46, F-Pnaz-MeG-OH) (1.38 g, 3.69 mmol) and N-ethyl-isopropylpropan-2-amine (DIPEA) (0.95 g, 7.38 mmol) were dissolved in DMF (28 mL), 2-bromoacetonitrile (1.74 g, 14.75 mmol) was added at room temperature, and the mixture was stirred at room temperature for 16 hours. The reaction solution was concentrated, and purified by normal-phase silica gel column chromatography (ethyl acetate/petroleum ether) to obtain cyanomethyl N-(((4-(2-(4-fluorophenyl)acetamide)benzyl)oxy)carbonyl)-N-methylglycinate (Compound SS47, F-Pnaz-MeG-$OCH_2CN$) (1.2 g, 79%).

LCMS (ESI) m/z=436 (M+Na)+

Retention time: 0.70 minutes (analysis condition SMD method 4)

Synthesis of (2R,3S,4R.5R)-2-((((((2R,3S,4K,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(((4-(2-(4-fluorophenyl)acetamide)benzyl)oxy)carbonyl)-N-methylglycinate(Compound SS45, F-Pnaz-MeG-pCpA)

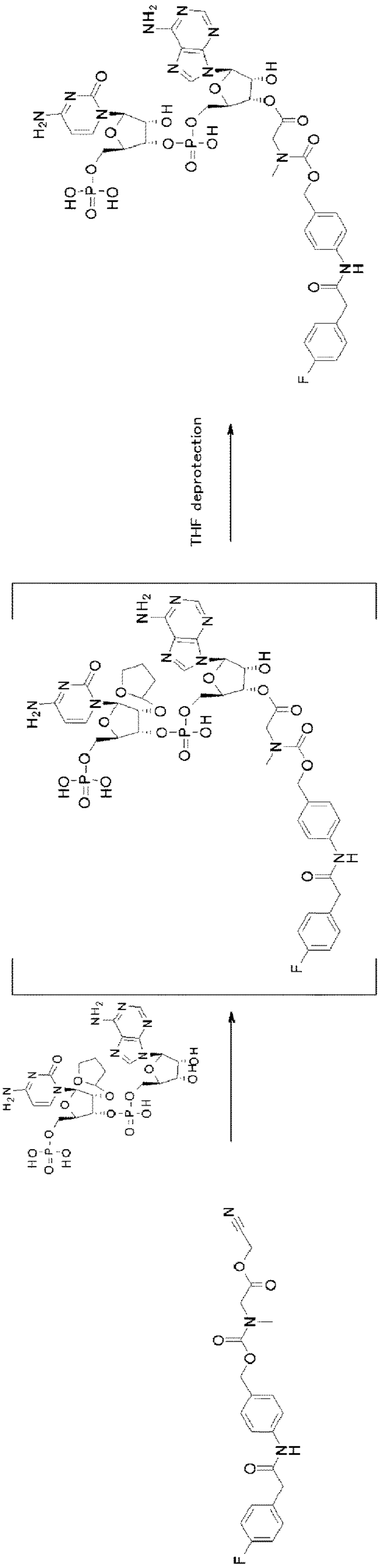


((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogenphosphate (Compound pc01) (422 mg, 0.58 mmol) synthesized by a method described in literature (Helv. Chim. Acta, 90, 297-310) was dissolved in Buffer A (100 mL), a solution of cyanomethyl N-(((4-(2-(4-fluorophenyl)acetamide)benzyl)oxy)carbonyl)-N-methylglycinate (Compound SS47, F-Pnaz-MeG-$OCH_2CN$) (120.7 mg, 0.29 mmol) in acetonitrile (5 mL) was added dropwise over 15 minutes or more using a syringe pump, and the mixture was stirred at room temperature for 5 hours. Trifluoroacetic acid (2.3 mL) was added to the reaction solution, and this reaction solution was freeze-dried and then purified by reverse-phase silica gel column chromatography (0.05% aqueous trifluoroacetic acid solution/0.05% trifluoroacetic acid-acetonitrile) to obtain the title compound (Compound SS45, F-Pnaz-MeG-pCpA) (76.7 mg, 26%).

LCMS (ESI) m/z=1007.5 (M-H)-

Retention time: 0.48 minutes (analysis condition SQDFA05_02)

Example 2. Synthesis of BdpFL-Phe-pCpA(MT01)

Synthesis of (3-(5, 5-difluoro-7,9-dimethyl-5H-4$\lambda^4$,5$\lambda^4$-dipyrrolo[1,2-c:2',1'-f][1,3,2]diazaborinin-3-yl)propanoyl)-L-phenylalanine (Compound MT02 BdpFL-Phe-OH)

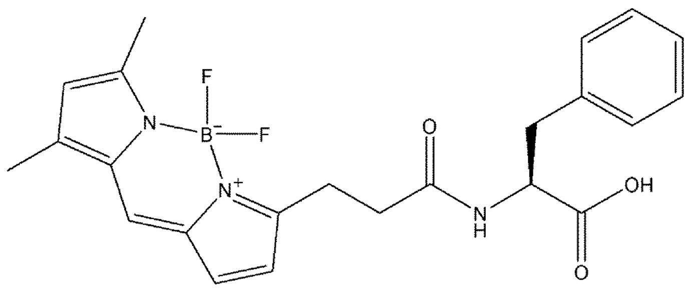

Under nitrogen atmosphere, DIC (0.128 mL, 0.822 mmol) was added to a solution of 3-(2-carboxyethyl)-5,5-difluoro-7,9-dimethyl-5H-5$\lambda^4$-dipyrrolo[1,2-c:2',1'-f][1,3,2]diazaborinin-4-ium (200 mg, 0.685 mmol) and 1-hydroxypyrrolidine-2,5-dione (87 mg, 0.753 mmol) in NMP (4.5 mL) at room temperature, and then the mixture was stirred at 40°C overnight. After returning to room temperature, L-phenylalanine (113 mg, 0.685 mmol) and TEA (0.191 mL, 1.369 mmol) were added to the reaction solution, and stirred at 40°C overnight. The reaction solution was purified by reverse-phase column chromatography (0.1% FA-MeCN/H2O) to obtain (3-(5, 5-difluoro-7,9-dimethyl-5H-4$\lambda^4$,5$\lambda^4$-dipyrrolo[1,2-c:2',1'-f][1,3,2]diazaborinin-3-yl)propanoyl)-L-phenylalanine (Compound MT02, BdpFL-Phe-OH) (102 mg, 34% yield).

LCMS (ESI) m/z = 438.3 (M-H)-

Retention time: 0.78 minutes (analysis condition SQDFA05_02)

Synthesis of (3-(5,5-difluoro-7,9-dimethyl-5H-4$\lambda^4$,5$\lambda^4$-dipyrrolo[1,2-c:2',1'-f][1,3,2]diazaborinin-3-yl)propanoyl)-L-phenylalanine cyanomethyl ester (Compound MT03,

BdpFL-Phe-$OCH_2CN$)

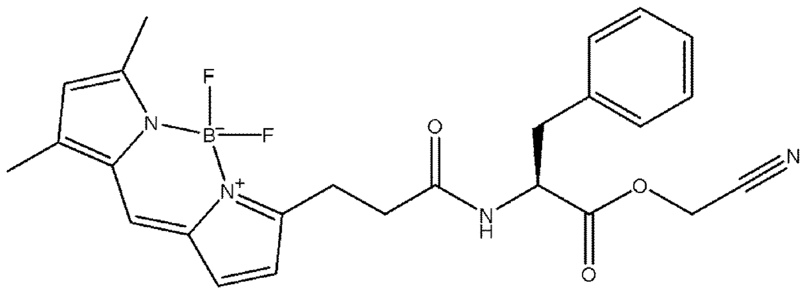

Under nitrogen atmosphere, (3-(5,5-difluoro-7,9-dimethyl-5H-4λ$^4$,5λ$^4$-dipyrrolo[1,2-c:2',1'-f][1,3,2]diazaborinin-3-yl)propanoyl)-L-phenylalanine (50 mg, 0.114 mmol) and N-ethyl-isopropylpropan-2-amine (DIPEA) (31.0 μL, 0.177 mmol) were dissolved in acetonitrile (500 μL), 2-bromoacetonitrile (12 μL, 0.177 mmol) was added at 0°C, and then the mixture was stirred at 40°C for 3hours. The reaction solution was concentrated to obtain (3-(5,5-difluoro-7,9-dimethyl-5H-4λ$^4$,5λ$^4$-dipyrrolo[1,2-c:2',1'-f][1,3,2]diazaborinin-3-yl)propanoyl)-L-phenylalanine cyanomethyl ester (Compound MT03, BdpFL-Phe-$OCH_2CN$) as a crude product. The obtained crude product was directly used in the next step.

LCMS (ESI) m/z = 477.3 (M-H)-

Retention time: 0.86 minutes (analysis condition SQDFA05_01)

Synthesis of 3-(3-(((2S)-1-(((2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl)oxy)-1-oxo-3-phenylpropan-2-yl)amino)-3-oxopropyl)-5.5-difluoro-7,9-dimethyl-5H-5λ$^4$-dipyrrolo [1,2-c:2',1'-f1[1,3,2]diazaborinin-4-ium (Compound MT01, BdpFL-Phe-pCpA)

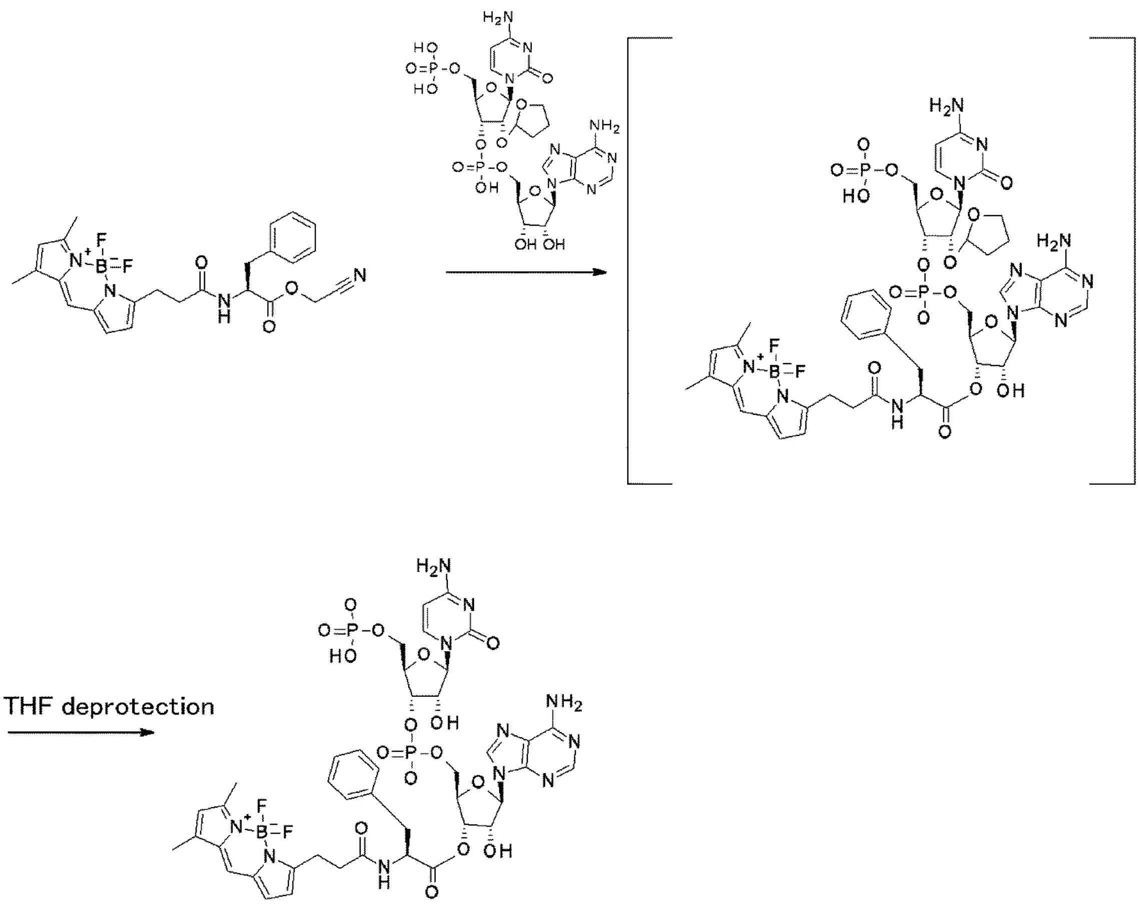


((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (33.2 mg, 0.046 mmol) was dissolved in Buffer A (11.3 mL), a solution of (3-(5,5-difluoro-7,9-dimethyl-5H-4$\lambda^4$,$\lambda^4$-dipyrrolo[1,2-c:2',1'-f][1,3,2]diazaborinin-3-yl)propanoyl)-L-phenylalanine cyanomethyl ester (Compound MT03, BdpFL-Phe-$OCH_2CN$) (11 mg, 0.023 mmol) in acetonitrile (0.13 mL) was added, and then the mixture was stirred at room temperature for 45 minutes. TFA (0.56 mL) was added to the reaction solution at 0°C and stirred for 5minutes, and then stirred at room temperature for 10 minutes. The reaction solution was purified by reverse-phase silica gel column chromatography (0.05% TFA-MeCN/H2O) to obtain the title compound (Compound MT01, BdpFL-Phe-pCpA) (2.1 mg, 8.5% yield).

LCMS (ESI) m/z = 1072.5 (M-H)-

Retention time: 0.56 minutes (analysis condition SQDFA05_02)

Synthesis of lysidine-diphosphate for introducing a lysidine unit at the 3' end of a tRNA fragment by a ligation method - an alternative method

The method for synthesizing the diphosphate of lysidine used for introducing a lysidine unit at the 3' end of a tRNA fragment by a ligation method was improved. More specifically, lysidine-diphosphate (SS04, pLp) was synthesized according to the following scheme.

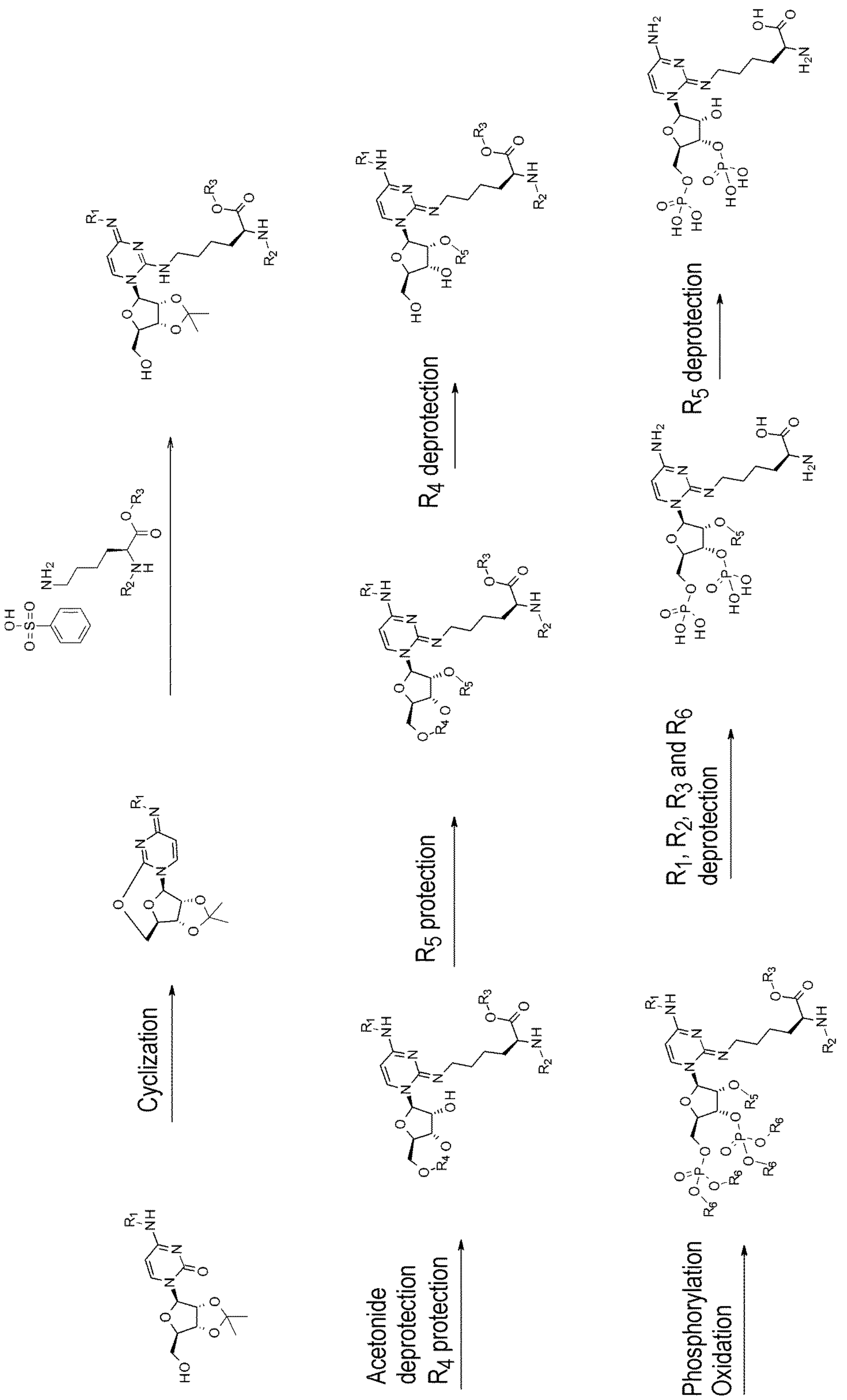


Synthesis of benzyl ((3aR,4R,12R,12aR)-2,2-dimethyl-3a,4,12,12a-tetrahydro-5H,8H-4,12-epoxy[1.3]dioxolo[4.5-e]pyrimido[2.1-b][1.3]oxazocin-8-ylidene)carbamate(Compound SS24)

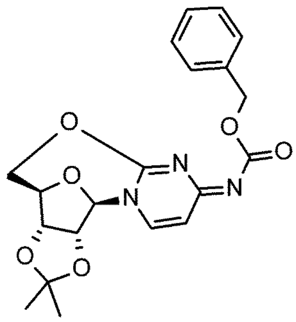

Under nitrogen atmosphere, DCM (17.2 mL) was added to a mixture of 2',3'-O-isopropylidene-4-N-(benzyloxy-carbonyl)-cytidine (718.2 mg, 1.72 mmol), which is a literature (Antiviral Chemistry & Chemotherapy, 2003, 14(4), 183-194)-known compound, and triphenylphosphine (474 mg, 1.81 mmol), at room temperature. After cooling the mixture in an ice bath, diisopropyl azodicarbonate (385 μL, 1.98 mmol) was added, then the mixture was warmed to room temperature, and stirred at room temperature for 1.5 hours. The reaction solution was concentrated, toluene (20 mL) was added, and then the produced precipitates were recovered by filtration. The obtained solid was washed three times using toluene to obtain benzyl ((3aR,4R,12R,12aR)-2,2-dimethyl-3a,4,12,12a-tetrahydro-5H,8H-4,12-epoxy[1,3]dioxolo[4,5-e]pyrimido[2,1-b][1,3]oxazocin-8-ylidene)carbamate (Compound SS24) (525.7 mg, 76%).

LCMS (ESI) m/z = 400.3 (M+H)+

Retention time: 0.48 minutes (analysis condition SQDFA05_02)

Synthesis of benzyl (2S)-6-[[1-[(3aR,4K,6K,6aR)-6-(hydroxymethyl)-22-dimethyl-3a,4,6,6a-tetrahydrofuror[3,4-d][1,3]dioxol-4-yl]-4-(benzyloxycarbonylamino)pyrimidin-2-ylidene]amino]-2-(benzyloxycarbonylamino)hexanoate; 2,2,2-trifluoroacetic acid (Compound SS25)

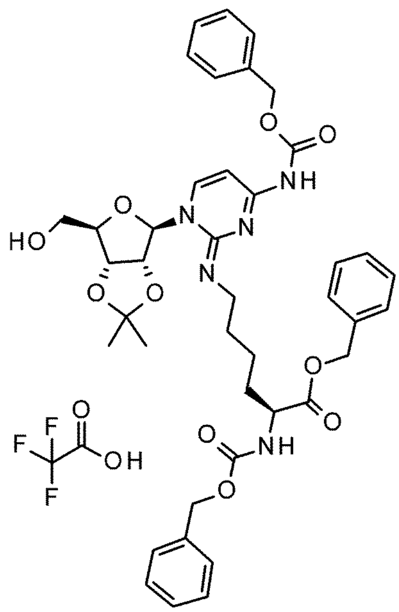

Under nitrogen atmosphere, THF (7.5 mL) was added to a mixture of benzyl ((3aR,4R,12R,12aR)-2,2-dimethyl-3a,4,12,12a-tetrahydro-5H,8H-4,12-epoxy[1,3]dioxolo[4,5-e]pyrimido[2,1-b][1,3]oxazocin-8-ylidene)carbamate (Compound SS24) (300 mg, 0.75 mmol) and lithium chloride (159 mg, 3.76 mmol), at room temperature, and the mixture was cooled in an ice bath. To this mixture, a mixture of benzyl ((benzyloxy)carbonyl)-L-lysinate benzenesulfonate (813 mg, 2.01 mmol) and DBU (673 μL, 4.51 mmol)added with THF (7.5 mL) was added in an ice bath, and the reaction mixture was stirred at 0°C for 30 minutes. DMSO was added to the reaction solution in an ice bath, the mixture was warmed to

room temperature, and then the reaction solution was concentrated to remove THF. The residue was purified by reverse-phase silica gel column chromatography (0.05% aqueous TFA solution/0.05% TFA-acetonitrile solution) to obtain benzyl (2S)-6-[[1-[(3aR,4R,6R,6aR)-6-(hydroxymethyl)-2,2-dimethyl-3a,4,6,6a-tetrahydrofuro[3,4-d][1,3]dioxol-4-yl]-4-(benzyloxycarbonylamino)pyrimidin-2-ylidene]amino]-2-(benzyloxycarbonylamino)hexanoate; 2,2,2-trifluoroacetic acid (Compound SS25) (726.6 mg) quantitatively.

LCMS (ESI) m/z = 768.6 (M-H)-

Retention time: 0.74 minutes (analysis condition SQDFA05_02)

Synthesis of benzyl (2S)-2-(benzyloxycarbonylamino)-6-[[4-(benzyloxyarbonylamino)-1-[(2R,3R,4S,5R)-3,4-dihydroxy-5-(hydroxymethyl)tetrahydrofuran-2-yl]pyrimidin-2-ylidene]amino]hexanoate: 2,2,2-trifluoroacetic acid (Compound SS26)

Benzyl (2S)-6-[[1-[(3aR,4R,6R,6aR)-6-(hydroxymethyl)-2,2-dimethyl-3a,4,6,6a-tetrahydrofuro[3,4-d][1,3]dioxol-4-yl]-4-(benzyloxycarbonylamino)pyrimidin-2-ylidene]amino]-2-(benzyloxycarbonylamino)hexanoate; 2,2,2-trifluoroacetic acid (Compound SS25) (281.1 mg, 0.318 mmol) was dissolved in a mixed solvent of TFA (4.24 mL) and ultrapure water (2.12 mL) while cooling in an ice bath, and the mixture was stirred at room temperature for 50 minutes. Toluene and acetonitrile were added, and the reaction solution was concentrated. This operation was repeated several times to distill off water and TFA, and benzyl (2S)-2-(benzyloxycarbonylamino)-6-[[4-(benzyloxycarbonylamino)-1-[(2R,3R,4S,5R)-3,4-dihydroxy-5-(hydroxymethyl)tetrahydrofuran-2-yl]pyrimidin-2-ylidene]amino] hexanoate; 2,2,2-trifluoroacetic acid (Compound SS26) (272.6 mg) was obtained as a crude product. The obtained crude product, benzyl (2S)-2-(benzyloxycarbonylamino)-6-[[4-(benzyloxycarbonylamino)-1-[(2R,3R,4S,5R)-3,4-dihydroxy-5-(hydroxymethyl)tetrahydrofuran-2-yl]pyrimidin-2-ylidene]amino]hexanoate; 2,2,2-trifluoroacetic acid (Compound SS26), was used directly in the next step.

LCMS (ESI) m/z=728.5 (M-H)-

Retention time: 0.69 minutes (analysis condition SQDFA05_02)

Synthesis of benzyl (2S)-6-[[1-(4aR,6R,7R,7aS)-2,2-ditert-butyl-7-hydroxy-4a,6,7,7a-tetrahydro-4H-furo[3,2-d][1,3,2]dioxasilin-6-yl]-4-(benzyloxycarbonylamino)pyrimidin-2-ylidene]amino]-2-(benzyloxycarbonylamino)hexanoate; 2,2,2-trifluoroacetic acid (Compound SS27)

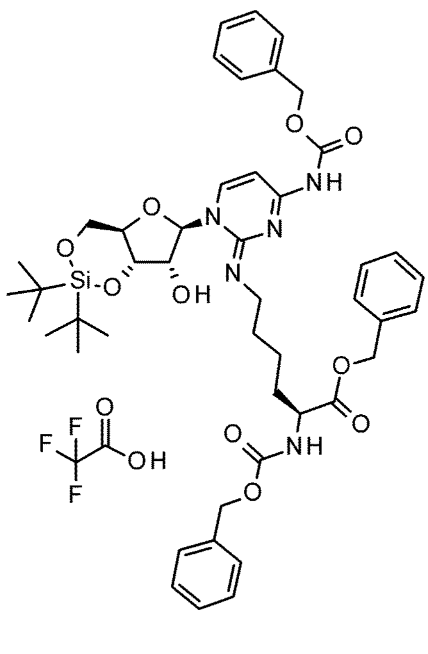

Under nitrogen atmosphere, the crude product obtained in the previous step, benzyl (2S)-2-(benzyloxycarbonylamino)-6-[[4-(benzyloxyarbonylamino)-1-[(2R,3R,4S,5R)-3,4-dihydroxy-5-(hydroxymethyl)tetrahydrofuran-2-yl]pyrimidin-2-ylidene]amino]hexanoate; 2,2,2-trifluoroacetic acid (Compound SS26) (258 mg, 0.306 mmol), was dissolved in DMF (3.06 mL). After the mixture was cooled in an ice bath, di-tert-butylsilyl bis(trifluoromethanesulfonate) (396 μL, 1.22 mmol) was added, and the mixture was stirred in an ice bath for 2hours. In an ice bath, saturated aqueous sodium bicarbonate solution was added to the reaction solution, and the obtained mixture was purified by reverse-phase silica gel column chromatography (0.05% aqueous TFA solution/0.05% TFA-acetonitrile solution) to obtain benzyl (2S)-6-[[1-[(4aR,6R,7R,7aS)-2,2-ditert-butyl-7-hydroxy-4a,6,7,7a-tetrahydro-4H-furo[3,2-d][1,3,2]dioxasilin-6-yl]-4-(benzyloxycarbonylamino)pyrimidin-2-ylidene]amino]-2-(benzyloxycarbonylamino)hexanoate; 2,2,2-trifluoroacetic acid (Compound SS27) (234.0 mg, 78%, two steps).

LCMS (ESI) m/z = 868.8 (M-H)-

Retention time: 0.88 minutes (analysis condition SQDFA05_02)

Synthesis of benzyl (2S)-6-[[1-[(4aR,6R,7R,7aR)-2,2-ditert-buiyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate, 2,2,2-trifluoroacetic acid (Compound SS28)

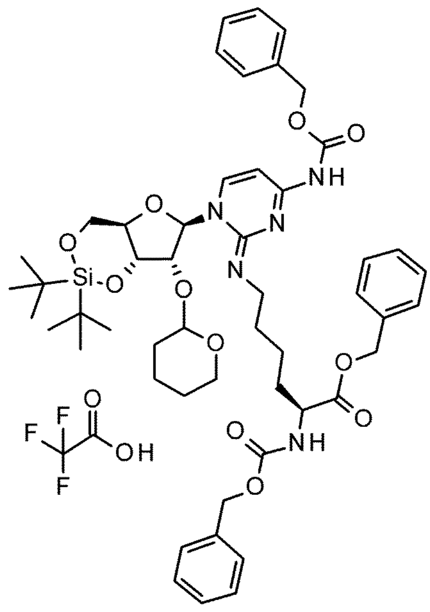

Under nitrogen atmosphere, benzyl (2S)-6-[[1-[(4aR,6R,7R,7aS)-2,2-ditert-butyl-7-hydroxy-4a,6,7,7a-tetrahydro-4H-furo[3,2-d][1,3,2]dioxasilin-6-yl]-4-(benzyloxycarbonylamino)pyrimidin-2-ylidene]amino]-2-(benzyloxycarbonylamino)hexanoate; 2,2,2-trifluoroacetic acid (Compound SS27) (30 mg, 0.03 mmol) and TFA (6.98 μL, 0.09 mmol) were dissolved in DCM (610 μL) at room temperature, and 3,4-dihydro-2H-pyran (83 μL, 0.915 mmol) was added. After stirring the reaction mixture at room temperature for 13 hours, toluene was added, and the reaction solution was concentrated to obtain a crude product, benzyl (2S)-6-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28), as a mixture of diastereomers derived from the asymmetric carbon on the THP protecting group. The obtained crude product, benzyl (2S)-6-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28), was directly used in the next step.

LCMS (ESI) m/z = 952.8 (M-H)-

Retention time: 3.17 minutes, 3.38 minutes (analysis condition SQDAA05long)

Synthesis of benzyl (2S)-2-(benzyloxycarbonylamino)-6-[[4-(benzyloxycarbonylamino)-1 - [(2R,3R,4R,5R)-4-hydroxy-5-(hydroxymethyl)-3-tetrahydropyran-2-yloxy-tetrahydrofuran-2-yl]pyrimidin-2-ylidene]amino]hexanoate (Compound SS29)

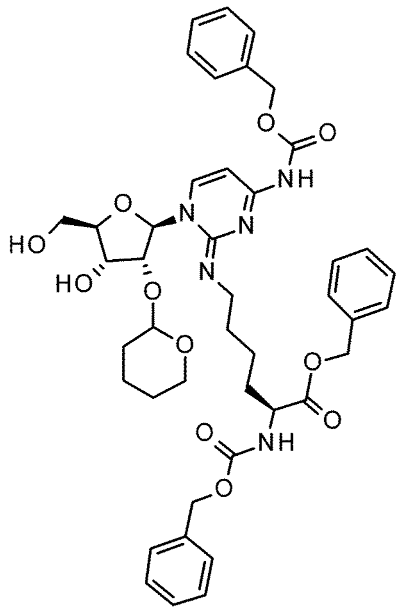

Under nitrogen atmosphere, the crude product obtained in the previous step, benzyl (2S)-6-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28), was dissolved in THF (610 μL) at room temperature, then tetrabutylammonium fluoride (tetrahydrofuran solution of approximately 1 mol/L) (305 μL, approximately 0.305 mmol) was added at room temperature, and the reaction mixture was stirred at room temperature for 30 minutes. The reaction solution was added with DMSO, and then concentrated to distill off THF. The residue was purified by reverse-phase silica gel column chromatography (10 mM aqueous AA solution/ 10 mM AA-acetonitrile solution) to obtain benzyl (2S)-2-(benzyloxycarbonylamino)-6-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]hexanoate (Compound SS29) (21.51 mg, 87%, two steps) as a mixture of diastereomers derived from the asymmetric carbon on the THP protecting group.

LCMS (ESI) m/z = 812.7 (M-H)-

Retention time: 1.74 minutes (analysis condition SQDAA05long)

Synthesis of benzyl (2S)-2-(benzyloxycarbonylamino)-6-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl1pyrimidin-2-ylidene]amino]hexanoate (Compound SS30)

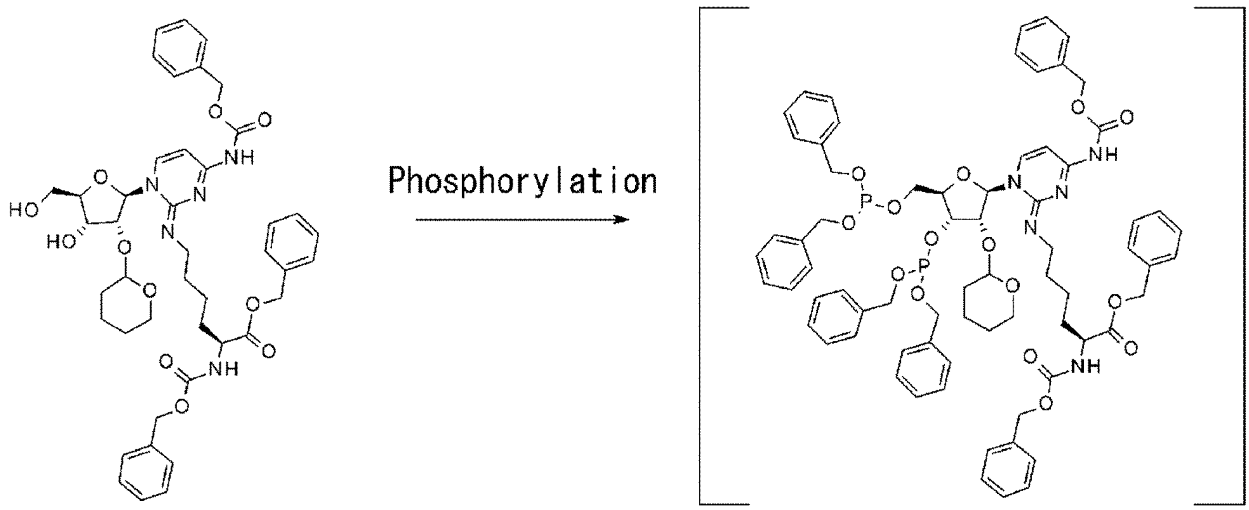


Compound SS29

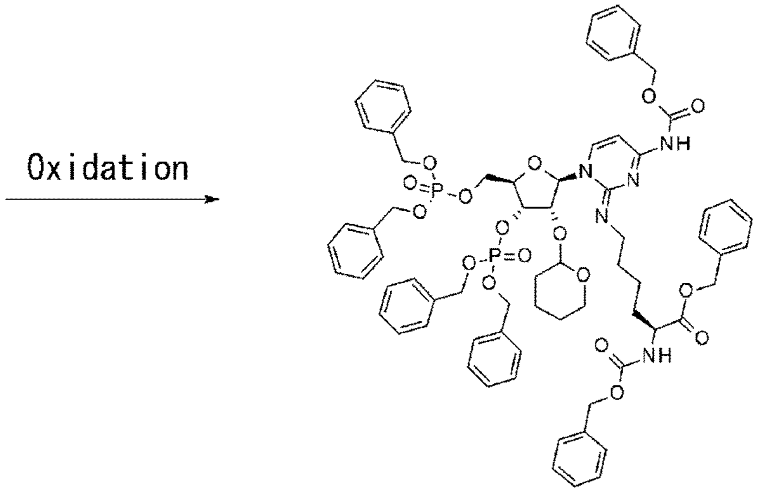


Compound SS30

Under nitrogen atmosphere, benzyl (2S)-2-(benzyloxycarbonylamino)-6-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]hexanoate (Compound SS29) (21.51 mg, 0.026 mmol) and 1H-tetrazole (22.22 mg, 0.317 mmol) were dissolved in acetonitrile (1.06 mL) at room temperature, dibenzyl N,N-diisopropylphosphoroamidite (53.2 μL, 0.159 mmol) was added, and the mixture was stirred at room temperature for 1hour. The mixture was added with Dess-Martin Periodinane (135 mg, 0.317 mmol) and stirred at room temperature for 15 minutes, then the reaction solution was purified by reverse-phase silica gel column chromatography (10 mM aqueous AA solution /10 mM AA solution in acetonitrile) to obtain benzyl (2S)-2-(benzyloxycarbonylamino)-6-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]hexanoate (Compound SS30) (36.59 mg, two steps) quantitatively, as a mixture of diastereomers derived from the asymmetric carbon on the THP protecting group.

LCMS (ESI) m/z = 1332.8 (M-H)-

Retention time: 3.08 minutes, 3.11 minutes (analysis condition SQDAA05long)

Synthesis of (2S)-2-amino-6-[[4-amino-1-[(2R,3R,4S,5R)-3-hydroxy-4-phosphonooxy-5-(phosphonooxymethyl)tetrahydrofuran-2-yl]pyrimidin-2-ylidene]amino]hexanoic acid (Compound SS04, pLp)

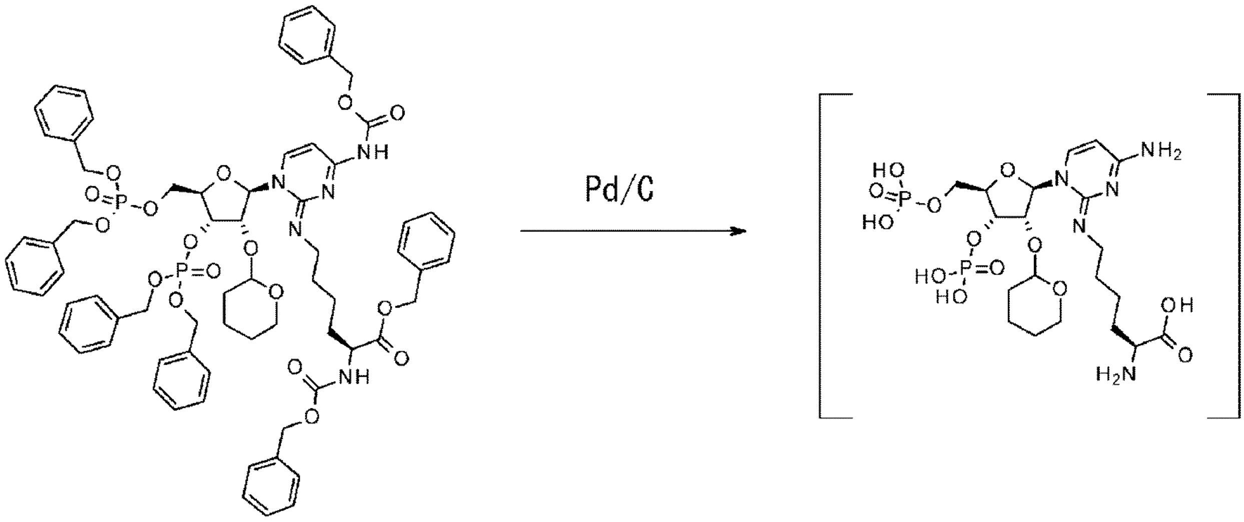


**Compound SS30**

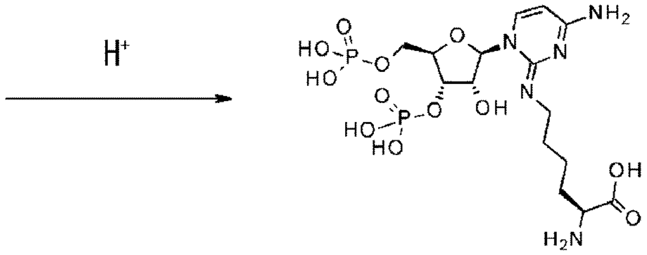


**Compound SS04**

Benzyl (2S)-2-(benzyloxycarbonylamino)-6-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]hexanoate (Compound SS30) (36.59 mg, 0.027 mmol) was dissolved in a mixed solvent of methanol (649 μL) and ultrapure water (152 μL) at room temperature, and palladium on carbon (10% Pd) (5.84 mg, 5.48 μmol) was added under nitrogen atmosphere. Under hydrogen atmosphere, this mixture was stirred at room temperature for 18 hours. The reaction solution was filtered through Celite, and washed several times using ultrapure water. To the obtained filtrate (24.66 mL), 1 mol/L hydrogen chloride (2.74 mL, 2.74 mmol) was added, and was left to stand at room temperature for one hour. The reaction solution was filtered through Celite, and washed several times using ultrapure water. After freeze-drying the filtrate, the obtained powder was redissolved using ultrapure water (1.52 mL), and then centrifugation was performed and the supernatant was recovered to obtain an aqueous solution of (2S)-2-amino-6-[[4-amino-1-[(2R,3R,4S,5R)-3-hydroxy-4-phosphonooxy-5-(phosphonooxymethyl)tetrahydrofuran-2-yl]pyrimidin-2-ylidene]amino]hexanoic acid (Compound SS04, pLp) (1.37mL, 17.47 mM, 87%, two steps).

LCMS (ESI) m/z = 530.1 (M-H)-

Retention time: 1.60 minutes (analysis condition LTQTEA/HFIP05_02)

Column exchange was performed during the time after analyzing Compound SS04 synthesized in Example 2 and before analyzing Compound SS04 synthesized in Example 3. Compound SS04 synthesized in Example 2 was analyzed again after column exchange, and was confirmed to be the same as Compound SS04 synthesized in Example 3. The results are shown below.

LCMS (ESI) m/z = 530.1 (M-H)-

Retention time: 1.60 minutes (analysis condition LTQTEA/HFIP05_02)

Example 3

Example 3-1. Synthesis of a peptide bearing BdpFL at its N terminus (LCT-67)

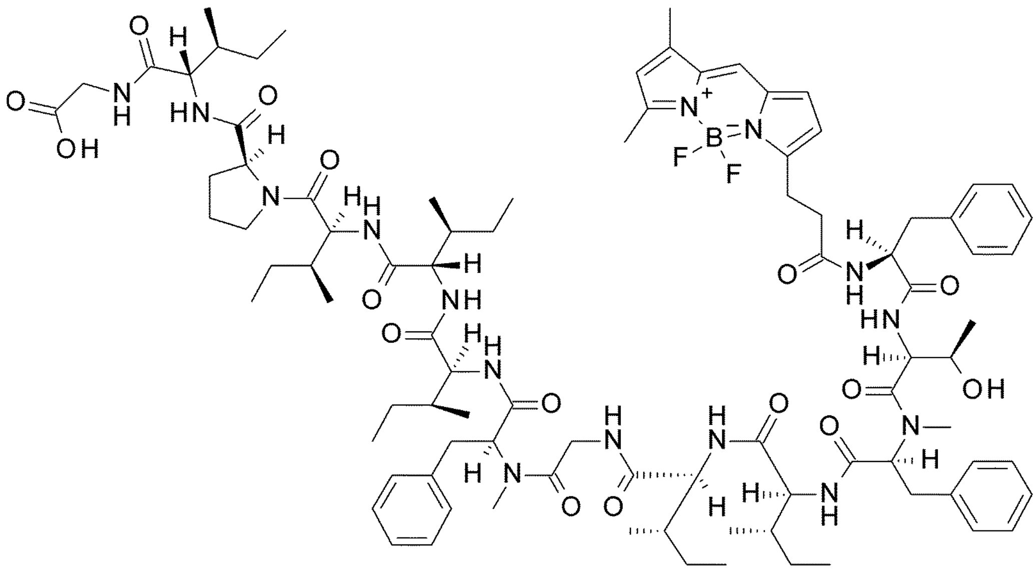

Using 2-chlorotrityl resin bearing Fmoc-Gly-OH (100 mg), and using Fmoc-Gly-OH, Fmoc-Thr(THP)-OH (aa01) synthesized by a method described in patent literature (WO2018225864), Fmoc-Ile-OH, Fmoc-Leu-OH, Fmoc-Phe-OH, Fmoc-MePhe-OH, and Fmoc-Pro-OH as Fmoc amino acids, peptide elongation was performed on a peptide synthesizer (abbreviations of amino acids are described separately in this specification). Peptide elongation was performed according to a peptide synthesis method using the Fmoc method (WO2013100132B2). After the peptide elongation, N-terminal Fmoc group was removed on the peptide synthesizer, and then the resin was washed with DCM.

TFE/DCM (1:1, v/v, 2 mL) was added to the resin, this was shaken for 1hour, and the peptides were cleaved off from the resin. After completion of the reaction, the resin was removed by filtering the solution inside the tube through a synthesis column, and the resin was washed twice with TFE/DCM (1:1, v/v, 1 mL). All of the extracts were mixed, DMF (2 mL) was added, and then the mixture was concentrated under reduced pressure. The obtained residue was dissolved in NMP (1 mL), 4,4-difluoro-5,7-dimethyl-4-bora-3a,4a-diaza-s-indacene-3-propionic acid N-succinimidyl ester (5 mg, 0.013 mmol) was added at room temperature, the mixture was stirred for 19 hours, and then the reaction solution was subjected to reverse-phase silica gel column chromatography (0.1% FA MeCN/$H_2O$), and the fraction containing the intermediate was concentrated under reduced pressure. The obtained residue was dissolved in 5% TFA in DCM (2 mL) and stirred at room temperature for 2hours. The reaction solution was concentrated under reduced pressure, and then the obtained residue was purified by reverse-phase silica gel column chromatography (0.1% FA MeCN/$H_2O$) to obtain the title compound (LCT-67) (13 mg). The amino acid sequence of LCT-67 is shown in SEQ ID NO: 237.

LCMS (ESI) m/z = 1751.2 (M-H)-

Retention time: 0.97 minutes (analysis condition SQDFA05_02)

Example 3-2. Synthesis of a peptide bearing BdpFL at its N-terminus (LCT-12)

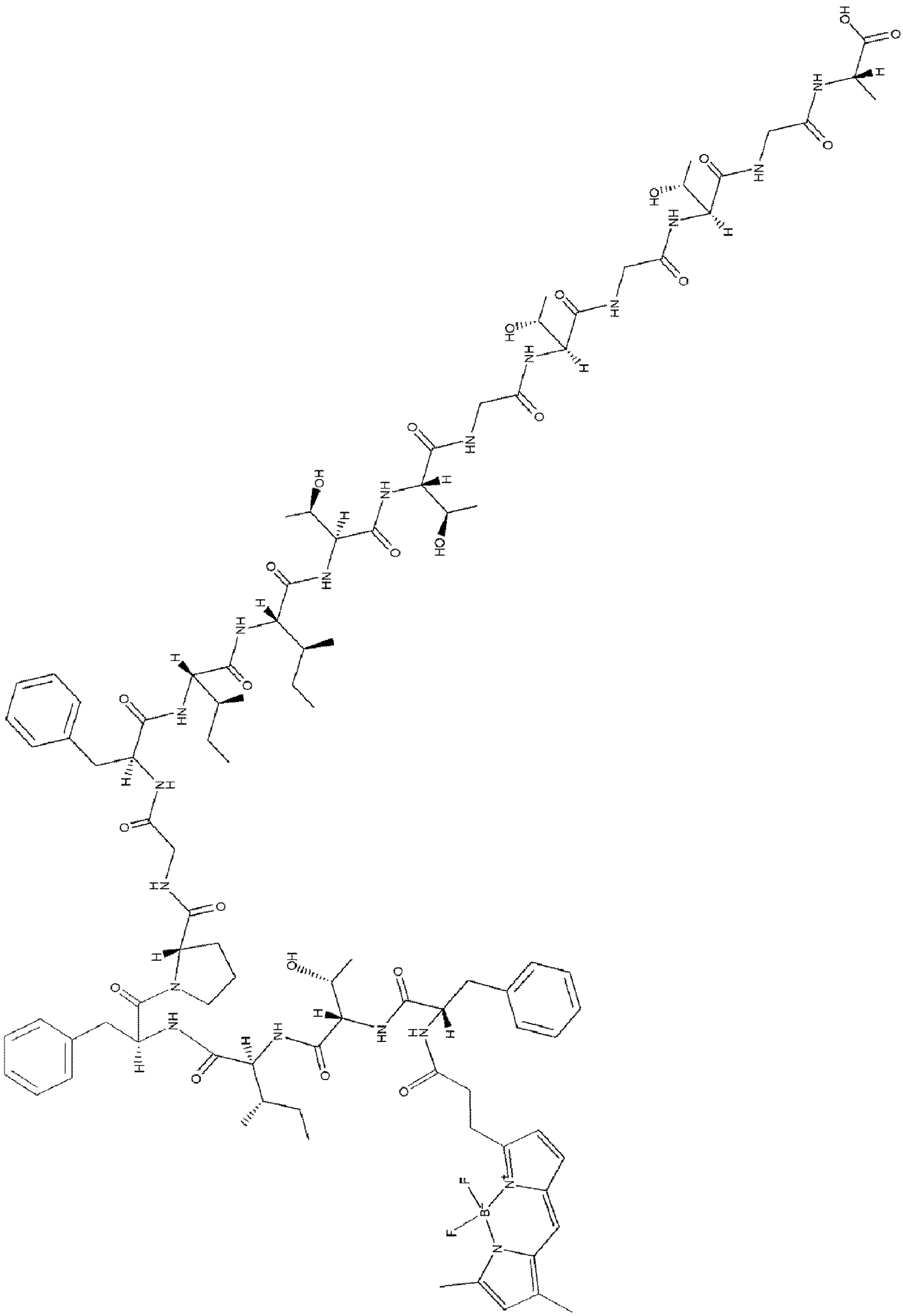

Using 2-chlorotrityl resin bearing Fmoc-Ala-OH (100 mg), and using Fmoc-Gly-OH, Fmoc-Thr(THP)-OH (aa01) synthesized by a method described in patent literature (WO2018225864), Fmoc-Ile-OH, Fmoc-Phe-OH, and Fmoc-Pro-OH as Fmoc amino acids, peptide elongation was performed on a peptide synthesizer (abbreviations of amino acids are described separately in this specification). Peptide elongation was performed according to a peptide synthesis method using the Fmoc method (WO2013100132B2). After the peptide elongation, removal of the N-terminal Fmoc group was performed on the peptide synthesizer, and then the resin was washed with DCM.

TFE/DCM (1:1, v/v, 2 mL) was added to the resin, this was shaken for 1hour, and the peptides were cleaved off from the resin. After completion of the reaction, the resin was removed by filtering the solution inside the tube through a synthesis column, and the resin was washed twice with TFE/DCM (1:1, v/v, 1 mL). All of the extract solutions were mixed, DMF (2 mL) was added, and then the mixture was concentrated under reduced pressure. The obtained residue was dissolved in NMP (0.5 mL), and one-fourth (125 μL) of it was used in the next reaction. To the peptide solution in NMP, 4,4-difluoro-5,7-dimethyl-4-bora-3a,4a-diaza-s-indacene-3-propionic acid N-succinimide ester (140 μL) adjusted to 76.5 mM was added at room temperature, the mixture was stirred overnight at 40°C, and then concentrated under reduced pressure. The obtained residue was dissolved in 0.05 M tetramethylammonium hydrogen sulfate in HFIP (1.2 mL, 0.060 mmol) and stirred at room temperature for 2hours. The reaction solution was purified by reverse-phase silica gel column chromatography (0.1% FA MeCN/$H_2O$) to obtain the title compound (LCT-12) (0.3 mg). The amino acid sequence of LCT-12 is shown in SEQ ID NO: 238.

LCMS (ESI) m/z = 1972.9 (M-H)-

Retention time: 0.74 minutes (analysis condition SQDFA05_01)

Example 4 Synthesis of aminoacyl tRNAs

Example 4-1. Preparation of tRNAs

From template DNAs (TD-1 to TD-107), tRNAs (TR-1 to TR-107) were synthesized by *in vitro* transcription reaction using T7 RNA polymerase, and were purified by RNeasy kit (Qiagen).

Template DNA SEQ ID NO: 1 (TD-1)
DNA sequence:

GGCGTAATACGACTCACTATAGTCCCCTTCGTCTAGAGGCCCAGGACACCGCCCTaag
AAGGCGGTAACAGGGGTTCGAATCCCCTAGGGGACGC

Template DNA SEQ ID NO: 2 (TD-2)
DNA sequence:

GGCGTAATACGACTCACTATAGTCCCCTTCGTCTAGAGGCCCAGGACACCGCCTTaag
ACGGCGGTAACAGGGGTTCGAATCCCCTAGGGGACGC

Template DNA SEQ ID NO: 3 (TD-3)
DNA sequence:

GGCGTAATACGACTCACTATAGTCCCCTTCGTCTAGAGGCCCAGGACACCGCCTTaag
AAGGCGGTAACAGGGGTTCGAATCCCCTAGGGGACGC

Template DNA SEQ ID NO: 4 (TD-4)
DNA sequence:

GGCGTAATACGACTCACTATAGTCCCCTTCGTCTAGAGGCCCAGGACACCGCCCTaag
ACGGCGGTAACAGGGGTTCGAATCCCCTAGGGGACGC

Template DNA SEQ ID NO: 5 (TD-5)
DNA sequence:

GGCGTAATACGACTCACTATAGTCCCCTTCGTCTAGAGGCCCAGGACACCGCCCTaag GAGGCGGTAACAGGGGTTCGAATCCCCTAGGGGACGC

Template DNA SEQ ID NO: 6 (TD-6)
DNA sequence:

GGCGTAATACGACTCACTATAGTCCCCTTCGTCTAGAGGCCCAGGACACCGCCTTaag ATGGCGGTAACAGGGGTTCGAATCCCCTAGGGGACGC

Template DNA SEQ ID NO: 7 (TD-7)
DNA sequence:

GGCGTAATACGACTCACTATAGTCCCCTTCGTCTAGAGGCCCAGGACACCGCCTTaag GTGGCGGTAACAGGGGTTCGAATCCCCTAGGGGACGC

Template DNA SEQ ID NO: 8 (TD-8)
DNA sequence:

GGCGTAATACGACTCACTATAGTCCCCTTCGTCTAGAGGCCCAGGACACCGCCTTaag GAGGCGGTAACAGGGGTTCGAATCCCCTAGGGGACGC

Template DNA SEQ ID NO: 9 (TD-9)
DNA sequence:

GGCGTAATACGACTCACTATAGTCCCCTTCGTCTAGAGGCCCAGGACACCGCCATaag GTGGCGGTAACAGGGGTTCGAATCCCCTAGGGGACGC

Template DNA SEQ ID NO: 10 (TD-10)
DNA sequence:

GGCGTAATACGACTCACTATAGTCCCCTTCGTCTAGAGGCCCAGGACACCGCCATaag ATGGCGGTAACAGGGGTTCGAATCCCCTAGGGGACGC

Template DNA SEQ ID NO: 11 (TD-11)
DNA sequence:

GGCGTAATACGACTCACTATAGTCCCCTTCGTCTAGAGGCCCAGGACACCGCCCTcag AAGGCGGTAACAGGGGTTCGAATCCCCTAGGGGACGC

Template DNA SEQ ID NO: 12 (TD-12)
DNA sequence:

GGCGTAATACGACTCACTATAGTCCCCTTCGTCTAGAGGCCCAGGACACCGCCTTcag ACGGCGGTAACAGGGGTTCGAATCCCCTAGGGGACGC

Template DNA SEQ ID NO: 13 (TD-13)
DNA sequence:

GGCGTAATACGACTCACTATAGTCCCCTTCGTCTAGAGGCCCAGGACACCGCCTTcag AAGGCGGTAACAGGGGTTCGAATCCCCTAGGGGACGC

Template DNA SEQ ID NO: 14 (TD-14)
DNA sequence:

GGCGTAATACGACTCACTATAGTCCCCTTCGTCTAGAGGCCCAGGACACCGCCCTcag ACGGCGGTAACAGGGGTTCGAATCCCCTAGGGGACGC

Template DNA SEQ ID NO: 15 (TD-15)
DNA sequence:

GGCGTAATACGACTCACTATAGTCCCCTTCGTCTAGAGGCCCAGGACACCGCCCTcag GAGGCGGTAACAGGGGTTCGAATCCCCTAGGGGACGC

Template DNA SEQ ID NO: 16 (TD-16)
DNA sequence:

GGCGTAATACGACTCACTATAGTCCCCTTCGTCTAGAGGCCCAGGACACCGCCTTcag ATGGCGGTAACAGGGGTTCGAATCCCCTAGGGGACGC

Template DNA SEQ ID NO: 17 (TD-17)
DNA sequence:

GGCGTAATACGACTCACTATAGTCCCCTTCGTCTAGAGGCCCAGGACACCGCCTTcag GTGGCGGTAACAGGGGTTCGAATCCCCTAGGGGACGC

Template DNA SEQ ID NO: 18 (TD-18)
DNA sequence:

GGCGTAATACGACTCACTATAGTCCCCTTCGTCTAGAGGCCCAGGACACCGCCTTcag GAGGCGGTAACAGGGGTTCGAATCCCCTAGGGGACGC

Template DNA SEQ ID NO: 19 (TD-19)
DNA sequence:

GGCGTAATACGACTCACTATAGTCCCCTTCGTCTAGAGGCCCAGGACACCGCCATcag GTGGCGGTAACAGGGGTTCGAATCCCCTAGGGGACGC

Template DNA SEQ ID NO: 20 (TD-20)
DNA sequence:

GGCGTAATACGACTCACTATAGTCCCCTTCGTCTAGAGGCCCAGGACACCGCCATcag ATGGCGGTAACAGGGGTTCGAATCCCCTAGGGGACGC

Template DNA SEQ ID NO: 21 (TD-21)
DNA sequence:

GGCGTAATACGACTCACTATAGTCCCCTTCGTCTAGAGGCCCAGGACACCGCCCTagg AAGGCGGTAACAGGGGTTCGAATCCCCTAGGGGACGC

Template DNA SEQ ID NO: 22 (TD-22)
DNA sequence:

GGCGTAATACGACTCACTATAGTCCCCTTCGTCTAGAGGCCCAGGACACCGCCTTagg ACGGCGGTAACAGGGGTTCGAATCCCCTAGGGGACGC

Template DNA SEQ ID NO: 23 (TD-23)
DNA sequence:

GGCGTAATACGACTCACTATAGTCCCCTTCGTCTAGAGGCCCAGGACACCGCCTTagg AAGGCGGTAACAGGGGTTCGAATCCCCTAGGGGACGC

Template DNA SEQ ID NO: 24 (TD-24)
DNA sequence:

GGCGTAATACGACTCACTATAGTCCCCTTCGTCTAGAGGCCCAGGACACCGCCCTagg ACGGCGGTAACAGGGGTTCGAATCCCCTAGGGGACGC

Template DNA SEQ ID NO: 25 (TD-25)
DNA sequence:

GGCGTAATACGACTCACTATAGTCCCCTTCGTCTAGAGGCCCAGGACACCGCCCTagg GAGGCGGTAACAGGGGTTCGAATCCCCTAGGGGACGC

Template DNA SEQ ID NO: 26 (TD-26)
DNA sequence:

GGCGTAATACGACTCACTATAGTCCCCTTCGTCTAGAGGCCCAGGACACCGCCTTagg ATGGCGGTAACAGGGGTTCGAATCCCCTAGGGGACGC

Template DNA SEQ ID NO: 27 (TD-27)
DNA sequence:

GGCGTAATACGACTCACTATAGTCCCCTTCGTCTAGAGGCCCAGGACACCGCCTTagg GTGGCGGTAACAGGGGTTCGAATCCCCTAGGGGACGC

Template DNA SEQ ID NO: 28 (TD-28)
DNA sequence:

GGCGTAATACGACTCACTATAGTCCCCTTCGTCTAGAGGCCCAGGACACCGCCTTagg GAGGCGGTAACAGGGGTTCGAATCCCCTAGGGGACGC

Template DNA SEQ ID NO: 29 (TD-29)
DNA sequence:

GGCGTAATACGACTCACTATAGTCCCCTTCGTCTAGAGGCCCAGGACACCGCCATagg
GTGGCGGTAACAGGGGTTCGAATCCCCTAGGGGACGC

Template DNA SEQ ID NO: 30 (TD-30)
DNA sequence:

GGCGTAATACGACTCACTATAGTCCCCTTCGTCTAGAGGCCCAGGACACCGCCATagg

ATGGCGGTAACAGGGGTTCGAATCCCCTAGGGGACGC

Template DNA SEQ ID NO: 31 (TD-31)
DNA sequence:

GGCGTAATACGACTCACTATAGTCCCCTTCGTCTAGAGGCCCAGGACACCGCCCTcgg
AAGGCGGTAACAGGGGTTCGAATCCCCTAGGGGACGC

Template DNA SEQ ID NO: 32 (TD-32)
DNA sequence:

GGCGTAATACGACTCACTATAGTCCCCTTCGTCTAGAGGCCCAGGACACCGCCTTcgg
ACGGCGGTAACAGGGGTTCGAATCCCCTAGGGGACGC

Template DNA SEQ ID NO: 33 (TD-33)
DNA sequence:

GGCGTAATACGACTCACTATAGTCCCCTTCGTCTAGAGGCCCAGGACACCGCCTTcgg
AAGGCGGTAACAGGGGTTCGAATCCCCTAGGGGACGC

Template DNA SEQ ID NO: 34 (TD-34)
DNA sequence:

GGCGTAATACGACTCACTATAGTCCCCTTCGTCTAGAGGCCCAGGACACCGCCCTcgg
ACGGCGGTAACAGGGGTTCGAATCCCCTAGGGGACGC

Template DNA SEQ ID NO: 35 (TD-35)
DNA sequence:

GGCGTAATACGACTCACTATAGTCCCCTTCGTCTAGAGGCCCAGGACACCGCCCTcgg
GAGGCGGTAACAGGGGTTCGAATCCCCTAGGGGACGC

Template DNA SEQ ID NO: 36 (TD-36)
DNA sequence:

GGCGTAATACGACTCACTATAGTCCCCTTCGTCTAGAGGCCCAGGACACCGCCTTcgg
ATGGCGGTAACAGGGGTTCGAATCCCCTAGGGGACGC

Template DNA SEQ ID NO: 37 (TD-37)
DNA sequence:

GGCGTAATACGACTCACTATAGTCCCCTTCGTCTAGAGGCCCAGGACACCGCCTTcgg
GTGGCGGTAACAGGGGTTCGAATCCCCTAGGGGACGC

Template DNA SEQ ID NO: 38 (TD-38)
DNA sequence:

GGCGTAATACGACTCACTATAGTCCCCTTCGTCTAGAGGCCCAGGACACCGCCTTcgg
GAGGCGGTAACAGGGGTTCGAATCCCCTAGGGGACGC

Template DNA SEQ ID NO: 39 (TD-39)
DNA sequence:

GGCGTAATACGACTCACTATAGTCCCCTTCGTCTAGAGGCCCAGGACACCGCCATcgg
GTGGCGGTAACAGGGGTTCGAATCCCCTAGGGGACGC

Template DNA SEQ ID NO: 40 (TD-40)
DNA sequence:

GGCGTAATACGACTCACTATAGTCCCCTTCGTCTAGAGGCCCAGGACACCGCCATcgg
ATGGCGGTAACAGGGGTTCGAATCCCCTAGGGGACGC

Template DNA SEQ ID NO: 41 (TD-41)
DNA sequence:

GGCGTAATACGACTCACTATAGTCCCCTTCGTCTAGAGGCCCAGGACACCGCCCTacc
AAGGCGGTAACAGGGGTTCGAATCCCCTAGGGGACGC

Template DNA SEQ ID NO: 42 (TD-42)
DNA sequence:

GGCGTAATACGACTCACTATAGTCCCCTTCGTCTAGAGGCCCAGGACACCGCCTTacc
ACGGCGGTAACAGGGGTTCGAATCCCCTAGGGGACGC

Template DNA SEQ ID NO: 43 (TD-43)
DNA sequence:

GGCGTAATACGACTCACTATAGTCCCCTTCGTCTAGAGGCCCAGGACACCGCCTTacc
AAGGCGGTAACAGGGGTTCGAATCCCCTAGGGGACGC

Template DNA SEQ ID NO: 44 (TD-44)
DNA sequence:

GGCGTAATACGACTCACTATAGTCCCCTTCGTCTAGAGGCCCAGGACACCGCCCTacc
ACGGCGGTAACAGGGGTTCGAATCCCCTAGGGGACGC

Template DNA SEQ ID NO: 45 (TD-45)
DNA sequence:

GGCGTAATACGACTCACTATAGTCCCCTTCGTCTAGAGGCCCAGGACACCGCCCTacc GAGGCGGTAACAGGGGTTCGAATCCCCTAGGGGACGC

Template DNA SEQ ID NO: 46 (TD-46)
DNA sequence:

GGCGTAATACGACTCACTATAGTCCCCTTCGTCTAGAGGCCCAGGACACCGCCTTacc ATGGCGGTAACAGGGGTTCGAATCCCCTAGGGGACGC

Template DNA SEQ ID NO: 47 (TD-47)
DNA sequence:

GGCGTAATACGACTCACTATAGTCCCCTTCGTCTAGAGGCCCAGGACACCGCCTTacc GTGGCGGTAACAGGGGTTCGAATCCCCTAGGGGACGC

Template DNA SEQ ID NO: 48 (TD-48)
DNA sequence:

GGCGTAATACGACTCACTATAGTCCCCTTCGTCTAGAGGCCCAGGACACCGCCTTacc GAGGCGGTAACAGGGGTTCGAATCCCCTAGGGGACGC

Template DNA SEQ ID NO: 49 (TD-49)
DNA sequence:

GGCGTAATACGACTCACTATAGTCCCCTTCGTCTAGAGGCCCAGGACACCGCCATacc GTGGCGGTAACAGGGGTTCGAATCCCCTAGGGGACGC

Template DNA SEQ ID NO: 50 (TD-50)
DNA sequence:

GGCGTAATACGACTCACTATAGTCCCCTTCGTCTAGAGGCCCAGGACACCGCCATacc ATGGCGGTAACAGGGGTTCGAATCCCCTAGGGGACGC

Template DNA SEQ ID NO: 51 (TD-51)
DNA sequence:

GGCGTAATACGACTCACTATAGTCCCCTTCGTCTAGAGGCCCAGGACACCGCCCTccc AAGGCGGTAACAGGGGTTCGAATCCCCTAGGGGACGC

Template DNA SEQ ID NO: 52 (TD-52)
DNA sequence:

GGCGTAATACGACTCACTATAGTCCCCTTCGTCTAGAGGCCCAGGACACCGCCTTccc ACGGCGGTAACAGGGGTTCGAATCCCCTAGGGGACGC

Template DNA SEQ ID NO: 53 (TD-53)
DNA sequence:

GGCGTAATACGACTCACTATAGTCCCCTTCGTCTAGAGGCCCAGGACACCGCCTTccc AAGGCGGTAACAGGGGTTCGAATCCCCTAGGGGACGC

Template DNA SEQ ID NO: 54 (TD-54)
DNA sequence:

GGCGTAATACGACTCACTATAGTCCCCTTCGTCTAGAGGCCCAGGACACCGCCCTccc ACGGCGGTAACAGGGGTTCGAATCCCCTAGGGGACGC

Template DNA SEQ ID NO: 55 (TD-55)
DNA sequence:

GGCGTAATACGACTCACTATAGTCCCCTTCGTCTAGAGGCCCAGGACACCGCCCTccc GAGGCGGTAACAGGGGTTCGAATCCCCTAGGGGACGC

Template DNA SEQ ID NO: 56 (TD-56)
DNA sequence:

GGCGTAATACGACTCACTATAGTCCCCTTCGTCTAGAGGCCCAGGACACCGCCTTccc ATGGCGGTAACAGGGGTTCGAATCCCCTAGGGGACGC

Template DNA SEQ ID NO: 57 (TD-57)
DNA sequence:

GGCGTAATACGACTCACTATAGTCCCCTTCGTCTAGAGGCCCAGGACACCGCCTTccc GTGGCGGTAACAGGGGTTCGAATCCCCTAGGGGACGC

Template DNA SEQ ID NO: 58 (TD-58)
DNA sequence:

GGCGTAATACGACTCACTATAGTCCCCTTCGTCTAGAGGCCCAGGACACCGCCTTccc GAGGCGGTAACAGGGGTTCGAATCCCCTAGGGGACGC

Template DNA SEQ ID NO: 59 (TD-59)
DNA sequence:

GGCGTAATACGACTCACTATAGTCCCCTTCGTCTAGAGGCCCAGGACACCGCCATccc GTGGCGGTAACAGGGGTTCGAATCCCCTAGGGGACGC

Template DNA SEQ ID NO: 60 (TD-60)
DNA sequence:

GGCGTAATACGACTCACTATAGTCCCCTTCGTCTAGAGGCCCAGGACACCGCCATccc ATGGCGGTAACAGGGGTTCGAATCCCCTAGGGGACGC

Template DNA SEQ ID NO: 61 (TD-61)
DNA sequence:

GGCGTAATACGACTCACTATAGGAGCGGTAGTTCAGTCGGTTAGAATACCTGCCTaag AAGCAGGGGGTCGCGGGTTCGAGTCCCGTCCGTTCCGC

Template DNA SEQ ID NO: 62 (TD-62)
DNA sequence:

GGCGTAATACGACTCACTATAGGAGCGGTAGTTCAGTCGGTTAGAATACCTGCTTaag ACGCAGGGGGTCGCGGGTTCGAGTCCCGTCCGTTCCGC

Template DNA SEQ ID NO: 63 (TD-63)
DNA sequence:

GGCGTAATACGACTCACTATAGGAGCGGTAGTTCAGTCGGTTAGAATACCTGCTTaag AAGCAGGGGGTCGCGGGTTCGAGTCCCGTCCGTTCCGC

Template DNA SEQ ID NO: 64 (TD-64)
DNA sequence:

GGCGTAATACGACTCACTATAGGAGCGGTAGTTCAGTCGGTTAGAATACCTGCCTaag ACGCAGGGGGTCGCGGGTTCGAGTCCCGTCCGTTCCGC

Template DNA SEQ ID NO: 65 (TD-65)
DNA sequence:

GGCGTAATACGACTCACTATAGGAGCGGTAGTTCAGTCGGTTAGAATACCTGCCTaag GAGCAGGGGGTCGCGGGTTCGAGTCCCGTCCGTTCCGC

Template DNA SEQ ID NO: 66 (TD-66)
DNA sequence:

GGCGTAATACGACTCACTATAGGAGCGGTAGTTCAGTCGGTTAGAATACCTGCTTaag

ATGCAGGGGGTCGCGGGTTCGAGTCCCGTCCGTTCCGC

Template DNA SEQ ID NO: 67 (TD-67)
DNA sequence:

GGCGTAATACGACTCACTATAGGAGCGGTAGTTCAGTCGGTTAGAATACCTGCTTaag GTGCAGGGGGTCGCGGGTTCGAGTCCCGTCCGTTCCGC

Template DNA SEQ ID NO: 68 (TD-68)
DNA sequence:

GGCGTAATACGACTCACTATAGGAGCGGTAGTTCAGTCGGTTAGAATACCTGCTTaag GAGCAGGGGGTCGCGGGTTCGAGTCCCGTCCGTTCCGC

Template DNA SEQ ID NO: 69 (TD-69)
DNA sequence:

GGCGTAATACGACTCACTATAGGAGCGGTAGTTCAGTCGGTTAGAATACCTGCATaag GTGCAGGGGGTCGCGGGTTCGAGTCCCGTCCGTTCCGC

Template DNA SEQ ID NO: 70 (TD-70)
DNA sequence:

GGCGTAATACGACTCACTATAGGAGCGGTAGTTCAGTCGGTTAGAATACCTGCATaag ATGCAGGGGGTCGCGGGTTCGAGTCCCGTCCGTTCCGC

Template DNA SEQ ID NO: 71 (TD-71)
DNA sequence:

GGCGTAATACGACTCACTATAGGAGCGGTAGTTCAGTCGGTTAGAATACCTGCCTcag AAGCAGGGGGTCGCGGGTTCGAGTCCCGTCCGTTCCGC

Template DNA SEQ ID NO: 72 (TD-72)
DNA sequence:

GGCGTAATACGACTCACTATAGGAGCGGTAGTTCAGTCGGTTAGAATACCTGCTTcag ACGCAGGGGGTCGCGGGTTCGAGTCCCGTCCGTTCCGC

Template DNA SEQ ID NO: 73 (TD-73)
DNA sequence:

GGCGTAATACGACTCACTATAGGAGCGGTAGTTCAGTCGGTTAGAATACCTGCTTcag AAGCAGGGGGTCGCGGGTTCGAGTCCCGTCCGTTCCGC

Template DNA SEQ ID NO: 74 (TD-74)
DNA sequence:

GGCGTAATACGACTCACTATAGGAGCGGTAGTTCAGTCGGTTAGAATACCTGCCTcag ACGCAGGGGGTCGCGGGTTCGAGTCCCGTCCGTTCCGC

Template DNA SEQ ID NO: 75 (TD-75)
DNA sequence:

GGCGTAATACGACTCACTATAGGAGCGGTAGTTCAGTCGGTTAGAATACCTGCCTcag GAGCAGGGGGTCGCGGGTTCGAGTCCCGTCCGTTCCGC

Template DNA SEQ ID NO: 76 (TD-76)
DNA sequence:

GGCGTAATACGACTCACTATAGGAGCGGTAGTTCAGTCGGTTAGAATACCTGCTTcag ATGCAGGGGGTCGCGGGTTCGAGTCCCGTCCGTTCCGC

Template DNA SEQ ID NO: 77 (TD-77)
DNA sequence:

GGCGTAATACGACTCACTATAGGAGCGGTAGTTCAGTCGGTTAGAATACCTGCTTcagGTGCAGGGGGTCGCGGGTTCGAGTCCCGTCCGTTCCGC

Template DNA SEQ ID NO: 78 (TD-78)
DNA sequence:

GGCGTAATACGACTCACTATAGGAGCGGTAGTTCAGTCGGTTAGAATACCTGCTTcagGAGCAGGGGGTCGCGGGTTCGAGTCCCGTCCGTTCCGC

Template DNA SEQ ID NO: 79 (TD-79)
DNA sequence:

GGCGTAATACGACTCACTATAGGAGCGGTAGTTCAGTCGGTTAGAATACCTGCATcagGTGCAGGGGGTCGCGGGTTCGAGTCCCGTCCGTTCCGC

Template DNA SEQ ID NO: 80 (TD-80)
DNA sequence:

GGCGTAATACGACTCACTATAGGAGCGGTAGTTCAGTCGGTTAGAATACCTGCATcagATGCAGGGGGTCGCGGGTTCGAGTCCCGTCCGTTCCGC

Template DNA SEQ ID NO: 81 (TD-81)
DNA sequence:

GGCGTAATACGACTCACTATAGGCTCTGTAGTTCAGTCGGTAGAACGGCGGACTaagAATCCGTATGTCACTGGTTCGAGTCCAGTCAGAGCCGC

Template DNA SEQ ID NO: 82 (TD-82)
DNA sequence:

GGCGTAATACGACTCACTATAGGCTCTGTAGTTCAGTCGGTAGAACGGCGGATTaagACTCCGTATGTCACTGGTTCGAGTCCAGTCAGAGCCGC

Template DNA SEQ ID NO: 83 (TD-83)
DNA sequence:

GGCGTAATACGACTCACTATAGGCTCTGTAGTTCAGTCGGTAGAACGGCGGATTaagAATCCGTATGTCACTGGTTCGAGTCCAGTCAGAGCCGC

Template DNA SEQ ID NO: 84 (TD-84)
DNA sequence:

GGCGTAATACGACTCACTATAGGCTCTGTAGTTCAGTCGGTAGAACGGCGGACTaagACTCCGTATGTCACTGGTTCGAGTCCAGTCAGAGCCGC

Template DNA SEQ ID NO: 85 (TD-85)
DNA sequence:

GGCGTAATACGACTCACTATAGGCTCTGTAGTTCAGTCGGTAGAACGGCGGACTaag
GATCCGTATGTCACTGGTTCGAGTCCAGTCAGAGCCGC

Template DNA SEQ ID NO: 86 (TD-86)
DNA sequence:

GGCGTAATACGACTCACTATAGGCTCTGTAGTTCAGTCGGTAGAACGGCGGATTaagA
TTCCGTATGTCACTGGTTCGAGTCCAGTCAGAGCCGC

Template DNA SEQ ID NO: 87 (TD-87)
DNA sequence:

GGCGTAATACGACTCACTATAGGCTCTGTAGTTCAGTCGGTAGAACGGCGGATTaagG
TTCCGTATGTCACTGGTTCGAGTCCAGTCAGAGCCGC

Template DNA SEQ ID NO: 88 (TD-88)
DNA sequence:

GGCGTAATACGACTCACTATAGGCTCTGTAGTTCAGTCGGTAGAACGGCGGATTaagG
ATCCGTATGTCACTGGTTCGAGTCCAGTCAGAGCCGC

Template DNA SEQ ID NO: 89 (TD-89)
DNA sequence:

GGCGTAATACGACTCACTATAGGCTCTGTAGTTCAGTCGGTAGAACGGCGGAATaag
GTTCCGTATGTCACTGGTTCGAGTCCAGTCAGAGCCGC

Template DNA SEQ ID NO: 90 (TD-90)
DNA sequence:

GGCGTAATACGACTCACTATAGGCTCTGTAGTTCAGTCGGTAGAACGGCGGAATaag
ATTCCGTATGTCACTGGTTCGAGTCCAGTCAGAGCCGC

Template DNA SEQ ID NO: 91 (TD-91)
DNA sequence:

GGCGTAATACGACTCACTATAGGCTCTGTAGTTCAGTCGGTAGAACGGCGGACTcag
AATCCGTATGTCACTGGTTCGAGTCCAGTCAGAGCCGC

Template DNA SEQ ID NO: 92 (TD-92)
DNA sequence:

GGCGTAATACGACTCACTATAGGCTCTGTAGTTCAGTCGGTAGAACGGCGGATTcagA
CTCCGTATGTCACTGGTTCGAGTCCAGTCAGAGCCGC

Template DNA SEQ ID NO: 93 (TD-93)
DNA sequence:

GGCGTAATACGACTCACTATAGGCTCTGTAGTTCAGTCGGTAGAACGGCGGATTcagA ATCCGTATGTCACTGGTTCGAGTCCAGTCAGAGCCGC

Template DNA SEQ ID NO: 94 (TD-94)
DNA sequence:

GGCGTAATACGACTCACTATAGGCTCTGTAGTTCAGTCGGTAGAACGGCGGACTcag ACTCCGTATGTCACTGGTTCGAGTCCAGTCAGAGCCGC

Template DNA SEQ ID NO: 95 (TD-95)
DNA sequence:

GGCGTAATACGACTCACTATAGGCTCTGTAGTTCAGTCGGTAGAACGGCGGACTcag GATCCGTATGTCACTGGTTCGAGTCCAGTCAGAGCCGC

Template DNA SEQ ID NO: 96 (TD-96)
DNA sequence:

GGCGTAATACGACTCACTATAGGCTCTGTAGTTCAGTCGGTAGAACGGCGGATTcagA TTCCGTATGTCACTGGTTCGAGTCCAGTCAGAGCCGC

Template DNA SEQ ID NO: 97 (TD-97)
DNA sequence:

GGCGTAATACGACTCACTATAGGCTCTGTAGTTCAGTCGGTAGAACGGCGGATTcagG TTCCGTATGTCACTGGTTCGAGTCCAGTCAGAGCCGC

Template DNA SEQ ID NO: 98 (TD-98)
DNA sequence:

GGCGTAATACGACTCACTATAGGCTCTGTAGTTCAGTCGGTAGAACGGCGGATTcagG ATCCGTATGTCACTGGTTCGAGTCCAGTCAGAGCCGC

Template DNA SEQ ID NO: 99 (TD-99)
DNA sequence:

GGCGTAATACGACTCACTATAGGCTCTGTAGTTCAGTCGGTAGAACGGCGGAATcag GTTCCGTATGTCACTGGTTCGAGTCCAGTCAGAGCCGC

Template DNA SEQ ID NO: 100 (TD-100)
DNA sequence:

GGCGTAATACGACTCACTATAGGCTCTGTAGTTCAGTCGGTAGAACGGCGGAATcag ATTCCGTATGTCACTGGTTCGAGTCCAGTCAGAGCCGC

Template DNA SEQ ID NO: 101 (TD-101)
DNA sequence:

GGCGTAATACGACTCACTATAGTCCCCTTCGTCTAGAGGCCCAGGACACCGCCCTgcc ACGGCGGTAACAGGGGTTCGAATCCCCTAGGGGACGC

Template DNA SEQ ID NO: 102 (TD-102)
DNA sequence:

GGCGTAATACGACTCACTATAGTCCCCTTCGTCTAGAGGCCCAGGACACCGCCTTgcc GTGGCGGTAACAGGGGTTCGAATCCCCTAGGGGACGC

Template DNA SEQ ID NO: 103 (TD-103)
DNA sequence:

GGCGTAATACGACTCACTATAGTCCCCTTCGTCTAGAGGCCCAGGACACCGCCTTgcc GAGGCGGTAACAGGGGTTCGAATCCCCTAGGGGACGC

Template DNA SEQ ID NO: 104 (TD-104)
DNA sequence:

GGCGTAATACGACTCACTATAGTCCCCTTCGTCTAGAGGCCCAGGACACCGCCCTtcc ACGGCGGTAACAGGGGTTCGAATCCCCTAGGGGACGC

Template DNA SEQ ID NO: 105 (TD-105)
DNA sequence:

GGCGTAATACGACTCACTATAGGCGGGGTGGAGCAGCCTGGTAGCTCGTCGGGCTcat AACCCGAAGATCGTCGGTTCAAATCCGGCCCCCGCAAC

Template DNA SEQ ID NO: 106 (TD-106)
DNA sequence:

GGCGTAATACGACTCACTATAGGAGCGGTAGTTCAGTCGGTTAGAATACCTGCTTtag GTGCAGGGGGTCGCGGGTTCGAGTCCCGTCCGTTCCGC

Template DNA SEQ ID NO: 107 (TD-107)
DNA sequence:

GGCGTAATACGACTCACTATAGGCTCTGTAGTTCAGTCGGTAGAACGGCGGATTtagG TTCCGTATGTCACTGGTTCGAGTCCAGTCAGAGCCGC

tRNA SEQ ID NO: 108 (TR-1)
tRNA(Glu2+Ser5)aag-CA RNA sequence:

GUCCCCUUCGUCUAGAGGCCCAGGACACCGCCCUaagAAGGCGGUAACAGGGGUUC GAAUCCCCUAGGGGACGC

tRNA SEQ ID NO: 109 (TR-2)
tRNA(Glu2+Ala1B)aag-CA RNA sequence:

GUCCCCUUCGUCUAGAGGCCCAGGACACCGCCUUaagACGGCGGUAACAGGGGUUCGAAUCCCCUAGGGGACGC

tRNA SEQ ID NO: 110 (TR-3)
tRNA(Glu2+Phe)aag-CA RNA sequence:

GUCCCCUUCGUCUAGAGGCCCAGGACACCGCCUUaagAAGGCGGUAACAGGGGUUCGAAUCCCCUAGGGGACGC

tRNA SEQ ID NO: 111 (TR-4)
tRNA(Glu2)aag-CA RNA sequence:

GUCCCCUUCGUCUAGAGGCCCAGGACACCGCCCUaagACGGCGGUAACAGGGGUUCGAAUCCCCUAGGGGACGC

tRNA SEQ ID NO: 112 (TR-5)
tRNA(Glu2+Arg3)aag-CA RNA sequence:

GUCCCCUUCGUCUAGAGGCCCAGGACACCGCCCUaagGAGGCGGUAACAGGGGUUCGAAUCCCCUAGGGGACGC

tRNA SEQ ID NO: 113 (TR-6)
tRNA(Glu2+Val2)aag-CA RNA sequence:

GUCCCCUUCGUCUAGAGGCCCAGGACACCGCCUUaagAUGGCGGUAACAGGGGUUCGAAUCCCCUAGGGGACGC

tRNA SEQ ID NO: 114 (TR-7)
tRNA(Glu2+Leu2)aag-CA RNA sequence:

GUCCCCUUCGUCUAGAGGCCCAGGACACCGCCUUaagGUGGCGGUAACAGGGGUUCGAAUCCCCUAGGGGACGC

tRNA SEQ ID NO: 115 (TR-8)
tRNA(Glu2+Pro3)aag-CA RNA sequence:

GUCCCCUUCGUCUAGAGGCCCAGGACACCGCCUUaagGAGGCGGUAACAGGGGUUCGAAUCCCCUAGGGGACGC

tRNA SEQ ID NO: 116 (TR-9)
tRNA(Glu2+Pro2)aag-CA RNA sequence:

GUCCCCUUCGUCUAGAGGCCCAGGACACCGCCAUaagGUGGCGGUAACAGGGGUUCGAAUCCCCUAGGGGACGC

tRNA SEQ ID NO: 117 (TR-10)
tRNA(Glu2+Ala2)aag-CA RNA sequence:

GUCCCCUUCGUCUAGAGGCCCAGGACACCGCCAUaagAUGGCGGUAACAGGGGUUC GAAUCCCCUAGGGGACGC

tRNA SEQ ID NO: 118 (TR-11)
tRNA(Glu2+Ser5)cag-CA RNA sequence:

GUCCCCUUCGUCUAGAGGCCCAGGACACCGCCCUcagAAGGCGGUAACAGGGGUUC GAAUCCCCUAGGGGACGC

tRNA SEQ ID NO:119 (TR-12)
tRNA(Glu2+Ala1B)cag-CA RNA sequence:

GUCCCCUUCGUCUAGAGGCCCAGGACACCGCCUUcagACGGCGGUAACAGGGGUUC GAAUCCCCUAGGGGACGC

tRNA SEQ ID NO:120 (TR-13)
tRNA(Glu2+Phe)cag-CA RNA sequence:

GUCCCCUUCGUCUAGAGGCCCAGGACACCGCCUUcagAAGGCGGUAACAGGGGUUC GAAUCCCCUAGGGGACGC

tRNA SEQ ID NO:121 (TR-14)
tRNA(Glu2)cag-CA RNA sequence:

GUCCCCUUCGUCUAGAGGCCCAGGACACCGCCCUcagACGGCGGUAACAGGGGUUC GAAUCCCCUAGGGGACGC

tRNA SEQ ID NO:122 (TR-15)
tRNA(Glu2+Arg3)cag-CA RNA sequence:

GUCCCCUUCGUCUAGAGGCCCAGGACACCGCCCUcagGAGGCGGUAACAGGGGUUC GAAUCCCCUAGGGGACGC

tRNA SEQ ID NO:123 (TR-16)
tRNA(Glu2+Val2)cag-CA RNA sequence:

GUCCCCUUCGUCUAGAGGCCCAGGACACCGCCUUcagAUGGCGGUAACAGGGGUUC GAAUCCCCUAGGGGACGC

tRNA SEQ ID NO:124 (TR-17)
tRNA(Glu2+Leu2)cag-CA RNA sequence:

GUCCCCUUCGUCUAGAGGCCCAGGACACCGCCUUcagGUGGCGGUAACAGGGGUUC GAAUCCCCUAGGGGACGC

tRNA SEQ ID NO:125 (TR-18)
tRNA(Glu2+Pro3)cag-CA RNA sequence:

GUCCCCUUCGUCUAGAGGCCCAGGACACCGCCUUcagGAGGCGGUAACAGGGGUUC GAAUCCCCUAGGGGACGC

tRNA SEQ ID NO:126 (TR-19)
tRNA(Glu2+Pro2)cag-CA RNA sequence:

GUCCCCUUCGUCUAGAGGCCCAGGACACCGCCAUcagGUGGCGGUAACAGGGGUUC GAAUCCCCUAGGGGACGC

tRNA SEQ ID NO:127 (TR-20)
tRNA(Glu2+Ala2)cag-CA RNA sequence:

GUCCCCUUCGUCUAGAGGCCCAGGACACCGCCAUcagAUGGCGGUAACAGGGGUUC GAAUCCCCUAGGGGACGC

tRNA SEQ ID NO:128 (TR-21)
tRNA(Glu2+Ser5)agg-CA RNA sequence:

GUCCCCUUCGUCUAGAGGCCCAGGACACCGCCCUaggAAGGCGGUAACAGGGGUUC GAAUCCCCUAGGGGACGC

tRNA SEQ ID NO:129 (TR-22)
tRNA(Glu2+Ala1B)agg-CA RNA sequence:

GUCCCCUUCGUCUAGAGGCCCAGGACACCGCCUUaggACGGCGGUAACAGGGGUUC GAAUCCCCUAGGGGACGC

tRNA SEQ ID NO:130 (TR-23)
tRNA(Glu2+Phe)agg-CA RNA sequence:

GUCCCCUUCGUCUAGAGGCCCAGGACACCGCCUUaggAAGGCGGUAACAGGGGUUC GAAUCCCCUAGGGGACGC

tRNA SEQ ID NO:131 (TR-24)
tRNA(Glu2)agg-CA RNA sequence:

GUCCCCUUCGUCUAGAGGCCCAGGACACCGCCCUaggACGGCGGUAACAGGGGUUC GAAUCCCCUAGGGGACGC

tRNA SEQ ID NO:132 (TR-25)
tRNA(Glu2+Arg3)agg-CA RNA sequence:

GUCCCCUUCGUCUAGAGGCCCAGGACACCGCCCUaggGAGGCGGUAACAGGGGUUC GAAUCCCCUAGGGGACGC

tRNA SEQ ID NO:133 (TR-26)
tRNA(Glu2+Val2)agg-CA RNA sequence:

GUCCCCUUCGUCUAGAGGCCCAGGACACCGCCUUaggAUGGCGGUAACAGGGGUUC GAAUCCCCUAGGGGACGC

tRNA SEQ ID NO:134 (TR-27)
tRNA(Glu2+Leu2)agg-CA RNA sequence:

GUCCCCUUCGUCUAGAGGCCCAGGACACCGCCUUaggGUGGCGGUAACAGGGGUUC GAAUCCCCUAGGGGACGC

tRNA SEQ ID NO:135 (TR-28)
tRNA(Glu2+Pro3)agg-CA RNA sequence:

GUCCCCUUCGUCUAGAGGCCCAGGACACCGCCUUaggGAGGCGGUAACAGGGGUUC GAAUCCCCUAGGGGACGC

tRNA SEQ ID NO:136 (TR-29)
tRNA(Glu2+Pro2)agg-CA RNA sequence:

GUCCCCUUCGUCUAGAGGCCCAGGACACCGCCAUaggGUGGCGGUAACAGGGGUUC GAAUCCCCUAGGGGACGC

tRNA SEQ ID NO:137 (TR-30)
tRNA(Glu2+Ala2)agg-CA RNA sequence:

GUCCCCUUCGUCUAGAGGCCCAGGACACCGCCAUaggAUGGCGGUAACAGGGGUUC GAAUCCCCUAGGGGACGC

tRNA SEQ ID NO:138 (TR-31)
tRNA(Glu2+Ser5)cgg-CA RNA sequence:

GUCCCCUUCGUCUAGAGGCCCAGGACACCGCCCUcggAAGGCGGUAACAGGGGUUC

GAAUCCCCUAGGGGACGC

tRNA SEQ ID NO:139 (TR-32)
tRNA(Glu2+Ala1B)cgg-CA RNA sequence:

GUCCCCUUCGUCUAGAGGCCCAGGACACCGCCUUcggACGGCGGUAACAGGGGUUC GAAUCCCCUAGGGGACGC

tRNA SEQ ID NO:140 (TR-33)

tRNA(Glu2+Phe)cgg-CA RNA sequence:

GUCCCCUUCGUCUAGAGGCCCAGGACACCGCCUUcggAAGGCGGUAACAGGGGUUC GAAUCCCCUAGGGGACGC

tRNA SEQ ID NO:141 (TR-34)
tRNA(Glu2)cgg-CA RNA sequence:

GUCCCCUUCGUCUAGAGGCCCAGGACACCGCCCUcggACGGCGGUAACAGGGGUUC GAAUCCCCUAGGGGACGC

tRNA SEQ ID NO:142 (TR-35)
tRNA(Glu2+Arg3)cgg-CA RNA sequence:

GUCCCCUUCGUCUAGAGGCCCAGGACACCGCCCUcggGAGGCGGUAACAGGGGUUC GAAUCCCCUAGGGGACGC

tRNA SEQ ID NO:143 (TR-36)
tRNA(Glu2+Val2)cgg-CA RNA sequence:

GUCCCCUUCGUCUAGAGGCCCAGGACACCGCCUUcggAUGGCGGUAACAGGGGUUC GAAUCCCCUAGGGGACGC

tRNA SEQ ID NO:144 (TR-37)
tRNA(Glu2+Leu2)cgg-CA RNA sequence:

GUCCCCUUCGUCUAGAGGCCCAGGACACCGCCUUcggGUGGCGGUAACAGGGGUUC GAAUCCCCUAGGGGACGC

tRNA SEQ ID NO:145 (TR-38)
tRNA(Glu2+Pro3)cgg-CA RNA sequence:

GUCCCCUUCGUCUAGAGGCCCAGGACACCGCCUUcggGAGGCGGUAACAGGGGUUC GAAUCCCCUAGGGGACGC

tRNA SEQ ID NO:146 (TR-39)
tRNA(Glu2+Pro2)cgg-CA RNA sequence:

GUCCCCUUCGUCUAGAGGCCCAGGACACCGCCAUcggGUGGCGGUAACAGGGGUUC GAAUCCCCUAGGGGACGC

tRNA SEQ ID NO:147 (TR-40)
tRNA(Glu2+Ala2)cgg-CA RNA sequence:

GUCCCCUUCGUCUAGAGGCCCAGGACACCGCCAUcggAUGGCGGUAACAGGGGUUC GAAUCCCCUAGGGGACGC

tRNA SEQ ID NO:148 (TR-41)
tRNA(Glu2+Ser5)acc-CA RNA sequence:

GUCCCCUUCGUCUAGAGGCCCAGGACACCGCCCUaccAAGGCGGUAACAGGGGUUC GAAUCCCCUAGGGGACGC

tRNA SEQ ID NO:149 (TR-42)
tRNA(Glu2+Ala1B)acc-CA RNA sequence:

GUCCCCUUCGUCUAGAGGCCCAGGACACCGCCUUaccACGGCGGUAACAGGGGUUC GAAUCCCCUAGGGGACGC

tRNA SEQ ID NO:150 (TR-43)
tRNA(Glu2+Phe)acc-CA RNA sequence:

GUCCCCUUCGUCUAGAGGCCCAGGACACCGCCUUaccAAGGCGGUAACAGGGGUUC GAAUCCCCUAGGGGACGC

tRNA SEQ ID NO:151 (TR-44)
tRNA(Glu2)acc-CA RNA sequence:

GUCCCCUUCGUCUAGAGGCCCAGGACACCGCCCUaccACGGCGGUAACAGGGGUUC GAAUCCCCUAGGGGACGC

tRNA SEQ ID NO:152 (TR-45)
tRNA(Glu2+Arg3)acc-CA RNA sequence:

GUCCCCUUCGUCUAGAGGCCCAGGACACCGCCCUaccGAGGCGGUAACAGGGGUUC GAAUCCCCUAGGGGACGC

tRNA SEQ ID NO:153 (TR-46)
tRNA(Glu2+Val2)acc-CA RNA sequence:

GUCCCCUUCGUCUAGAGGCCCAGGACACCGCCUUaccAUGGCGGUAACAGGGGUUC GAAUCCCCUAGGGGACGC

tRNA SEQ ID NO:154 (TR-47)
tRNA(Glu2+Leu2)acc-CA RNA sequence:

GUCCCCUUCGUCUAGAGGCCCAGGACACCGCCUUaccGUGGCGGUAACAGGGGUUC GAAUCCCCUAGGGGACGC

tRNA SEQ ID NO:155 (TR-48)
tRNA(Glu2+Pro3)acc-CA RNA sequence:

GUCCCCUUCGUCUAGAGGCCCAGGACACCGCCUUaccGAGGCGGUAACAGGGGUUC GAAUCCCCUAGGGGACGC

tRNA SEQ ID NO:156 (TR-49)
tRNA(Glu2+Pro2)acc-CA RNA sequence:

GUCCCCUUCGUCUAGAGGCCCAGGACACCGCCAUaccGUGGCGGUAACAGGGGUUC GAAUCCCCUAGGGGACGC

tRNA SEQ ID NO:157 (TR-50)
tRNA(Glu2+Ala2)acc-CA RNA sequence:

GUCCCCUUCGUCUAGAGGCCCAGGACACCGCCAUaccAUGGCGGUAACAGGGGUUC GAAUCCCCUAGGGGACGC

tRNA SEQ ID NO:158 (TR-51)
tRNA(Glu2+Ser5)ccc-CA RNA sequence:

GUCCCCUUCGUCUAGAGGCCCAGGACACCGCCCUcccAAGGCGGUAACAGGGGUUC GAAUCCCCUAGGGGACGC

tRNA SEQ ID NO:159 (TR-52)
tRNA(Glu2+Ala1B)ccc-CA RNA sequence:

GUCCCCUUCGUCUAGAGGCCCAGGACACCGCCUUcccACGGCGGUAACAGGGGUUC GAAUCCCCUAGGGGACGC

tRNA SEQ ID NO:160 (TR-53)
tRNA(Glu2+Phe)ccc-CA RNA sequence:

GUCCCCUUCGUCUAGAGGCCCAGGACACCGCCUUcccAAGGCGGUAACAGGGGUUC GAAUCCCCUAGGGGACGC

tRNA SEQ ID NO:161 (TR-54)
tRNA(Glu2)ccc-CA RNA sequence:

GUCCCCUUCGUCUAGAGGCCCAGGACACCGCCCUcccACGGCGGUAACAGGGGUUC GAAUCCCCUAGGGGACGC

tRNA SEQ ID NO:162 (TR-55)
tRNA(Glu2+Arg3)ccc-CA RNA sequence:

GUCCCCUUCGUCUAGAGGCCCAGGACACCGCCCUcccGAGGCGGUAACAGGGGUUC GAAUCCCCUAGGGGACGC

tRNA SEQ ID NO:163 (TR-56)
tRNA(Glu2+Val2)ccc-CA RNA sequence:

GUCCCCUUCGUCUAGAGGCCCAGGACACCGCCUUcccAUGGCGGUAACAGGGGUUC GAAUCCCCUAGGGGACGC

tRNA SEQ ID NO:164 (TR-57)
tRNA(Glu2+Leu2)ccc-CA RNA sequence:

GUCCCCUUCGUCUAGAGGCCCAGGACACCGCCUUcccGUGGCGGUAACAGGGGUUC GAAUCCCCUAGGGGACGC

tRNA SEQ ID NO:165 (TR-58)
tRNA(Glu2+Pro3)ccc-CA RNA sequence:

GUCCCCUUCGUCUAGAGGCCCAGGACACCGCCUUcccGAGGCGGUAACAGGGGUUC GAAUCCCCUAGGGGACGC

tRNA SEQ ID NO:166 (TR-59)
tRNA(Glu2+Pro2)ccc-CA RNA sequence:

GUCCCCUUCGUCUAGAGGCCCAGGACACCGCCAUcccGUGGCGGUAACAGGGGUUC GAAUCCCCUAGGGGACGC

tRNA SEQ ID NO:167 (TR-60)
tRNA(Glu2+Ala2)ccc-CA RNA sequence:

GUCCCCUUCGUCUAGAGGCCCAGGACACCGCCAUcccAUGGCGGUAACAGGGGUUC GAAUCCCCUAGGGGACGC

tRNA SEQ ID NO:168 (TR-61)
tRNA(Asp1+Ser5)aag-CA RNA sequence:

GGAGCGGUAGUUCAGUCGGUUAGAAUACCUGCCUaagAAGCAGGGGGUCGCGGGU UCGAGUCCCGUCCGUUCCGC

tRNA SEQ ID NO:169 (TR-62)
tRNA(Asp1+Ala1B)aag-CA RNA sequence:

GGAGCGGUAGUUCAGUCGGUUAGAAUACCUGCUUaagACGCAGGGGGUCGCGGGU UCGAGUCCCGUCCGUUCCGC

tRNA SEQ ID NO: 170 (TR-63)
tRNA(Asp1+Phe)aag-CA RNA sequence:

GGAGCGGUAGUUCAGUCGGUUAGAAUACCUGCUUaagAAGCAGGGGGUCGCGGGU UCGAGUCCCGUCCGUUCCGC

tRNA SEQ ID NO:171 (TR-64)
tRNA(Asp1)aag-CA RNA sequence:

GGAGCGGUAGUUCAGUCGGUUAGAAUACCUGCCUaagACGCAGGGGGUCGCGGGU UCGAGUCCCGUCCGUUCCGC

tRNA SEQ ID NO:172 (TR-65)
tRNA(Asp1+Arg3)aag-CA RNA sequence:

GGAGCGGUAGUUCAGUCGGUUAGAAUACCUGCCUaagGAGCAGGGGGUCGCGGGU UCGAGUCCCGUCCGUUCCGC

tRNA SEQ ID NO: 173 (TR-66)
tRNA(Asp1+Val2)aag-CA RNA sequence:

GGAGCGGUAGUUCAGUCGGUUAGAAUACCUGCUUaagAUGCAGGGGGUCGCGGGUUCGAGUCCCGUCCGUUCCGC

tRNA SEQ ID NO: 174 (TR-67)
tRNA(Asp1+Leu2)aag-CA RNA sequence:

GGAGCGGUAGUUCAGUCGGUUAGAAUACCUGCUUaagGUGCAGGGGGUCGCGGGUUCGAGUCCCGUCCGUUCCGC

tRNA SEQ ID NO:175 (TR-68)
tRNA(Asp1+Pro3)aag-CA RNA sequence:

GGAGCGGUAGUUCAGUCGGUUAGAAUACCUGCUUaagGAGCAGGGGGUCGCGGGUUCGAGUCCCGUCCGUUCCGC

tRNA SEQ ID NO:176 (TR-69)
tRNA(Asp1+Pro2)aag-CA RNA sequence:

GGAGCGGUAGUUCAGUCGGUUAGAAUACCUGCAUaagGUGCAGGGGGUCGCGGGUUCGAGUCCCGUCCGUUCCGC

tRNA SEQ ID NO: 177 (TR-70)
tRNA(Asp1+Ala2)aag-CA RNA sequence:

GGAGCGGUAGUUCAGUCGGUUAGAAUACCUGCAUaagAUGCAGGGGGUCGCGGGUUCGAGUCCCGUCCGUUCCGC

tRNA SEQ ID NO:178 (TR-71)
tRNA(Asp1+Ser5)cag-CA RNA sequence:

GGAGCGGUAGUUCAGUCGGUUAGAAUACCUGCCUcagAAGCAGGGGGUCGCGGGUUCGAGUCCCGUCCGUUCCGC

tRNA SEQ ID NO:179 (TR-72)
tRNA(Asp1+Ala1B)cag-CA RNA sequence:

GGAGCGGUAGUUCAGUCGGUUAGAAUACCUGCUUcagACGCAGGGGGUCGCGGGUUCGAGUCCCGUCCGUUCCGC

tRNA SEQ ID NO:180 (TR-73)
tRNA(Asp1+Ph2)cag-CA RNA sequence:

GGAGCGGUAGUUCAGUCGGUUAGAAUACCUGCUUcagAAGCAGGGGGUCGCGGGUUCGAGUCCCGUCCGUUCCGC

tRNA SEQ ID NO:181 (TR-74)
tRNA(Asp1)cag-CA RNA sequence:

GGAGCGGUAGUUCAGUCGGUUAGAAUACCUGCCUcagACGCAGGGGGUCGCGGGUUCGAGUCCCGUCCGUUCCGC

tRNA SEQ ID NO:182 (TR-75)
tRNA(Asp1+Arg3)cag-CA RNA sequence:

GGAGCGGUAGUUCAGUCGGUUAGAAUACCUGCCUcagGAGCAGGGGGUCGCGGGUUCGAGUCCCGUCCGUUCCGC

tRNA SEQ ID NO:183 (TR-76)
tRNA(Asp1+Val2)cag-CA RNA sequence:

GGAGCGGUAGUUCAGUCGGUUAGAAUACCUGCUUcagAUGCAGGGGGUCGCGGGUUCGAGUCCCGUCCGUUCCGC

tRNA SEQ ID NO:184 (TR-77)
tRNA(Asp1+Leu2)cag-CA RNA sequence:

GGAGCGGUAGUUCAGUCGGUUAGAAUACCUGCUUcagGUGCAGGGGGUCGCGGGUUCGAGUCCCGUCCGUUCCGC

tRNA SEQ ID NO:185 (TR-78)
tRNA(Asp1+Pro3)cag-CA RNA sequence:

GGAGCGGUAGUUCAGUCGGUUAGAAUACCUGCUUcagGAGCAGGGGGUCGCGGGUUCGAGUCCCGUCCGUUCCGC

tRNA SEQ ID NO:186 (TR-79)
tRNA(Asp1+Pro2)cag-CA RNA sequence:

GGAGCGGUAGUUCAGUCGGUUAGAAUACCUGCAUcagGUGCAGGGGGUCGCGGGUUCGAGUCCCGUCCGUUCCGC

tRNA SEQ ID NO:187 (TR-80)
tRNA(Asp1+Ala2)cag-CA RNA sequence:

GGAGCGGUAGUUCAGUCGGUUAGAAUACCUGCAUcagAUGCAGGGGGUCGCGGGUUCGAGUCCCGUCCGUUCCGC

tRNA SEQ ID NO:188 (TR-81)
tRNA(AsnE2)aag-CA RNA sequence:

GGCUCUGUAGUUCAGUCGGUAGAACGGCGGACUaagAAUCCGUAUGUCACUGGUUCGAGUCCAGUCAGAGCCGC

tRNA SEQ ID NO:189 (TR-82)
tRNA(AsnE2+Ala1B)aag-CA RNA sequence:

GGCUCUGUAGUUCAGUCGGUAGAACGGCGGAUUaagACUCCGUAUGUCACUGGUU CGAGUCCAGUCAGAGCCGC

tRNA SEQ ID NO:190 (TR-83)
tRNA(AsnE2+Phe)aag-CA RNA sequence:

GGCUCUGUAGUUCAGUCGGUAGAACGGCGGAUUaagAAUCCGUAUGUCACUGGUU CGAGUCCAGUCAGAGCCGC

tRNA SEQ ID NO:191 (TR-84)
tRNA(AsnE2+Glu2)aag-CA RNA sequence:

GGCUCUGUAGUUCAGUCGGUAGAACGGCGGACUaagACUCCGUAUGUCACUGGUUC GAGUCCAGUCAGAGCCGC

tRNA SEQ ID NO:192 (TR-85)
tRNA(AsnE2+Arg3)aag-CA RNA sequence:

GGCUCUGUAGUUCAGUCGGUAGAACGGCGGACUaagGAUCCGUAUGUCACUGGUU CGAGUCCAGUCAGAGCCGC

tRNA SEQ ID NO:193 (TR-86)
tRNA(AsnE2+Val2)aag-CA RNA sequence:

GGCUCUGUAGUUCAGUCGGUAGAACGGCGGAUUaagAUUCCGUAUGUCACUGGUU CGAGUCCAGUCAGAGCCGC

tRNA SEQ ID NO:194 (TR-87)
tRNA(AsnE2+Leu2)aag-CA RNA sequence:

GGCUCUGUAGUUCAGUCGGUAGAACGGCGGAUUaagGUUCCGUAUGUCACUGGUU CGAGUCCAGUCAGAGCCGC

tRNA SEQ ID NO:195 (TR-88)
tRNA(AsnE2+Pro3)aag-CA RNA sequence:

GGCUCUGUAGUUCAGUCGGUAGAACGGCGGAUUaagGAUCCGUAUGUCACUGGUU CGAGUCCAGUCAGAGCCGC

tRNA SEQ ID NO:196 (TR-89)
tRNA(AsnE2+Pro2)aag-CA RNA sequence:

GGCUCUGUAGUUCAGUCGGUAGAACGGCGGAAUaagGUUCCGUAUGUCACUGGUU CGAGUCCAGUCAGAGCCGC

tRNA SEQ ID NO:197 (TR-90)
tRNA(AsnE2+Ala2)aag-CA RNA sequence:

GGCUCUGUAGUUCAGUCGGUAGAACGGCGGAAUaagAUUCCGUAUGUCACUGGUUCGAGUCCAGUCAGAGCCGC

tRNA SEQ ID NO:198 (TR-91)
tRNA(AsnE2)cag-CA RNA sequence:

GGCUCUGUAGUUCAGUCGGUAGAACGGCGGACUcagAAUCCGUAUGUCACUGGUUCGAGUCCAGUCAGAGCCGC

tRNA SEQ ID NO:199 (TR-92)
tRNA(AsnE2+Ala1B)cag-CA RNA sequence:

GGCUCUGUAGUUCAGUCGGUAGAACGGCGGAUUcagACUCCGUAUGUCACUGGUUCGAGUCCAGUCAGAGCCGC

tRNA SEQ ID NO:200 (TR-93)
tRNA(AsnE2+Phe)cag-CA RNA sequence:

GGCUCUGUAGUUCAGUCGGUAGAACGGCGGAUUcagAAUCCGUAUGUCACUGGUUCGAGUCCAGUCAGAGCCGC

tRNA SEQ ID NO:201 (TR-94)
tRNA(AsnE2+Glu2)cag-CA RNA sequence:

GGCUCUGUAGUUCAGUCGGUAGAACGGCGGACUcagACUCCGUAUGUCACUGGUUCGAGUCCAGUCAGAGCCGC

tRNA SEQ ID NO:202 (TR-95)
tRNA(AsnE2+Arg3)cag-CA RNA sequence:

GGCUCUGUAGUUCAGUCGGUAGAACGGCGGACUcagGAUCCGUAUGUCACUGGUUCGAGUCCAGUCAGAGCCGC

tRNA SEQ ID NO:203 (TR-96)
tRNA(AsnE2+Val2)cag-CA RNA sequence:

GGCUCUGUAGUUCAGUCGGUAGAACGGCGGAUUcagAUUCCGUAUGUCACUGGUUCGAGUCCAGUCAGAGCCGC

tRNA SEQ ID NO:204 (TR-97)
tRNA(AsnE2+Leu2)cag-CA RNA sequence:

GGCUCUGUAGUUCAGUCGGUAGAACGGCGGAUUcagGUUCCGUAUGUCACUGGUUCGAGUCCAGUCAGAGCCGC

tRNA SEQ ID NO:205 (TR-98)
tRNA(AsnE2+Pro3)cag-CA RNA sequence:

GGCUCUGUAGUUCAGUCGGUAGAACGGCGGAUUcagGAUCCGUAUGUCACUGGUU CGAGUCCAGUCAGAGCCGC

tRNA SEQ ID NO:206 (TR-99)
tRNA(AsnE2+Pro2)cag-CA RNA sequence:

GGCUCUGUAGUUCAGUCGGUAGAACGGCGGAAUcagGUUCCGUAUGUCACUGGUU CGAGUCCAGUCAGAGCCGC

tRNA SEQ ID NO:207 (TR-100)
tRNA(AsnE2+Ala2)cag-CA RNA sequence:

GGCUCUGUAGUUCAGUCGGUAGAACGGCGGAAUcagAUUCCGUAUGUCACUGGUU CGAGUCCAGUCAGAGCCGC

tRNA SEQ ID NO:208 (TR-101)
tRNA(Glu2)gcc-CA RNA sequence:

GUCCCCUUCGUCUAGAGGCCCAGGACACCGCCCUgccACGGCGGUAACAGGGGUUC GAAUCCCCUAGGGGACGC

tRNA SEQ ID NO:209 (TR-102)
tRNA(Glu2+Leu2)gcc-CA RNA sequence:

GUCCCCUUCGUCUAGAGGCCCAGGACACCGCCUUgccGUGGCGGUAACAGGGGUUC GAAUCCCCUAGGGGACGC

tRNA SEQ ID NO:210 (TR-103)
tRNA(Glu2+Pro3)gcc-CA RNA sequence:

GUCCCCUUCGUCUAGAGGCCCAGGACACCGCCUUgccGAGGCGGUAACAGGGGUUC

GAAUCCCCUAGGGGACGC

tRNA SEQ ID NO:211 (TR-104)
tRNA(Glu2)ucc-CA RNA sequence:

GUCCCCUUCGUCUAGAGGCCCAGGACACCGCCCUuccACGGCGGUAACAGGGGUUC GAAUCCCCUAGGGGACGC

tRNA SEQ ID NO:212 (TR-105)
tRNA(fMet)cau-CA RNA sequence:

GGCGGGGUGGAGCAGCCUGGUAGCUCGUCGGGCUcauAACCCGAAGAUCGUCGGUU CAAAUCCGGCCCCCGCAAC

tRNA SEQ ID NO:213 (TR-106)
tRNA(Asp1+Leu2)_uag-CA RNA sequence:

GGAGCGGUAGUUCAGUCGGUUAGAAUACCUGCUUuagGUGCAGGGGGUCGCGGGU UCGAGUCCCGUCCGUUCCGC

tRNA SEQ ID NO:214 (TR-107)
tRNA(AsnE2+Leu2)_uag-CA RNA sequence:

GGCUCUGUAGUUCAGUCGGUAGAACGGCGGAUUuagGUUCCGUAUGUCACUGGUU CGAGUCCAGUCAGAGCCGC

Example 4-2. Production of tRNA-CAs by ligation reaction

By the procedure described below, tRNA5' fragments, pLp, and tRNA3' fragments were ligated using a ligation reaction to produce various tRNA-CAs. Chemically synthesized products (Gene Design Co., Ltd.) were used for the tRNA 5' fragments and tRNA 3' fragments. Each tRNA fragment and its full-length sequences are shown below. FR-1 and FR-2 were used as the tRNA 5' fragment and the tRNA 3' fragment, respectively, to produce TR-108, and FR-3 and FR-4 were used as the tRNA 5' fragment and the tRNA 3' fragment, respectively, to produce TR-109.

SEQ ID NO: 217 (FR-1)
tRNA(Asp1)5' RNA sequence GGAGCGGUAGUUCAGUCGGUUAGAAUACCUGCUU
SEQ ID NO: 218 (FR-2)
tRNA(Asp1)3' agRNA sequence AGGUGCAGGGGGUCGCGGGUUCGAGUCCCGUCCGUUCCGC tRNA SEQ ID NO: 215 (TR-108)
tRNA(Asp1+Leu2)Lag-CA RNA sequence:

GGAGCGGUAGUUCAGUCGGUUAGAAUACCUGCUULagGUGCAGGGGGUCGCGGGU UCGAGUCCCGUCCGUUCCGC

SEQ ID NO: 219 (FR-3)
tRNA(AsnE2)5' RNA sequence GGCUCUGUAGUUCAGUCGGUAGAACGGCGGAUU
SEQ ID NO: 220 (FR-4)
tRNA(AsnE2)3'ag RNA sequence AGGUUCCGUAUGUCACUGGUUCGAGUCCAGUCAGAGCCGC
tRNA SEQ ID NO: 216 (TR-109)
tRNA(AsnE2+Leu2)Lag-CA RNA sequence

GGCUCUGUAGUUCAGUCGGUAGAACGGCGGAUULagGUUCCGUAUGUCACUGGUU CGAGUCCAGUCAGAGCCGC

A reaction solution composed of 50 mM HEPES-KOH (pH 7.5), 20 mM MgC12, 1 mM ATP, 0.125-0.25 mM pLp, 25 μM tRNA 5' fragment, 0.6 U/μL T4 RNA ligase (New England Biolabs), and 10% DMSO was left to stand overnight at 15°C to perform a ligation reaction between the tRNA 5' fragment and pLp. The ligation product was extracted with phenol-chloroform, and recovered by ethanol precipitation.

To prevent the unreacted tRNA 5' fragment from being carried over to the next ligation reaction, sodium periodate (NaIO4) was used to cleave the ribose at the 3' end of the tRNA 5' fragment. Specifically, 10 μM ligation product was cleaved by allowing it to stand on ice for 30 minutes in the dark in the presence of 10 mM sodium periodate. After the reaction, one-tenth volume of 100 mM glucose was added, and the mixture was allowed to stand on ice for 30 minutes in the dark to decompose the excess sodium periodate. The reaction product was collected by ethanol precipitation.

After the periodic acid treatment, T4 polybase kinase (T4 PNK) treatment was performed to phosphorylate the 5' end and dephosphorylate the 3' end of the ligation product. The reaction solution composed of 10 μM ligation product after periodic acid treatment, 50 mM Tris-HCl (pH 8.0), 10 mM MgCl2, 5 mM DTT, 300 μM ATP, and 0.5 U/μL T4 PNK (TaKaRa) was reacted by allowing it to stand at 37°C for 30 to 60 minutes. The reaction product was extracted with phenol-chloroform and collected by ethanol precipitation.

A ligation reaction was performed between the post-PNK-treatment reaction product and the tRNA 3' fragment. First, a solution composed of 10 μM PNK-treated reaction product, 10 μM tRNA 3' fragment, 50 mM HEPES-KOH (pH 7.5), and 15 mM MgC12 was heated at 65°C for 7 minutes and then allowed to stand at room temperature for 30 minutes

to 1 hour to anneal the PNK-treated reaction product and the tRNA 3' fragment. Next, T4 PNK treatment was performed to phosphorylate the 5' end of the tRNA 3' fragment. T4 PNK treatment was performed by adding DTT (final concentration of 3.5 mM), ATP (final concentration of 300 μM), and T4 PNK (final concentration of 0.5 U/μL) to the annealed solution, and allowing this to stand at 37°C for 30 minutes. Next, T4 RNA ligase (New England Biolabs) was added at a final concentration of 0.9 U/μL to this solution, and ligation reaction was performed by allowing this mixture to stand at 37°C for 30 to 40 minutes. The ligation product was extracted with phenol-chloroform and collected by ethanol precipitation.

The tRNA-CAs produced by the ligation method were subjected to preparative purification by high-performance reverse-phase chromatography (HPLC) (aqueous solution of 15 mM TEA and 400 mM HFIP/ methanol solution of 15 mM TEA and 400 mM HFIP) and then subjected to denatured urea-10% polyacrylamide electrophoresis, to confirm whether they had the desired length.

Analyses of tRNA fragments cleaved by RNase$T_1$

tRNA-CAs prepared using a ligation reaction were fragmented by RNase, and then analyzed to confirm incorporation of lysidine (L) introduced by pLp at the intended site.

A reaction solution containing 10 μM tRNA-CA, 5 U/μL RNase$T_1$ (Epicentre or ThermoFisher Scientific), and 10 mM ammonium acetate (pH 5.3) was allowed to stand at 37°C for 1 hour to cleave the RNA specifically at the 3' side of the G base to analyze the RNA fragment containing lysidine (L) introduced by pLp.

CUULAGp
LCMS(ESI) m/z = 1020 ((M-2H)/2)-
Retention time: 3.84 minutes (analysis condition LTQTEA/HFIP05_03)

Since the molecular weight of the fragment (CUUAGp) expected when pLp is not ligated and the molecular weight of the fragment (UUCAGp) derived from other parts of RNA are the same, unfragmeneted RNA (TR-108) was also analyzed.

LCMS(ESI) m/z = 1109 ((M-22H)/22)-
Retention time: 3.92 minutes (analysis condition LTQTEA/HFIP05_01)

Comparison to the mass chromatogram of the RNA expected when pLp is not ligated and to that of the RNA expected when uridine is present instead of lysidine, confirmed that most of the pLp ligation took place.

AUULAGp
LCMS(ESI) m/z = 1032 ((M-2H)/2)-
Retention time: 4.16 minutes (analysis condition LTQTEA/HFIP05_03)

Comparison to the mass chromatogram of the fragment (AUUAGp) expected when pLp is not ligated and to that of the fragment (AUUUAGp) expected when uridine is present instead of lysidine, confirmed that most of the pLp ligation took place.

Preparation of elongator aminoacyl tRNA using aminoacyl pCpA

A reaction solution was prepared by adding nuclease-free water to adjust the solution to 25 μM transcribed tRNA(Glu2+Ser5)aag-CA (TR-1), 50 mM HEPES-KOH pH7.5, 20 mM $MgCl_2$, 1 mM ATP, 0.6 unit/μL T4 RNA ligase (New England Biolabs), and 0.25 mM aminoacyl pCpA (a DMSO solution of SS15), and ligation reaction was performed at 15°C for 45 minutes. Before adding T4 RNA ligase and aminoacyl pCpA, the reaction solution was heated to 95°C for 2 minutes and then allowed to stand at room temperature for 5 minutes to refold the tRNA in advance.

To the ligation reaction solution, sodium acetate was added to make a concentration of 0.3 M, and phenol-chloroform extraction was performed to prepare the elongator aminoacyl tRNA (AAtR-1). AAtR-1 was recovered by ethanol precipitation, and before adding it to a translation mixture, it was dissolved in a 1 mM aqueous sodium acetate solution.

Similarly, the transcribed tRNAs (TR-2 to TR-103, TR-106 to TR-109) were subjected to ligation reaction with aminoacyl pCpA (SS15) by the method described above, phenol-chloroform extraction, and ethanol precipitation, to prepare elongator aminoacyl tRNAs (AAtR-2 to AAtR-103, AAtR-132, AAtR-133, AAtR-136, and AAtR-137). These aminoacyl tRNAs were dissolved in 1 mM aqueous sodium acetate solution before addition to a translation mixture.

Similarly, the transcribed tRNAs (TR-4, TR-14, TR-24, TR-34, TR-44, TR-54, TR-64, TR-74, TR-81, TR-91, and TR-104) were subjected to ligation reaction with aminoacyl pCpA (SS16) by the method described above, phenol-chloroform extraction, and ethanol precipitation, to prepare elongator aminoacyl tRNAs (AAtR-104 to AAtR-114). These aminoacyl tRNAs were dissolved in 1 mM aqueous sodium acetate solution before addition to a translation mixture.

Similarly, the transcribed tRNAs (TR-44, TR-47, TR-48, and TR-50) were subjected to ligation reaction with aminoacyl pCpA (SS14) by the method described above, phenol-chloroform extraction, and ethanol precipitation, to prepare elongator aminoacyl tRNAs (AAtR-115 to AAtR-118). These aminoacyl tRNAs were dissolved in 1 mM aqueous sodium acetate solution before addition to a translation mixture.

Similarly, the transcribed tRNAs (TR-44, TR-47, TR-48, TR-50, TR-24, and TR-34) were subjected to ligation reaction with aminoacyl pCpA (SS45) by the method described above, phenol-chloroform extraction, and ethanol precipitation, to prepare elongator aminoacyl tRNAs (AAtR-119 to AAtR-122, AAtR-129, and AAtR-130). These aminoacyl tRNAs were dissolved in 1 mM aqueous sodium acetate solution before addition to a translation mixture.

Similarly, the transcribed tRNAs (TR-44, TR-47, TR-48, TR-50, TR-77, and TR-97) were subjected to ligation reaction with aminoacyl pCpA (Compound TS24 synthesized by a method described in Patent Literature (WO2018143145A1)) by the method described above, phenol-chloroform extraction, and ethanol precipitation, to prepare elongator aminoacyl tRNAs (AAtR-123 to AAtR-126, AAtR-134, and AAtR-138). These aminoacyl tRNAs were dissolved in 1 mM aqueous sodium acetate solution before addition to a translation mixture.

Similarly, the transcribed tRNAs (TR-54, TR-67, and TR-87) were subjected to ligation reaction with aminoacyl pCpA (Compound ts14 synthesized by a method described in Patent Literature (WO2018143145A1)) by the method described above, phenol-chloroform extraction, and ethanol precipitation, to prepare elongator aminoacyl tRNAs (AAtR-127, AAtR-131, and AAtR-135). These aminoacyl tRNAs were dissolved in 1 mM aqueous sodium acetate solution before addition to a translation mixture.

AAtR-1 SPh2Cl-tRNA(Glu2+Ser5)aag

GUCCCCUUCGUCUAGAGGCCCAGGACACCGCCCUaagAAGG
CGGUAACAGGGGUUCGAAUCCCCUAGGGGACGC-CA

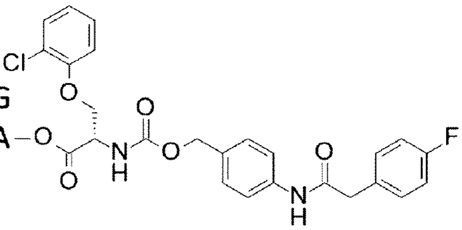

AAtR-2 SPh2Cl-tRNA(Glu2+Ala1B)aag

GUCCCCUUCGUCUAGAGGCCCAGGACACCGCCUUaagACGG
CGGUAACAGGGGUUCGAAUCCCCUAGGGGACGC-CA

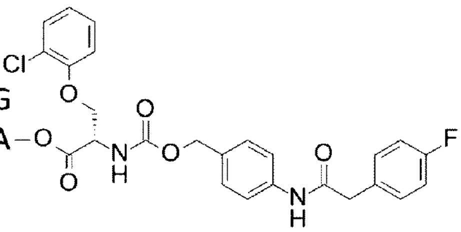

AAtR-3 SPh2Cl-tRNA(Glu2+Phe)aag

GUCCCCUUCGUCUAGAGGCCCAGGACACCGCCUUaagAAGG
CGGUAACAGGGGUUCGAAUCCCCUAGGGGACGC-CA

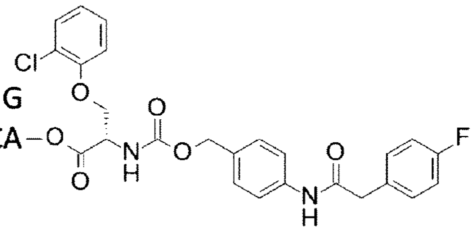

AAtR-4 SPh2Cl-tRNA(Glu2)aag

GUCCCCUUCGUCUAGAGGCCCAGGACACCGCCCUaagACGG
CGGUAACAGGGGUUCGAAUCCCCUAGGGGACGC-CA

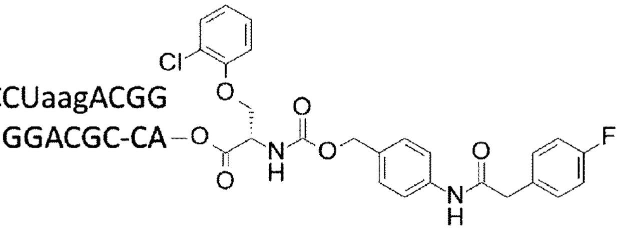

AAtR-5 SPh2Cl-tRNA(Glu2+Arg3)aag

GUCCCCUUCGUCUAGAGGCCCAGGACACCGCCCUaagGAGG
CGGUAACAGGGGUUCGAAUCCCCUAGGGGACGC-CA

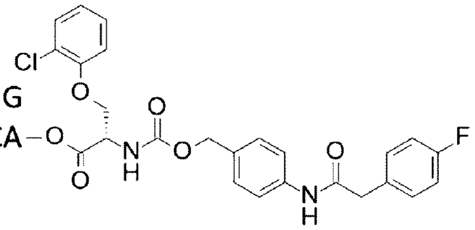

AAtR-6 SPh2Cl-tRNA(Glu2+Val2)aag

GUCCCCUUCGUCUAGAGGCCCAGGACACCGCCUUaagAUGG
CGGUAACAGGGGUUCGAAUCCCCUAGGGGACGC-CA

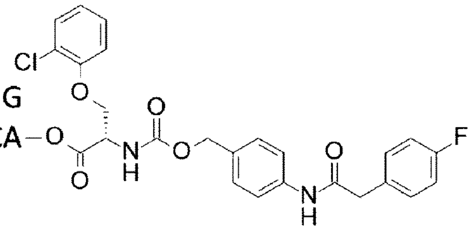

AAtR-7 SPh2Cl-tRNA(Glu2+Leu2)aag

GUCCCCUUCGUCUAGAGGCCCAGGACACCGCCUUaagGUGG
CGGUAACAGGGGUUCGAAUCCCCUAGGGGACGC-CA

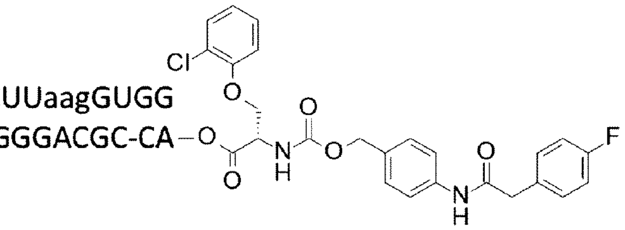

AAtR-8 SPh2Cl-tRNA(Glu2+Pro3)aag

GUCCCCUUCGUCUAGAGGCCCAGGACACCGCCUUaagGAGG
CGGUAACAGGGGUUCGAAUCCCCUAGGGGACGC-CA

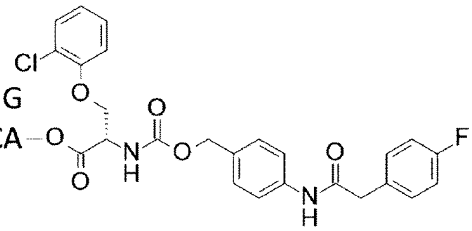

AAtR-9 SPh2Cl-tRNA(Glu2+Pro2)aag

GUCCCCUUCGUCUAGAGGCCCAGGACACCGCCAUaagGUGG
CGGUAACAGGGGUUCGAAUCCCCUAGGGGACGC-CA

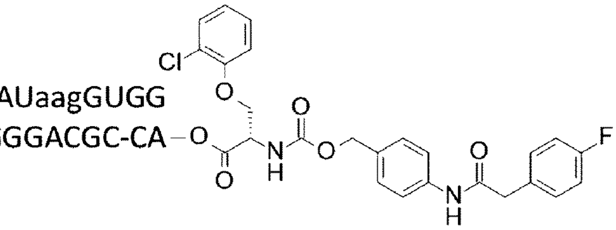

AAtR-10 SPh2Cl-tRNA(Glu2+Ala2)aag

GUCCCCUUCGUCUAGAGGCCCAGGACACCGCCAUaagAUGG
CGGUAACAGGGGUUCGAAUCCCCUAGGGGACGC-CA

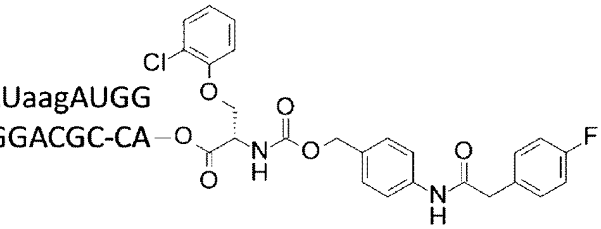

AAtR-11 SPh2Cl-tRNA(Glu2+Ser5)cag

GUCCCCUUCGUCUAGAGGCCCAGGACACCGCCCUcagAAGG
CGGUAACAGGGGUUCGAAUCCCCUAGGGGACGC-CA

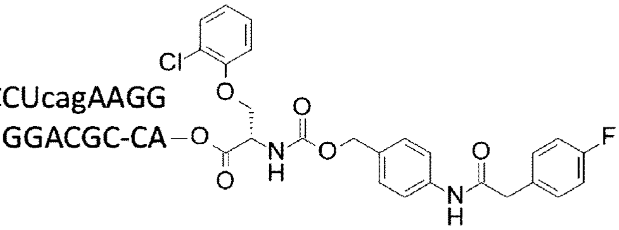

AAtR-12 SPh2Cl-tRNA(Glu2+Ala1B)cag

GUCCCCUUCGUCUAGAGGCCCAGGACACCGCCUUcagACGG
CGGUAACAGGGGUUCGAAUCCCCUAGGGGACGC-CA

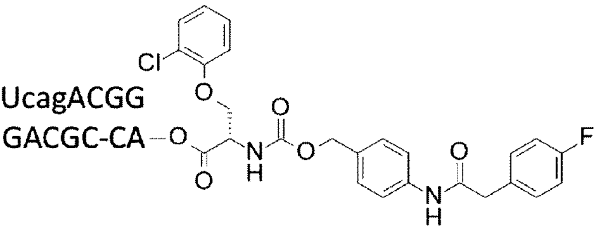

AAtR-13 SPh2Cl-tRNA(Glu2+Phe)cag

GUCCCCUUCGUCUAGAGGCCCAGGACACCGCCUUcagAAGG
CGGUAACAGGGGUUCGAAUCCCCUAGGGGACGC-CA

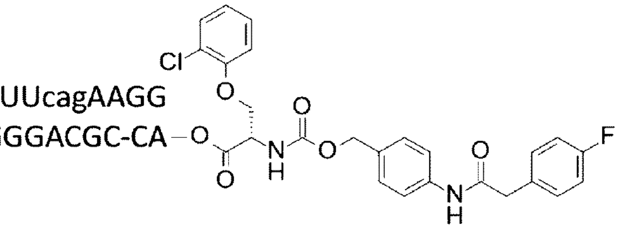

AAtR-14 SPh2Cl-tRNA(Glu2)cag

GUCCCCUUCGUCUAGAGGCCCAGGACACCGCCCUcagACGG
CGGUAACAGGGGUUCGAAUCCCCUAGGGGACGC-CA

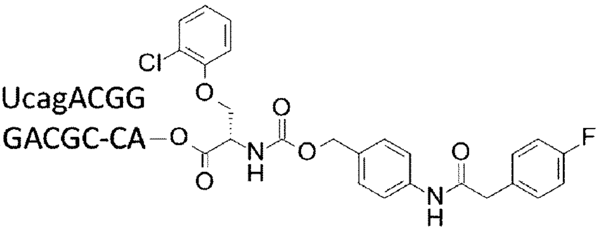

AAtR-15 SPh2Cl-tRNA(Glu2+Arg3)cag

GUCCCCUUCGUCUAGAGGCCCAGGACACCGCCCUcagGAGG
CGGUAACAGGGGUUCGAAUCCCCUAGGGGACGC-CA

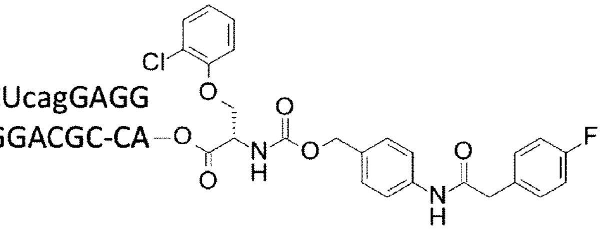

AAtR-16 SPh2Cl-tRNA(Glu2+Val2)cag

GUCCCCUUCGUCUAGAGGCCCAGGACACCGCCUUcagAUGG
CGGUAACAGGGGUUCGAAUCCCCUAGGGGACGC-CA

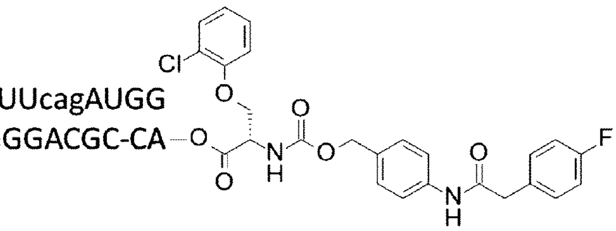

AAtR-17 SPh2Cl-tRNA(Glu2+Leu2)cag

GUCCCCUUCGUCUAGAGGCCCAGGACACCGCCUUcagGUGG
CGGUAACAGGGGUUCGAAUCCCCUAGGGGACGC-CA

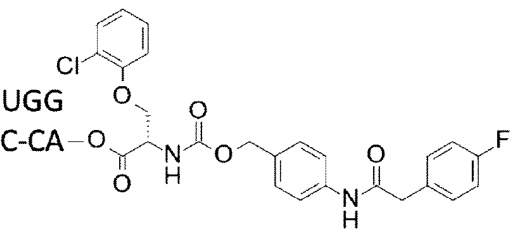

AAtR-18 SPh2Cl-tRNA(Glu2+Pro3)cag

GUCCCCUUCGUCUAGAGGCCCAGGACACCGCCUUcagGAGG
CGGUAACAGGGGUUCGAAUCCCCUAGGGGACGC-CA

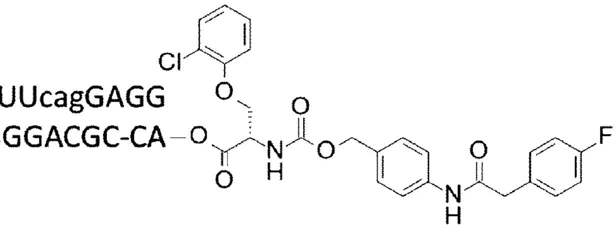

AAtR-19 SPh2Cl-tRNA(Glu2+Pro2)cag

GUCCCCUUCGUCUAGAGGCCCAGGACACCGCCAUcagGUGG
CGGUAACAGGGGUUCGAAUCCCCUAGGGGACGC-CA

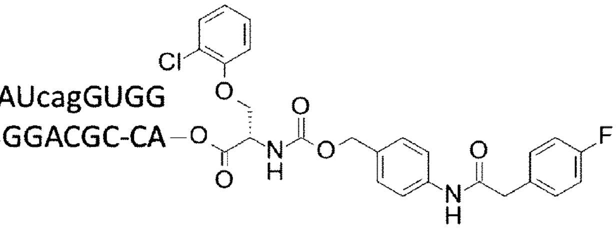

AAtR-20 SPh2Cl-tRNA(Glu2+Ala2)cag

GUCCCCUUCGUCUAGAGGCCCAGGACACCGCCAUcagAUGG
CGGUAACAGGGGUUCGAAUCCCCUAGGGGACGC-CA

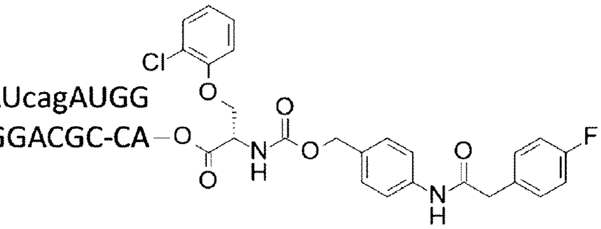

AAtR-21 SPh2Cl-tRNA(Glu2+Ser5)agg

GUCCCCUUCGUCUAGAGGCCCAGGACACCGCCCUaggAAGG
CGGUAACAGGGGUUCGAAUCCCCUAGGGGACGC-CA

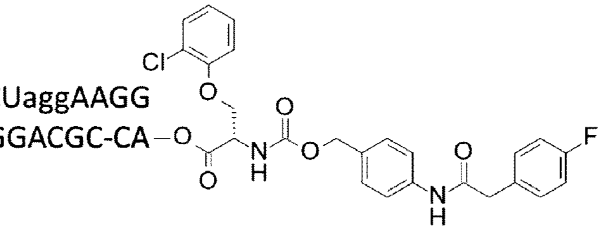

AAtR-22 SPh2Cl-tRNA(Glu2+AlaIB)agg

GUCCCCUUCGUCUAGAGGCCCAGGACACCGCCUUaggACGG
CGGUAACAGGGGUUCGAAUCCCCUAGGGGACGC-CA

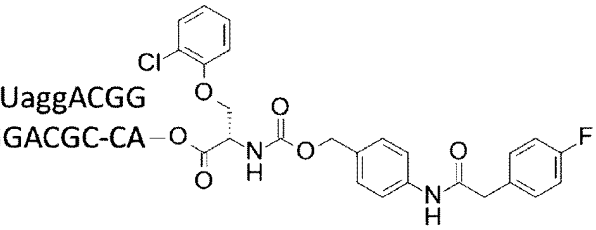

AAtR-23 SPh2Cl-tRNA(Glu2+Phe)agg

GUCCCCUUCGUCUAGAGGCCCAGGACACCGCCUUaggAAGG
CGGUAACAGGGGUUCGAAUCCCCUAGGGGACGC-CA

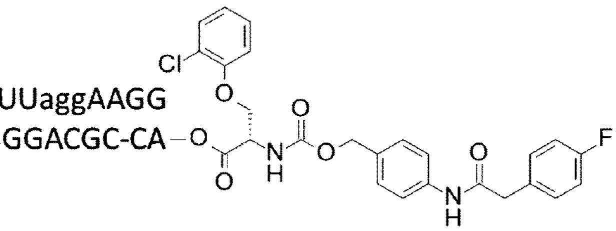

AAtR-24 SPh2Cl-tRNA(Glu2)agg

GUCCCCUUCGUCUAGAGGCCCAGGACACCGCCCUaggACGG
CGGUAACAGGGGUUCGAAUCCCCUAGGGGACGC-CA

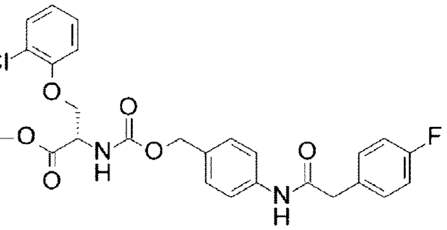

AAtR-25 SPh2Cl-tRNA(Glu2+Arg3)agg

GUCCCCUUCGUCUAGAGGCCCAGGACACCGCCCUaggGAGG
CGGUAACAGGGGUUCGAAUCCCCUAGGGGACGC-CA

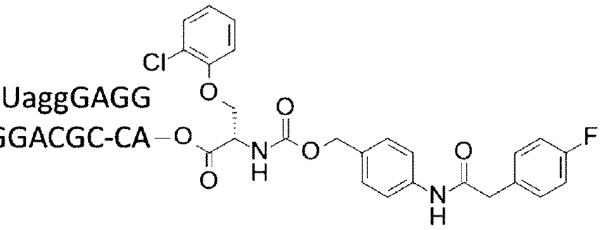

AAtR-26 SPh2Cl-tRNA(Glu2+Val2)agg

GUCCCCUUCGUCUAGAGGCCCAGGACACCGCCUUaggAUGG
CGGUAACAGGGGUUCGAAUCCCCUAGGGGACGC-CA

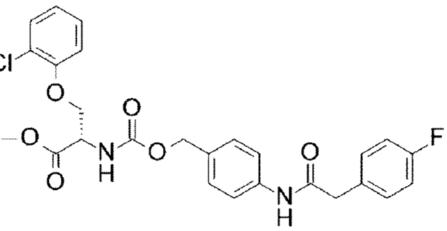

AAtR-27 SPh2Cl-tRNA(Glu2+Leu2)agg

GUCCCCUUCGUCUAGAGGCCCAGGACACCGCCUUaggGUGG
CGGUAACAGGGGUUCGAAUCCCCUAGGGGACGC-CA

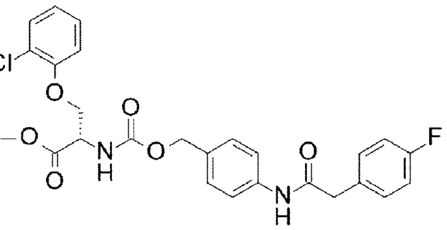

AAtR-28 SPh2Cl-tRNA(Glu2+Pro3)agg

GUCCCCUUCGUCUAGAGGCCCAGGACACCGCCUUaggGAGG
CGGUAACAGGGGUUCGAAUCCCCUAGGGGACGC-CA

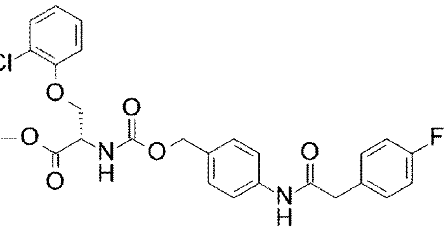

AAtR-29 SPh2Cl-tRNA(Glu2+Pro2)agg

GUCCCCUUCGUCUAGAGGCCCAGGACACCGCCAUaggGUGG
CGGUAACAGGGGUUCGAAUCCCCUAGGGGACGC-CA

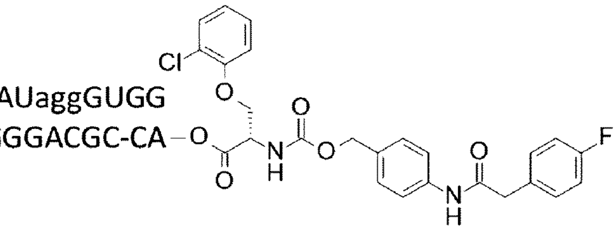

AAtR-30 SPh2Cl-tRNA(Glu2+Ala2)agg

GUCCCCUUCGUCUAGAGGCCCAGGACACCGCCAUaggAUGG
CGGUAACAGGGGUUCGAAUCCCCUAGGGGACGC-CA

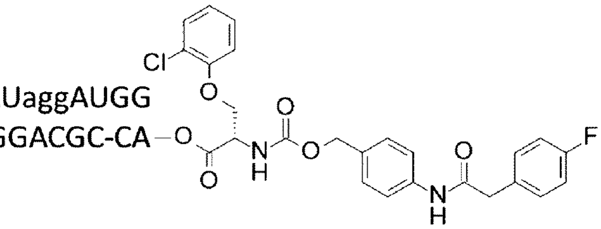

AAtR-31 SPh2Cl-tRNA(Glu2+Ser5)cgg

GUCCCCUUCGUCUAGAGGCCCAGGACACCGCCCUcggAAGG
CGGUAACAGGGGUUCGAAUCCCCUAGGGGACGC-CA

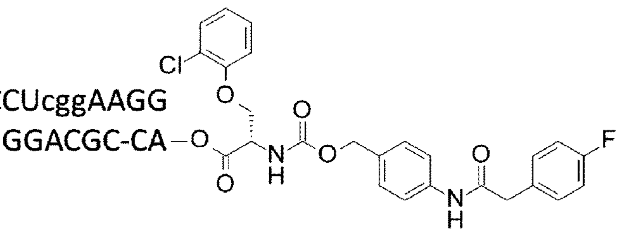

AAtR-32 SPh2Cl-tRNA(Glu2+AlaIB)cgg

GUCCCCUUCGUCUAGAGGCCCAGGACACCGCCUUcggACGG
CGGUAACAGGGGUUCGAAUCCCCUAGGGGACGC-CA

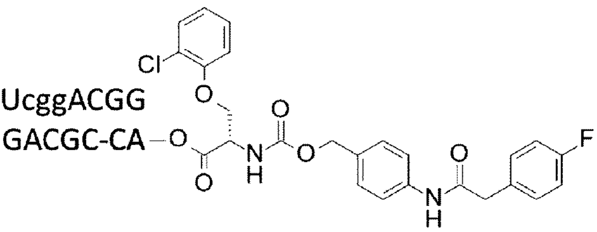

AAtR-33 SPh2Cl-tRNA(Glu2+Phe)cgg

GUCCCCUUCGUCUAGAGGCCCAGGACACCGCCUUcggAAGG
CGGUAACAGGGGUUCGAAUCCCCUAGGGGACGC-CA

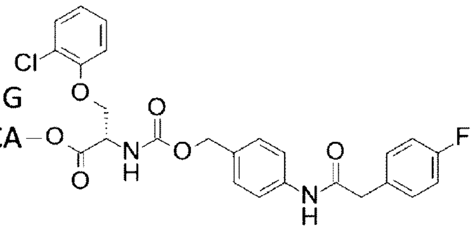

AAtR-34 SPh2Cl-tRNA(Glu2)cgg

GUCCCCUUCGUCUAGAGGCCCAGGACACCGCCCUcggACGG
CGGUAACAGGGGUUCGAAUCCCCUAGGGGACGC-CA

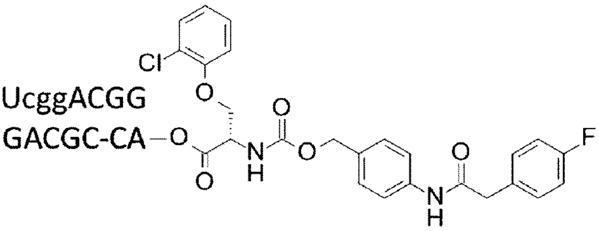

AAtR-35 SPh2Cl-tRNA(Glu2+Arg3)cgg

GUCCCCUUCGUCUAGAGGCCCAGGACACCGCCCUcggGAGG
CGGUAACAGGGGUUCGAAUCCCCUAGGGGACGC-CA

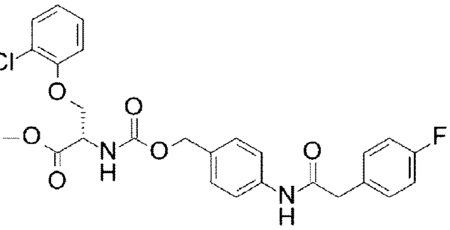

AAtR-36 SPh2Cl-tRNA(Glu2+Val2)cgg

GUCCCCUUCGUCUAGAGGCCCAGGACACCGCCUUcggAUGG
CGGUAACAGGGGUUCGAAUCCCCUAGGGGACGC-CA

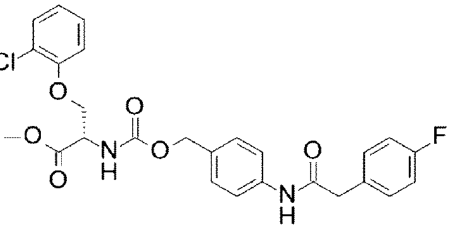

AAtR-37 SPh2Cl-tRNA(Glu2+Leu2)cgg

GUCCCCUUCGUCUAGAGGCCCAGGACACCGCCUUcggGUGG
CGGUAACAGGGGUUCGAAUCCCCUAGGGGACGC-CA

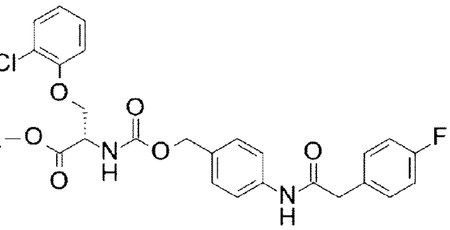

AAtR-38 SPh2Cl-tRNA(Glu2+Pro3)cgg

GUCCCCUUCGUCUAGAGGCCCAGGACACCGCCUUcggGAGG
CGGUAACAGGGGUUCGAAUCCCCUAGGGGACGC-CA

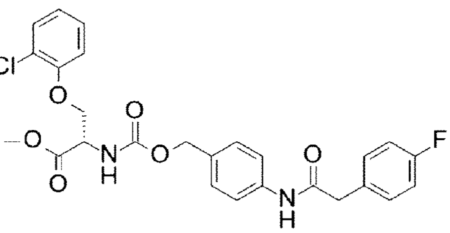

AAtR-39 SPh2Cl-tRNA(Glu2+Pro2)cgg

GUCCCCUUCGUCUAGAGGCCCAGGACACCGCCAUcggGUGG
CGGUAACAGGGGUUCGAAUCCCCUAGGGGACGC-CA

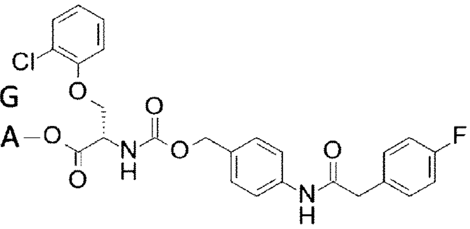

AAtR-40 SPh2Cl-tRNA(Glu2+Ala2)cgg

GUCCCCUUCGUCUAGAGGCCCAGGACACCGCCAUcggAUGG
CGGUAACAGGGGUUCGAAUCCCCUAGGGGACGC-CA

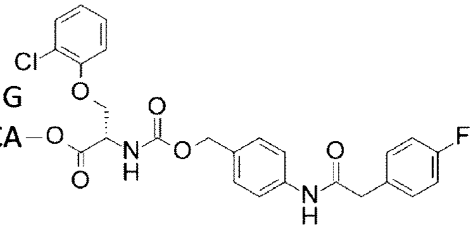

AAtR-41 SPh2Cl-tRNA(Glu2+Ser5)acc

GUCCCCUUCGUCUAGAGGCCCAGGACACCGCCCUaccAAGG
CGGUAACAGGGGUUCGAAUCCCCUAGGGGACGC-CA

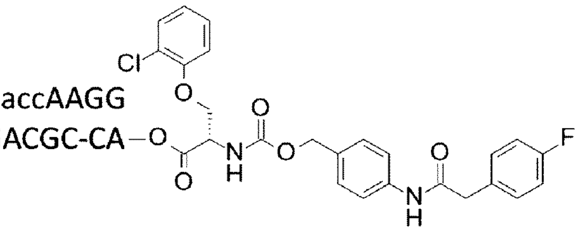

AAtR-42 SPh2Cl-tRNA(Glu2+Ala1B)acc

GUCCCCUUCGUCUAGAGGCCCAGGACACCGCCUUaccACGG
CGGUAACAGGGGUUCGAAUCCCCUAGGGGACGC-CA

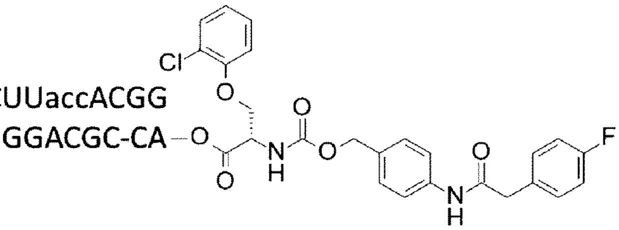

AAtR-43 SPh2Cl-tRNA(Glu2+Phe)acc

GUCCCCUUCGUCUAGAGGCCCAGGACACCGCCUUaccAAGG
CGGUAACAGGGGUUCGAAUCCCCUAGGGGACGC-CA

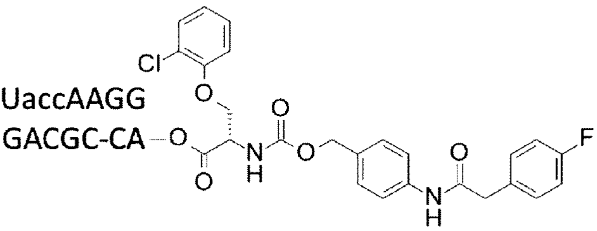

AAtR-44 SPh2Cl-tRNA(Glu2)acc

GUCCCCUUCGUCUAGAGGCCCAGGACACCGCCCUaccACGG
CGGUAACAGGGGUUCGAAUCCCCUAGGGGACGC-CA

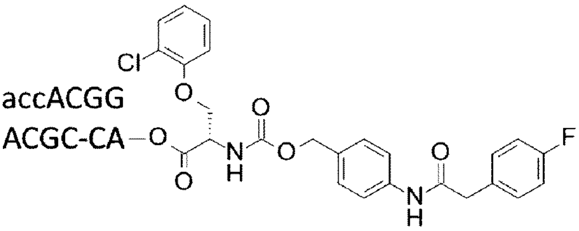

AAtR-45 SPh2Cl-tRNA(Glu2+Arg3)acc

GUCCCCUUCGUCUAGAGGCCCAGGACACCGCCCUaccGAGG
CGGUAACAGGGGUUCGAAUCCCCUAGGGGACGC-CA

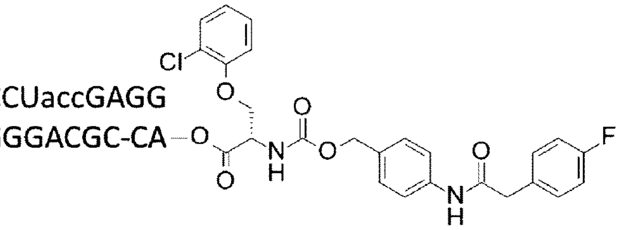

AAtR-46 SPh2Cl-tRNA(Glu2+Val2)acc

GUCCCCUUCGUCUAGAGGCCCAGGACACCGCCUUaccAUGG
CGGUAACAGGGGUUCGAAUCCCCUAGGGGACGC-CA

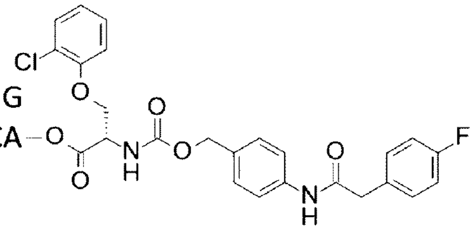

AAtR-47 SPh2Cl-tRNA(Glu2+Leu2)acc

GUCCCCUUCGUCUAGAGGCCCAGGACACCGCCUUaccGUGG
CGGUAACAGGGGUUCGAAUCCCCUAGGGGACGC-CA

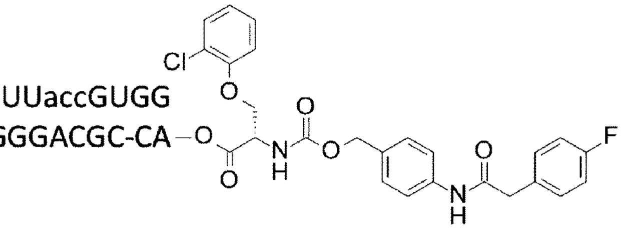

AAtR-48 SPh2Cl-tRNA(Glu2+Pro3)acc

GUCCCCUUCGUCUAGAGGCCCAGGACACCGCCUUaccGAGG
CGGUAACAGGGGUUCGAAUCCCCUAGGGGACGC-CA

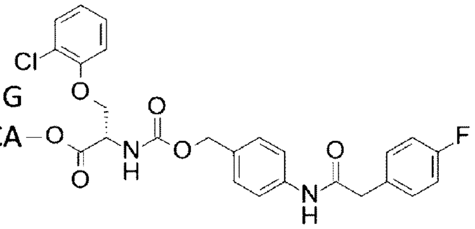

AAtR-49 SPh2Cl-tRNA(Glu2+Pro2)acc

GUCCCCUUCGUCUAGAGGCCCAGGACACCGCCAUaccGUGG
CGGUAACAGGGGUUCGAAUCCCCUAGGGGACGC-CA

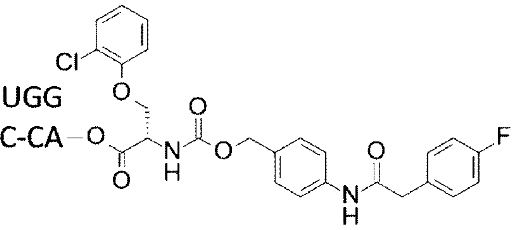

AAtR-50 SPh2Cl-tRNA(Glu2+Ala2)acc

GUCCCCUUCGUCUAGAGGCCCAGGACACCGCCAUaccAUGG
CGGUAACAGGGGUUCGAAUCCCCUAGGGGACGC-CA

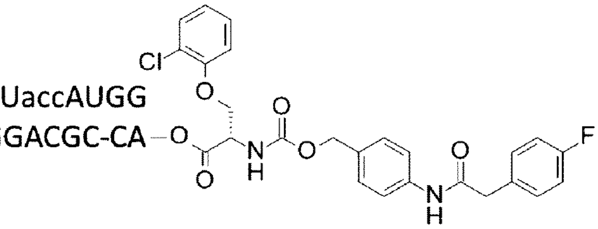

AAtR-51 SPh2Cl-tRNA(Glu2+Ser5)ccc

GUCCCCUUCGUCUAGAGGCCCAGGACACCGCCCUcccAAGGC
GGUAACAGGGGUUCGAAUCCCCUAGGGGACGC-CA

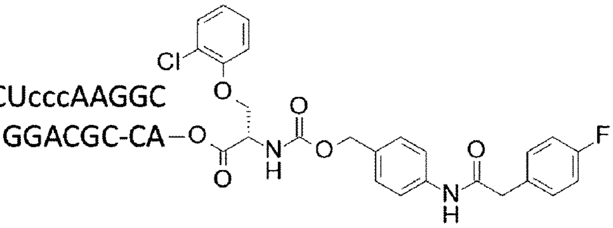

AAtR-52 SPh2Cl-tRNA(Glu2+Ala1B)ccc

GUCCCCUUCGUCUAGAGGCCCAGGACACCGCCUUcccACGG
CGGUAACAGGGGUUCGAAUCCCCUAGGGGACGC-CA

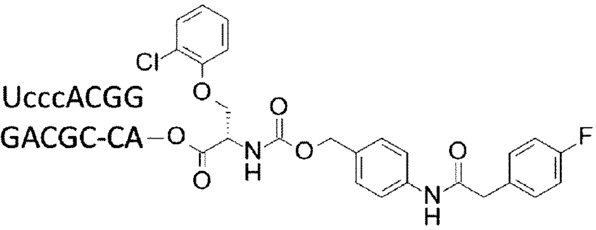

AAtR-53 SPh2Cl-tRNA(Glu2+Phe)ccc

GUCCCCUUCGUCUAGAGGCCCAGGACACCGCCUUcccAAGG
CGGUAACAGGGGUUCGAAUCCCCUAGGGGACGC-CA

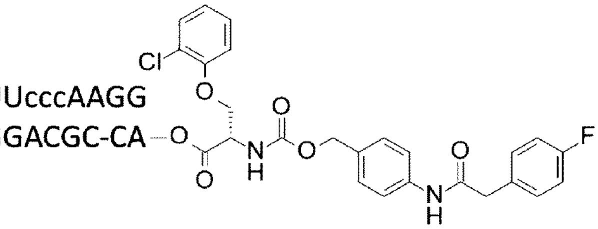

AAtR-54 SPh2Cl-tRNA(Glu2)ccc

GUCCCCUUCGUCUAGAGGCCCAGGACACCGCCCUcccACGGC
GGUAACAGGGGUUCGAAUCCCCUAGGGGACGC-CA

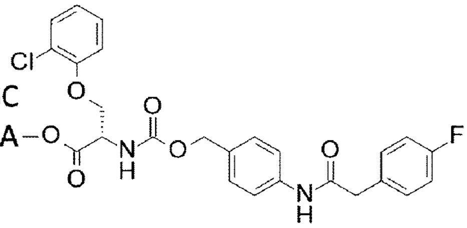

AAtR-55 SPh2Cl-tRNA(Glu2+Arg3)ccc

GUCCCCUUCGUCUAGAGGCCCAGGACACCGCCCUcccGAGG
CGGUAACAGGGGUUCGAAUCCCCUAGGGGACGC-CA

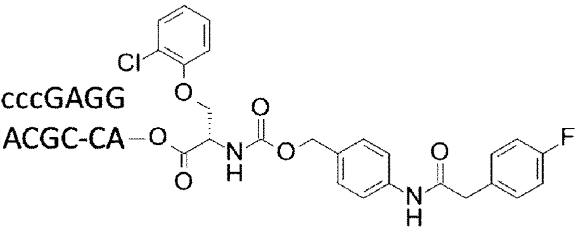

AAtR-56 SPh2Cl-tRNA(Glu2+Val2)ccc

GUCCCCUUCGUCUAGAGGCCCAGGACACCGCCUUcccAUGG
CGGUAACAGGGGUUCGAAUCCCCUAGGGGACGC-CA

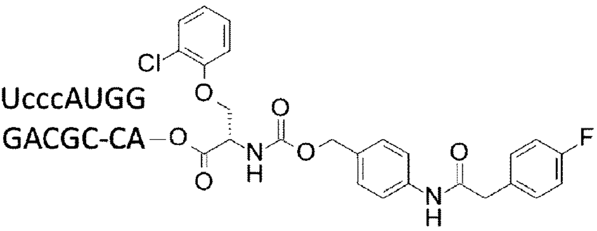

AAtR-57 SPh2Cl-tRNA(Glu2+Leu2)ccc

GUCCCCUUCGUCUAGAGGCCCAGGACACCGCCUUcccGUGG
CGGUAACAGGGGUUCGAAUCCCCUAGGGGACGC-CA

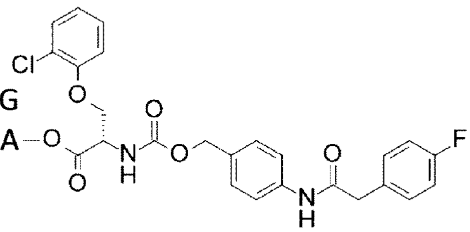

AAtR-58 SPh2Cl-tRNA(Glu2+Pro3)ccc

GUCCCCUUCGUCUAGAGGCCCAGGACACCGCCUUcccGAGG
CGGUAACAGGGGUUCGAAUCCCCUAGGGGACGC-CA

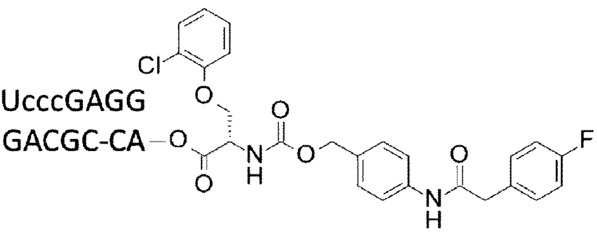

AAtR-59 SPh2Cl-tRNA(Glu2+Pro2)ccc

GUCCCCUUCGUCUAGAGGCCCAGGACACCGCCAUcccGUGG
CGGUAACAGGGGUUCGAAUCCCCUAGGGGACGC-CA

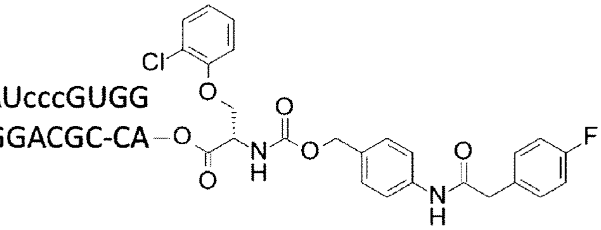

AAtR-60 SPh2Cl-tRNA(Glu2+Ala2)ccc

GUCCCCUUCGUCUAGAGGCCCAGGACACCGCCAUcccAUGG
CGGUAACAGGGGUUCGAAUCCCCUAGGGGACGC-CA

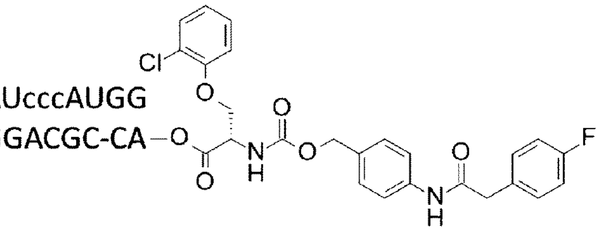

AAtR-61 SPh2Cl-tRNA(Aspl+Ser5)aag

GGAGCGGUAGUUCAGUCGGUUAGAAUACCUGCCUaagAAG
CAGGGGGUCGCGGGUUCGAGUCCCGUCCGUUCCGC-CA

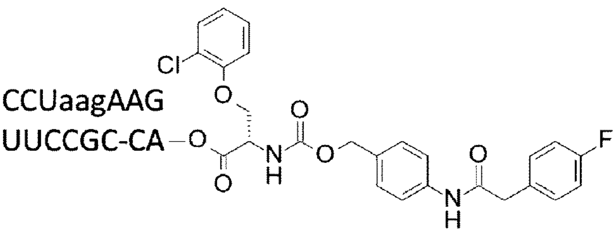

AAtR-62 SPh2Cl-tRNA(Aspl+Ala1B)aag

GGAGCGGUAGUUCAGUCGGUUAGAAUACCUGCUUaagACG
CAGGGGGUCGCGGGUUCGAGUCCCGUCCGUUCCGC-CA

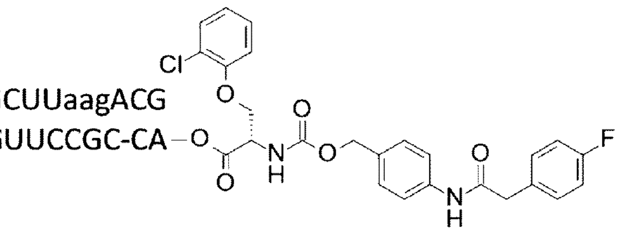

AAtR-63 SPh2Cl-tRNA(Aspl+Phe)aag

GGAGCGGUAGUUCAGUCGGUUAGAAUACCUGCUUaagAAG
CAGGGGGUCGCGGGUUCGAGUCCCGUCCGUUCCGC-CA

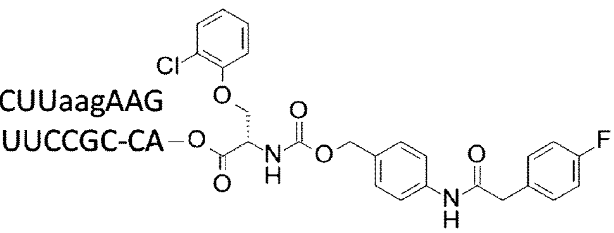

AAtR-64 SPh2Cl-tRNA(Asp1)aag

GGAGCGGUAGUUCAGUCGGUUAGAAUACCUGCCUaagACG
CAGGGGGUCGCGGGUUCGAGUCCCGUCCGUUCCGC-CA

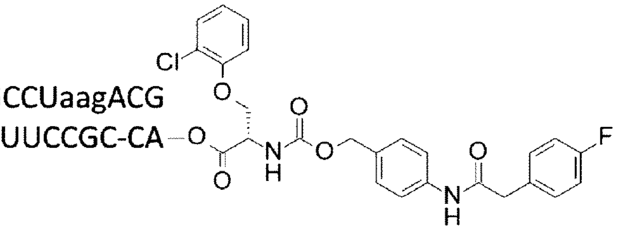

AAtR-65 SPh2Cl-tRNA(Asp1+Arg3)aag

GGAGCGGUAGUUCAGUCGGUUAGAAUACCUGCCUaagGAG
CAGGGGGUCGCGGGUUCGAGUCCCGUCCGUUCCGC-CA

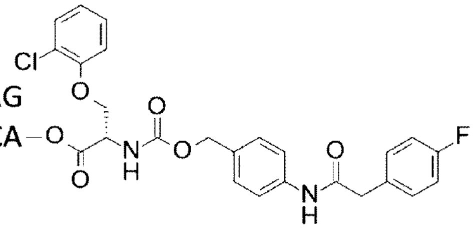

AAtR-66 SPh2Cl-tRNA(Asp1+Val2)aag

GGAGCGGUAGUUCAGUCGGUUAGAAUACCUGCUUaagAUG
CAGGGGGUCGCGGGUUCGAGUCCCGUCCGUUCCGC-CA

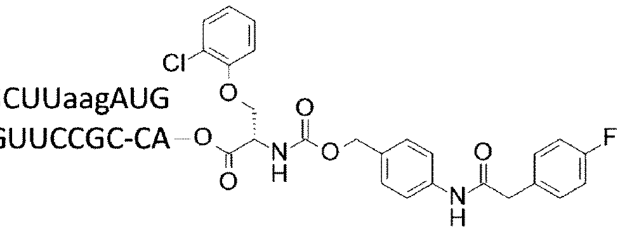

AAtR-67 SPh2Cl-tRNA(Asp1+Leu2)aag

GGAGCGGUAGUUCAGUCGGUUAGAAUACCUGCUUaagGUG
CAGGGGGUCGCGGGUUCGAGUCCCGUCCGUUCCGC-CA

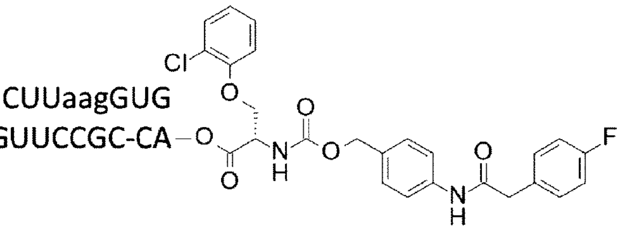

AAtR-68 SPh2Cl-tRNA(Asp1+Pro3)aag

GGAGCGGUAGUUCAGUCGGUUAGAAUACCUGCUUaagGAG
CAGGGGGUCGCGGGUUCGAGUCCCGUCCGUUCCGC-CA

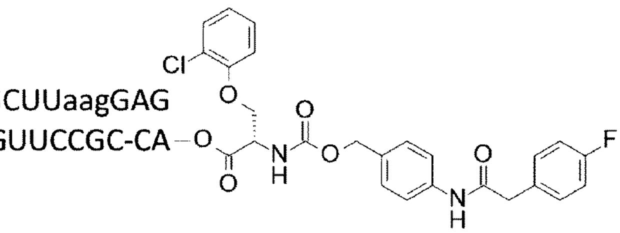

AAtR-69 SPh2Cl-tRNA(Aspl+Pro2)aag

GGAGCGGUAGUUCAGUCGGUUAGAAUACCUGCAUaagGUG
CAGGGGGUCGCGGGUUCGAGUCCCGUCCGUUCCGC-CA

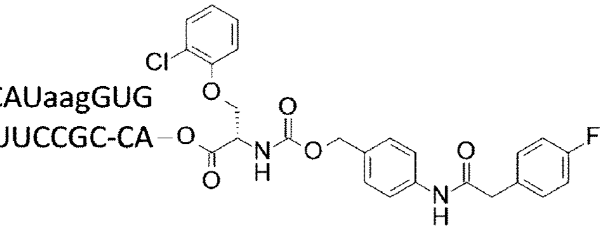

AAtR-70 SPh2Cl-tRNA(Asp1+Ala2)aag

GGAGCGGUAGUUCAGUCGGUUAGAAUACCUGCAUaagAUG
CAGGGGGUCGCGGGUUCGAGUCCCGUCCGUUCCGC-CA

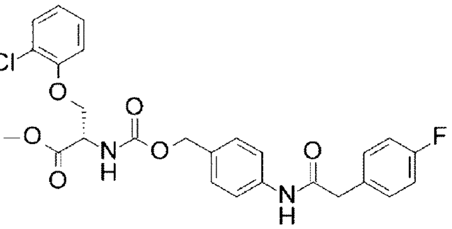

AAtR-71 SPh2Cl-tRNA(Asp1+Ser5)cag

GGAGCGGUAGUUCAGUCGGUUAGAAUACCUGCCUcagAAG
CAGGGGGUCGCGGGUUCGAGUCCCGUCCGUUCCGC-CA

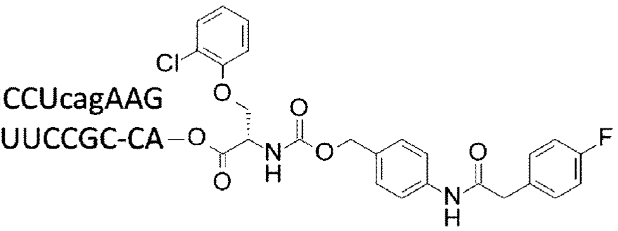

AAtR-72 SPh2Cl-tRNA(Asp1+Ala1B)cag

GGAGCGGUAGUUCAGUCGGUUAGAAUACCUGCUUcagACG
CAGGGGGUCGCGGGUUCGAGUCCCGUCCGUUCCGC-CA

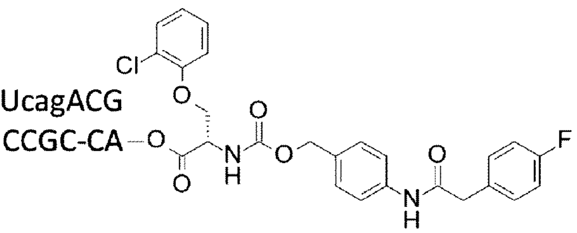

AAtR-73 SPh2Cl-tRNA(Asp1+Phe)cag

GGAGCGGUAGUUCAGUCGGUUAGAAUACCUGCUUcagAAG
CAGGGGGUCGCGGGUUCGAGUCCCGUCCGUUCCGC-CA

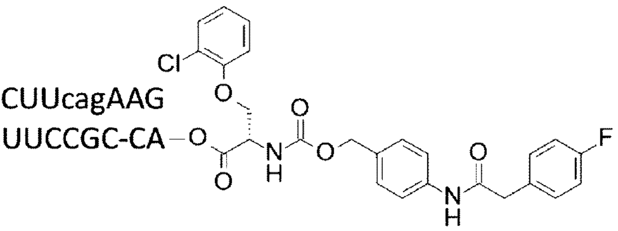

AAtR-74 SPh2Cl-tRNA(Asp1)cag

GGAGCGGUAGUUCAGUCGGUUAGAAUACCUGCCUcagACG
CAGGGGGUCGCGGGUUCGAGUCCCGUCCGUUCCGC-CA

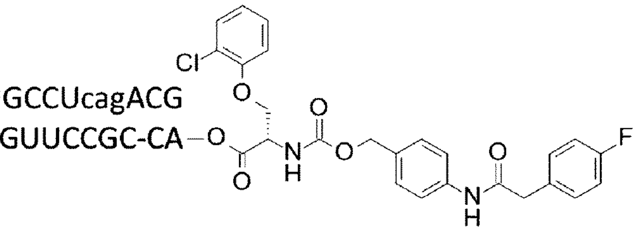

AAtR-75 SPh2Cl-tRNA(Aspl+Arg3)cag

GGAGCGGUAGUUCAGUCGGUUAGAAUACCUGCCUcagGAG
CAGGGGGUCGCGGGUUCGAGUCCCGUCCGUUCCGC-CA

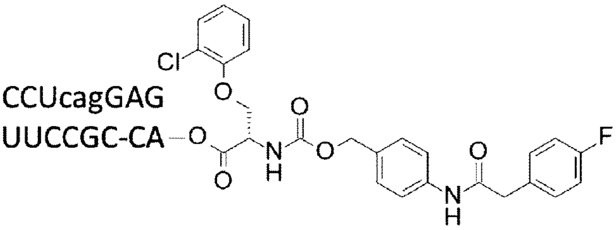

AAtR-76 SPh2Cl-tRNA(Asp1+Val2)cag

GGAGCGGUAGUUCAGUCGGUUAGAAUACCUGCUUcagAUG
CAGGGGGUCGCGGGUUCGAGUCCCGUCCGUUCCGC-CA

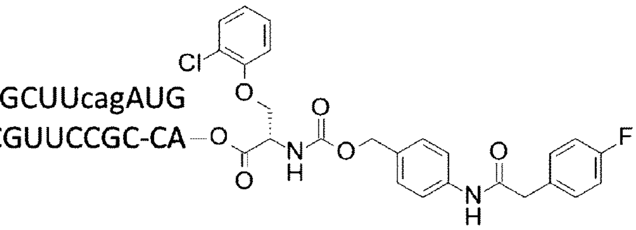

AAtR-77 SPh2Cl-tRNA(Asp1+Leu2)cag

GGAGCGGUAGUUCAGUCGGUUAGAAUACCUGCUUcagGUG
CAGGGGGUCGCGGGUUCGAGUCCCGUCCGUUCCGC-CA

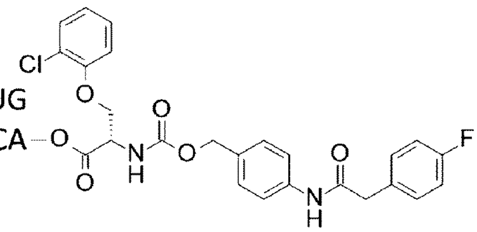

AAtR-78 SPh2Cl-tRNA(Asp1+Pro3)cag

GGAGCGGUAGUUCAGUCGGUUAGAAUACCUGCUUcagGAG
CAGGGGGUCGCGGGUUCGAGUCCCGUCCGUUCCGC-CA

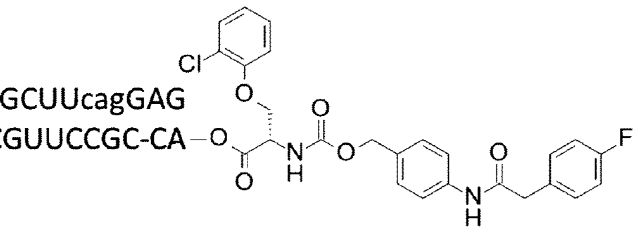

AAtR-79 SPh2Cl-tRNA(Asp1+Pro2)cag

GGAGCGGUAGUUCAGUCGGUUAGAAUACCUGCAUcagGUG
CAGGGGGUCGCGGGUUCGAGUCCCGUCCGUUCCGC-CA

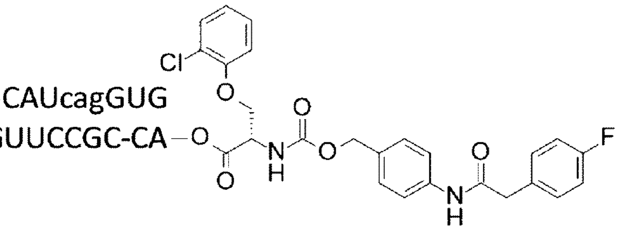

AAtR-80 SPh2Cl-tRNA(Asp1+Ala2)cag

GGAGCGGUAGUUCAGUCGGUUAGAAUACCUGCAUcagAUG
CAGGGGGUCGCGGGUUCGAGUCCCGUCCGUUCCGC-CA

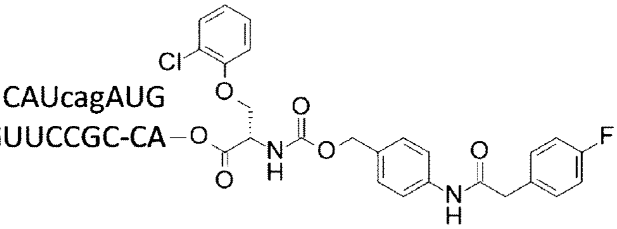

AAtR-81 SPh2Cl-tRNA(AsnE2)aag

GGCUCUGUAGUUCAGUCGGUAGAACGGCGGACUaagAAUC
CGUAUGUCACUGGUUCGAGUCCAGUCAGAGCCGC-CA

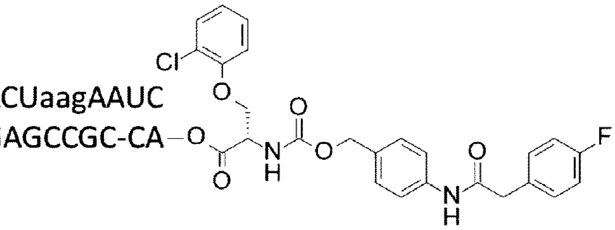

AAtR-82 SPh2Cl-tRNA(AsnE2+Ala1B)aag

GGCUCUGUAGUUCAGUCGGUAGAACGGCGGAUUaagACUC
CGUAUGUCACUGGUUCGAGUCCAGUCAGAGCCGC-CA

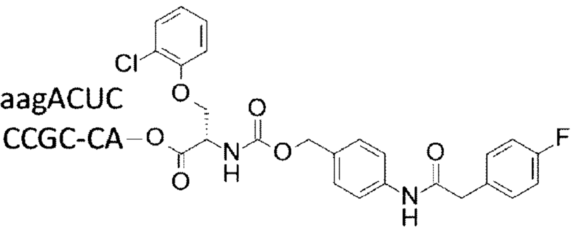

AAtR-83 SPh2Cl-tRNA(AsnE2+Phe)aag

GGCUCUGUAGUUCAGUCGGUAGAACGGCGGAUUaagAAUC
CGUAUGUCACUGGUUCGAGUCCAGUCAGAGCCGC-CA

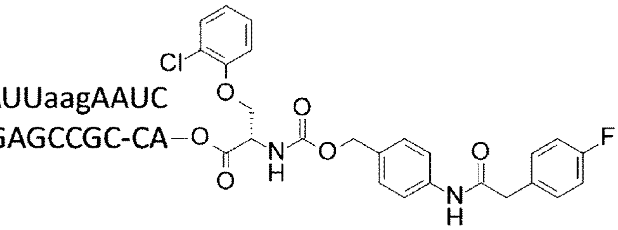

AAtR-84 SPh2Cl-tRNA(AsnE2+Glu2)aag

GGCUCUGUAGUUCAGUCGGUAGAACGGCGGACUaagACUC
CGUAUGUCACUGGUUCGAGUCCAGUCAGAGCCGC-CA

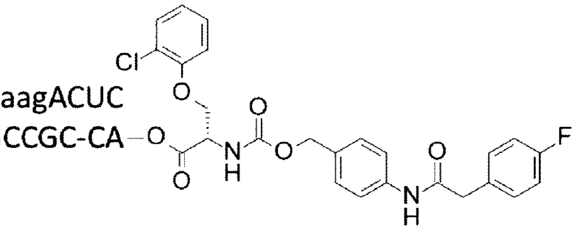

AAtR-85 SPh2Cl-tRNA(AsnE2+Arg3)aag

GGCUCUGUAGUUCAGUCGGUAGAACGGCGGACUaagGAUC
CGUAUGUCACUGGUUCGAGUCCAGUCAGAGCCGC-CA

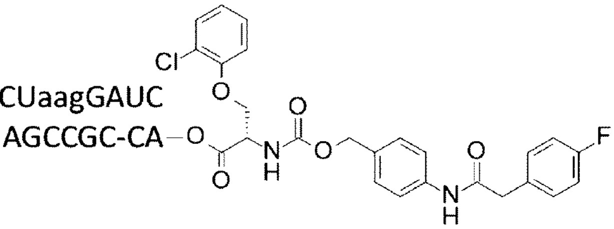

AAtR-86 SPh2Cl-tRNA(AsnE2+Val2)aag

GGCUCUGUAGUUCAGUCGGUAGAACGGCGGAUUaagAUUC
CGUAUGUCACUGGUUCGAGUCCAGUCAGAGCCGC-CA

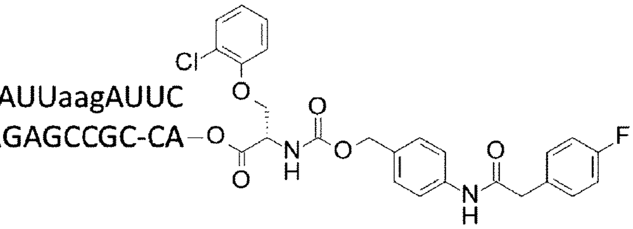

AAtR-87 SPh2Cl-tRNA(AsnE2+Leu2)aag

GGCUCUGUAGUUCAGUCGGUAGAACGGCGGAUUaagGUUC
CGUAUGUCACUGGUUCGAGUCCAGUCAGAGCCGC-CA

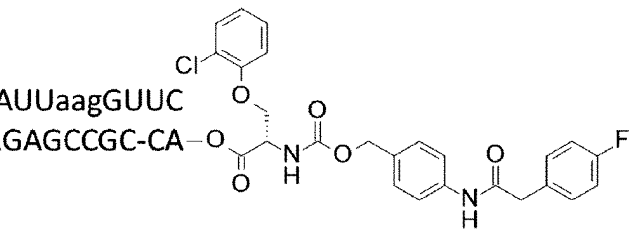

AAtR-88 SPh2Cl-tRNA(AsnE2+Pro3)aag

GGCUCUGUAGUUCAGUCGGUAGAACGGCGGAUUaagGAUC
CGUAUGUCACUGGUUCGAGUCCAGUCAGAGCCGC-CA

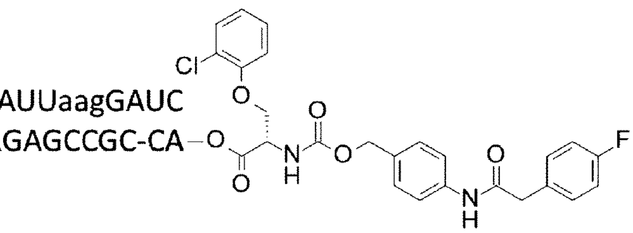

AAtR-89 SPh2Cl-tRNA(AsnE2+Pro2)aag

GGCUCUGUAGUUCAGUCGGUAGAACGGCGGAAUaagGUUC
CGUAUGUCACUGGUUCGAGUCCAGUCAGAGCCGC-CA

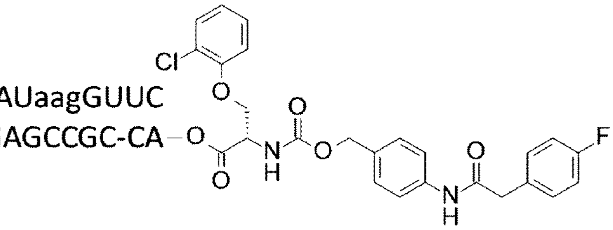

AAtR-90 SPh2Cl-tRNA(AsnE2+Ala2)aag

GGCUCUGUAGUUCAGUCGGUAGAACGGCGGAAUaagAUUC
CGUAUGUCACUGGUUCGAGUCCAGUCAGAGCCGC-CA

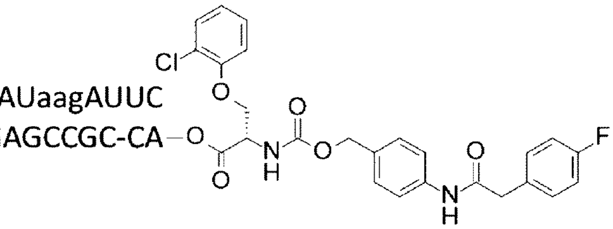

AAtR-91 SPh2Cl-tRNA(AsnE2)cag

GGCUCUGUAGUUCAGUCGGUAGAACGGCGGACUcagAAUC
CGUAUGUCACUGGUUCGAGUCCAGUCAGAGCCGC-CA

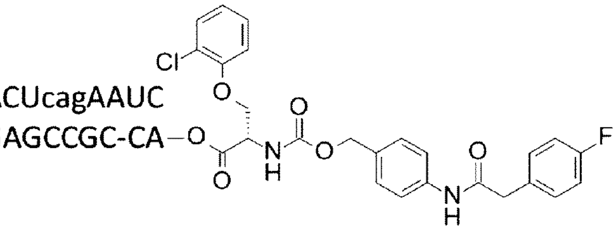

AAtR-92 SPh2Cl-tRNA(AsnE2+Ala1B)cag

GGCUCUGUAGUUCAGUCGGUAGAACGGCGGAUUcagACUC
CGUAUGUCACUGGUUCGAGUCCAGUCAGAGCCGC-CA

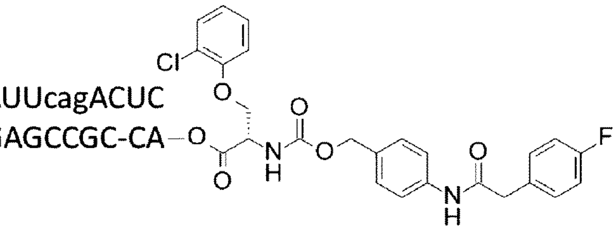

AAtR-93 SPh2Cl-tRNA(AsnE2+Phe)cag

GGCUCUGUAGUUCAGUCGGUAGAACGGCGGAUUcagAAUC
CGUAUGUCACUGGUUCGAGUCCAGUCAGAGCCGC-CA

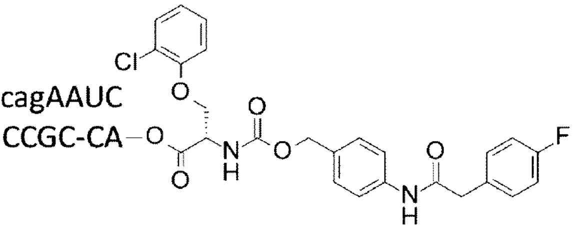

AAtR-94 SPh2Cl-tRNA(AsnE2+Glu2)cag

GGCUCUGUAGUUCAGUCGGUAGAACGGCGGACUcagACUC
CGUAUGUCACUGGUUCGAGUCCAGUCAGAGCCGC-CA

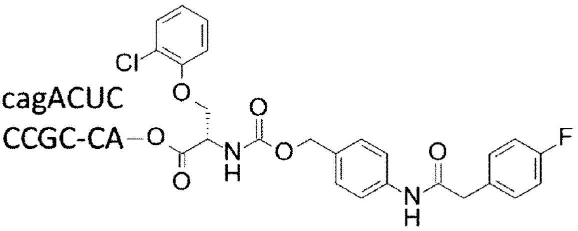

AAtR-95 SPh2Cl-tRNA(AsnE2+Arg3)cag

GGCUCUGUAGUUCAGUCGGUAGAACGGCGGACUcagGAUC
CGUAUGUCACUGGUUCGAGUCCAGUCAGAGCCGC-CA

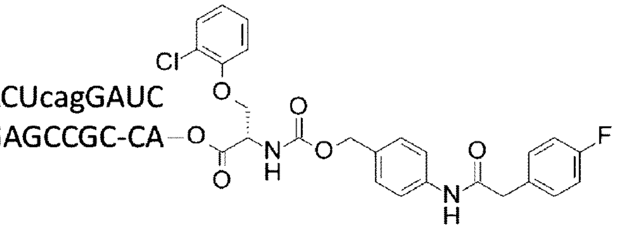

AAtR-96 SPh2Cl-tRNA(AsnE2+Val2)cag

GGCUCUGUAGUUCAGUCGGUAGAACGGCGGAUUcagAUUC
CGUAUGUCACUGGUUCGAGUCCAGUCAGAGCCGC-CA

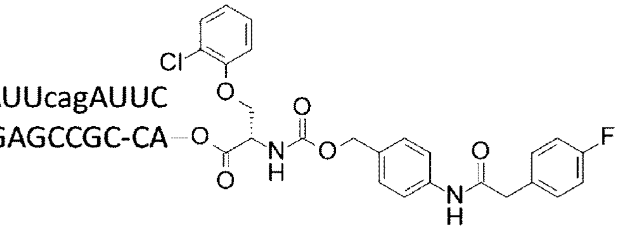

AAtR-97 SPh2Cl-tRNA(AsnE2+Leu2)cag

GGCUCUGUAGUUCAGUCGGUAGAACGGCGGAUUcagGUUC
CGUAUGUCACUGGUUCGAGUCCAGUCAGAGCCGC-CA

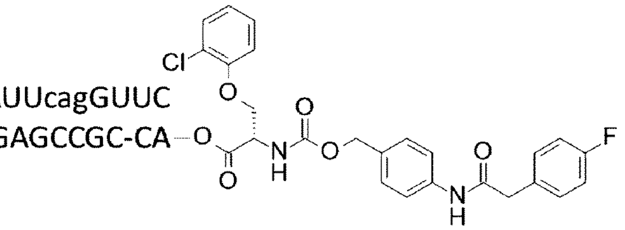

AAtR-98 SPh2Cl-tRNA(AsnE2+Pro3)cag

GGCUCUGUAGUUCAGUCGGUAGAACGGCGGAUUcagGAUC
CGUAUGUCACUGGUUCGAGUCCAGUCAGAGCCGC-CA

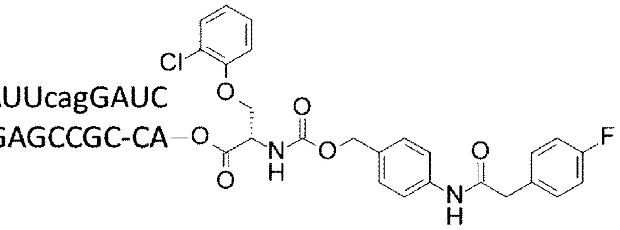

AAtR-99 SPh2Cl-tRNA(AsnE2+Pro2)cag

GGCUCUGUAGUUCAGUCGGUAGAACGGCGGAAUcagGUUC
CGUAUGUCACUGGUUCGAGUCCAGUCAGAGCCGC-CA

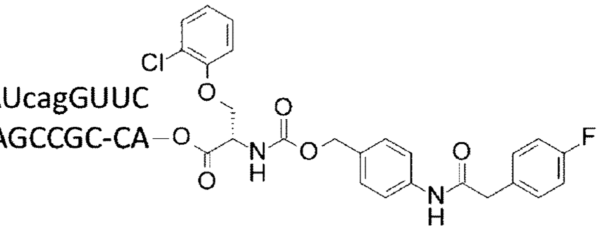

AAtR-100 SPh2Cl-tRNA(AsnE2+Ala2)cag

GGCUCUGUAGUUCAGUCGGUAGAACGGCGGAAUcagAUUC
CGUAUGUCACUGGUUCGAGUCCAGUCAGAGCCGC-CA

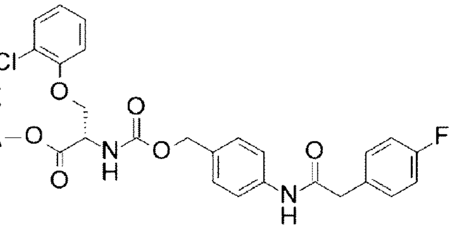

AAtR-101 SPh2Cl-tRNA(Glu2)gcc

GUCCCCUUCGUCUAGAGGCCCAGGACACCGCCCUgccACGG
CGGUAACAGGGGUUCGAAUCCCCUAGGGGACGC-CA

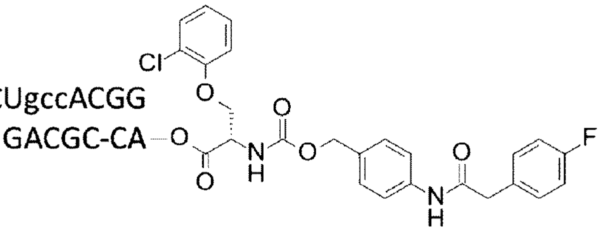

AAtR-102 SPh2Cl-tRNA(Glu2+Leu2)gcc

GUCCCCUUCGUCUAGAGGCCCAGGACACCGCCUUgccGUGG
CGGUAACAGGGGUUCGAAUCCCCUAGGGGACGC-CA

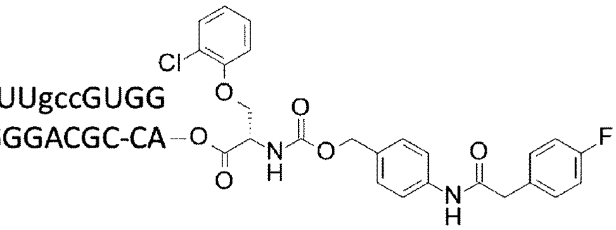

AAtR-103 SPh2Cl-tRNA(Glu2+Pro3)gcc

GUCCCCUUCGUCUAGAGGCCCAGGACACCGCCUUgccGAGG
CGGUAACAGGGGUUCGAAUCCCCUAGGGGACGC-CA

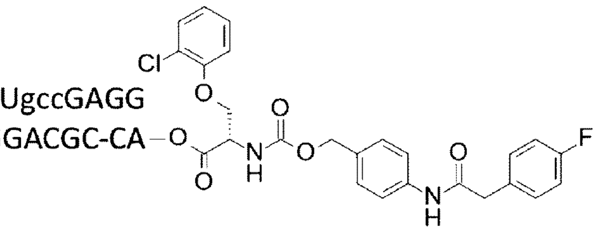

AAtR-104 MeHph-tRNA(Glu2)aag

GUCCCCUUCGUCUAGAGGCCCAGGACACCGCCCUaagACGG
CGGUAACAGGGGUUCGAAUCCCCUAGGGGACGC-CA

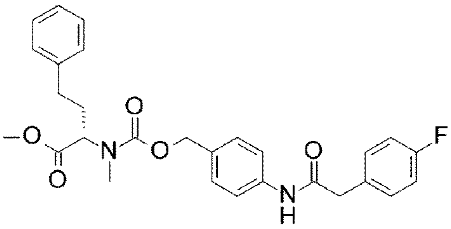

AAtR-105 MeHph-tRNA(Glu2)cag

GUCCCCUUCGUCUAGAGGCCCAGGACACCGCCCUcagACGG
CGGUAACAGGGGUUCGAAUCCCCUAGGGGACGC-CA

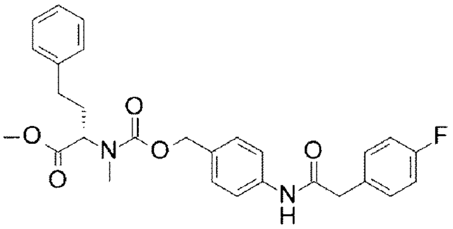

AAtR-106 MeHph-tRNA(Glu2)agg

GUCCCCUUCGUCUAGAGGCCCAGGACACCGCCCUaggACGG
CGGUAACAGGGGUUCGAAUCCCCUAGGGGACGC-CA

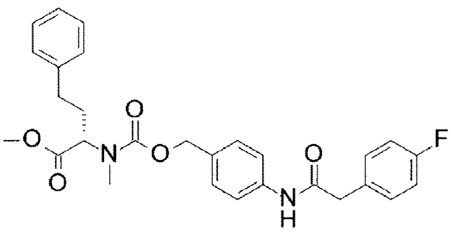

AAtR-107 MeHph-tRNA(Glu2)cgg

GUCCCCUUCGUCUAGAGGCCCAGGACACCGCCCUcggACGG
CGGUAACAGGGGUUCGAAUCCCCUAGGGGACGC-CA

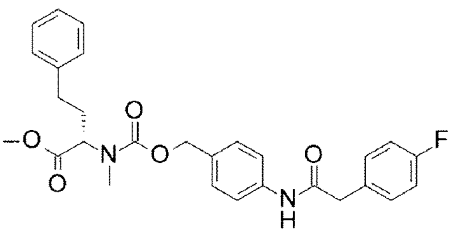

AAtR-108 MeHph-tRNA(Glu2)acc

GUCCCCUUCGUCUAGAGGCCCAGGACACCGCCCUaccACGG
CGGUAACAGGGGUUCGAAUCCCCUAGGGGACGC-CA

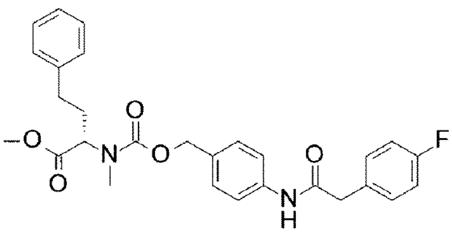

AAtR-109 MeHph-tRNA(Glu2)ccc

GUCCCCUUCGUCUAGAGGCCCAGGACACCGCCCUcccACGG
CGGUAACAGGGGUUCGAAUCCCCUAGGGGACGC-CA 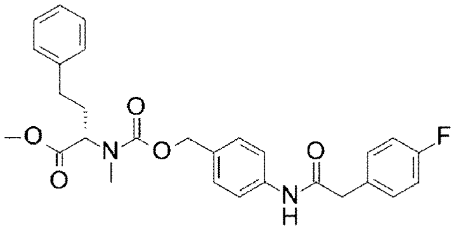

AAtR-110 MeHph-tRNA(Asp1)aag

GGAGCGGUAGUUCAGUCGGUUAGAAUACCUGCCUaagACG
CAGGGGGUCGCGGGUUCGAGUCCCGUCCGUUCCGC-CA 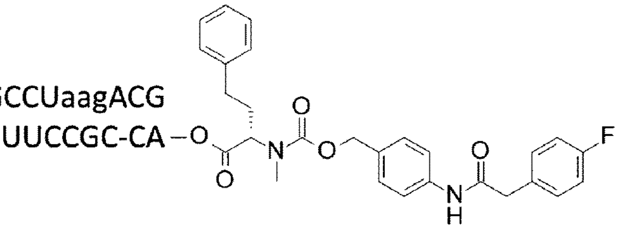

AAtR-111 MeHph-tRNA(Asp1)cag

GGAGCGGUAGUUCAGUCGGUUAGAAUACCUGCCUcagACG
CAGGGGGUCGCGGGUUCGAGUCCCGUCCGUUCCGC-CA 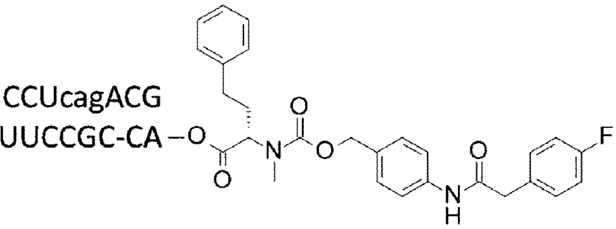

AAtR-112 MeHph-tRNA(AsnE2)aag

GGCUCUGUAGUUCAGUCGGUAGAACGGCGGACUaagAAUC
CGUAUGUCACUGGUUCGAGUCCAGUCAGAGCCGC-CA 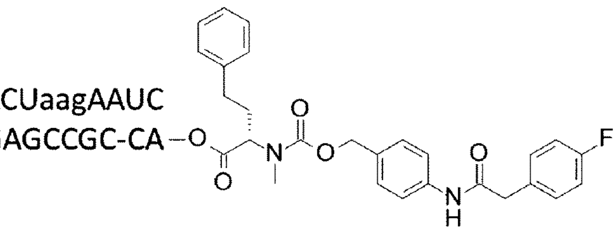

AAtR-113 MeHph-tRNA(AsnE2)cag

GGCUCUGUAGUUCAGUCGGUAGAACGGCGGACUcagAAUC
CGUAUGUCACUGGUUCGAGUCCAGUCAGAGCCGC-CA 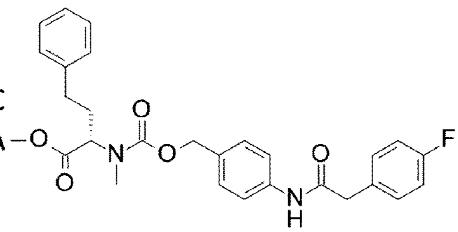

AAtR-114 MeHph-tRNA(Glu2)ucc

GUCCCCUUCGUCUAGAGGCCCAGGACACCGCCCUuccACGG
CGGUAACAGGGGUUCGAAUCCCCUAGGGGACGC-CA

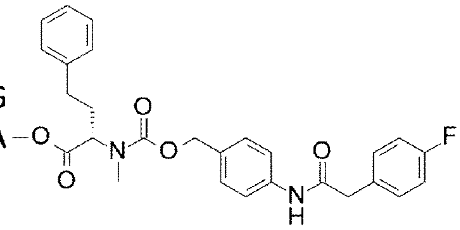

AAtR-115 Pic2- tRNA(Glu2)acc

GUCCCCUUCGUCUAGAGGCCCAGGACACCGCCCUaccACGG
CGGUAACAGGGGUUCGAAUCCCCUAGGGGACGC-CA

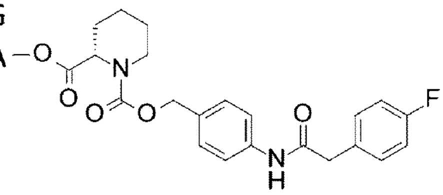

AAtR-116 Pic2- tRNA(Glu2+Leu2)acc

GUCCCCUUCGUCUAGAGGCCCAGGACACCGCCUUaccGUGG
CGGUAACAGGGGUUCGAAUCCCCUAGGGGACGC-CA

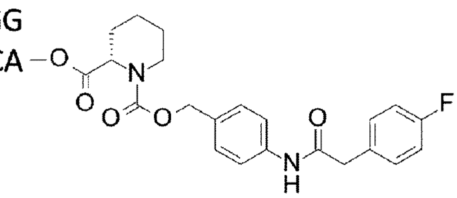

AAtR-117 Pic2- tRNA(Glu2+Pro3)acc

GUCCCCUUCGUCUAGAGGCCCAGGACACCGCCUUaccGAGG
CGGUAACAGGGGUUCGAAUCCCCUAGGGGACGC-CA

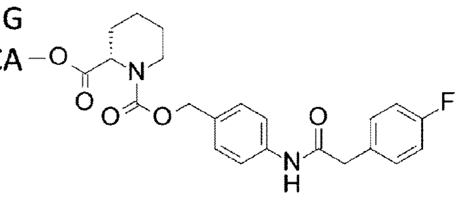

AAtR-118 Pic2- tRNA(Glu2+Ala2)acc

GUCCCCUUCGUCUAGAGGCCCAGGACACCGCCAUaccAUGG
CGGUAACAGGGGUUCGAAUCCCCUAGGGGACGC-CA

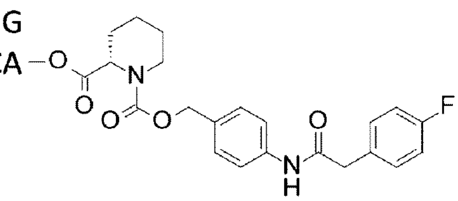

AAtR-119 MeG- tRNA(Glu2)acc

GUCCCCUUCGUCUAGAGGCCCAGGACACCGCCCUaccACGG
CGGUAACAGGGGUUCGAAUCCCCUAGGGGACGC-CA

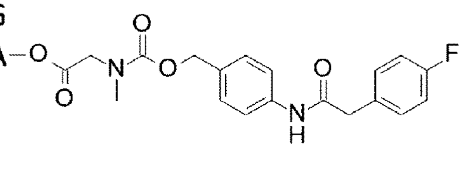

AAtR-120 MeG- tRNA(Glu2+Leu2)acc

GUCCCCUUCGUCUAGAGGCCCAGGACACCGCCUUaccGUGG
CGGUAACAGGGGUUCGAAUCCCCUAGGGGACGC-CA

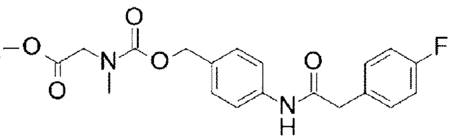

AAtR-121 MeG- tRNA(Glu2+Pro3)acc

GUCCCCUUCGUCUAGAGGCCCAGGACACCGCCUUaccGAGG
CGGUAACAGGGGUUCGAAUCCCCUAGGGGACGC-CA

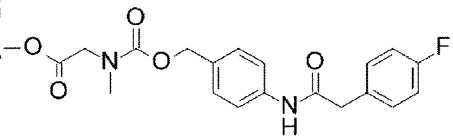

AAtR-122 MeG- tRNA(Glu2+Ala2)acc

GUCCCCUUCGUCUAGAGGCCCAGGACACCGCCAUaccAUGG
CGGUAACAGGGGUUCGAAUCCCCUAGGGGACGC-CA

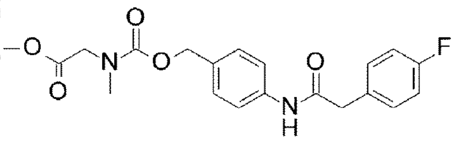

AAtR-123 dA- tRNA(Glu2)acc

GUCCCCUUCGUCUAGAGGCCCAGGACACCGCCCUaccACGG
CGGUAACAGGGGUUCGAAUCCCCUAGGGGACGC-CA

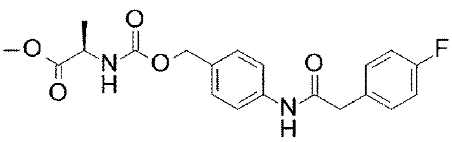

AAtR-124 dA- tRNA(Glu2+Leu2)acc

GUCCCCUUCGUCUAGAGGCCCAGGACACCGCCUUaccGUGG
CGGUAACAGGGGUUCGAAUCCCCUAGGGGACGC-CA

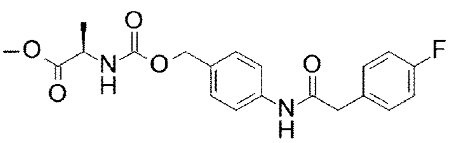

AAtR-125 dA- tRNA(Glu2+Pro3)acc

GUCCCCUUCGUCUAGAGGCCCAGGACACCGCCUUaccGAGG
CGGUAACAGGGGUUCGAAUCCCCUAGGGGACGC-CA

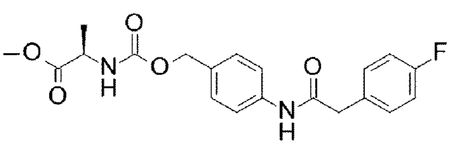

AAtR-126 dA- tRNA(Glu2+Ala2)acc

GUCCCCUUCGUCUAGAGGCCCAGGACACCGCCAUaccAUGG
CGGUAACAGGGGUUCGAAUCCCCUAGGGGACGC-CA

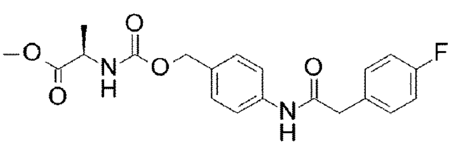

AAtR-127 nBuG- tRNA(Glu2)ccc

GUCCCCUUCGUCUAGAGGCCCAGGACACCGCCCUcccACGG
CGGUAACAGGGGUUCGAAUCCCCUAGGGGACGC-CA

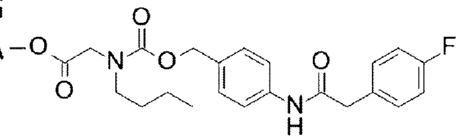

AAtR-129 MeG- tRNA(Glu2)agg

GUCCCCUUCGUCUAGAGGCCCAGGACACCGCCCUaggACGG
CGGUAACAGGGGUUCGAAUCCCCUAGGGGACGC-CA

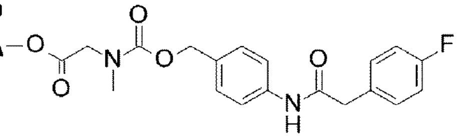

AAtR-130 MeG- tRNA(Glu2)cgg

GUCCCCUUCGUCUAGAGGCCCAGGACACCGCCCUcggACGG
CGGUAACAGGGGUUCGAAUCCCCUAGGGGACGC-CA

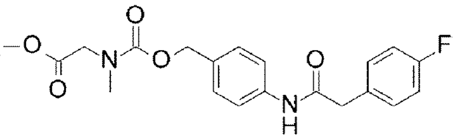

AAtR-131 nBuG-tRNA(Asp1+Leu2)aag

GGAGCGGUAGUUCAGUCGGUUAGAAUACCUGCUUAAGGUGC
AGGGGGUCGCGGGUUCGAGUCCCGUCCGUUCCGC-CA

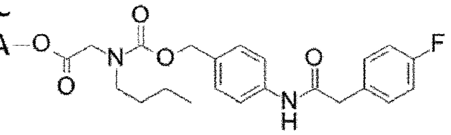

AAtR-132 SPh2Cl-tRNA(Asp1+Leu2)uag

GGAGCGGUAGUUCAGUCGGUUAGAAUACCUGCUUUAGGUGC
AGGGGGUCGCGGGUUCGAGUCCCGUCCGUUCCGC-CA

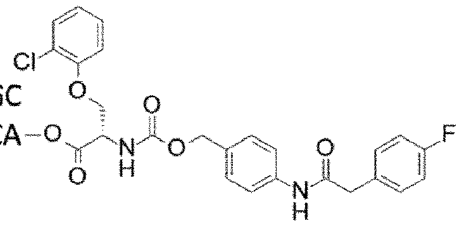

AAtR-133 SPh2Cl-tRNA(Asp1+Leu2)Lag

GGAGCGGUAGUUCAGUCGGUUAGAAUACCUGCUULAGGUGC
AGGGGGUCGCGGGUUCGAGUCCCGUCCGUUCCGC-CA

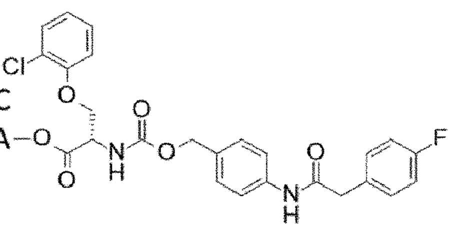

AAtR-134 dA-tRNA(Asp1+Leu2)cag

GGAGCGGUAGUUCAGUCGGUUAGAAUACCUGCUUCAGGUGC
AGGGGGUCGCGGGUUCGAGUCCCGUCCGUUCCGC-CA

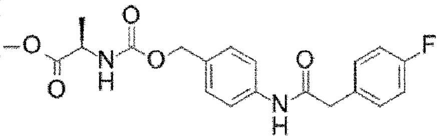

AAtR-135 nBuG-tRNA(AsnE2+Leu2)aag

GGCUCUGUAGUUCAGUCGGUAGAACGGCGGAUUAAGGUUC
CGUAUGUCACUGGUUCGAGUCCAGUCAGAGCCGC-CA

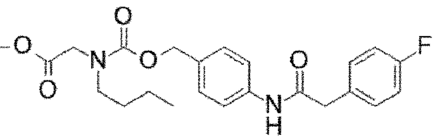

AAtR-136 SPh2Cl-tRNA(AsnE2+Leu2)uag

GGCUCUGUAGUUCAGUCGGUAGAACGGCGGAUUUAGGUUC
CGUAUGUCACUGGUUCGAGUCCAGUCAGAGCCGC-CA

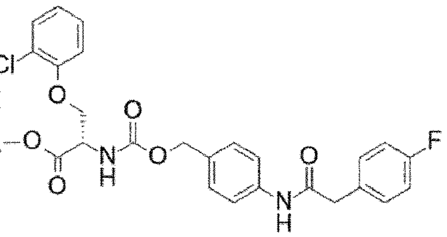

AAtR-137 SPh2Cl-tRNA(AsnE2+Leu2)Lag

GGCUCUGUAGUUCAGUCGGUAGAACGGCGGAUULAGGUUC
CGUAUGUCACUGGUUCGAGUCCAGUCAGAGCCGC-CA

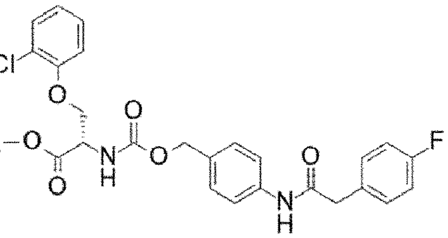

AAtR-138 dA-tRNA(AsnE2+Leu2)cag

GGCUCUGUAGUUCAGUCGGUAGAACGGCGGAUUCAGGUUC
CGUAUGUCACUGGUUCGAGUCCAGUCAGAGCCGC-CA

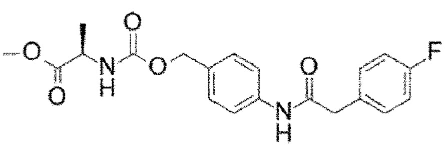

Preparation of initiator aminoacyl tRNA using aminoacyl pCpA

A reaction solution was prepared by adding nuclease-free water to adjust the solution to 25 μM transcribed tRNA(fMet)cau-CA (TR-105), 50 mM HEPES-KOH pH7.5, 20 mM $MgCl_2$, 1 mM ATP, 0.6 unit/μL T4 RNA ligase (New England Biolabs), and 0.25 mM aminoacyl pCpA (MT01), and ligation reaction was performed at 15°C for 45 minutes. Before adding T4 RNA ligase and aminoacyl pCpA, the reaction solution was heated to 95°C for 2 minutes and then allowed to stand at room temperature for 5 minutes to refold the tRNA in advance.

To the ligation reaction solution, sodium acetate was added to make a concentration of 0.3 M, and phenol-chloroform extraction was performed to prepare the initiator aminoacyl tRNA (AAtR-128). AAtR-128 was recovered by ethanol precipitation, and before adding it to a translation mixture, it was dissolved in a 1 mM aqueous sodium acetate solution.

AAtR-128 BdpFL-Phe-tRNA(fMet)cau

GGCGGGGUGGAGCAGCCUGGUAGCUCGUCGGGCUcauAAC
CCGAAGAUCGUCGGUUCAAAUCCGGCCCCCGCAAC-CA

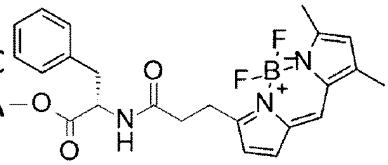

Example 5. Preparation of mRNAs

From the template DNAs (MD-1 to MD-8), template mRNAs (MR-1 to MR-8) were synthesized by *in vitro* transcription reaction using RiboMAX Large Scale RNA production System T7 (Promega, P1300), and then purified by RNeasy Mini kit (Qiagen).

Template DNA SEQ ID NO:221 (MD-1)
DNA sequence:

GGCGTAATACGACTCACTATAGGGTTAACTTTAAGAAGGAGATATACATATGACTTT
TATTATTGGTTTTcttATTATTCCGATTGGTTAAGCTTCG

Template DNA SEQ ID NO:222 (MD-2)
DNA sequence:

GGCGTAATACGACTCACTATAGGGTTAACTTTAAGAAGGAGATATACATATGACTTT
TATTATTGGTTTTctgATTATTCCGATTGGTTAAGCTTCG

Template DNA SEQ ID NO:223 (MD-3)
DNA sequence:

GGCGTAATACGACTCACTATAGGGTTAACTTTAAGAAGGAGATATACATATGACTTT
TATTATTGGTTTTcctATTATTGCTATTGGTTAAGCTTCG

Template DNA SEQ ID NO:224 (MD-4)
DNA sequence:

GGCGTAATACGACTCACTATAGGGTTAACTTTAAGAAGGAGATATACATATGACTTT
TATTATTGGTTTTccgATTATTGCTATTGGTTAAGCTTCG

Template DNA SEQ ID NO:225 (MD-5)
DNA sequence:

GGCGTAATACGACTCACTATAGGGTTAACTTTAAGAAGGAGATATACATATGACTTT
TATTATTCTATTTggtATTATTCCGATTCTATAAGCTTCG

Template DNA SEQ ID NO:226 (MD-6)
DNA sequence:

GGCGTAATACGACTCACTATAGGGTTAACTTTAAGAAGGAGATATACATATGACTTT
TATTATTCTATTTgggATTATTCCGATTCTATAAGCTTCG

Template DNA SEQ ID NO:227 (MD-7)
DNA sequence:

GGCGTAATACGACTCACTATAGGGTTAACTTTAAGAAGGAGATATACATATGACTTT TATTATTCTATTTggaATTATTCCGATTCTATAAGCTTCG

Template DNA SEQ ID NO:228 (MD-8)
DNA sequence:

GGCGTAATACGACTCACTATAGGGTTAACTTTAAGAAGGAGATATACATATGACTTT TATTATTGGTTTTctaATTATTCCGATTGGTTAAGCTTCG

Template mRNA SEQ ID NO:229 (MR-1)
RNA sequence:

GGGUUAACUUUAAGAAGGAGAUAUACAUAUGACUUUUAUUAUUGGUUUUcuuAUU AUUCCGAUUGGUUAAGCUUCG

Template mRNA SEQ ID NO:230 (MR-2)
RNA sequence:

GGGUUAACUUUAAGAAGGAGAUAUACAUAUGACUUUUAUUAUUGGUUUUcugAUU AUUCCGAUUGGUUAAGCUUCG

Template mRNA SEQ ID NO:231 (MR-3)
RNA sequence:

GGGUUAACUUUAAGAAGGAGAUAUACAUAUGACUUUUAUUAUUGGUUUUccuAUU AUUGCUAUUGGUUAAGCUUCG

Template mRNA SEQ ID NO:232 (MR-4)
RNA sequence:

GGGUUAACUUUAAGAAGGAGAUAUACAUAUGACUUUUAUUAUUGGUUUUccgAUU AUUGCUAUUGGUUAAGCUUCG

Template mRNA SEQ ID NO:233 (MR-5)
RNA sequence:

GGGUUAACUUUAAGAAGGAGAUAUACAUAUGACUUUUAUUAUUCUAUUUgguAUU AUUCCGAUUCUAUAAGCUUCG

Template mRNA SEQ ID NO:234 (MR-6)
RNA sequence:

GGGUUAACUUUAAGAAGGAGAUAUACAUAUGACUUUUAUUAUUCUAUUUgggAUU AUUCCGAUUCUAUAAGCUUCG

Template mRNA SEQ ID NO:235 (MR-7)
RNA sequence:

GGGUUAACUUUAAGAAGGAGAUAUACAUAUGACUUUUAUUAUUCUAUUUggaAUU AUUCCGAUUCUAUAAGCUUCG

Template mRNA SEQ ID NO:236 (MR-8)
RNA sequence:

GGGUUAACUUUAAGAAGGAGAUAUACAUAUGACUUUUAUUAUUGGUUUUcuaAUU AUUCCGAUUGGUUAAGCUUCG

Example 6. Translational synthesis of peptides

Example 6-1

To evaluate the effects of combinations of bases at positions 32, 33, 37, and 38 of tRNAs on accuracy of translation, a translation experiment was performed in the presence of two types of elongator aminoacyl tRNAs carrying anticodons that match different codons in the same codon box.

Specifically, peptides were translationally synthesized by translating template mRNA (MR-2) using aminoacyl tRNAs (AAtR-105 and any one of AAtR-1 to AAtR-10), translating template mRNA (MR-1) using aminoacyl tRNAs (AAtR-104 and any one of AAtR-11 to AAtR-20), translating template mRNA (MR-4) using aminoacyl tRNAs (AAtR-107 and any one of AAtR-21 to AAtR-30), translating template mRNA (MR-3) using aminoacyl tRNAs (AAtR-106 and any one of AAtR-31 to AAtR-40), translating template mRNA (MR-6) using aminoacyl tRNAs (AAtR-109 and any one of AAtR-41 to AAtR-50), and translating template mRNA (MR-5) using aminoacyl tRNAs (AAtR-108 and any one of AAtR-51 to AAtR-60). Herein, "AAtR" and "AATR" are used synonymously. More specifically, "AAtR-105" and "AATR-105" refer to the same tRNA.

Translation Condition 1

A translation experiment for evaluating the amount of misreading of the CCG codon by tRNAs carrying the agg anticodon was performed. The translation system used was the PURE system, a prokaryote-derived reconstituted cell-free protein synthesis system. Specifically, the translation was carried out as follows: ARS mix (selection of ARS of amino acids encoded in mRNA from among 0.09 μM GlyRS, 0.4 μM or 0.97 μM IleRS, 0.68 μM or 1.64 μM PheRS, 0.16 μM or 0.39 μM ProRS, 0.09 μM or 0.22 μM ThrRS, 2.73 μM AlaRS, 0.04 μM or 0.097 μM LeuRS, 0.04 μM SerRS, and 0.02 μM ValRS), 1 μM template mRNA (MR-4), and 0.25 mM each of the group of natural amino acids encoded in the template mRNA, 10 μM initiator aminoacylated tRNA (AAtR-128) and 10 μM each of the elongator aminoacyl tRNAs (AAtR-107 and any one selected from AAtR-21 to AAtR-30) were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL *E. coli* MRE600 (RNase-negative)-derived tRNA (Roche), 0.24 μM or 0.26 μM EF-G, 0.24 μM RF2, 0.17 μM RF3, 0.5 μM RRF, 3.7 μM or 4 μg/mL creatine kinase, 2.8 μM or 3 μg/mL myokinase, 1.9 unit/mL or 2 unit/mL inorganic pyrophosphatase, 1.0 μg/mL or 1.1 μg/mL nucleoside diphosphate kinase, 2.5 μM or 2.7 μM IF1, 0.37 μM or 0.4 μM IF2, 1.4 μM or 1.5 μM IF3, 37.2 μM or 40 μM EF-Tu, 49 μM or 54.1 μM or 54.9 μM or 59 μM EF-Ts, 0.93 μM or 1 μM EF-P-Lys, 0.4 unit/μL RNasin® Ribonuclease inhibitor (Promega, N2111), 1.1 μM or 1.2 μM ribosome, 0.5 mM PGA), and the mixture was left to stand at 37° C for one hour. Hereafter, this translation condition may be called "Translation Condition 1".

The template mRNA was designed so that translation gives BdpF:Thr:Phe:Ile:Ile:Gly:Phe:MeHph:Ile:Ile:Ala:Ile:Gly (SEQ ID NO: 241 (Pep-3); herein, an amino acid sequence may be written by separating the amino acids with a colon) as the translation product. When the codon is misread by an aminoacyl tRNA, the translation gives BdpF:Thr:Phe:Ile:Ile:Gly:Phe:SPh2Cl:Ile:Ile:Ala:Ile:Gly (SEQ ID NO: 242 (Pep-4)).

A translation experiment for evaluating the amount of misreading of the CCU codon by tRNAs carrying the cgg anticodon was performed. Translation was carried out according to the above-mentioned Translation Condition 1, except that template mRNA (MR-3) and elongator aminoacyl tRNAs (AAtR-106 and any one of AAtR-31 to AAtR-40) were used.

The template mRNA was designed so that translation gives BdpF:Thr:Phe:Ile:Ile:Gly:Phe:MeHph:Ile:Ile:Ala:Ile:Gly (SEQ ID NO: 241 (Pep-3)) as the correctly read translation product. When misreading by an aminoacyl tRNA takes place, the translation gives BdpF:Thr:Phe:Ile:Ile:Gly:Phe:SPh2Cl:Ile:Ile:Ala:Ile:Gly (SEQ ID NO: 242 (Pep-4)).

A translation experiment for evaluating the amount of misreading of the GGG codon by tRNAs carrying the acc anticodon was performed. Translation was carried out according to the above-mentioned Translation Condition 1, except

that template mRNA (MR-6) and elongator aminoacyl tRNAs (AAtR-109 and any one of AAtR-41 to AAtR-50) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:SPh2Cl:Ile:Ile:Pro:Ile:Leu (SEQ ID NO: 244 (Pep-6)).

A translation experiment for evaluating the amount of misreading of the GGU codon by tRNAs carrying the ccc anticodon was performed. Translation was carried out according to the above-mentioned Translation Condition 1, except that template mRNA (MR-5) and elongator aminoacyl tRNAs (AAtR-108 and any one of AAtR-51 to AAtR-60) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:SPh2Cl:Ile:Ile:Pro:Ile:Leu (SEQ ID NO: 244 (Pep-6)).

A translation experiment for evaluating the amount of misreading of the CUG codon by tRNAs carrying the aag anticodon was performed. Translation was carried out according to the above-mentioned Translation Condition 1, except that template mRNA (MR-2) and elongator aminoacyl tRNAs (AAtR-105 and any one of AAtR-1 to AAtR-10) were used.

The template mRNA was designed so that translation gives BdpF:Thr:Phe:Ile:Ile:Gly:Phe:MeHph:Ile:Ile:Pro:Ile:Gly (SEQ ID NO: 239 (Pep-1)) as the correctly read translation product. When misreading by an aminoacyl tRNA takes place, the translation gives BdpF:Thr:Phe:Ile:Ile:Gly:Phe:SPh2Cl:Ile:Ile:Pro:Ile:Gly (SEQ ID NO: 240 (Pep-2)).

A translation experiment for evaluating the amount of misreading of the CUU codon by tRNAs carrying the cag anticodon was performed. Translation was carried out according to the above-mentioned Translation Condition 1, except that template mRNA (MR-1) and elongator aminoacyl tRNAs (AAtR-104 and any one of AAtR-11 to AAtR-20) were used.

The template mRNA was designed so that translation gives BdpF:Thr:Phe:Ile:Ile:Gly:Phe:MeHph:Ile:Ile:Pro:Ile:Gly (SEQ ID NO: 239 (Pep-1)) as the correctly read translation product. When misreading by an aminoacyl tRNA takes place, the translation gives BdpF:Thr:Phe:Ile:Ile:Gly:Phe:SPh2Cl:Ile:Ile:Pro:Ile:Gly (SEQ ID NO: 240 (Pep-2)).

Example 6-2

The tRNA bodies were changed, and experiments similar to those of Example 6-1 were performed. Specifically, peptides were translationally synthesized by translating template mRNA (MR-2) using aminoacyl tRNAs (AAtR-111 and any one of AAtR-61 to AAtR-70), translating template mRNA (MR-1) using aminoacyl tRNAs (AAtR-110 and any one of AAtR-71 to AAtR-80), translating template mRNA (MR-2) using aminoacyl tRNAs (AAtR-113 and any one of AAtR-81 to AAtR-90), and translating template mRNA (MR-1) using aminoacyl tRNAs (AAtR-112 and any one of AAtR-91 to AAtR-100).

Translation condition

A translation experiment for evaluating the amount of misreading of the CUG codon by tRNAs carrying the aag anticodon was performed. Translation was carried out according to the above-mentioned Translation Condition 1, except that template mRNA (MR-2) and elongator aminoacyl tRNAs (AAtR-113 and any one of AAtR-81 to AAtR-90) were used.

The template mRNA was designed so that translation gives BdpF:Thr:Phe:Ile:Ile:Gly:Phe:MeHph:Ile:Ile:Pro:Ile:Gly (SEQ ID NO: 239 (Pep-1)) as the correctly read translation product. When misreading by an aminoacyl tRNA takes place, the translation gives BdpF:Thr:Phe:Ile:Ile:Gly:Phe:SPh2Cl:Ile:Ile:Pro:Ile:Gly (SEQ ID NO: 240 (Pep-2)).

A translation experiment for evaluating the amount of misreading of the CUU codon by tRNAs carrying the cag anticodon was performed. Translation was carried out according to the above-mentioned Translation Condition 1, except that template mRNA (MR-1) and elongator aminoacyl tRNAs (AAtR-112 and any one of AAtR-91 to AAtR-100) were used.

The template mRNA was designed so that translation gives BdpF:Thr:Phe:Ile:Ile:Gly:Phe:MeHph:Ile:Ile:Pro:Ile:Gly (SEQ ID NO: 239 (Pep-1)) as the correctly read translation product. When misreading by an aminoacyl tRNA takes place, the translation gives BdpF:Thr:Phe:Ile:Ile:Gly:Phe:SPh2Cl:Ile:Ile:Pro:Ile:Gly (SEQ ID NO: 240 (Pep-2)).

A translation experiment for evaluating the amount of misreading of the CUG codon by tRNAs carrying the aag anticodon was performed. Translation was carried out according to the above-mentioned Translation Condition 1, except that template mRNA (MR-2) and elongator aminoacyl tRNAs (AAtR-111 and any one of AAtR-61 to AAtR-70) were used.

The template mRNA was designed so that translation gives BdpF:Thr:Phe:Ile:Ile:Gly:Phe:MeHph:Ile:Ile:Pro:Ile:Gly (SEQ ID NO: 239 (Pep-1)) as the correctly read translation product. When misreading by an aminoacyl tRNA takes place, the translation gives

BdpF:Thr:Phe:Ile:Ile:Gly:Phe:SPh2Cl:Ile:Ile:Pro:Ile:Gly (SEQ ID NO: 240 (Pep-2)).

A translation experiment for evaluating the amount of misreading of the CUU codon by tRNAs carrying the cag anticodon was performed. Translation was carried out according to the above-mentioned Translation Condition 1, except that template mRNA (MR-1) and elongator aminoacyl tRNAs (AAtR-110 and any one of AAtR-71 to AAtR-80) were used.

The template mRNA was designed so that translation gives BdpF:Thr:Phe:Ile:Ile:Gly:Phe:MeHph:Ile:Ile:Pro:Ile:Gly (SEQ ID NO: 239 (Pep-1)) as the correctly read translation product. When misreading by an aminoacyl tRNA takes place, the translation gives BdpF:Thr:Phe:Ile:Ile:Gly:Phe:SPh2Cl:Ile:Ile:Pro:Ile:Gly (SEQ ID NO: 240 (Pep-2)).

Example 6-3

The codons used were changed, and experiments similar to those of Examples 6-1 and 6-2 were performed. Specifically, peptides were translationally synthesized by translating template mRNA (MR-7) using aminoacyl tRNAs (AAtR-114 and any one of AAtR-44, AAtR-47, and AAtR-48), translating template mRNA (MR-7) using aminoacyl tRNAs (AAtR-114 and any one of AAtR-101 to AAtR-103), and translating template mRNA (MR-6) using aminoacyl tRNAs (AAtR-109 and any one of AAtR-101 to AAtR-103).

Translation condition

A translation experiment for evaluating the amount of misreading of the GGA codon by tRNAs carrying the acc anticodon was performed. Translation was carried out according to the above-mentioned Translation Condition 1, except that template mRNA (MR-7) and elongator aminoacyl tRNAs (AAtR-114 and any one of AAtR-44, AAtR-47, and AAtR-48) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:SPh2Cl:Ile:Ile:Pro:Ile:Leu (SEQ ID NO: 244 (Pep-6)).

A translation experiment for evaluating the amount of misreading of the GGA codon by tRNAs carrying the gcc anticodon was performed. Translation was carried out according to the above-mentioned Translation Condition 1, except that template mRNA (MR-7) and elongator aminoacyl tRNAs (AAtR-114 and any one of AAtR-101 to AAtR-103) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:SPh2Cl:Ile:Ile:Pro:Ile:Leu (SEQ ID NO: 244 (Pep-6)).

A translation experiment for evaluating the amount of misreading of the GGG codon by tRNAs carrying the gcc anticodon was performed. Translation was carried out according to the above-mentioned Translation Condition 1, except that template mRNA (MR-6) and elongator aminoacyl tRNAs (AAtR-109 and any one of AAtR-101 to AAtR-103) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:SPh2Cl:Ile:Ile:Pro:Ile:Leu (SEQ ID NO: 244 (Pep-6)).

Example 6-4

Using tRNAs in which the first letter of the anticodon has been engineered (tRNAs whose base at the first letter of the anticodon is lysidine), experiments similar to those of Examples 6-1 to 6-3 were performed. Specifically, peptides were translationally synthesized by translating according to the above-mentioned Translation Condition 1, except that template mRNA (MR-1, MR-8, or MR-2) and aminoacyl tRNAs (AAtR-131, AAtR-134, and either AAtR-132 or AAtR-133) or aminoacyl tRNAs (AAtR-135, AAtR-138, and either AAtR-136 or AAtR-137) were used. The amount of translation of the following translation products were compared:

BdpF:Thr:Phe:Ile:Ile:Gly:Phe:nBuG:Ile:Ile:Pro:Ile:Gly (SEQ ID NO: 250 (Pep-12));
BdpF:Thr:Phe:Ile:Ile:Gly:Phe:SPh2Cl:Ile:Ile:Pro:Ile:Gly (SEQ ID NO: 251 (Pep-13)); and
BdpF:Thr:Phe:Ile:Ile:Gly:Phe:dA:Ile:Ile:Pro:Ile:Gly (SEQ ID NO: 252 (Pep-14)).

Example 6-5

The amino acids were changed, and experiments similar to those of Examples 6-1 to 6-4 were performed. Specifically, peptides were translationally synthesized by translating template mRNA (MR-6) using aminoacyl tRNAs (AAtR-109 and any one of AAtR-115 to AAtR-118; or AAtR-109 and any one of AAtR-119 to AAtR-122; or AAtR-127 and any one of AAtR-123 to AAtR-126), translating template mRNA (MR-4) using aminoacyl tRNAs (AAtR-130 and any one of AAtR-21 to AAtR-30), and translating template mRNA (MR-3) using aminoacyl tRNAs (AAtR-129 and any one of AAtR-31 to AAtR-40).

Translation condition

A translation experiment for evaluating the amount of misreading of the GGG codon by tRNAs carrying the acc anticodon was performed. Translation was carried out according to the above-mentioned Translation Condition 1, except that template mRNA (MR-6) and elongator aminoacyl tRNAs (AAtR-109 and any one of AAtR-115 to AAtR-118) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:Pic2:Ile:Ile:Pro:Ile:Leu (SEQ ID NO: 245 (Pep-7)).

A translation experiment for evaluating the amount of misreading of the GGG codon by tRNAs carrying the acc anticodon was performed. Translation was carried out according to the above-mentioned Translation Condition 1, except that template mRNA (MR-6) and elongator aminoacyl tRNAs (AAtR-109 and any one of AAtR-119 to AAtR-122) were used.

The template mRNA was designed so that translation gives BdpF:Thr:Phe:Ile:Ile:Leu:Phe:MeHph:Ile:Ile:Pro:Ile:Leu (SEQ ID NO: 243 (Pep-5)) as the correctly read translation product. When misreading by an aminoacyl tRNA takes place, the translation gives BdpF:Thr:Phe:Ile:Ile:Leu:Phe:MeG:Ile:Ile:Pro:Ile:Leu (SEQ ID NO: 246 (Pep-8)).

A translation experiment for evaluating the amount of misreading of the GGG codon by tRNAs carrying the acc anticodon was performed. Translation was carried out according to the above-mentioned Translation Condition 1, except that template mRNA (MR-6) and elongator aminoacyl tRNAs (AAtR-127 and any one of AAtR-123 to AAtR-126) were used.

The template mRNA was designed so that translation gives BdpF:Thr:Phe:Ile:Ile:Leu:Phe:nBuG:Ile:Ile:Pro:Ile:Leu (SEQ ID NO: 247 (Pep-9)) as the correctly read translation product. When misreading by an aminoacyl tRNA takes place, the translation gives BdpF:Thr:Phe:Ile:Ile:Leu:Phe:dA:Ile:Ile:Pro:Ile:Leu (SEQ ID NO: 248 (Pep-10)).

A translation experiment for evaluating the amount of misreading of the CCG codon by tRNAs carrying the agg anticodon was performed. Translation was carried out according to the above-mentioned Translation Condition 1, except that template mRNA (MR-4) and elongator aminoacyl tRNAs (AAtR-130 and any one of AAtR-21 to AAtR-30) were used.

The template mRNA was designed so that translation gives BdpF:Thr:Phe:Ile:Ile:Gly:Phe:MeG:Ile:Ile:Ala:Ile:Gly (SEQ ID NO: 249 (Pep-11)) as the correctly read translation product. When misreading by an aminoacyl tRNA takes place, the translation gives BdpF:Thr:Phe:Ile:Ile:Gly:Phe:SPh2Cl:Ile:Ile:Ala:Ile:Gly (SEQ ID NO: 242 (Pep-4)).

A translation experiment for evaluating the amount of misreading of the CCU codon by tRNAs carrying the cgg anticodon was performed. Translation was carried out according to the above-mentioned Translation Condition 1, except that template mRNA (MR-3) and elongator aminoacyl tRNAs (AAtR-129 and any one of AAtR-31 to AAtR-40) were used.

The template mRNA was designed so that translation gives BdpF:Thr:Phe:Ile:Ile:Gly:Phe:MeG:Ile:Ile:Ala:Ile:Gly (SEQ ID NO: 249 (Pep-11)) as the correctly read translation product. When misreading by an aminoacyl tRNA takes place, the translation gives BdpF:Thr:Phe:Ile:Ile:Gly:Phe:SPh2Cl:Ile:Ile:Ala:Ile:Gly (SEQ ID NO: 242 (Pep-4)).

Example 7 Analyses of the translated peptides

Translation product solutions obtained by the translation reactions of Examples 6-1 to 6-5 were diluted ten-fold, and then analyzed using a LC-FLR-MS system. The amount of translated peptide was evaluated from the analysis data by identifying the retention time of the target translated peptide from the MS data, and quantifying the fluorescence peak at the relevant retention time. Unless indicated otherwise, in the quantitative evaluation, LCT-67 synthesized in Example 3 was used as a standard to prepare a calibration curve, and the content was calculated by relative quantification. Unless stated otherwise, the LC-MS was analyzed according to the conditions of Method 1 shown in Table 5 below. Furthermore, based on the determined amount of peptide translation, the percentage (%) of misread peptides relative to an intended product was calculated using the following equation. Herein, the peptide obtained when correct reading

takes place may be referred to as the correctly read translation product or intended product.

[Equation 1]

$$\text{Percentage of misread peptides relative to the intended product (\%)} = \frac{\text{Amount of translated peptide obtained when misreading takes place (μM)}}{\text{Amount of translated peptide obtained when correctly read (μM)}} \times 100$$

Analysis conditions

[Table 5]

| | Method1 | Method2 |
|---|---|---|
| System | Aquity UPLC-FLR-Xevo G2-XS Tof | Aquity UPLC-FLR-Xevo G2-XS Tof |
| Column | waters BEHC18(2.1×50mm, $\phi$1.7$\mu$m) | waters BEHC18(2.1×100mm, $\phi$1.7 $\mu$m) |
| Mobile phase | A=0.1% FA with $H_20$<br>B=0.1% FA with $CH_3CN$ | A=0.1% FA with $H_20$<br>B=0.1% FA with $CH_3CN$ |
| Gradient (%B) | 0-0.2min=10%<br>0. 2-3. 6min=98%<br>3.6-4.0min=10% | 0-0.4min=60%<br>0.4-9.0min=98%<br>9.0-10min=60% |
| Flow rate (ml/min) | 0.5 | 0.5 |
| Column temp. | 40 | 40 |
| **Fluorometry** wavelength (Ex/Em) | 491nm/515nm | 491nm/515nm |
| Ms mode | ESI- | ESI- |

Results

As a result of the evaluation, the rate of codon misreading by tRNAs whose combination of bases at positions 32, 33, 37, and 38 had been engineered tended to increase when the combination of bases at those positions was the Ser5 sequence, the AlaIB sequence, and the Phe sequence, and tended to decrease when this combination was the Pro3 sequence, the Pro2 sequence, the Ala2 sequence, the Leu2 sequence, the Arg3 sequence, and the Val2 sequence (Figs. 1 to 6, and Tables 6 to 11). Among them, decreasing tendencies were greater for the Pro3 sequence, the Pro2 sequence, the Ala2 sequence, and the Leu2 sequence. The ranking of the rate of misreading was not affected, even when the tRNA body was changed (Figs. 7 to 10, and Tables 12 to 15). Effects of reducing codon misreading due to engineering the combination of bases at positions 32, 33, 37, and 38 in the tRNA to a specific combination were also confirmed for cases where the combination of the codon and the anticodon to be evaluated was changed (Figs. 11 to 13, Tables 16 to 18). Effects of reducing misreading due to engineering the combination of bases at positions 32, 33, 37, and 38 in the tRNA to a specific combination were also confirmed for cases where the amino acid to be aminoacylated was changed (Figs. 14 to 18, and Tables 21 to 25). These results showed that engineering the combination of bases at positions 32, 33, 37, and 38 in the tRNA can reduce codon misreading. The likelihood of occurring codon misreading was elucidated for combinations of bases regarding positions 32, 33, 37, and 38 in the tRNA; and this enables customizing tRNA sequences to reduce codon misreading, while maintaining the amount of translated amino acid above a certain level, depending on the tRNA used.

The following table shows the translation results of CCG codon discrimination by a tRNA carrying agg or cgg as the anticodon, whose combination of bases at positions 32, 33, 37, and 38 had been engineered. Compared to tRNA Glu2 (AAtR-24), whose combination of bases at positions 32, 33, 37, and 38 had not been engineered, tRNAs (AAtR-27 to 30) in which the base combination had been engineered to be the Pro2 sequence, the Leu2 sequence, the Ala2

sequence, or the Pro3 sequence were found to have reduced codon misreading (Fig. 1).

[Table 6]

| Aminoacylated tRNA | Template mRNA sequence | Translated peptide compound | Amount ot translation (μM) |
|---|---|---|---|
| AATR-21 | MR-4 | BdpF:Thr:Phe:Ile:Ile:Gly:Phe:SPh2Cl:Ile:Ile:Ala:Ile:Gly | 0.58 |
| AATR-107 | | BdpF:ThrPhe:Ile:Ile:Gly:Phe:MeHph:Ile:Ile:Ala:Ile:Gly | 1.75 |
| AATR-22 | | BdpF:Thr:Phe:Ile:Ile:Gly:Phe:SPh2Cl:Ile:Ile:Ala:Ile:Gly | 0.51 |
| AATR-107 | | BdpF:Thr:Phe:Ile:Ile:Gly:Phe:MeHph:Ile:Ile:Ala:Ile:Gly | 1.66 |
| AATR-23 | | BdpF:Thr:Phe:Ile:Ile:Gly:Phe:SPh2Cl:Ile:Ile:Ala:Ile:Gly | 0.58 |
| AATR-107 | | BdpF:Thr:Phe:Ile:Ile:Gly:Phe:MeHph:Ile:Ile:Ala:Ile:Gly | 2.05 |
| AATR-24 | | BdpF: Thr:Phe:Ile:Ile:Gly:Phe:SPh2Cl:Ile:Ile:Ala:Ile:Gly | 0.36 |
| AATR-107 | | BdpF:Thr:Phe:Ile:Ile:Gly:Phe:MeHph:Ile:Ile:Ala:Ile:Gly | 1.93 |
| AATR-25 | | BdpF:Thr:Phe:Ile:Ile:Gly:Phe:SPh2Cl:Ile:Ile:Ala:Ile:Gly | 0.48 |
| AATR-107 | | BdpF:Thr:Phe:Ile:Ile:Gly:Phe:MeHph:Ile:Ile:Ala:Ile:Gly | 2.19 |
| AATR-26 | | BdpF:Thr:Phe:Ile:Ile:Gly:Phe:SPh2Cl:Ile:Ile:Ala:Ile:Gly | 0.44 |
| AATR-107 | | BdpF:Thr:Phe:Ile:Ile:Gly:Phe:MeHph:Ile:Ile:Ala:Ile:Gly | 2.15 |
| AATR-27 | | BdpF: Thr:Phe:Ile:Ile:Gly:Phe:SPh2Cl:Ile:Ile:Ala:Ile:Gly | 0.25 |
| AATR-107 | | BdpF:Thr:Phe:Ile:Ile:Gly:Phe:MeHph:Ile:Ile:Ala:Ile:Gly | 2.32 |
| AATR-28 | | BdpF:Thr:Phe:Ile:Ile:Gly:Phe:SPh2Cl:Ile:Ile:Ala:Ile:Gly | 0.35 |
| AATR-107 | | BdpF:Thr:Phe:Ile:Ile:Gly:Phe:MeHph:Ile:Ile:Ala:Ile:Gly | 2.41 |
| AATR-29 | | BdpF:Thr:Phe:Ile:Ile:Gly:Phe:SPh2Cl:Ile:Ile:Ala:Ile:Gly | 0.11 |
| AATR-107 | | BdpF:Thr:Phe:Ile:Ile:Gly:Phe:MeHph:Ile:Ile:Ala:Ile:Gly | 2.89 |
| AATR-30 | | BdpF:Thr:Phe:Ile:Ile:Gly:Phe:SPh2Cl:Ile:Ile:Ala:Ile:Gly | 0.11 |
| AATR-107 | | BdpF:Thr:Phe:Ile:Ile:Gly:Phe:MeHph:Ile:Ile:Ala:Ile:Gly | 2.60 |

The following table shows the translation results of CCU codon discrimination by a tRNA carrying cgg or agg as the anticodon, whose combination of bases at positions 32, 33, 37, and 38 had been engineered. Compared to tRNA

Glu2 (AAtR-34), whose combination of bases at positions 32, 33, 37, and 38 had not been engineered, tRNAs (AAtR-33 and AAtR-35 to 40) in which the base combination had been engineered to be the Phe sequence, the Arg3 sequence, the Val2 sequence, the Pro2 sequence, the Leu2 sequence, the Ala2 sequence, or the Pro3 sequence were found to have reduced codon misreading, and particularly the tRNAs engineered to have the Pro2 sequence, the Leu2 sequence, the Ala2 sequence, or the Pro3 sequence showed highly effective reduction of codon misreading (Fig. 2).

[Table 7]

| Aminoacvlated tRNA | Template mRNA seauence | Translated peptide compound | Amount of translation (μM |
|---|---|---|---|
| AATR-31 | MR-3 | BdpF:Thr:Phe:Ile:Ile:Gly:Phe:SPh2Cl:Ile:Ile:Ala:Ile:Gly | 0.44 |
| AATR-106 | | BdpF:Thr:Phe:Ile:Ile:Gly:Phe:MeHph:Ile:Ile:Ala:Ile:Gly | 1.96 |
| AATR-32 | | BdpF:Thr:Phe:Ile:Ile:Gly:Phe:SPh2Cl:Ile:Ile:Ala:Ile:Gly | 0.39 |
| AATR-106 | | BdpF:Thr:Phe:Ile:Ile:Gly:Phe:MeHph:Ile:Ile:Ala:Ile:Gly | 1.70 |
| AATR-33 | | BdpF:Thr:Phe:Ile:Ile:Gly:Phe:SPh2Cl:Ile:Ile:Ala:Ile:Gly | 0.35 |
| AATR-106 | | BdpF:Thr:Phe:Ile:Ile:Gly:Phe:MeHph:Ile:Ile:Ala:Ile:Gly | 2.45 |
| AATR-34 | | BdpF: Thr:Phe:Ile:Ile:Gly:Phe:SPh2Cl:Ile:Ile:Ala:Ile:Gly | 0.32 |
| AATR-106 | | BdpF:Thr:Phe:Ile:Ile:Gly:Phe:MeHph:Ile:Ile:Ala:Ile:Gly | 2.05 |
| AATR-35 | | BdpF:Thr:Phe:Ile:Ile:Gly:Phe:SPh2Cl:Ile:Ile:Ala:Ile:Gly | 0.21 |
| AATR-106 | | BdpF:Thr:Phe:Ile:Ile:Gly:Phe:MeHph:Ile:Ile:Ala:Ile:Gly | 1.94 |
| AATR-36 | | BdpF: Thr:Phe:Ile:Ile:Gly:Phe:SPh2Cl:Ile:Ile:Ala:Ile:Gly | 0.25 |
| AATR-106 | | BdpF:Thr:Phe:Ile:Ile:Gly:Phe:MeHph:Ile:Ile:Ala:Ile:Gly | 2.40 |
| AATR-37 | | BdpF:Thr:Phe:Ile:Ile:Gly:Phe:SPh2Cl:Ile:Ile:Ala:Ile:Gly | 0.13 |
| AATR-106 | | BdpF:Thr:Phe:Ile:Ile:Gly:Phe:MeHph:Ile:Ile:Ala:Ile:Gly | 2.42 |
| AATR-38 | | BdpF:Thr:Phe:Ile:Ile:Gly:Phe:SPh2Cl:Ile:Ile:Ala:Ile:Gly | 0.14 |
| AATR-106 | | BdpF:ThrPhe:Ile:Ile:Gly:Phe:MeHph:Ile:Ile:Ala:Ile:Gly | 2.68 |
| AATR-39 | | BdpF:Thr:Phe:Ile:Ile:Gly:Phe:SPh2Cl:Ile:Ile:Ala:Ile:Gly | 0.07 |
| AATR-106 | | BdpF:Thr:Phe:Ile:Ile:Gly:Phe:MeHph:Ile:Ile:Ala:Ile:Gly | 3.00 |
| AATR-40 | | BdpF:Thr:Phe:Ile:Ile:Gly:Phe:SPh2Cl:Ile:Ile:Ala:Ile:Gly | 0.06 |
| AATR-106 | | BdpF:Thr:Phe:Ile:Ile:Gly:Phe:MeHph:Ile:Ile:Ala:Ile:Gly | 2.60 |

The following table shows the translation results of GGG codon discrimination by a tRNA carrying acc or ccc as the anticodon, whose combination of bases at positions 32, 33, 37, and 38 had been engineered. Compared to tRNA Glu2 (AAtR-44), whose combination of bases at positions 32, 33, 37, and 38 had not been engineered, tRNAs (AAtR-43 and AAtR45 to 50) in which the base combination had been engineered to be the Arg3 sequence, the Phe sequence,

the Val2 sequence, the Pro2 sequence, the Leu2 sequence, the Ala2 sequence, or the Pro3 sequence were found to have reduced codon misreading, and particularly the tRNAs engineered to have the Pro2 sequence, the Leu2 sequence, the Ala2 sequence, or the Pro3 sequence showed highly effective reduction of codon misreading (Fig. 3).

[Table 8]

| Aminoacylated tRNA | Template mRNA sequence | Translated peptide compound | Amount of translation (μM) |
|---|---|---|---|
| AA TR-41 | MR-6 | BdpF:Thr:Phe:Ile:Ile:Leu:Phe: SPh2Cl:Ile:Ile:Pro:Ile:Leu | 0.91 |
| AATR-109 | | BdpF:Thr:Phe:Ile:Ile:Leu:Phe:MeHph: Ile:Ile:Pro:Ile:Leu | 2.51 |
| AA TR-42 | | BdpF:Thr:Phe:Ile:Ile:Leu:Phe: SPh2Cl:Ile:Ile:Pro:Ile:Leu | 0.68 |
| AATR-109 | | BdpF:Thr:Phe:Ile:Ile:Leu:Phe:MeHph: Ile:Ile:Pro:Ile:Leu | 2.48 |
| AA TR-43 | | BdpF:Thr:Phe:Ile:Ile:Leu:Phe: SPh2Cl:Ile:Ile:Pro:Ile:Leu | 0.57 |
| AATR-109 | | BdpF:Thr:Phe:Ile:Ile:Leu:Phe:MeHph: Ile:Ile:Pro:Ile:Leu | 3.29 |
| AA TR-44 | | BdpF:Thr:Phe:Ile:Ile:Leu:Phe: SPh2Cl:Ile:Ile:Pro:Ile:Leu | 0.77 |
| AATR-109 | | BdpF:Thr:Phe:Ile:Ile:Leu:Phe:MeHph: Ile:Ile:Pro:Ile:Leu | 2.79 |
| AATR-45 | | BdpF:Thr:Phe:Ile:Ile:Leu:Phe: SPh2Cl:Ile:Ile:Pro:Ile:Leu | 0.58 |
| AATR-109 | | BdpF:Thr:Phe:Ile:Ile:Leu:Phe:MeHph: Ile:Ile:Pro:Ile:Leu | 3.31 |
| AATR-46 | | BdpF:Thr:Phe:Ile:Ile:Leu:Phe: SPh2Cl:Ile:Ile:Pro:Ile:Leu | 0.34 |
| AATR-109 | | BdpF:Thr:Phe:Ile:Ile:Leu:Phe:MeHph: Ile:Ile:Pro:Ile:Leu | 3.53 |
| AATR-47 | | BdpF:Thr:Phe:Ile:Ile:Leu:Phe: SPh2Cl:Ile:Ile:Pro:Ile:Leu | 0.05 |
| AATR-109 | | BdpF:ThrPhe:Ile:Ile:Leu:Phe:MeHph: Ile:Ile:Pro:Ile:Leu | 3.23 |
| AA TR-48 | | BdpF:Thr:Phe:Ile:Ile:Leu:Phe: SPh2Cl:Ile:Ile:Pro:Ile:Leu | 0.07 |
| AATR-109 | | BdpF:Thr:Phe:Ile:Ile:Leu:Phe:MeHph: Ile:Ile:Pro:Ile:Leu | 3.68 |
| AA TR-49 | | BdpF:Thr:Phe:Ile:Ile:Leu:Phe: SPh2Cl:Ile:Ile:Pro:Ile:Leu | 0.08 |
| AATR-109 | | BdpF:Thr:Phe:Ile:Ile:Leu:Phe:MeHph: Ile:Ile:Pro:Ile:Leu | 3.15 |
| AATR-50 | | BdpF:Thr:Phe:Ile:Ile:Leu:Phe: SPh2Cl:Ile:Ile:Pro:Ile:Leu | 0.07 |
| AATR-109 | | BdpF:Thr:Phe:Ile:Ile:Leu:Phe:MeHph: Ile:Ile:Pro:Ile:Leu | 3.23 |

The following table shows the translation results of GGU codon discrimination by a tRNA carrying ccc or acc as the anticodon, whose combination of bases at positions 32, 33, 37, and 38 had been engineered. Although the amount of translated peptides produced by codon misreading was low for tRNA Glu2 (AAtR-54), whose combination of bases at positions 32, 33, 37, and 38 had not been engineered, the amount of translated peptides obtained by codon misreading was also kept low for tRNAs (AAtR-52, AAtR-53, and AAtR-55 to 60) in which the base combination had been engineered to be the Phe sequence, the AlalB sequence, the Pro2 sequence, the Leu2 sequence, the Ala2 sequence, the Pro3 sequence, the Arg3 sequence, or the Val2 sequence, and a trend similar to the above Examples were observed (Fig. 4).

[Table 9]

| Aminoacylated tRNA | Template mRNA sequence | Translated peptide compound | Amount of translation (μM) |
|---|---|---|---|
| AATR-51 | MR-5 | BdpF:Thr:Phe:Ile:Ile:Leu:Phe: SPh2Cl:Ile:Ile:Pro:Ile:Leu | 0.15 |
| AATR-108 | | BdpF:Thr:Phe:Ile:Ile:Leu:Phe:MeHph: Ile:Ile:Pro:Ile:Leu | 2.54 |
| AATR-52 | | BdpF:Thr:Phe:Ile:Ile:Leu:Phe: SPh2Cl:Ile:Ile:Pro:Ile:Leu | 0.03 |
| AATR-108 | | BdpF:Thr:Phe:Ile:Ile:Leu:Phe:MeHph: Ile:Ile:Pro:Ile:Leu | 2.43 |
| AA TR-53 | | BdpF:Thr:Phe:Ile:Ile:Leu:Phe: SPh2Cl:Ile:Ile:Pro:Ile:Leu | 0.04 |
| AATR-108 | | BdpF:Thr:Phe:Ile:Ile:Leu:Phe:MeHph: Ile:Ile:Pro:Ile:Leu | 2.97 |
| AATR-54 | | BdpF:Thr:Phe:Ile:Ile:Leu:Phe: SPh2Cl:Ile:Ile:Pro:Ile:Leu | 0.04 |
| AATR-108 | | BdpF:Thr:Phe:Ile:Ile:Leu:Phe:MeHph: Ile:Ile:Pro:Ile:Leu | 2.40 |
| AATR-55 | | BdpF:Thr:Phe:Ile:Ile:Leu:Phe: SPh2Cl:Ile:Ile:Pro:Ile:Leu | 0.02 |
| AATR-108 | | BdpF:Thr:Phe:Ile:Ile:Leu:Phe:MeHph: Ile:Ile:Pro:Ile:Leu | 2.57 |
| AATR-56 | | BdpF:Thr:Phe:Ile:Ile:Leu:Phe: SPh2Cl:Ile:Ile:Pro:Ile:Leu | 0.02 |
| AATR-108 | | BdpF:Thr:Phe:Ile:Ile:Leu:Phe:MeHph: Ile:Ile:Pro:Ile:Leu | 2.22 |
| AATR-57 | | BdpF:Thr:Phe:Ile:Ile:Leu:Phe: SPh2Cl:Ile:Ile:Pro:Ile:Leu | 0.01 |
| AATR-108 | | BdpF:Thr:Phe:Ile:Ile:Leu:Phe:MeHph: Ile:Ile:Pro:Ile:Leu | 2.39 |
| AATR-58 | | BdpF:Thr:Phe:Ile:Ile:Leu:Phe: SPh2Cl:Ile:Ile:Pro:Ile:Leu | 0.02 |
| AATR-108 | | BdpF:Thr:Phe:Ile:Ile:Leu:Phe:MeHph: Ile:Ile:Pro:Ile:Leu | 2.63 |
| AATR-59 | | BdpF:Thr:Phe:Ile:Ile:Leu:Phe: SPh2Cl:Ile:Ile:Pro:Ile:Leu | 0.03 |
| AATR-108 | | BdpF:Thr:Phe:Ile:Ile:Leu:Phe:MeHph: Ile:Ile:Pro:Ile:Leu | 2.71 |
| AATR-60 | | BdpF:Thr:Phe:Ile:Ile:Leu:Phe: SPh2Cl:Ile:Ile:Pro:Ile:Leu | 0.02 |
| AATR-108 | | BdpF:Thr:Phe:Ile:Ile:Leu:Phe:MeHph: Ile:Ile:Pro:Ile:Leu | 2.55 |

The following table shows the results of translating the CUG codon by a tRNA carrying cag or aag as the anticodon, whose combination of bases at positions 32, 33, 37, and 38 had been engineered. Compared to tRNA Glu2 (AAtR-4), whose combination of bases at positions 32, 33, 37, and 38 had not been engineered, tRNAs (AAtR-8 and 9) in which the base-derived combination had been engineered to be the Pro2 sequence or the Pro3 sequence were found

to have reduced codon misreading, and the amount of translated peptide produced by codon misreading was kept at a low level for the Ala2 sequence as well (Fig. 5).

[Table 10]

| Aminoacylated tRNA | Template mRNA sequence | Translated peptide compound | Amount of translation (μM) |
|---|---|---|---|
| AATR-1 | MR-2 | BdpF:Thr:Phe:Ile:Ile:Gly:Phe:SPh2Cl: Ile:Ile:Pro:Ile:Gly | 0.28 |
| AATR-105 | | BdpF:Thr:Phe:Ile:Ile:Gly:Phe:MeHph: Ile:Ile:Pro:Ile:Gly | 1.85 |
| AATR-2 | | BdpF:Thr:Phe:Ile:Ile:Gly:Phe:SPh2Cl: Ile:Ile:Pro:Ile:Gly | 0.35 |
| AATR-105 | | BdpF:Thr:Phe:Ile:Ile:Gly:Phe:MeHph: Ile:Ile:Pro:Ile:Gly | 1.86 |
| AATR-3 | | BdpF:Thr:Phe:Ile:Ile:Gly:Phe:SPh2Cl: Ile:Ile:Pro:Ile:Gly | 0.26 |
| AATR-105 | | BdpF:Thr:Phe:Ile:Ile:Gly:Phe:MeHph: Ile:Ile:Pro:Ile:Gly | 1.92 |
| AATR-4 | | BdpF:Thr:Phe:Ile:Ile:Gly:Phe:SPh2Cl: Ile:Ile:Pro:Ile:Gly | 0.13 |
| AATR-105 | | BdpF:Thr:Phe:Ile:Ile:Gly:Phe:MeHph: Ile:Ile:Pro:Ile:Gly | 1.62 |
| AATR-5 | | BdpF:Thr:Phe:Ile:Ile:Gly:Phe:SPh2Cl: Ile:Ile:Pro:Ile:Gly | 0.16 |
| AATR-105 | | BdpF:Thr:Phe:Ile:Ile:Gly:Phe:MeHph: Ile:Ile:Pro:Ile:Gly | 1.80 |
| AATR-6 | | BdpF: Thr:Phe:Ile:Ile:Gly:Phe: SPh2Cl:Ile:Ile:Pro:Ile:Gly | 0.17 |
| AATR-105 | | BdpF:Thr:Phe:Ile:Ile:Gly:Phe:MeHph: Ile:Ile:Pro:Ile:Gly | 1.82 |
| AATR-7 | | BdpF:Thr:Phe:Ile:Ile:Gly:Phe:SPh2Cl: Ile:Ile:Pro:Ile:Gly | 0.18 |
| AATR-105 | | BdpF:ThrPhe:Ile:Ile:Gly:Phe:MeHph: Ile:Ile:Pro:Ile:Gly | 1.56 |
| AATR-8 | | BdpF:Thr:Phe:Ile:Ile:Gly:Phe:SPh2Cl: Ile:Ile:Pro:Ile:Gly | 0.06 |
| AATR-105 | | BdpF:Thr:Phe:Ile:Ile:Gly:Phe:MeHph: Ile:Ile:Pro:Ile:Gly | 2.27 |
| AATR-9 | | BdpF:Thr:Phe:Ile:Ile:Gly:Phe:SPh2Cl: Ile:Ile:Pro:Ile:Gly | 0.05 |
| AATR-105 | | BdpF:Thr:Phe:Ile:Ile:Gly:Phe:MeHph: Ile:Ile:Pro:Ile:Gly | 1.85 |
| AATR-10 | | BdpF:Thr:Phe:Ile:Ile:Gly:Phe:SPh2Cl: Ile:Ile:Pro:Ile:Gly | 0.13 |
| AATR-105 | | BdpF:Thr:Phe:Ile:Ile:Gly:Phe:MeHph: Ile:Ile:Pro:Ile:Gly | 1.67 |

The following table shows the translation results of CUU codon discrimination by a tRNA carrying aag or cag

as the anticodon, whose combination of bases at positions 32, 33, 37, and 38 had been engineered. Although the amount of translated peptides produced by codon misreading was low for tRNA Glu2 (AAtR-14), whose combination of bases at positions 32, 33, 37, and 38 had not been engineered, the amount of translated peptides obtained by codon misreading was also kept low for tRNAs (AAtR-13, and AAtR-15 to 20) in which the combination derived from the above bases had been engineered to be the Phe sequence, the Pro2 sequence, the Leu2 sequence, the Ala2 sequence, the Pro3 sequence, the Arg3 sequence, or the Val2 sequence, and a trend similar to the above Examples were observed (Fig. 6).

[Table 11]

| Aminoacylated tRNA | Template mRNA seauence | Translated peptide compound | Amount of translation (μM) |
|---|---|---|---|
| AATR-11 | MR-1 | BdpF:Thr:Phe:Ile:Ile:Gly:Phe:SPh2Cl:Ile:Ile:Pro:Ile:Gly | 0.09 |
| AATR-104 | | BdpF:Thr:Phe:Ile:Ile:Gly:Phe:MeHph:Ile:Ile:Pro:Ile:Gly | 1.61 |
| AATR-12 | | BdpF:Thr:Phe:Ile:Ile:Gly:Phe:SPh2Cl:Ile:Ile:Pro:Ile:Gly | 0.10 |
| AATR-104 | | BdpF:Thr:Phe:Ile:Ile:Gly:Phe:MeHph:Ile:Ile:Pro:Ile:Gly | 1.62 |
| AATR-13 | | BdpF:Thr:Phe:Ile:Ile:Gly:Phe:SPh2Cl:Ile:Ile:Pro:Ile:Gly | 0.02 |
| AATR-104 | | BdpF:Thr:Phe:Ile:Ile:Gly:Phe:MeHph:Ile:Ile:Pro:Ile:Gly | 1.74 |
| AATR-14 | | BdpF:Thr:Phe:Ile:Ile:Gly:Phe:SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 1.51 |
| AATR-15 | | BdpF:Thr:Phe:Ile:Ile:Gly:Phe:SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 1.56 |
| AATR-16 | | BdpF:Thr:Phe:Ile:Ile:Gly:Phe:SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 1.48 |
| AATR-17 | | BdpF:Thr:Phe:Ile:Ile:Gly:Phe:SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 1.54 |
| AATR-18 | | BdpF: Thr:Phe:Ile:Ile:Gly:Phe: SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 1.65 |
| AATR-19 | | BdpF:Thr:Phe:Ile:Ile:Gly:Phe:SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 0.94 |
| AATR-20 | | BdpF:Thr:Phe:Ile:Ile:Gly:Phe:SPh2Cl:Ile:Ile:Pro:Ile:Gly | 0.01 |
| AATR-104 | | BdpF:Thr:Phe:Ile:Ile:Gly:Phe:MeHph:Ile:Ile:Pro:Ile:Gly | 1.62 |

These results indicated that when the combination of bases at positions 32, 33, 37, and 38 in a tRNA is the Ser5 sequence or the AlaIB sequence, the percentage of misread peptides relative to an intended product is high. By engineering the combination of bases at positions 32, 33, 37, and 38 in a tRNA having the Ser5 sequence as the above-mentioned base combination, the following experiments were performed to verify whether codon misreading can be

reduced.

tRNA AsnE2 was selected as the tRNA whose combination of bases at positions 32, 33, 37, and 38 is the Ser5 sequence. The following table shows the translation results of CUG codon discrimination by a tRNA which carries the tRNA(AsnE2) body with its combination of bases at positions 32, 33, 37, and 38 being engineered, and which carries aag or cag as the anticodon. Compared to tRNA(AsnE2) (AAtR-81), whose combination of bases at positions 32, 33, 37, and 38 had not been engineered, tRNAs (AAtR-85 to 90) in which the combination derived from the above-mentioned bases had been engineered to be the Arg3 sequence, the Val2 sequence, the Pro2 sequence, the Leu2 sequence, the Ala2 sequence, or the Pro3 sequence were found to have reduced codon misreading, and particularly the tRNAs engineered to have the Pro2 sequence, the Leu2 sequence, the Ala2 sequence, or the Pro3 sequence showed highly effective reduction of codon misreading (Fig. 7).

[Table 12]

| Aminoacylated tRNA | Template mRNA sequence | Translated peptide compound | Amount of translation (μM) |
|---|---|---|---|
| AATR-81 | MR-2 | BdpF:Thr:Phe:Ile:Ile:Gly:Phe:SPh2Cl: Ile:Ile:Pro:Ile:Gly | 0.69 |
| AATR-113 | | BdpF:Thr:Phe:Ile:Ile:Gly:Phe:MeHph: Ile:Ile:Pro:Ile:Gly | 0.60 |
| AATR-82 | | BdpF:Thr:Phe:Ile:Ile:Gly:Phe:SPh2Cl: Ile:Ile:Pro:Ile:Gly | 0.77 |
| AATR-113 | | BdpF:Thr:Phe:Ile:Ile:Gly:Phe:MeHph: Ile:Ile:Pro:Ile:Gly | 0.58 |
| AATR-83 | | BdpF:Thr:Phe:Ile:Ile:Gly:Phe:SPh2Cl: Ile:Ile:Pro:Ile:Gly | 0.84 |
| AATR-113 | | BdpF:Thr:Phe:Ile:Ile:Gly:Phe:MeHph: Ile:Ile:Pro:Ile:Gly | 0.63 |
| AATR-84 | | BdpF:Thr:Phe:Ile:Ile:Gly:Phe:SPh2Cl: Ile:Ile:Pro:Ile:Gly | 0.71 |
| AATR-113 | | BdpF:Thr:Phe:Ile:Ile:Gly:Phe:MeHph: Ile:Ile:Pro:Ile:Gly | 0.59 |
| AATR-85 | | BdpF:Thr:Phe:Ile:Ile:Gly:Phe:SPh2Cl: Ile:Ile:Pro:Ile:Gly | 0.57 |
| AATR-113 | | BdpF:Thr:Phe:Ile:Ile:Gly:Phe:MeHph: Ile:Ile:Pro:Ile:Gly | 0.63 |
| AATR-86 | | BdpF:Thr:Phe:Ile:Ile:Gly:Phe:SPh2Cl: Ile:Ile:Pro:Ile:Gly | 0.57 |
| AATR-113 | | BdpF:Thr:Phe:Ile:Ile:Gly:Phe:MeHph: Ile:Ile:Pro:Ile:Gly | 0.65 |
| AATR-87 | | BdpF:Thr:Phe:Ile:Ile:Gly:Phe:SPh2Cl: Ile:Ile:Pro:Ile:Gly | 0.19 |
| AATR-113 | | BdpF:ThrPhe:Ile:Ile:Gly:Phe:MeHph: Ile:Ile:Pro:Ile:Gly | 0.64 |
| AATR-88 | | BdpF: Thr:Phe:Ile:Ile:Gly:Phe: SPh2Cl:Ile:Ile:Pro:Ile:Gly | 0.30 |
| AATR-113 | | BdpF:Thr:Phe:Ile:Ile:Gly:Phe:MeHph: Ile:Ile:Pro:Ile:Gly | 0.57 |
| AATR-89 | | BdpF:Thr:Phe:Ile:Ile:Gly:Phe:SPh2Cl: Ile:Ile:Pro:Ile:Gly | 0.07 |
| AATR-113 | | BdpF:Thr:Phe:Ile:Ile:Gly:Phe:MeHph: Ile:Ile:Pro:Ile:Gly | 0.61 |
| AATR-90 | | BdpF:Thr:Phe:Ile:Ile:Gly:Phe:SPh2Cl: Ile:Ile:Pro:Ile:Gly | 0.08 |
| AATR-113 | | BdpF:Thr:Phe:Ile:Ile:Gly:Phe:MeHph: Ile:Ile:Pro:Ile:Gly | 0.70 |

The following table shows the translation results of CUU codon discrimination by a tRNA which carries the tRNA(AsnE2) body with its combination of bases at positions 32, 33, 37, and 38 being engineered, and which carries cag or aag as the anticodon. Compared to tRNA(AsnE2) (AAtR-91), whose combination of bases at positions 32, 33, 37, and 38 had not been engineered, tRNAs (AAtR-95 to 100) in which the combination derived from the above-mentioned

bases had been engineered to be the Arg3 sequence, the Val2 sequence, the Pro2 sequence, the Leu2 sequence, the Ala2 sequence, or the Pro3 sequence were found to have reduced codon misreading, and particularly the tRNAs engineered to have the Pro2 sequence, the Leu2 sequence, the Ala2 sequence, or the Pro3 sequence showed highly effective reduction of codon misreading (Fig. 8).

[Table 13]

| Aminoacylated tRNA | Template mRNA sequence | Translated peptide compound | Amount of translation (μM) |
|---|---|---|---|
| AATR-91 | MR-1 | BdpF:Thr:Phe:Ile:Ile:Gly:Phe:SPh2Cl:Ile:Ile:Pro:Ile:Gly | 0.36 |
| AATR-112 | | BdpF:Thr:Phe:Ile:Ile:Gly:Phe:MeHph:Ile:Ile:Pro:Ile:Gly | 0.69 |
| AATR-92 | | BdpF:Thr:Phe:Ile:Ile:Gly:Phe:SPh2Cl:Ile:Ile:Pro:Ile:Gly | 0.49 |
| AATR-112 | | BdpF:Thr:Phe:Ile:Ile:Gly:Phe:MeHph:Ile:Ile:Pro:Ile:Gly | 0.65 |
| AATR-93 | | BdpF:Thr:Phe:Ile:Ile:Gly:Phe:SPh2Cl:Ile:Ile:Pro:Ile:Gly | 0.40 |
| AATR-112 | | BdpF:Thr:Phe:Ile:Ile:Gly:Phe:MeHph:Ile:Ile:Pro:Ile:Gly | 0.68 |
| AATR-94 | | BdpF:Thr:Phe:Ile:Ile:Gly:Phe:SPh2Cl:Ile:Ile:Pro:Ile:Gly | 0.39 |
| AATR-112 | | BdpF:Thr:Phe:Ile:Ile:Gly:Phe:MeHph:Ile:Ile:Pro:Ile:Gly | 0.66 |
| AATR-95 | | BdpF:Thr:Phe:Ile:Ile:Gly:Phe:SPh2Cl:Ile:Ile:Pro:Ile:Gly | 0.13 |
| AATR-112 | | BdpF:Thr:Phe:Ile:Ile:Gly:Phe:MeHph:Ile:Ile:Pro:Ile:Gly | 0.74 |
| AATR-96 | | BdpF:Thr:Phe:Ile:Ile:Gly:Phe:SPh2Cl:Ile:Ile:Pro:Ile:Gly | 0.14 |
| AATR-112 | | BdpF:Thr:Phe:Ile:Ile:Gly:Phe:MeHph:Ile:Ile:Pro:Ile:Gly | 0.74 |
| AATR-97 | | BdpF:Thr:Phe:Ile:Ile:Gly:Phe:SPh2Cl:Ile:Ile:Pro:Ile:Gly | 0.05 |
| AATR-112 | | BdpF:ThrPhe:Ile:Ile:Gly:Phe:MeHph:Ile:Ile:Pro:Ile:Gly | 0.79 |
| AATR-98 | | BdpF: Thr:Phe:Ile:Ile:Gly:Phe:SPh2Cl:Ile:Ile:Pro:Ile:Gly | 0.07 |
| AATR-112 | | BdpF:Thr:Phe:Ile:Ile:Gly:Phe:MeHph:Ile:Ile:Pro:Ile:Gly | 0.84 |
| AATR-99 | | BdpF:Thr:Phe:Ile:Ile:Gly:Phe:SPh2Cl:Ile:Ile:Pro:Ile:Gly | 0.04 |
| AATR-112 | | BdpF:Thr:Phe:Ile:Ile:Gly:Phe:MeHph:Ile:Ile:Pro:Ile:Gly | 0.83 |
| AATR-100 | | BdpF: Thr:Phe:Ile:Ile:Gly:Phe:SPh2Cl:Ile:Ile:Pro:Ile:Gly | 0.04 |
| AATR-112 | | BdpF:Thr:Phe:Ile:Ile:Gly:Phe:MeHph:Ile:Ile:Pro:Ile:Gly | 0.84 |

The above results indicated that even for tRNAs prone to codon misreading, the misreading can be reduced by engineering the combination of bases at positions 32, 33, 37, and 38.

Reduction effects on codon misreading in other tRNA bodies were verified. The following table shows the translation results of CUG codon discrimination by a tRNA which carries the tRNA(Aspl) body with its combination of bases at positions 32, 33, 37, and 38 being engineered, and which carries aag or cag as the anticodon. Compared to tRNA(Aspl) (AAtR-64), whose combination of bases at positions 32, 33, 37, and 38 had not been engineered, tRNAs (AAtR-65 to 70) in which the combination derived from the above-mentioned bases had been engineered to be the Arg3 sequence, the Val2 sequence, the Pro2 sequence, the Leu2 sequence, the Ala2 sequence, or the Pro3 sequence were found to have reduced codon misreading (Fig. 9).

[Table 14]

| Aminoacvlated tRNA | Template mRNA sequence | Translated peptide compound | Amount of translation (μM) |
|---|---|---|---|
| AA TR-61 | MR-2 | BdpF:Thr:Phe:Ile:Ile:Gly:Phe:SPh2Cl:Ile:Ile:Pro:Ile:Gly | 0.52 |
| AATR-111 | | BdpF:Thr:Phe:Ile:Ile:Gly:Phe:MeHph:Ile:Ile:Pro:Ile:Gly | 1.37 |
| AATR-62 | | BdpF:Thr:Phe:Ile:Ile:Gly:Phe:SPh2Cl:Ile:Ile:Pro:Ile:Gly | 0.48 |
| AATR-111 | | BdpF:Thr:Phe:Ile:Ile:Gly:Phe:MeHph:Ile:Ile:Pro:Ile:Gly | 1.33 |
| AATR-63 | | BdpF:Thr:Phe:Ile:Ile:Gly:Phe:SPh2Cl:Ile:Ile:Pro:Ile:Gly | 0.35 |
| AATR-111 | | BdpF:Thr:Phe:Ile:Ile:Gly:Phe:MeHph:Ile:Ile:Pro:Ile:Gly | 1.42 |
| AATR-64 | | BdpF:Thr:Phe:Ile:Ile:Gly:Phe:SPh2Cl:Ile:Ile:Pro:Ile:Gly | 0.27 |
| AATR-111 | | BdpF:Thr:Phe:Ile:Ile:Gly:Phe:MeHph:Ile:Ile:Pro:Ile:Gly | 1.40 |
| AATR-65 | | BdpF:Thr:Phe:Ile:Ile:Gly:Phe:SPh2Cl:Ile:Ile:Pro:Ile:Gly | 0.22 |
| AATR-111 | | BdpF:Thr:Phe:Ile:Ile:Gly:Phe:MeHph:Ile:Ile:Pro:Ile:Gly | 1.37 |
| AATR-66 | | BdpF:Thr:Phe:Ile:Ile:Gly:Phe:SPh2Cl:Ile:Ile:Pro:Ile:Gly | 0.18 |
| AATR-111 | | BdpF:Thr:Phe:Ile:Ile:Gly:Phe:MeHph:Ile:Ile:Pro:Ile:Gly | 1.29 |
| AATR-67 | | BdpF:Thr:Phe:Ile:Ile:Gly:Phe:SPh2Cl:Ile:Ile:Pro:Ile:Gly | 0.15 |
| AATR-111 | | BdpF:Thr:Phe:Ile:Ile:Gly:Phe:MeHph:Ile:Ile:Pro:Ile:Gly | 1.22 |
| AATR-68 | | BdpF: Thr:Phe:Ile:Ile:Gly:Phe:SPh2Cl:Ile:Ile:Pro:Ile:Gly | 0.16 |
| AATR-111 | | BdpF:Thr:Phe:Ile:Ile:Gly:Phe:MeHph:Ile:Ile:Pro:Ile:Gly | 1.28 |
| AATR-69 | | BdpF:Thr:Phe:Ile:Ile:Gly:Phe:SPh2Cl:Ile:Ile:Pro:Ile:Gly | 0.15 |
| AATR-111 | | BdpF:Thr:Phe:Ile:Ile:Gly:Phe:MeHph:Ile:Ile:Pro:Ile:Gly | 1.23 |
| AATR-70 | | BdpF:Thr:Phe:Ile:Ile:Gly:Phe:SPh2Cl:Ile:Ile:Pro:Ile:Gly | 0.16 |
| AATR-111 | | BdpF:Thr:Phe:Ile:Ile:Gly:Phe:MeHph:Ile:Ile:Pro:Ile:Gly | 1.3? |

The following table shows the translation results of CUU codon discrimination by a tRNA which carries the tRNA(Aspl) body with its combination of bases at positions 32, 33, 37, and 38 being engineered, and which carries cag or aag as the anticodon. Although the amount of translated peptides obtained by codon misreading was low for tRNA(Aspl) (AAtR-74), whose combination of bases at positions 32, 33, 37, and 38 had not been engineered, codon misreading

was also reduced for tRNAs (AAtR-76 to 80) in which the combination derived from the above-mentioned bases had been engineered to be the Phe sequence, the Val2 sequence, the Pro2 sequence, the Leu2 sequence, the Ala2 sequence, or the Pro3 sequence, and a trend similar to the above Examples were observed (Fig. 10).

[Table 15]

| Aminoacvlated tRNA | Template mRNA sequence | Translated peptide compound | Amount of translation (μM) |
|---|---|---|---|
| AATR-71 | MR-1 | BdpF:Thr:Phe:Ile:Ile:Gly:Phe:SPh2Cl:Ile:Ile:Pro:Ile:Gly | 0.12 |
| AATR-110 | | BdpF:Thr:Phe:Ile:Ile:Gly:Phe:MeHph:Ile:Ile:Pro:Ile:Gly | 1.44 |
| AATR-72 | | BdpF:Thr:Phe:Ile:Ile:Gly:Phe:SPh2Cl:Ile:Ile:Pro:Ile:Gly | 0.11 |
| AATR-110 | | BdpF:Thr:Phe:Ile:Ile:Gly:Phe:MeHph:Ile:Ile:Pro:Ile:Gly | 1.45 |
| AATR-73 | | BdpF:Thr:Phe:Ile:Ile:Gly:Phe:SPh2Cl:Ile:Ile:Pro:Ile:Gly | 0.09 |
| AATR-110 | | BdpF:Thr:Phe:Ile:Ile:Gly:Phe:MeHph:Ile:Ile:Pro:Ile:Gly | 1.40 |
| AATR-74 | | BdpF:Thr:Phe:Ile:Ile:Gly:Phe:SPh2Cl:Ile:Ile:Pro:Ile:Gly | 0.09 |
| AATR-110 | | BdpF:Thr:Phe:Ile:Ile:Gly:Phe:MeHph:Ile:Ile:Pro:Ile:Gly | 1.38 |
| AATR-75 | | BdpF:Thr:Phe:Ile:Ile:Gly:Phe:SPh2Cl:Ile:Ile:Pro:Ile:Gly | 0.09 |
| AATR-110 | | BdpF:Thr:Phe:Ile:Ile:Gly:Phe:MeHph:Ile:Ile:Pro:Ile:Gly | 1.37 |
| AATR-76 | | BdpF:Thr:Phe:Ile:Ile:Gly:Phe:SPh2Cl:Ile:Ile:Pro:Ile:Gly | 0.08 |
| AATR-110 | | BdpF:Thr:Phe:Ile:Ile:Gly:Phe:MeHph:Ile:Ile:Pro:Ile:Gly | 1.37 |
| AATR-77 | | BdpF:Thr:Phe:Ile:Ile:Gly:Phe:SPh2Cl:Ile:Ile:Pro:Ile:Gly | 0.07 |
| AATR-110 | | BdpF:Thr:Phe:Ile:Ile:Gly:Phe:MeHph:Ile:Ile:Pro:Ile:Gly | 1.29 |
| AATR-78 | | BdpF:Thr:Phe:Ile:Ile:Gly:Phe:SPh2Cl:Ile:Ile:Pro:Ile:Gly | 0.08 |
| AATR-110 | | BdpF:Thr:Phe:Ile:Ile:Glv:Phe:MeHph:Ile:Ile:Pro:Ile:Gly | 1.37 |
| AATR-79 | | BdpF:Thr:Phe:Ile:Ile:Gly:Phe:SPh2Cl:Ile:Ile:Pro:Ile:Gly | 0.08 |
| AATR-110 | | BdpF:Thr:Phe:Ile:Ile:Gly:Phe:MeHph:Ile:Ile:Pro:Ile:Gly | 1.30 |
| AATR-80 | | BdpF: Thr:Phe:Ile:Ile:Gly:Phe:SPh2Cl:Ile:Ile:Pro:Ile:Gly | 0.08 |
| AATR-110 | | BdpF:Thr:Phe:Ile:Ile:Gly:Phe:MeHph:Ile:Ile:Pro:Ile:Gly | 1.26 |

Effects of reducing misreading when using codons whose bases at their third letters are different from those of the codons used in the above Examples were verified. The following table shows the translation results of GGA codon discrimination by a tRNA, whose combination of bases at positions 32, 33, 37, and 38 had been engineered, and which carries ucc or acc as the anticodon. When the combination of bases at positions 32, 33, 37, and 38 in a tRNA carrying the acc anticodon was engineered to be the Leu2 sequence or the Pro3 sequence, misreading of the GGA codon was reduced (Fig. 11).

[Table 16]

| Aminoacvlated tRNA | Template mRNA sequence | Translated peptide compound | Amount of translation (μM) |
|---|---|---|---|
| AA TR-44 | MR-7 | BdpF:Thr:Phe:Ile:Ile:Leu:Phe: SPh2Cl:Ile:Ile:Pro:Ile:Leu | 0.55 |
| AATR-114 | | BdpF:Thr:Phe:Ile:Ile:Leu:Phe:MeHph: Ile:Ile:Pro:Ile:Leu | 0.79 |
| AATR-47 | | BdpF:Thr:Phe:Ile:Ile:Leu:Phe: SPh2Cl:Ile:Ile:Pro:Ile:Leu | 0.14 |
| AATR-114 | | BdpF:Thr:Phe:Ile:Ile:Leu:Phe:MeH ph:Ile:Ile:Pro:Ile:Leu | 1.20 |
| AA TR-48 | | BdpF:Thr:Phe:Ile:Ile:Leu:Phe: SPh2Cl:Ile:Ile:Pro:Ile:Leu | 0.15 |
| AATR-114 | | BdpF:Thr:Phe:Ile:Ile:Leu:Phe:MeHph: Ile:Ile:Pro:Ile:Leu | 1.35 |

The following table shows the translation results of GGA codon discrimination by a tRNA, whose combination of bases at positions 32, 33, 37, and 38 had been engineered, and which carries ucc or gcc as the anticodon. When the combination of bases at positions 32, 33, 37, and 38 in a tRNA carrying the gcc anticodon was engineered to be the Leu2 sequence or the Pro3 sequence, misreading of the GGA codon was reduced (Fig. 12).

[Table 17]

| Aminoacylated tRNA | Template mRNA sequence | Translated peptide compound | Amount of translation (μM) |
|---|---|---|---|
| AATR-101 | MR-7 | BdpF:Thr:Phe:Ile:Ile:Leu:Phe: SPh2Cl:Ile:Ile:Pro:Ile:Leu | 0.87 |
| AATR-114 | | BdpF:Thr:Phe:Ile:Ile:Leu:Phe:MeHph: Ile:Ile:Pro:Ile:Leu | 0.97 |
| AATR-102 | | BdpF:Thr:Phe:Ile:Ile:Leu:Phe: SPh2Cl:Ile:Ile:Pro:Ile:Leu | 0.16 |
| AATR-114 | | BdpF:Thr:Phe:Ile:Ile:Leu:Phe:MeHph: Ile:Ile:Pro:Ile:Leu | 1.05 |
| AATR-103 | | BdpF:Thr:Phe:Ile:Ile:Leu:Phe: SPh2Cl:Ile:Ile:Pro:Ile:Leu | 0.24 |
| AATR-114 | | BdpF:Thr:Phe:Ile:Ile:Leu:Phe:MeHph: Ile:Ile:Pro:Ile:Leu | 1.18 |

The following table shows the translation results of GGG codon discrimination by a tRNA, whose combination of bases at positions 32, 33, 37, and 38 had been engineered, and which carries ccc or gcc as the anticodon. When the combination of bases at positions 32, 33, 37, and 38 in a tRNA carrying the gcc anticodon was engineered to be the Leu2 sequence or the Pro3 sequence, misreading of the GGG codon was reduced (Fig. 13).

[Table 18]

| Aminoacylated tRNA | Template mRNA sequence | Translated peptide compound | Amount of translation (μM) |
|---|---|---|---|
| AATR-101 | MR-6 | BdpF:Thr:Phe:Ile:Ile:Leu:Phe: SPh2Cl:Ile:Ile:Pro:Ile:Leu | 0.22 |
| AATR-109 | | BdpF:Thr:Phe:Ile:Ile:Leu:Phe:MeHph: Ile:Ile:Pro:Ile:Leu | 1.38 |
| AATR-102 | | BdpF:Thr:Phe:Ile:Ile:Leu:Phe: SPh2Cl:Ile:Ile:Pro:Ile:Leu | 0.13 |
| AATR-109 | | BdpF:Thr:Phe:Ile:Ile:Leu:Phe:MeHph: Ile:Ile:Pro:Ile:Leu | 1.47 |
| AATR-103 | | BdpF:Thr:Phe:Ile:Ile:Leu:Phe: SPh2Cl:Ile:Ile:Pro:Ile:Leu | 0.12 |
| AATR-109 | | BdpF:Thr:Phe:Ile:Ile:Leu:Phe:MeHph: Ile:Ile:Pro:Ile:Leu | 1.46 |

The above results indicated that regardless of the type of base at the third letter of the codon, effects of reducing codon misreading are observed. This shows that no matter which codon within the codon box is employed, effects of reducing codon misreading according to the present disclosure are achieved. When codons are reprogrammed to assign different types of amino acids to the same codon box, capability of achieving the effects of the present disclosure regardless of the codon used will allow greater degrees of freedom for the reprogramming and is thus beneficial.

Evaluation of discrimination of the CUU, CUA, or CUG codon by a tRNA, whose combination of bases at positions 32, 33, 37, and 38 had been engineered, and which carries aag, uag, or cag as the anticodon, was performed in the presence or absence of lysidine modification. As tRNA bodies, tRNA(Asp1+Leu2) and tRNA(AsnE2+Leu2) were used. The translation results are shown in the following table. It was shown that techniques to modify tRNAs can be combined. For quantitative evaluation, LCT-12 was used as a standard to prepare a calibration curve, and the content was calculated by relative quantification. The LC-MS was analyzed according to the conditions of Method 2 of Table 5.

[Table 19]

| Aminoacylated tRNA | Template mRNA sequence | Translated peptide compound | Amount of translation (μM) |
|---|---|---|---|
| AATR-131 | MR-1 | BdpF:Thr:Phe:Ile:Ile:Gly:Phe:nBuG:Ile: Ile:Pro:Ile:Gly | 0.14 |
| AATR-132 | | BdpF:Thr:Phe:Ile:Ile:Gly:Phe:SPh2Cl:Ile: Ile:Pro:Ile:Gly | 0.04 |
| AATR-134 | | BdpF:Thr:Phe:Ile:Ile:Gly:Phe:dA:Ile:Ile: Pro:Ile:Gly | 0.00 |
| AATR-131 | | BdpF:Thr:Phe:Ile:Ile:Gly:Phe:nBuG:Ile: Ile:Pro:Ile:Gly | 0.16 |
| AATR-133 | | BdpF:Thr:Phe:Ile:Ile:Gly:Phe:SPh2Cl:Ile: Ile:Pro:Ile:Gly | 0.00 |
| AATR-134 | | BdpF:Thr:Phe:Ile:Ile:Gly:Phe:dA:Ile:Ile: Pro:Ile:Gly | 0.00 |

(continued)

| Aminoacylated tRNA | Template mRNA sequence | Translated peptide compound | Amount of translation (μM) |
|---|---|---|---|
| AATR-131 | MR-8 | BdpF:Thr:Phe:Ile:Ile:Gly:Phe:nBuG:Ile:Ile:Pro:Ile:Gly | 0.00 |
| AATR-132 | | BdpF:Thr:Phe:Ile:Ile:Gly:Phe:SPh2Cl:Ile:Ile:Pro:Ile:Gly | 0.16 |
| AATR-134 | | BdpF:Thr:Phe:Ile:Ile:Gly:Phe:dA:Ile:Ile:Pro:Ile:Gly | 0.02 |
| AATR-131 | | BdpF:Thr:Phe:Ile:Ile:Gly:Phe:nBuG:Ile:Ile:Pro:Ile:Gly | 0.00 |
| AATR-133 | | BdpF:Thr:Phe:Ile:Ile:Gly:Phe:SPh2Cl:Ile:Ile:Pro:Ile:Gly | 0.39 |
| AATR-134 | | BdpF:Thr:Phe:Ile:Ile:Gly:Phe:dA:Ile:Ile:Pro:Ile:Gly | 0.03 |
| AATR-131 | MR-2 | BdpF:Thr:Phe:Ile:Ile:Gly:Phe:nBuG:Ile:Ile:Pro:Ile:Gly | 0.00 |
| AATR-132 | | BdpF:Thr:Phe:Ile:Ile:Gly:Phe:SPh2Cl:Ile:Ile:Pro:Ile:Gly | 0.00 |
| AATR-134 | | BdpF:Thr:Phe:Ile:Ile:Gly:Phe:dA:Ile:Ile:Pro:Ile:Gly | 0.13 |
| AATR-131 | | BdpF:Thr:Phe:Ile:Ile:Gly:Phe:nBuG:Ile:Ile:Pro:Ile:Gly | 0.00 |
| AATR-133 | | BdpF:Thr:Phe:Ile:Ile:Gly:Phe:SPh2Cl:Ile:Ile:Pro:Ile:Gly | 0.00 |
| AATR-134 | | BdpF:Thr:Phe:Ile:Ile:Gly:Phe:dA:Ile:Ile:Pro:Ile:Gly | 0.15 |

[Table 20]

| Aminoacylated tRNA | Template mRNA sequence | Translated peptide compound | Amount of translation (μM) |
|---|---|---|---|
| AATR-135 | MR-1 | BdpF:Thr:Phe:Ile:Ile:Gly:Phe:nBuG:Ile:Ile:Pro:Ile:Gly | 0.18 |
| AATR-136 | | BdpF:Thr:Phe:Ile:Ile:Gly:Phe:SPh2Cl:Ile:Ile:Pro:Ile:Gly | 0.22 |
| AATR-138 | | BdpF:Thr:Phe:Ile:Ile:Gly:Phe:dA:Ile:Ile:Pro:Ile:Gly | 0.00 |
| AATR-135 | | BdpF:Thr:Phe:Ile:Ile:Gly:Phe:nBuG:Ile:Ile:Pro:Ile:Gly | 0.26 |
| AATR-137 | | BdpF:Thr:Phe:Ile:Ile:Gly:Phe:SPh2Cl:Ile:Ile:Pro:Ile:Gly | 0.02 |
| AATR-138 | | BdpF:Thr:Phe:Ile:Ile:Gly:Phe:dA:Ile:Ile:Pro:Ile:Gly | 0.00 |

(continued)

| Aminoacylated tRNA | Template mRNA sequence | Translated peptide compound | Amount of translation (μM) |
|---|---|---|---|
| AATR-135 | MR-8 | BdpF:Thr:Phe:Ile:Ile:Gly:Phe:nBuG:Ile:Ile:Pro:Ile:Gly | 0.00 |
| AATR-136 | | BdpF:Thr:Phe:Ile:Ile:Gly:Phe:SPh2Cl:Ile:Ile:Pro:Ile:Gly | 0.45 |
| AATR-138 | | BdpF:Thr:Phe:Ile:Ile:Gly:Phe:dA:Ile:Ile:Pro:Ile:Gly | 0.01 |
| AATR-135 | | BdpF:Thr:Phe:Ile:Ile:Gly:Phe:nBuG:Ile:Ile:Pro:Ile:Gly | 0.00 |
| AATR-137 | | BdpF:Thr:Phe:Ile:Ile:Gly:Phe:SPh2Cl:Ile:Ile:Pro:Ile:Gly | 0.64 |
| AATR-138 | | BdpF:Thr:Phe:Ile:Ile:Gly:Phe:dA:Ile:Ile:Pro:Ile:Gly | 0.02 |
| AATR-135 | MR-2 | BdpF:Thr:Phe:Ile:Ile:Gly:Phe:nBuG:Ile:Ile:Pro:Ile:Gly | 0.00 |
| AATR-136 | | BdpF:Thr:Phe:Ile:Ile:Gly:Phe:SPh2Cl:Ile:Ile:Pro:Ile:Gly | 0.12 |
| AATR-138 | | BdpF:Thr:Phe:Ile:Ile:Gly:Phe:dA:Ile:Ile:Pro:Ile:Gly | 0.34 |
| AATR-135 | | BdpF:Thr:Phe:Ile:Ile:Gly:Phe:nBuG:Ile:Ile:Pro:Ile:Gly | 0.00 |
| AATR-137 | | BdpF:Thr:Phe:Ile:Ile:Gly:Phe:SPh2Cl:Ile:Ile:Pro:Ile:Gly | 0.00 |
| AATR-138 | | BdpF:Thr:Phe:Ile:Ile:Gly:Phe:dA:Ile:Ile:Pro:Ile:Gly | 0.45 |

Effects of reducing codon misreading was verified when the type of amino acid was changed. The following table shows the translation results of GGG codon discrimination by a tRNA, whose combination of bases at positions 32, 33, 37, and 38 had been engineered, and which carries acc or ccc as the anticodon. Reduction of codon misreading was observed in tRNAs whose combination of bases at positions 32, 33, 37, and 38 had been engineered to be the Leu2 sequence, the Ala2 sequence, or the Pro3 sequence (Fig. 14).

[Table 21]

| Aminoacylated tRNA | Template mRNA sequence | Translated peptide compound | Amount of translation (μM) |
|---|---|---|---|
| AATR-115 | MR-6 | BdpF:Thr:Phe:Ile:Ile:Leu:Phe:Pic2: Ile:Ile:Pro:Ile:Leu | 0.56 |
| AATR-109 | | BdF:Thr:Phe:Ile:Ile:Leu:Phe:MeHph: Ile:Ile:Pro:Ile:Leu | 1.47 |
| AATR-116 | | BdpF:Thr:Phe:Ile:Ile:Leu:Phe:Pic2: Ile:Ile:Pro:Ile:Leu | 0.06 |
| AATR-109 | | BdpF:Thr:Phe:Ile:Ile:Leu:Phe:MeHph: Ile:Ile:Pro:Ile:Leu | 1.24 |
| AATR-117 | | BdpF:Thr:Phe:Ile:Ile:Leu:Phe:Pic2: Ile:Ile:Pro:Ile:Leu | 0.08 |
| AATR-109 | | BdpF:Thr:Phe:Ile:Ile:Leu:Phe:MeHph: Ile:Ile:Pro:Ile:Leu | 1.37 |
| AATR-118 | | BdpF:Thr:Phe:Ile:Ile:Leu:Phe:Pic2: Ile:Ile:Pro:Ile:Leu | 0.07 |
| AATR-109 | | BdpF:Thr:Phe:Ile:Ile:Leu:Phe:MeHph: Ile:Ile:Pro:Ile:Leu | 1.18 |

The following table shows the translation results of GGG codon discrimination by a tRNA, whose combination of bases at positions 32, 33, 37, and 38 had been engineered and which carries acc or ccc as the anticodon. Reduction of codon misreading was observed in tRNAs whose combination of bases at positions 32, 33, 37, and 38 had been engineered to be the Leu2 sequence, the Ala2 sequence, or the Pro3 sequence (Fig. 15).

[Table 22]

| Aminoacylated tRNA | Template mRNA sequence | Translated peptide compound | Amount of translation (μM) |
|---|---|---|---|
| AATR-119 | MR-6 | BdpF:Thr:Phe:Ile:Ile:Leu:Phe:MeG: Ile:Ile:Pro:Ile:Leu | 0.28 |
| AATR-109 | | BdpF:Thr:Phe:Ile:Ile:Leu:Phe:MeHph: Ile:Ile:Pro:Ile:Leu | 0.95 |
| AATR-120 | | BdpF:Thr:Phe:Ile:Ile:Leu:Phe:MeG: Ile:Ile:Pro:Ile:Leu | 0.03 |
| AATR-109 | | BdpF:Thr:Phe:Ile:Ile:Leu:Phe:MeHph: Ile:Ile:Pro:Ile:Leu | 1.16 |
| AATR-121 | | BdpF:Thr:Phe:Ile:Ile:Leu:Phe:MeG: Ile:Ile:Pro.Ile:Leu | 0.04 |
| AATR-109 | | BdpF:Thr:Phe:Ile:Ile:Leu:Phe:MeHph: Ile:Ile:Pro:Ile:Leu | 1.29 |
| AATR-122 | | BdpF:Thr:Phe:Ile:Ile:Leu:Phe:MeG: Ile:Ile:Pro:Ile:Leu | 0.03 |
| AATR-109 | | BdpF:Thr:Phe:Ile:Ile:Leu:Phe:MeHph: Ile:Ile:Pro:Ile:Leu | 1.11 |

The following table shows the translation results of GGG codon discrimination by a tRNA, whose combination of bases at positions 32, 33, 37, and 38 had been engineered, and which carries acc or ccc as the anticodon. Reduction of codon misreading was observed in tRNAs whose combination of bases at positions 32, 33, 37, and 38 had been engineered to be the Leu2 sequence, the Ala2 sequence, or the Pro3 sequence (Fig. 16).

[Table 23]

| Aminoacylated tRNA | Template mRNA sequence | Translated peptide compound | Amount of translation (μM) |
|---|---|---|---|
| AATR-123 | MR-6 | BdpF:Thr:Phe:Ile:Ile:Leu:Phe:dA:Ile:Ile:Pro:Ile:Leu | 0.28 |
| AATR-127 | | BdpF:Thr:Phe:Ile:Ile:Leu:Phe:nBuG:Ile:Ile:Pro:Ile:Leu | 0.94 |
| AATR-124 | | BdpF:Thr:Phe:Ile:Ile:Leu:Phe:dA:Ile:Ile:Pro:Ile:Leu | 0.03 |
| AATR-127 | | BdpF:Thr:Phe:Ile:Ile:Leu:Phe:nBuG:Ile:Ile:Pro:Ile:Leu | 0.81 |
| AATR-125 | | BdpF:Thr:Phe:Ile:Ile:Leu:Phe:dA:Ile:Ile:Pro:Ile:Leu | 0.05 |
| AATR-127 | | BdpF:Thr:Phe:Ile:Ile:Leu:Phe:nBuG:Ile:Ile:Pro:Ile:Leu | 0.95 |
| AATR-126 | | BdpF:Thr:Phe:Ile:Ile:Leu:Phe:dA:Ile:Ile:Pro:Ile:Leu | 0.02 |
| AATR-127 | | BdpF:Thr:Phe:Ile:Ile:Leu:Phe:nBuG:Ile:Ile:Pro:Ile:Leu | 0.88 |

The following table shows the translation results of CCG codon discrimination by a tRNA, whose combination of bases at positions 32, 33, 37, and 38 had been engineered, and which carries cgg or agg as the anticodon. Reduction of codon misreading was observed in tRNAs whose combination of bases at positions 32, 33, 37, and 38 had been engineered to be the Pro2 sequence, the Leu2 sequence, the Ala2 sequence, or the Pro3 sequence (Fig. 17).

[Table 24]

| Aminoacylated tRNA | Template mRNA sequence | Translated peptide compound | Amount of translation (μM) |
|---|---|---|---|
| AATR-21 | MR-4 | BdpF:Thr:Phe:Ile:Ile:Gly:Phe:SPh2Cl:Ile:Ile:Ala:Ile:Gly | 0.57 |
| AATR-130 | | BdpF:Thr:Phe:Ile:Ile:Gly:Phe:MeG:Ile:Ile:Ala:Ile:Gly | 2.37 |
| AATR-22 | | BdpF:Thr:Phe:Ile:Ile:Gly:Phe:SPh2Cl:Ile:Ile:Ala:Ile:Gly | 0.43 |
| AATR-130 | | BdpF:Thr:Phe:Ile:Ile:Gly:Phe:MeG:Ile:Ile:Ala:Ile:Gly | 1.94 |
| AATR-23 | | BdpF:Thr:Phe:Ile:Ile:Gly:Phe:SPh2Cl:Ile:Ile:Ala:Ile:Gly | 0.53 |
| AATR-130 | | BdpF:Thr:Phe:Ile:Ile:Gly:Phe:MeG:Ile:Ile:Ala:Ile:Gly | 2.45 |
| AATR-24 | | BdpF:Thr:Phe:Ile:Ile:Gly:Phe:SPh2Cl:Ile:Ile:Ala:Ile:Gly | 0.32 |
| AATR-130 | | BdpF:Thr:Phe:Ile:Ile:Gly:Phe:MeG:Ile:Ile:Ala:Ile:Gly | 2.14 |
| AATR-25 | | BdpF:Thr:Phe:Ile:Ile:Gly:Phe:SPh2Cl:Ile:Ile:Ala:Ile:Gly | 0.27 |
| AATR-130 | | BdpF:Thr:Phe:Ile:Ile:Gly:Phe:MeG:Ile:Ile:Ala:Ile:Gly | 1.72 |
| AATR-26 | | BdpF:Thr:Phe:Ile:Ile:Gly:Phe:SPh2Cl:Ile:Ile:Ala:Ile:Gly | 0.36 |
| AATR-130 | | BdpF:Thr:Phe:Ile:Ile:Gly:Phe:MeG:Ile:Ile:Ala:Ile:Gly | 2.45 |
| AATR-27 | | BdpF:Thr:Phe:Ile:Ile:Gly:Phe:SPh2Cl:Ile:Ile:Ala:Ile:Gly | 0.18 |
| AATR-130 | | BdpF:Thr:Phe:Ile:Ile:Gly:Phe:MeG:Ile:Ile:Ala:Ile:Gly | 2.04 |
| AATR-28 | | BdpF:Thr:Phe:Ile:Ile:Gly:Phe:SPh2Cl:Ile:Ile:Ala:Ile:Gly | 0.25 |
| AATR-130 | | BdpF:Thr:Phe:Ile:Ile:Gly:Phe:MeG:Ile:Ile:Ala:Ile:Gly | 2.50 |
| AATR-29 | | BdpF:Thr:Phe:Ile:Ile:Gly:Phe:SPh2Cl:Ile:Ile:Ala:Ile:Gly | 0.12 |
| AATR-130 | | BdpF:Thr:Phe:Ile:Ile:Gly:Phe:MeG:Ile:Ile:Ala:Ile:Gly | 2.59 |
| AATR-30 | | BdpF:Thr:Phe:Ile:Ile:Gly:Phe:SPh2Cl:Ile:Ile:Ala:Ile:Gly | 0.10 |
| AATR-130 | | BdpF:Thr:Phe:Ile:Ile:Gly:Phe:MeG:Ile:Ile:Ala:Ile:Gly | 2.07 |

The following table shows the translation results of CCU codon discrimination by a tRNA, whose combination of bases at positions 32, 33, 37, and 38 had been engineered, and which carries agg or cgg as the anticodon. Reduction of codon misreading was observed in tRNAs whose combination of bases at positions 32, 33, 37, and 38 had been engineered to be the Phe sequence, the Pro2 sequence, the Leu2 sequence, the Ala2 sequence, the Pro3 sequence,

the Arg3 sequence, or the Val2 sequence, and among them, the reduction effects were remarkable in those with the Pro2 sequence, the Leu2 sequence, the Ala2 sequence, and the Pro3 sequence (Fig. 18).

[Table 25]

| Aminoacylated tRNA | Template mRNA sequence | Translated peptide compound | Amount of translation (μM) |
|---|---|---|---|
| AATR-31 | MR-3 | BdpF:Thr:Phe:Ile:Ile:Gly:Phe:SPh2Cl:Ile:Ile:Ala:Ile:Gly | 0.35 |
| AA TR-129 | | BdpF:Thr:Phe:Ile:Ile:Gly:Phe:MeG:Ile:Ile:Ala:Ile:Gly | 2.15 |
| AATR-32 | | BdpF:Thr:Phe:Ile:Ile:Gly:Phe:SPh2Cl:Ile:Ile:Ala:Ile:Gly | 0.33 |
| AA TR-129 | | BdpF:Thr:Phe:Ile:Ile:Gly:Phe:MeG:Ile:Ile:Ala:Ile:Gly | 1.90 |
| AATR-33 | | BdpF:ThrPhe:Ile:Ile:Gly:Phe:SPh2Cl:Ile:Ile:Ala:Ile:Gly | 0.26 |
| AATR-129 | | BdpF:Thr:Phe:Ile:Ile:Gly:Phe:MeG:Ile:Ile:Ala:Ile:Gly | 2.41 |
| AATR-34 | | BdpF:Thr:Phe:Ile:Ile:Gly:Phe:SPh2Cl:Ile:Ile:Ala:Ile:Gly | 0.22 |
| AATR-129 | | BdpF:Thr:Phe:Ile:Ile:Gly:Phe:MeG:Ile:Ile:Ala:Ile:Gly | 1.92 |
| AATR-35 | | BdpF:Thr:Phe:Ile:Ile:Gly:Phe:SPh2Cl:Ile:Ile:Ala:Ile:Gly | 0.19 |
| AATR-129 | | BdpF:Thr:Phe:Ile:Ile:Gly:Phe:MeG:Ile:Ile:Ala:Ile:Gly | 2.26 |
| AATR-36 | | BdpF:Thr:Phe:Ile:Ile:Gly:Phe:SPh2Cl:Ile:Ile:Ala:Ile:Gly | 0.18 |
| AATR-129 | | BdpF:Thr:Phe:Ile:Ile:Gly:Phe:MeG:Ile:Ile:Ala:Ile:Gly | 2.07 |
| AATR-37 | | BdpF:Thr:Phe:Ile:Ile:Gly:Phe:SPh2Cl:Ile:Ile:Ala:Ile:Gly | 0.09 |
| AATR-129 | | BdpF:Thr:Phe:Ile:Ile:Gly:Phe:MeG:Ile:Ile:Ala:Ile:Gly | 2.05 |
| AATR-38 | | BdpF:Thr:Phe:Ile:Ile:Gly:Phe:SPh2Cl:Ile:Ile:Ala:Ile:Gly | 0.09 |
| AATR-129 | | BdpF:Thr:Phe:Ile:Ile:Gly:Phe:MeG:Ile:Ile:Ala:Ile:Gly | 2.24 |
| AA TR-39 | | BdpF:Thr:Phe:Ile:Ile:Gly:Phe:SPh2Cl:Ile:Ile:Ala:Ile:Gly | 0.07 |
| AATR-129 | | BdpF:Thr:Phe:Ile:Ile:Gly:Phe:MeG:Ile:Ile:Ala:Ile:Gly | 2.38 |
| AA TR-40 | | BdpF:Thr:Phe:Ile:Ile:Gly:Phe:SPh2Cl:Ile:Ile:Ala:Ile:Gly | 0.06 |
| AATR-129 | | BdpF:Thr:Phe:Ile:Ile:Gly:Phe:MeG:Ile:Ile:Ala:Ile:Gly | 1.99 |

The above results showed that similar results were obtained even when the type of amino acid attached to the

tRNA was changed. Therefore, it is shown that effects of reducing codon misreading can be achieved regardless of the type of amino acid attached to the tRNA, and regardless of the combination of amino acids.

Although the above-mentioned invention has been described in detail using examples and illustrations for the purpose of facilitating a clear understanding, the descriptions and illustrations herein should not be construed as limiting the scope of the present invention. The disclosures of all patent literature and scientific literature cited herein are hereby expressly incorporated by reference in their entirety.

[Industrial Applicability]

Use of a composition for translation, a method for producing peptides, and such of the present disclosure can reduce the rate of mistranslation into unintended amino acids attributable to codon misreading by a tRNA when synthesizing a peptide by translation from a nucleic acid. Compositions, methods, and such of the present disclosure are particularly useful in the field of translational synthesis of peptides.

## Claims

1. A composition for translation, comprising a first tRNA to which a first amino acid is attached and a second tRNA to which a second amino acid is attached,

   wherein a combination of bases at positions 32, 33, 37, and 38 (tRNA numbering rule) in the first tRNA is:

   (1) 32U, 33U, 37G, and 38A;
   (2) 32A, 33U, 37G, and 38U;
   (3) 32A, 33U, 37A, and 38U;
   (4) 32U, 33U, 37G, and 38U;
   (5) 32U, 33U, 37A, and 38U; or
   (6) 32C, 33U, 37G, and 38A;

   bases at the first letters of the anticodons in the first tRNA and the second tRNA are different from each other, bases at the second letters of the anticodons in the first tRNA and the second tRNA are the same, bases at the third letters of the anticodons in the first tRNA and the second tRNA are the same, and
   at least one selected from the first amino acid and the second amino acid is an unnatural amino acid, and wherein A is adenine, C is cytosine, G is guanine, and U is uracil.

2. The composition of claim 1, wherein

   the base at the first letter of the anticodon in the first tRNA is A or G, and the base at the first letter of the anticodon in the second tRNA is C or U, or
   the base at the first letter of the anticodon in the first tRNA is C or U, and the base at the first letter of the anticodon in the second tRNA is A or G.

3. The composition of claim 1 or 2, wherein a combination of bases at the second and third letters of the anticodons

in the first and the second tRNAs is:

(i) G at the second letter and G at the third letter;
(ii) A at the second letter and G at the third letter;
(iii) C at the second letter and C at the third letter;
(iv) G at the second letter and C at the third letter;
(v) A at the second letter and C at the third letter;
(vi) G at the second letter and U at the third letter;
(vii) G at the second letter and A at the third letter; or
(viii) C at the second letter and G at the third letter.

**4.** The composition of any one of claims 1 to 3, wherein a tRNA body of the first tRNA is a chimeric tRNA body.

**5.** The composition of any one of claims 1 to 4, which is a composition for cell-free translation.

**6.** The composition of any one of claims 1 to 5, wherein the chimeric tRNA body is a chimeric tRNA body in which the bases at positions 32, 33, 37, and 38 (tRNA numbering rule) and the base sequence of the other portions have different origins.

**7.** The composition of any one of claims 1 to 6, wherein the first tRNA is an artificial tRNA.

**8.** The composition of any one of claims 1 to 7 (provided that, when the first tRNA and the second tRNA have a chimeric tRNA body which has the bases specified in (4) of claim 1 at positions 32, 33, 37, and 38 (tRNA numbering rule) and which at positions 1 to 31 and positions 39 to 74 (tRNA numbering rule) has the base sequences of positions 1 to 31 and positions 39 to 74 (tRNA numbering rule) of a tRNA having the base sequence of SEQ ID NO: 255, a composition in which the combination of the anticodons in the first tRNA and the second tRNA is GCG and CCG, and a composition in which the combination of the anticodons in the first tRNA and the second tRNA is CCG and GCG, are excluded.

**9.** The composition of any one of claims 1 to 8, wherein the first tRNA is a tRNA carrying a chimeric anticodon loop.

**10.** The composition of any one of claims 1 to 9, wherein the combination of bases at positions 32, 33, 37, and 38 (tRNA numbering rule) in the first tRNA is:

(1) 32U, 33U, 37G, and 38A;
(2) 32A, 33U, 37G, and 38U;
(3) 32A, 33U, 37A, and 38U; or
(4) 32U, 33U, 37G, and 38U.

**11.** The composition of any one of claims 1 to 10, wherein the second amino acid is an unnatural amino acid.

**12.** A method for producing the composition of any one of claims 1 to 11, which comprises preparing the first tRNA by attaching the first amino acid to a tRNA outside a translation system, and/or preparing the second tRNA by attaching the second amino acid to a tRNA outside a translation system.

**13.** A method for producing a peptide, comprising translating a nucleic acid using the composition of any one of claims 1 to 11.

**14.** A method for reducing misreading of a codon complementary to the anticodon in the second tRNA by the first tRNA, wherein the method comprises translating a nucleic acid using the composition of any one of claims 1 to 11.

**15.** A method for reducing misreading of a second codon by a tRNA carrying an anticodon complementary to a first codon, comprising substituting at least one base at a position selected from the group consisting of positions 32, 33, 37, and 38 (tRNA numbering rule) in said tRNA, wherein,

the bases at the first letters of the first codon and the second codon are the same,
the bases at the second letters of the first codon and the second codon are the same, and
the bases at the third letters of the first codon and the second codon are different from each other.

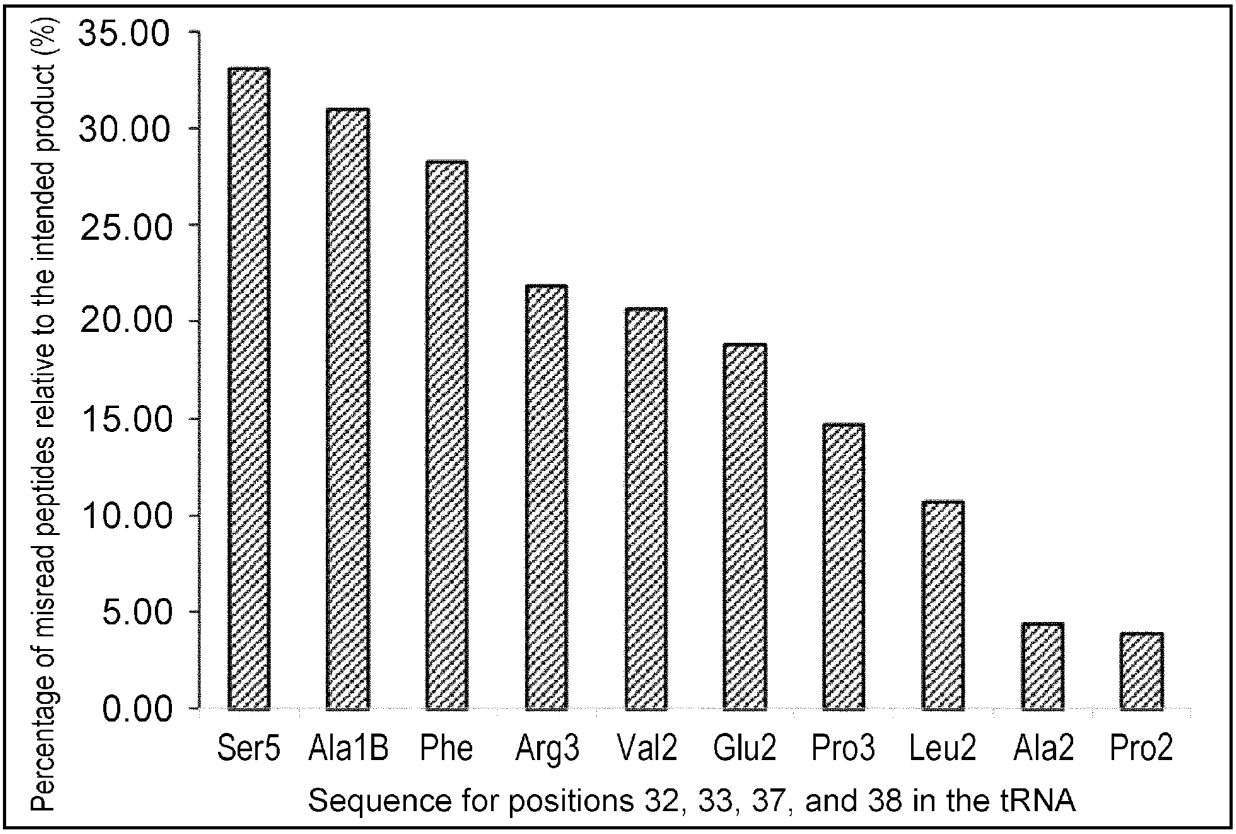
Percentage of misread peptides relative to the intended product (%)
35.00
30.00
25.00
20.00
15.00
10.00
5.00
0.00
Ser5
Ala1B
Phe
Arg3
Val2
Glu2
Pro3
Leu2
Ala2
Pro2
Sequence for positions 32, 33, 37, and 38 in the tRNA

FIG. 1

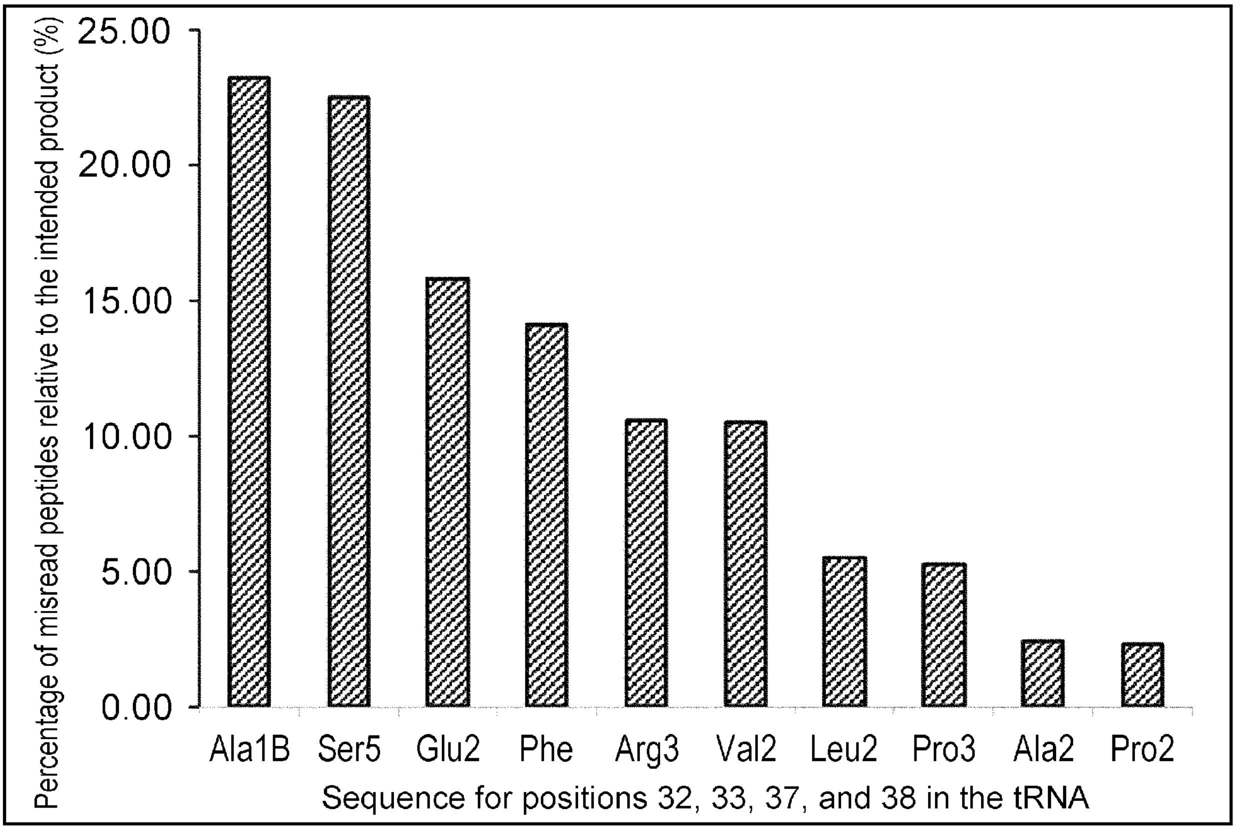
Percentage of misread peptides relative to the intended product (%)
25.00
20.00
15.00
10.00
5.00
0.00
Ala1B
Ser5
Glu2
Phe
Arg3
Val2
Leu2
Pro3
Ala2
Pro2
Sequence for positions 32, 33, 37, and 38 in the tRNA

FIG. 2

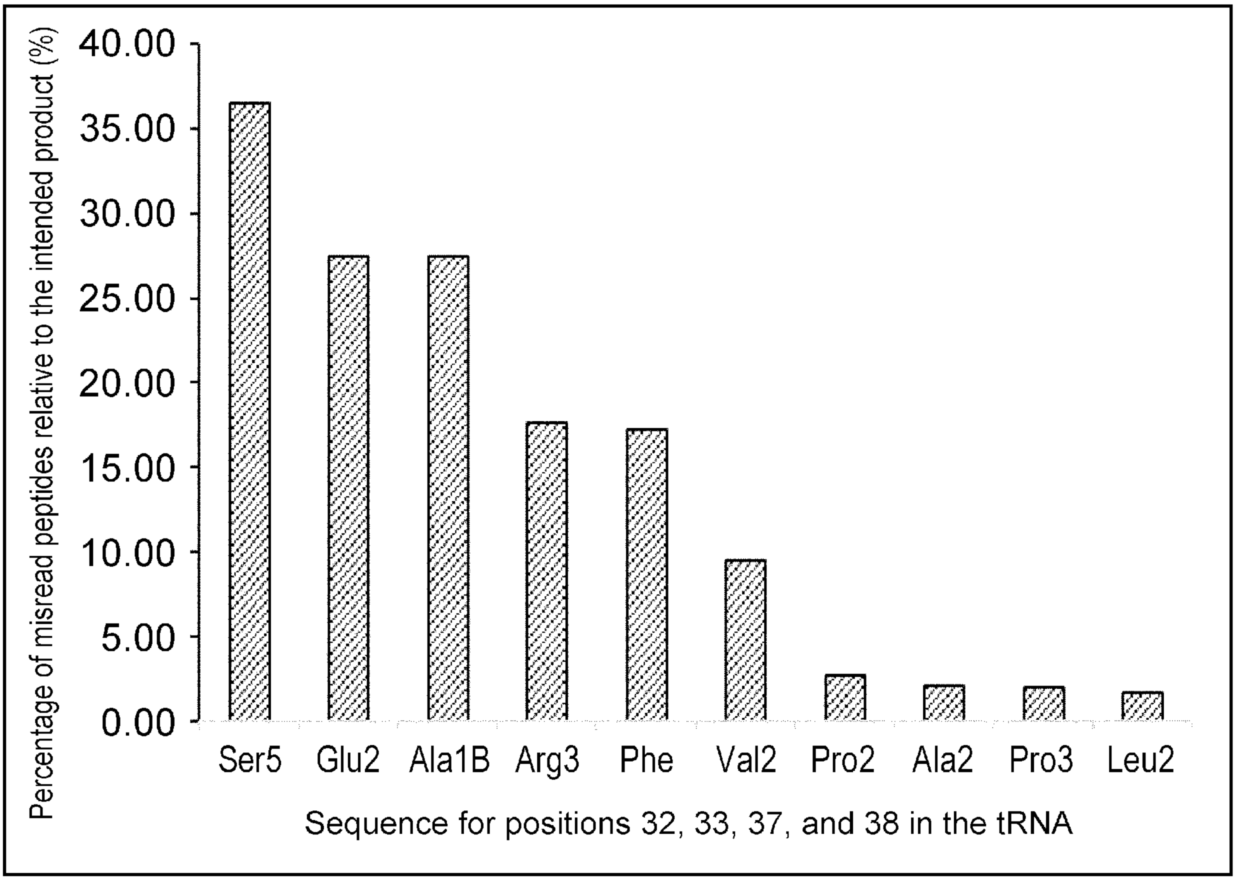
Percentage of misread peptides relative to the intended product (%)
40.00
35.00
30.00
25.00
20.00
15.00
10.00
5.00
0.00
Ser5
Glu2
Ala1B
Arg3
Phe
Val2
Pro2
Ala2
Pro3
Leu2
Sequence for positions 32, 33, 37, and 38 in the tRNA

FIG. 3

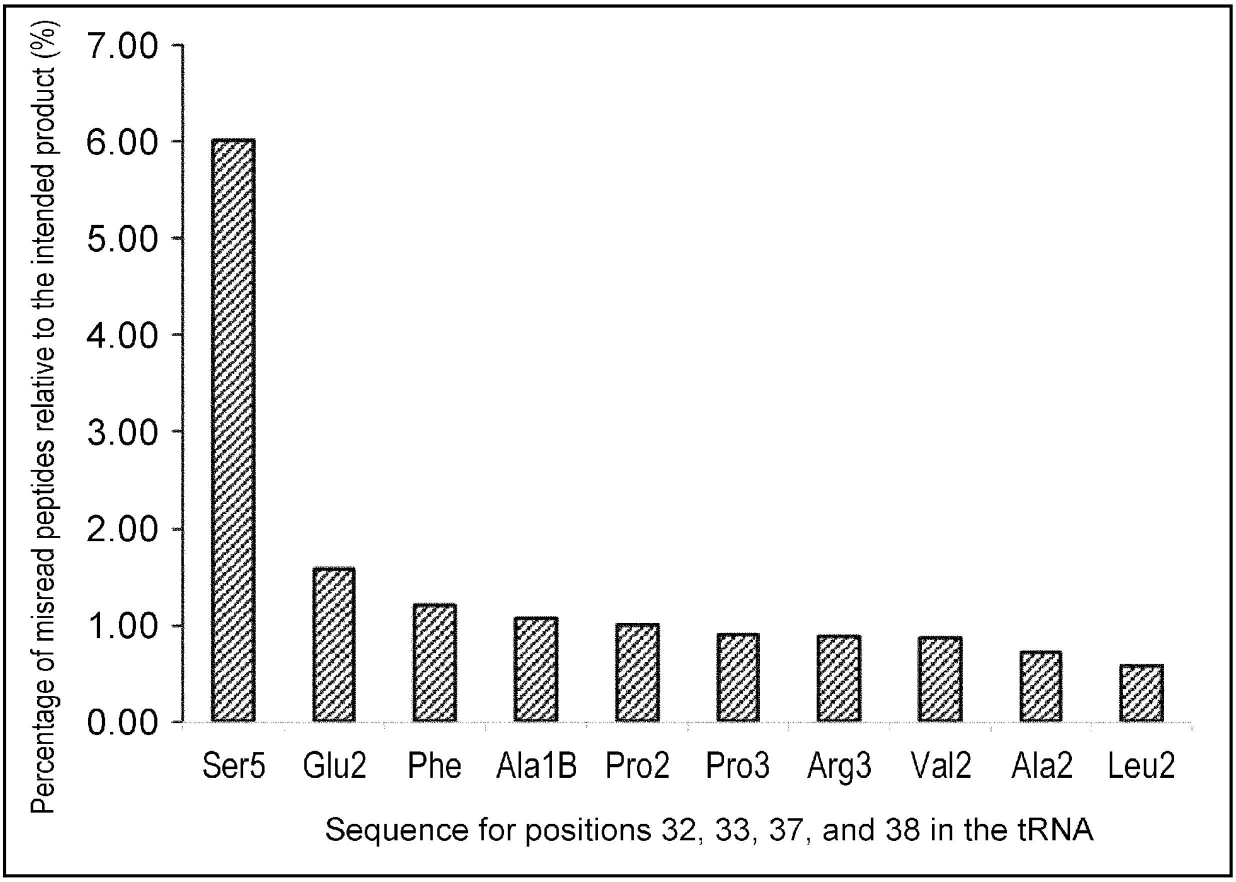
Percentage of misread peptides relative to the intended product (%)
7.00
6.00
5.00
4.00
3.00
2.00
1.00
0.00
Ser5
Glu2
Phe
Ala1B
Pro2
Pro3
Arg3
Val2
Ala2
Leu2
Sequence for positions 32, 33, 37, and 38 in the tRNA

FIG. 4

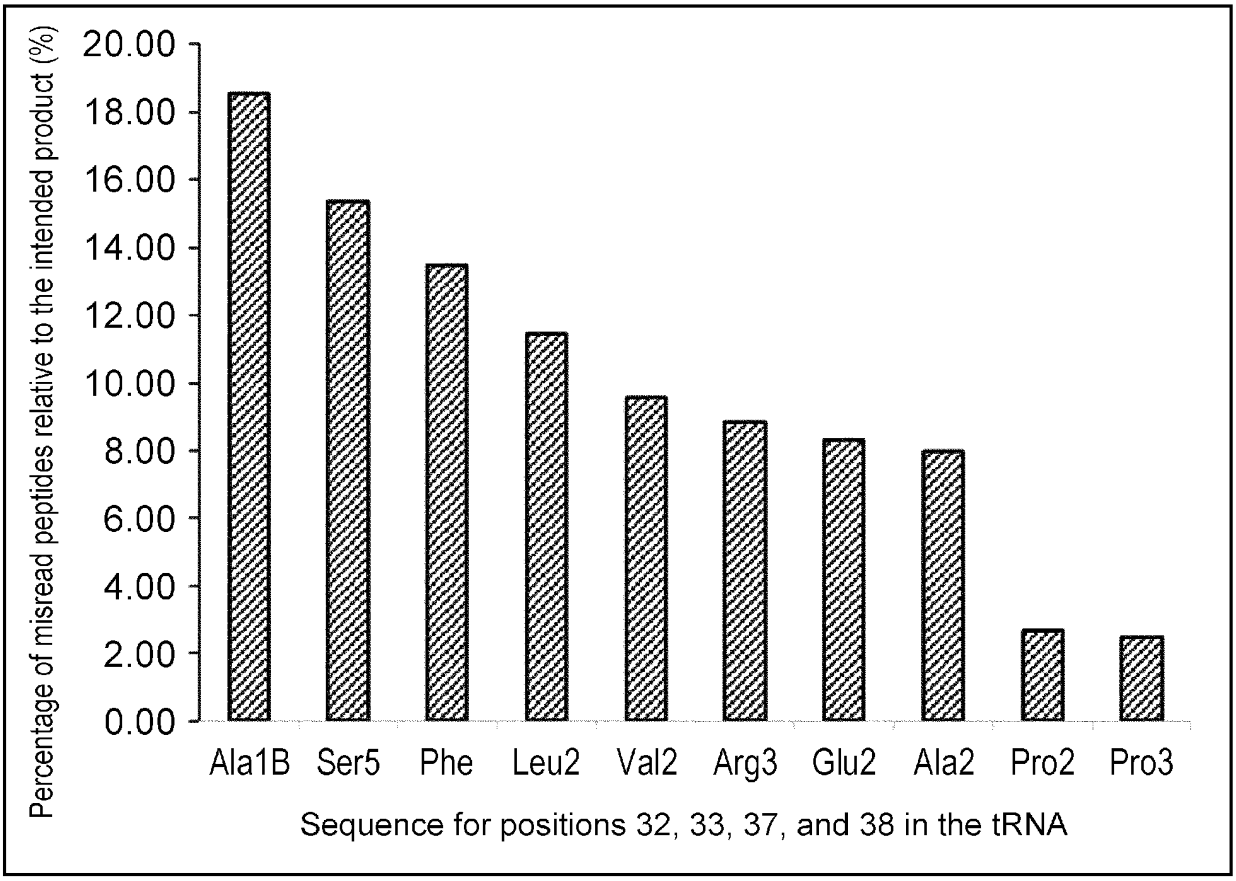
Percentage of misread peptides relative to the intended product (%)
20.00
18.00
16.00
14.00
12.00
10.00
8.00
6.00
4.00
2.00
0.00
Ala1B
Ser5
Phe
Leu2
Val2
Arg3
Glu2
Ala2
Pro2
Pro3
Sequence for positions 32, 33, 37, and 38 in the tRNA

FIG. 5

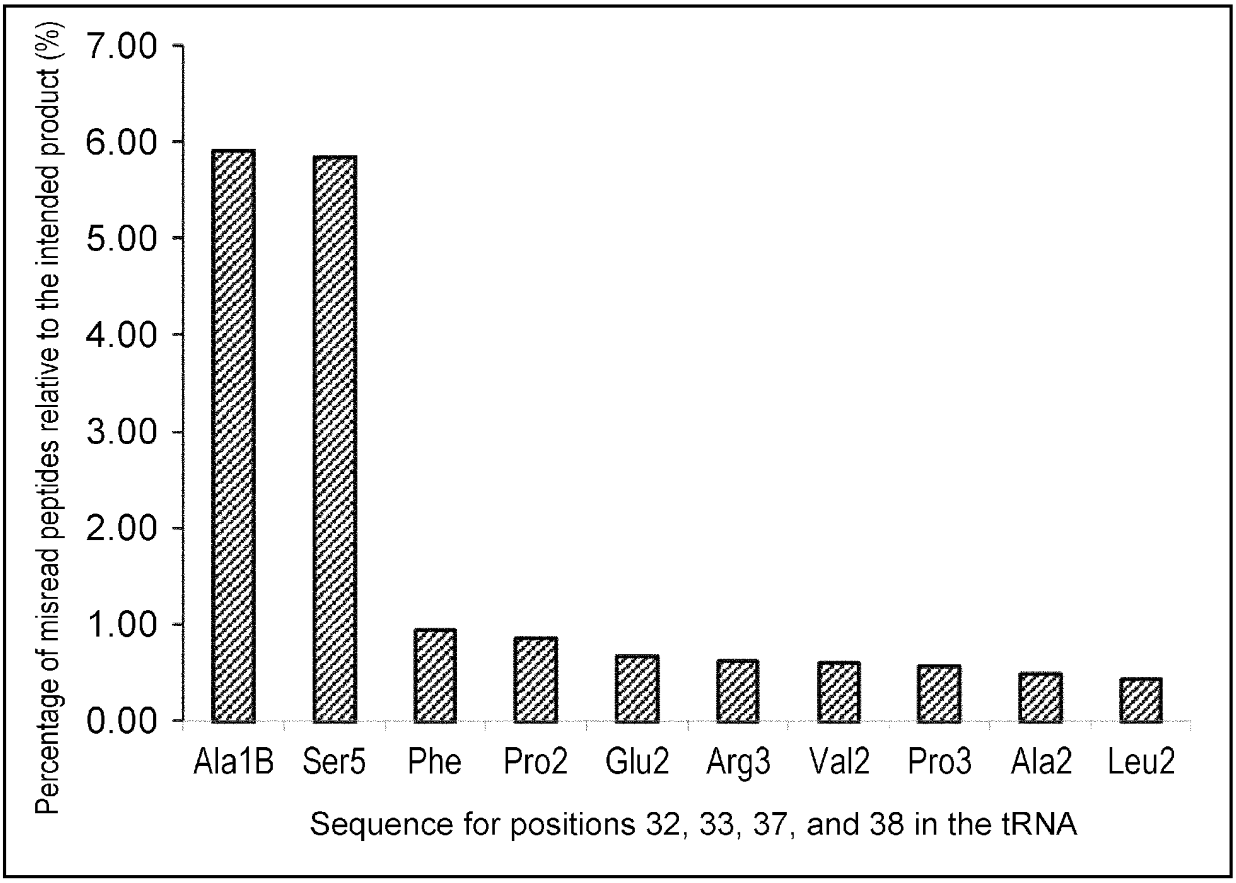
Percentage of misread peptides relative to the intended product (%)
7.00
6.00
5.00
4.00
3.00
2.00
1.00
0.00
Ala1B
Ser5
Phe
Pro2
Glu2
Arg3
Val2
Pro3
Ala2
Leu2
Sequence for positions 32, 33, 37, and 38 in the tRNA

FIG. 6

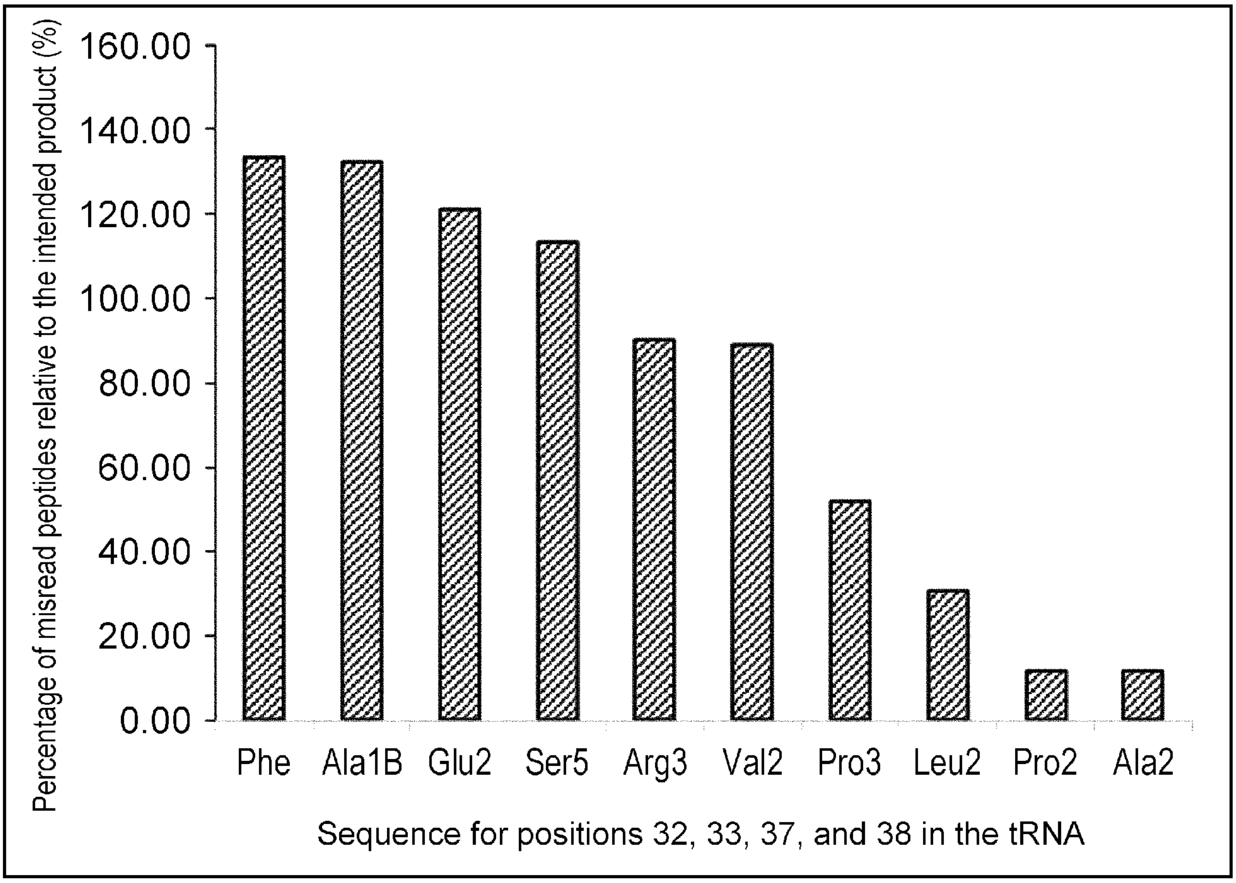
Percentage of misread peptides relative to the intended product (%)
160.00
140.00
120.00
100.00
80.00
60.00
40.00
20.00
0.00
Phe
Ala1B
Glu2
Ser5
Arg3
Val2
Pro3
Leu2
Pro2
Ala2
Sequence for positions 32, 33, 37, and 38 in the tRNA

FIG. 7

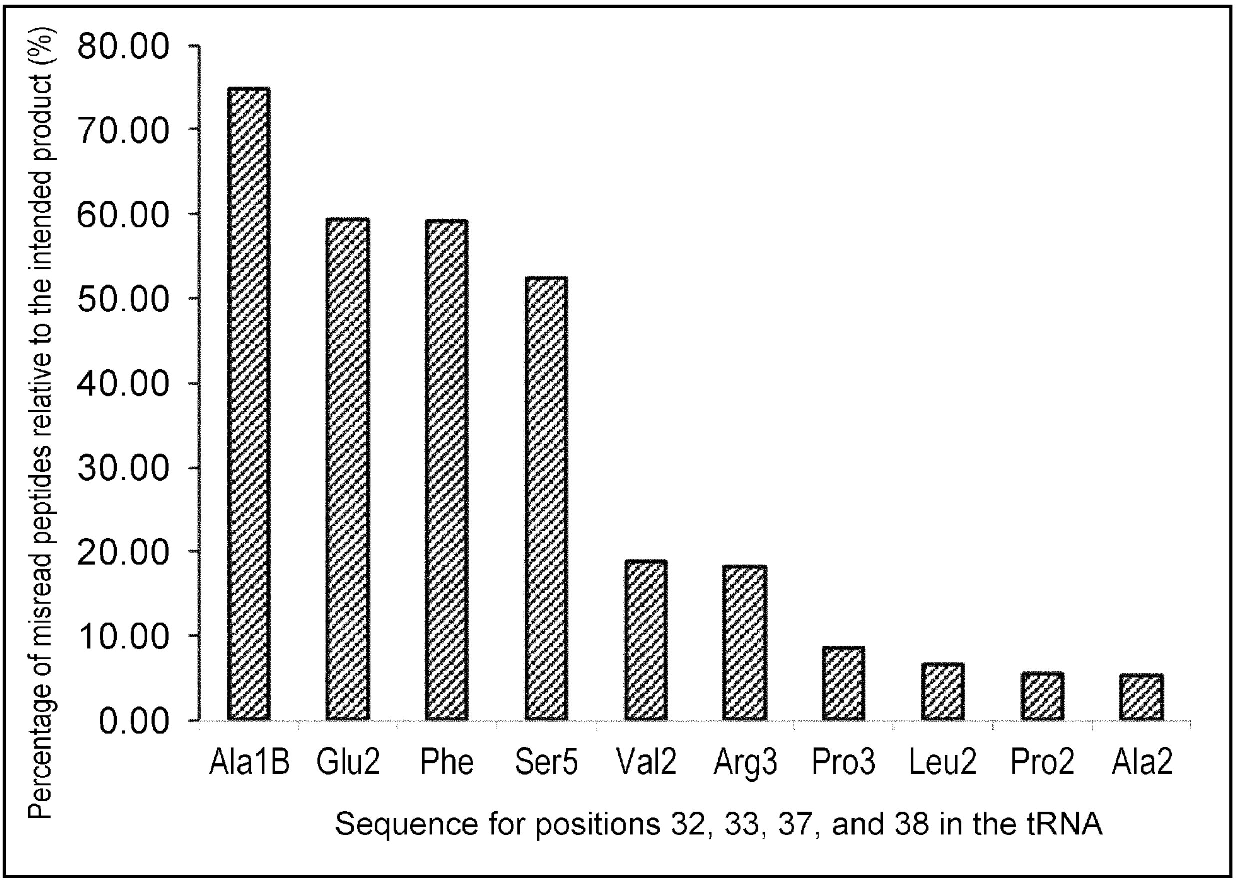
Percentage of misread peptides relative to the intended product (%)
80.00
70.00
60.00
50.00
40.00
30.00
20.00
10.00
0.00
Ala1B
Glu2
Phe
Ser5
Val2
Arg3
Pro3
Leu2
Pro2
Ala2
Sequence for positions 32, 33, 37, and 38 in the tRNA

FIG. 8

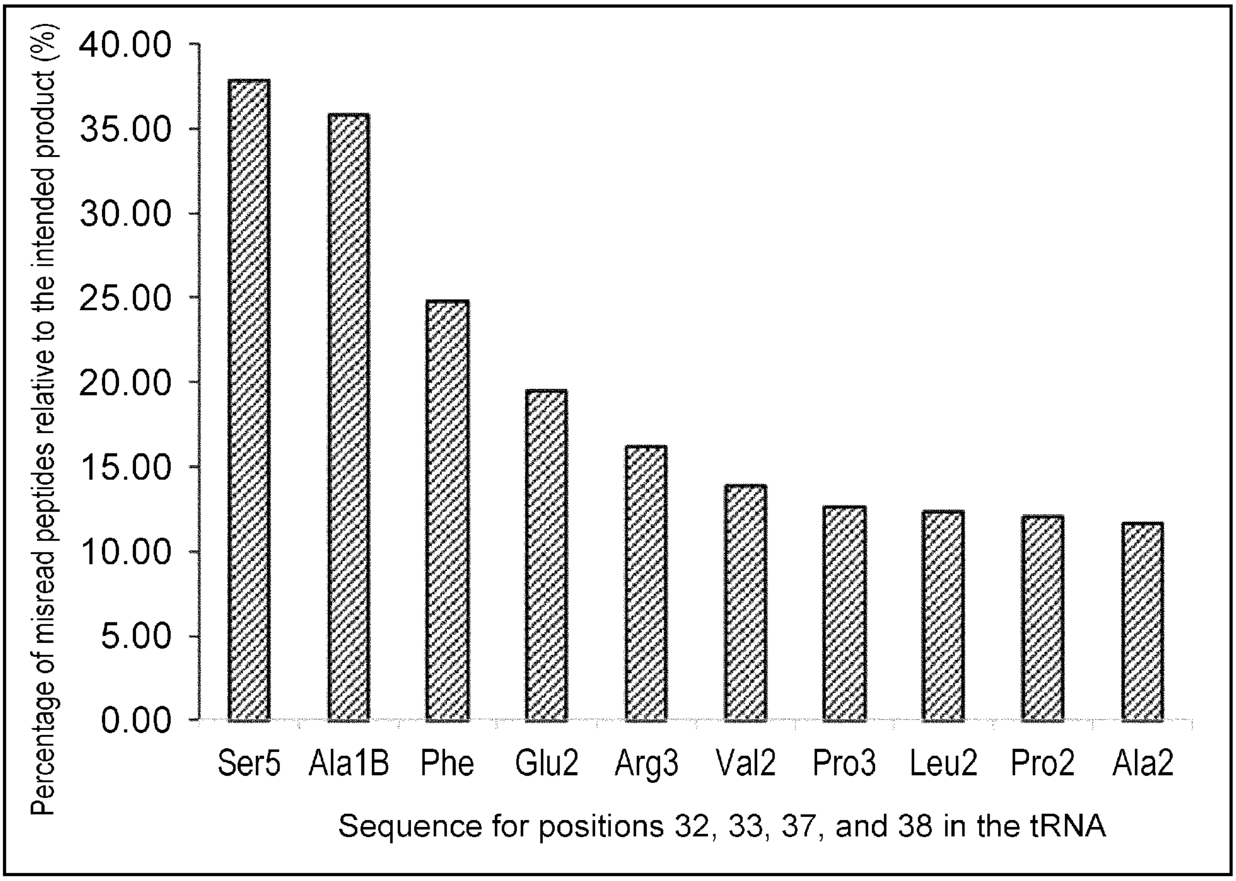
Percentage of misread peptides relative to the intended product (%)
40.00
35.00
30.00
25.00
20.00
15.00
10.00
5.00
0.00
Ser5
Ala1B
Phe
Glu2
Arg3
Val2
Pro3
Leu2
Pro2
Ala2
Sequence for positions 32, 33, 37, and 38 in the tRNA

FIG. 9

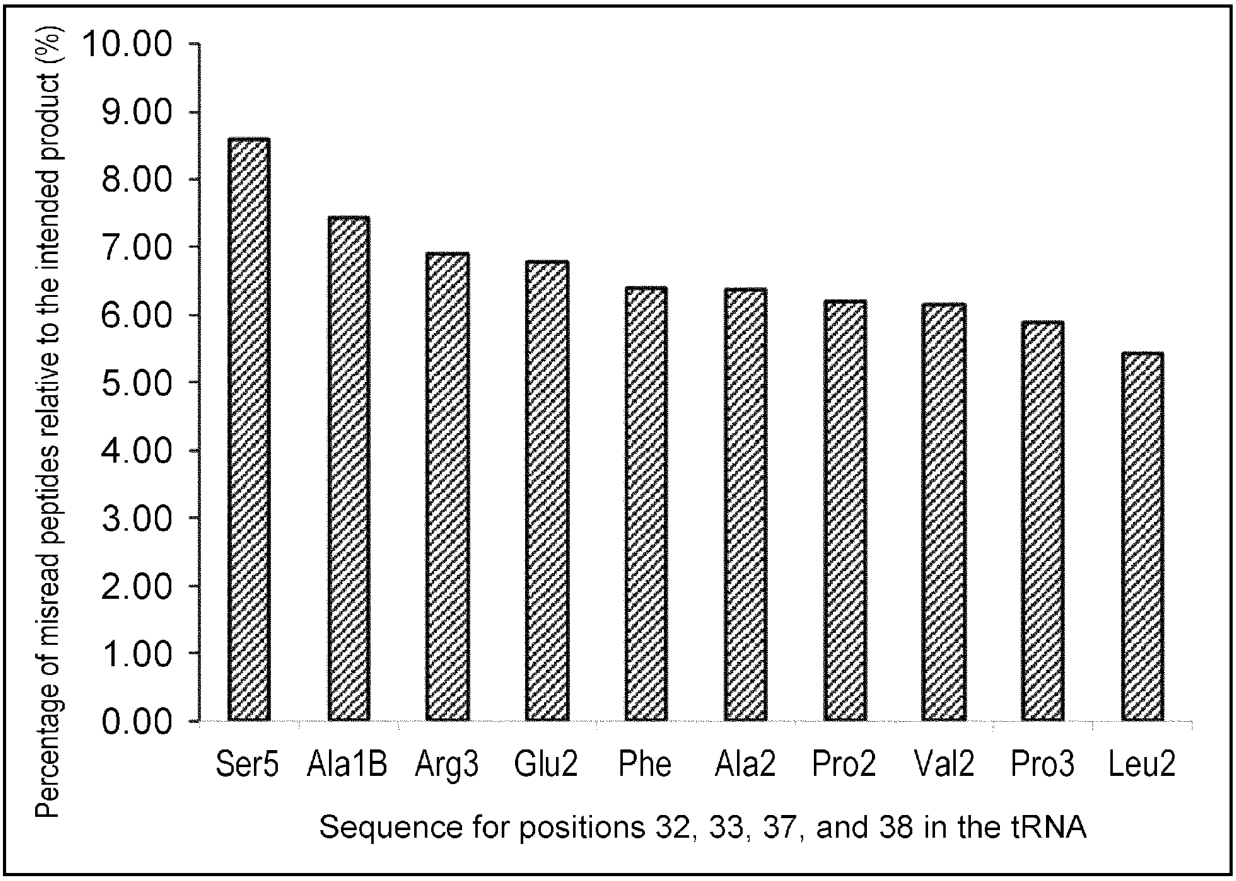
Percentage of misread peptides relative to the intended product (%)
10.00
9.00
8.00
7.00
6.00
5.00
4.00
3.00
2.00
1.00
0.00
Ser5
Ala1B
Arg3
Glu2
Phe
Ala2
Pro2
Val2
Pro3
Leu2
Sequence for positions 32, 33, 37, and 38 in the tRNA

FIG. 10

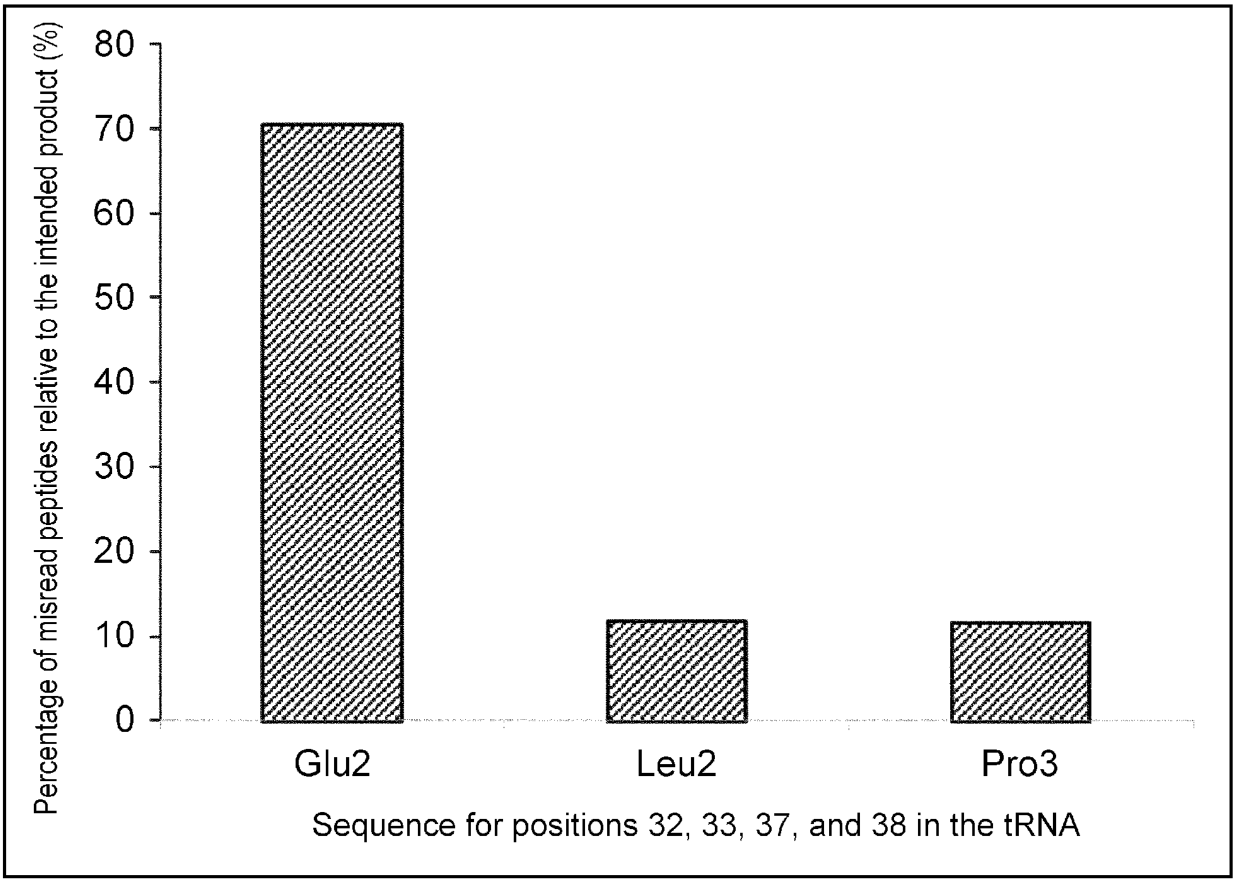
80
70
60
50
40
30
20
10
0
Percentage of misread peptides relative to the intended product (%)
Glu2
Leu2
Pro3
Sequence for positions 32, 33, 37, and 38 in the tRNA

FIG. 11

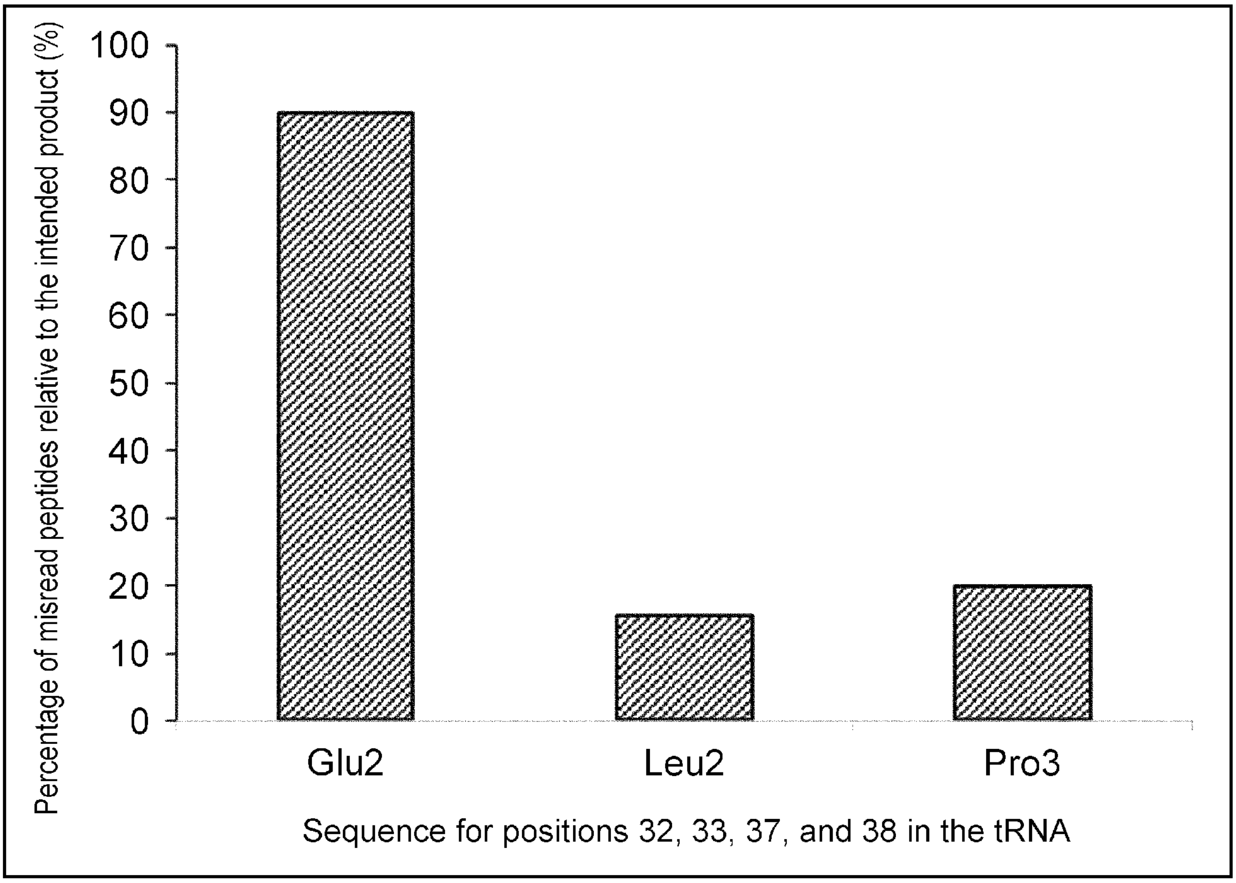
Percentage of misread peptides relative to the intended product (%)
100
90
80
70
60
50
40
30
20
10
0
Glu2
Leu2
Pro3
Sequence for positions 32, 33, 37, and 38 in the tRNA

FIG. 12

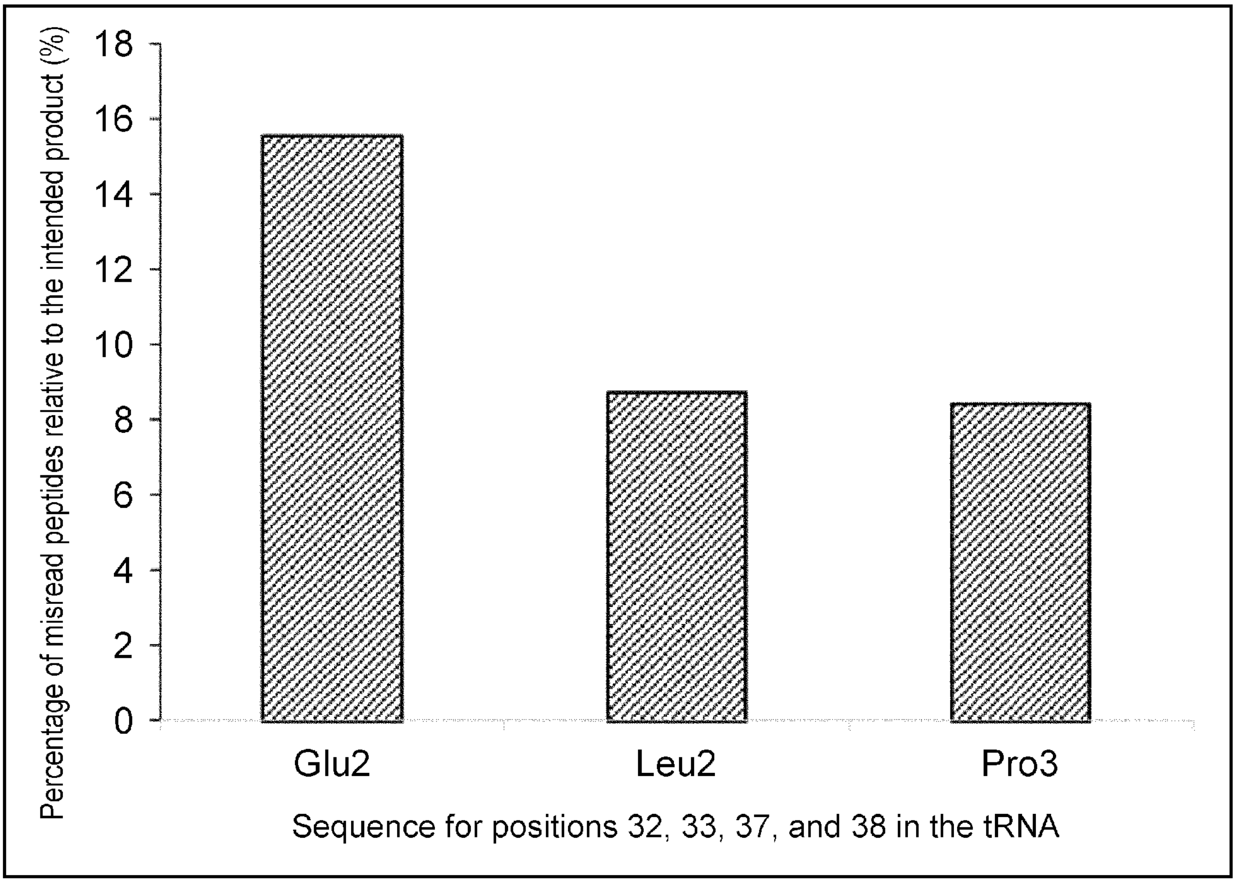
Percentage of misread peptides relative to the intended product (%)
18
16
14
12
10
8
6
4
2
0
Glu2
Leu2
Pro3
Sequence for positions 32, 33, 37, and 38 in the tRNA

FIG. 13

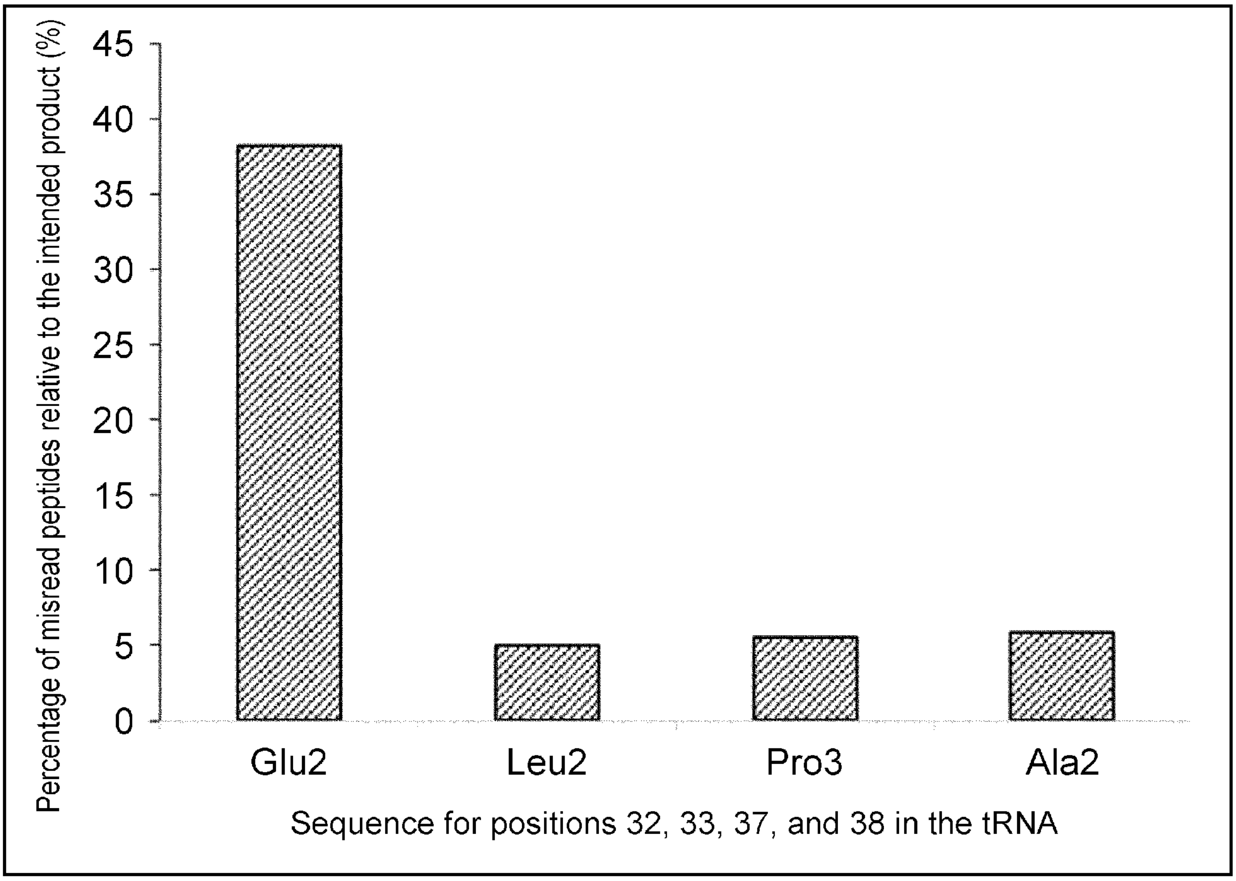
Percentage of misread peptides relative to the intended product (%)
45
40
35
30
25
20
15
10
5
0
Glu2
Leu2
Pro3
Ala2
Sequence for positions 32, 33, 37, and 38 in the tRNA

FIG. 14

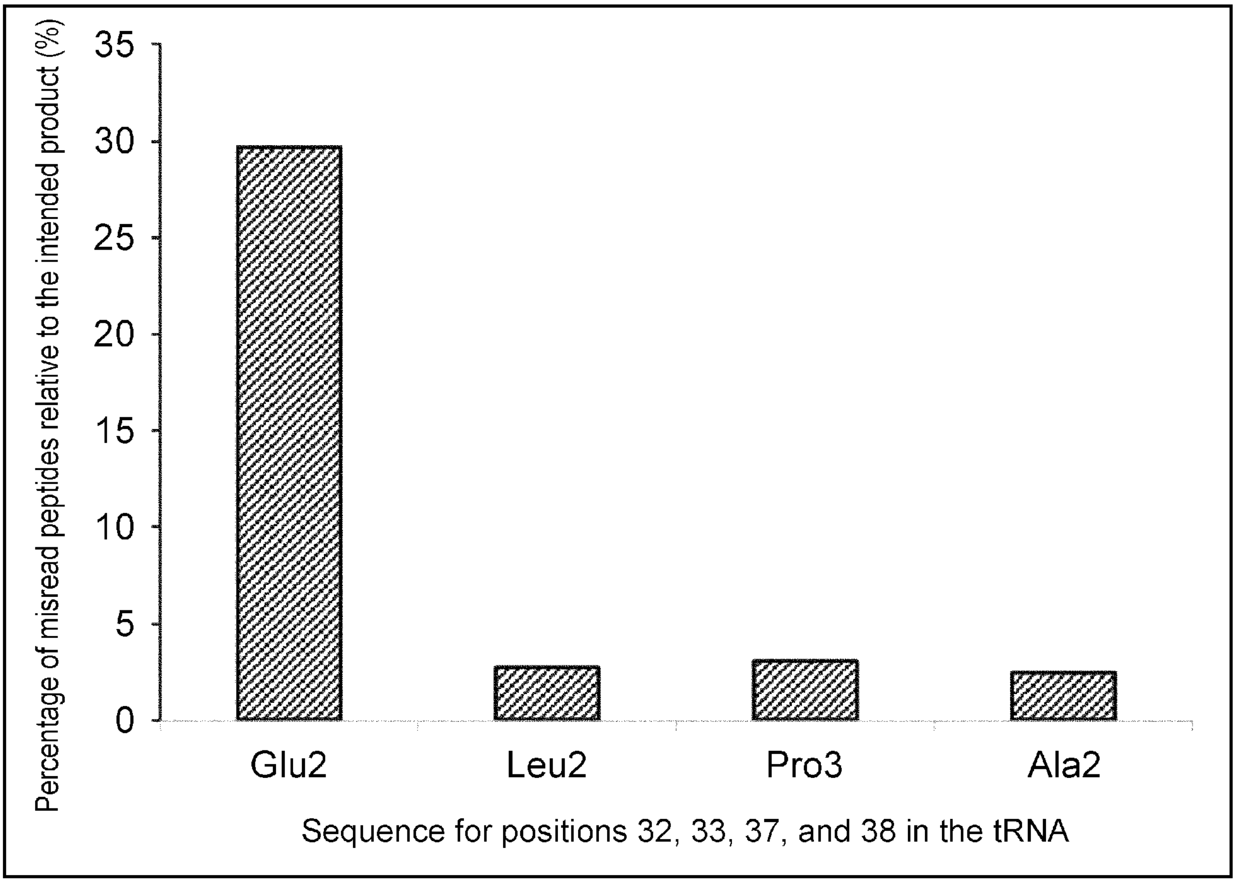
Percentage of misread peptides relative to the intended product (%)
35
30
25
20
15
10
5
0
Glu2
Leu2
Pro3
Ala2
Sequence for positions 32, 33, 37, and 38 in the tRNA

FIG. 15

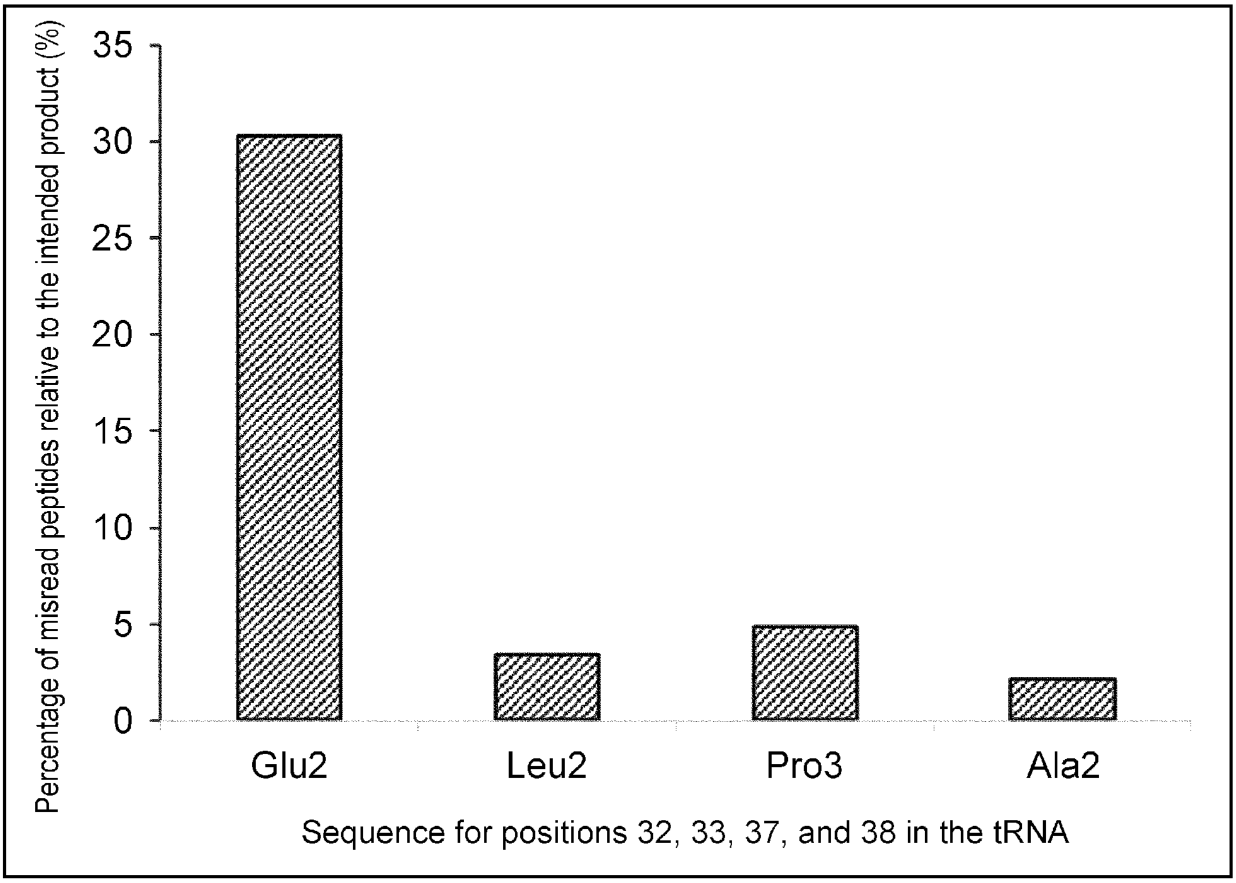
Percentage of misread peptides relative to the intended product (%)
35
30
25
20
15
10
5
0
Glu2
Leu2
Pro3
Ala2
Sequence for positions 32, 33, 37, and 38 in the tRNA

FIG. 16

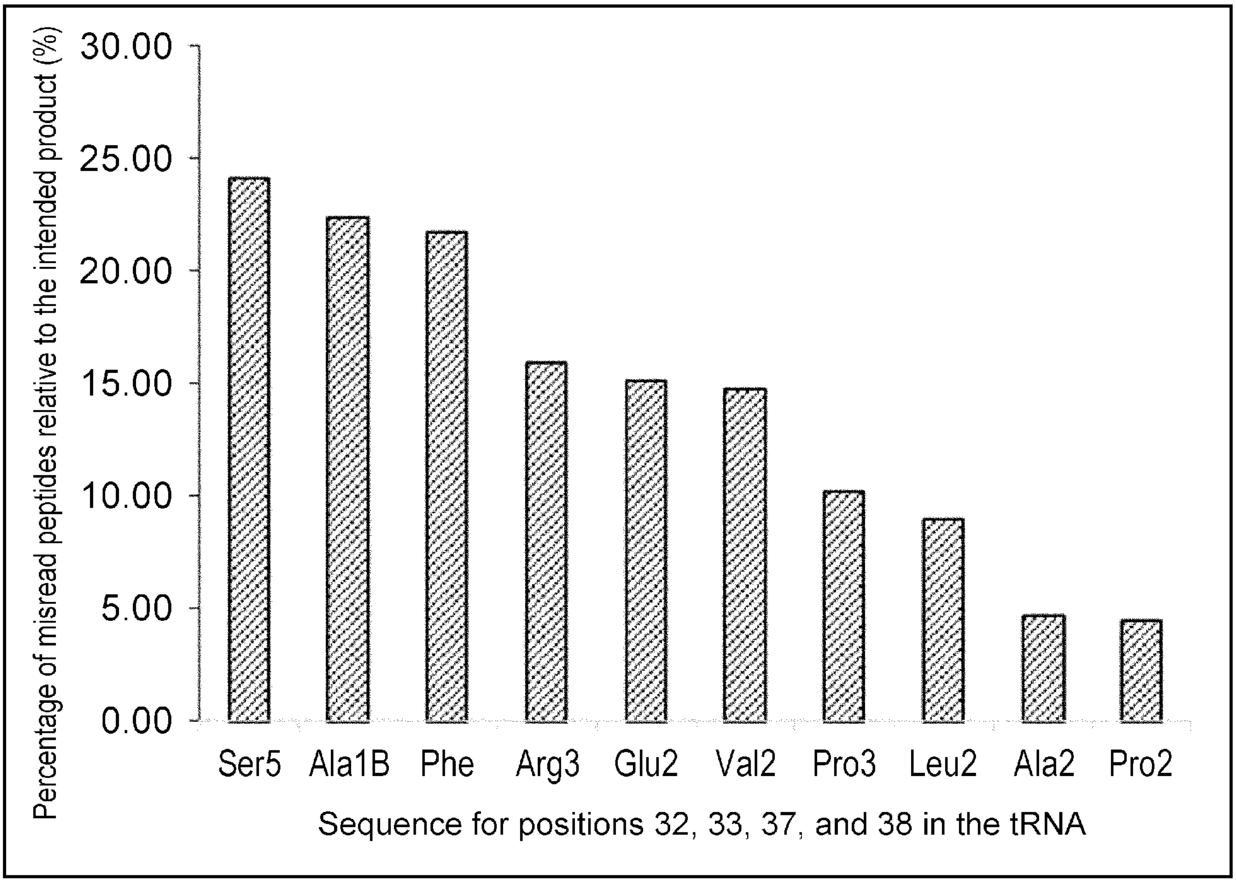
Percentage of misread peptides relative to the intended product (%)
30.00
25.00
20.00
15.00
10.00
5.00
0.00
Ser5
Ala1B
Phe
Arg3
Glu2
Val2
Pro3
Leu2
Ala2
Pro2
Sequence for positions 32, 33, 37, and 38 in the tRNA

FIG. 17

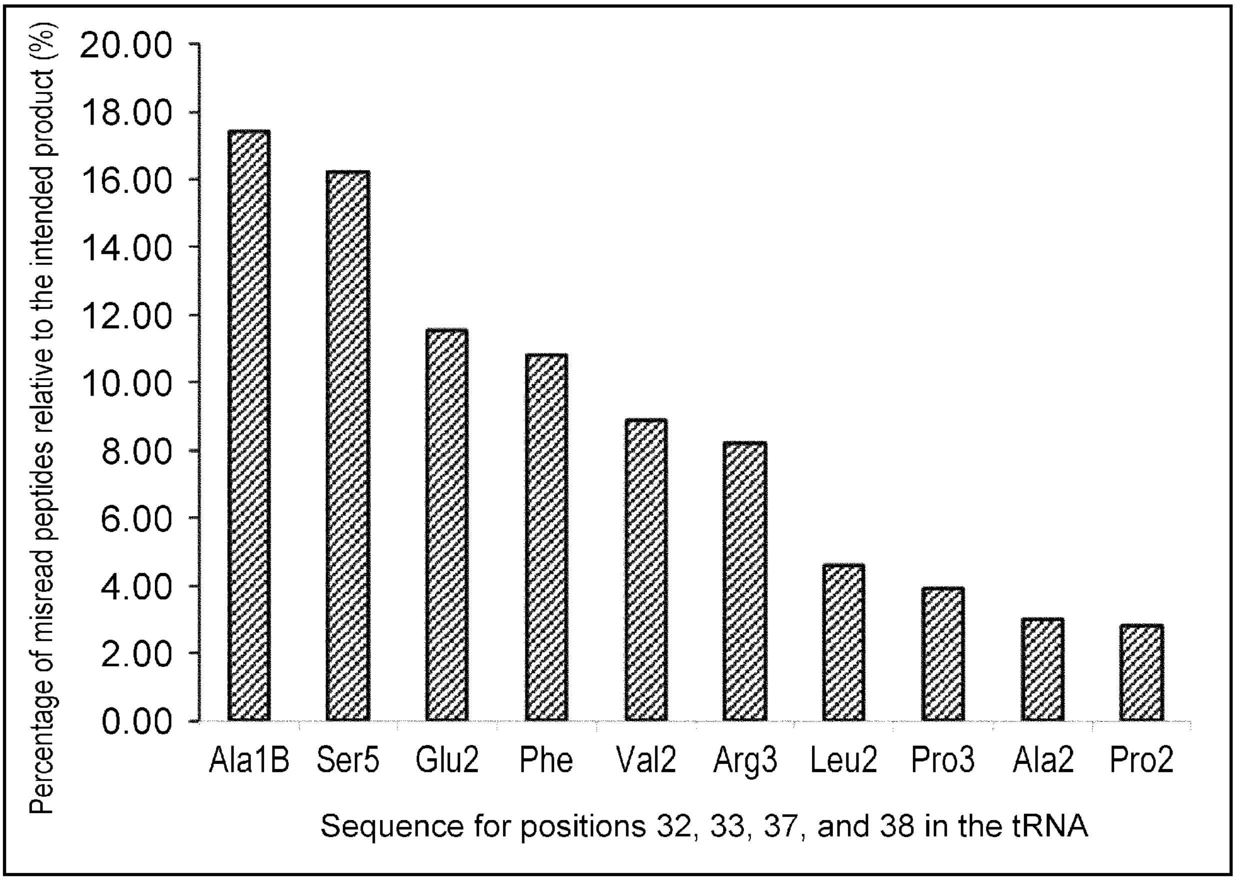
Percentage of misread peptides relative to the intended product (%)
20.00
18.00
16.00
14.00
12.00
10.00
8.00
6.00
4.00
2.00
0.00
Ala1B
Ser5
Glu2
Phe
Val2
Arg3
Leu2
Pro3
Ala2
Pro2
Sequence for positions 32, 33, 37, and 38 in the tRNA

FIG. 18

**INTERNATIONAL SEARCH REPORT**

International application No. PCT/JP2020/048652

A. CLASSIFICATION OF SUBJECT MATTER
C12N 15/11(2006.01)i; C12P 21/02(2006.01)i
FI: C12N15/11 Z; C12P21/02 C ZNA
According to International Patent Classification (IPC) or to both national classification and IPC

B. FIELDS SEARCHED

Minimum documentation searched (classification system followed by classification symbols)
C12N15/11; C12P21/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | KANDA, T. et al., "Sense Codon-Dependent Introduction of Unnatural Amino Acids into Multiple Sites of a Protein", Biochemical and Biophysical Research Communications, 2000, vol. 270, pp. 1136-1139, abstract, fig. 1-6, page 1139, left column, lines 13-16 | 1-3, 5, 7-8, 10-15 |
| Y | abstract, fig. 1-6, page 1139, left column, lines 13-16 | 4, 6, 9 |
| Y | JP 2017-063672 A (THE UNIVERSITY OF TOKYO) 06 April 2017 (2017-04-06) paragraphs [0010], [0078], fig. 2 | 4, 6, 9 |

☐ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 18 February 2021 (18.02.2021) | 09 March 2021 (09.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application no.
PCT/JP2020/048652

| Patent Documents referred in the Report | Publication Date | Patent Family | Publication Date |
|---|---|---|---|
| JP 2017-063672 A | 06 Apr. 2017 | WO 2017/057633 A1 paragraphs [0011], [0079], fig. 2<br>US 2018/0298390 A1 paragraphs [0082], [0190]-[0193], fig. 2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019139126 A
- WO 2012026566 A
- WO 2019077887 A
- WO 2013100132 A
- WO 2018143145 A
- WO 2006135096 A
- WO 2007061136 A
- WO 2007103307 A
- WO 2008001947 A
- WO 2010141851 A
- WO 2015120287 A
- WO 2007066627 A
- WO 2012033154 A
- WO 2012074130 A
- WO 2015030014 A
- WO 2018052002 A
- WO 2018143145 A1
- WO 2018225864 A
- WO 2013100132 B2


### Non-patent literature cited in the description


- **SHIMIZU et al.** *Nat. Biotechnol.,* August 2001, vol. 19 (8), 751-755
- **GAN et al.** *Biochem. Biophys. Acta,* December 2017, vol. 1861, 3047-3052
- **IWANE et al.** *Method Mol. Biol,* 2018, vol. 1728, 17-47
- **PASSIOURA et al.** *J Am. Chem. Soc.,* 2018, vol. 140, 11551-11555
- **PASSIOURA et al.** *Cell Chem. Biol.,* 2018, vol. 25, 906-915
- **IWANE et al.** *Nat. Chem,* 2016, vol. 8, 317-325
- **AZUSA et al.** *Peptide Science,* 2009, 17-18
- **URAKAMI et al.** *Nat. Struct. Mol. Biol,* March 2009, vol. 16, 353-358
- **SPRINZL et al.** *Nucleic Acids Res,* 1998, vol. 26, 148-153
- **NOWAK et al.** *Science,* 1995, vol. 268, 439-442
- **CHEN et al.** *Methods Enzymol,* 1983, vol. 101, 674-690
- **GASIOR et al.** *J Biol Chem,* 1979, vol. 254, 3965-3969
- **ERICKSON et al.** *Methods Enzymol,* 1983, vol. 96, 38-50
- **JACKSON et al.** *Methods Enzymol,* 1983, vol. 96, 50-74
- **BARTON et al.** *Methods Enzymol,* 1996, vol. 275, 35-57
- **SWERDEL et al.** *Comp Biochem Physiol B,* 1989, vol. 93, 803-806
- **SHIMIZU et al.** *Nat Biotech,* 2001, vol. 19, 751-755
- **OHTA, A ; MURAKAMI, H. ; HIGASHIMURA, E. ; SUGA, H.** *Chem. Biol.,* 2007, vol. 14, 1315-1322
- **HECHT et al.** *J Biol Chem,* 1978, vol. 253, 4517-4520
- **WANG et al.** *ACS Chem Biol,* 2015, vol. 10, 2187-2192
- **H. MURAKAMI et al.** *Chemistry & Biology,* 2003, vol. 10, 655-662
- **H. MURAKAMI et al.** *Chemistry & Biology,* 2003, vol. 10, 1077-1084
- **H. MURAKAMI et al.** *Nature Methods,* 2006, vol. 3, 357-359
- **N. NIWA et al.** *Bioorganic & Medicinal Chemistry Letters,* 2009, vol. 19, 3892-3894
- *Comb. Chem. High Throughput Screen,* 2010, vol. 13, 75-87
- *Nat. Chem. Biol,* 2009, vol. 5, 502-507
- *Nat. Chem. Biol.,* 2009, vol. 5, 888-90
- *Bioconjug. Chem,* 2007, vol. 18, 469-476
- *ChemBioChem,* 2009, vol. 10, 787-798
- *Chem. Commun. (Camb),* 2011, vol. 47, 9946-9958
- **ROBERTS ; SZOSTAK.** *Proc. Natl. Acad. Sci. USA,* 1997, vol. 94, 12297-12302
- **NEMOTO et al.** *FEBS Lett,* 1997, vol. 414, 405-408
- **YAMAGUCHI et al.** *Nucleic Acids Res.,* 2009, vol. 37, e108
- **MATTHEAKIS et al.** *Proc. Natl. Acad. Sci. USA,* 1994, vol. 91, 9022-9026
- **REIERSEN.** *Nucleic Acids Res.,* 2005, vol. 33, e10
- **ODEGRIP.** *Proc. Natl. Acad. Sci. USA,* 2004, vol. 101, 2806-2810
- **TAWFIK AND GRIFFITHS.** *Nat. Biotechnol.,* 1998, vol. 16, 652-656
- *Helv. Chim. Acta,* vol. 90, 297-310

- *Antiviral Chemistry & Chemotherapy,* 2003, vol. 14 (4), 183-194

SEQUENCE LISTING

<110> CHUGAI SEIYAKU KABUSHIKI KAISHA

<120> Composition for translation and a method for peptide synthesis

<130> C1-A2016P

<150> PCT/JP2019/051241
<151> 2019-12-26

<150> JP 2020-109292
<151> 2020-06-25

<160> 324

<170> PatentIn version 3.5

<210> 1
<211> 95
<212> DNA
<213> Artificial sequence

<220>
<223> an artificially synthesized sequence

<400> 1
ggcgtaatac gactcactat agtccccttc gtctagaggc ccaggacacc gccctaagaa 60

ggcggtaaca ggggttcgaa tcccctaggg gacgc 95

<210> 2
<211> 95
<212> DNA
<213> Artificial sequence

<220>
<223> an artificially synthesized sequence

<400> 2
ggcgtaatac gactcactat agtccccttc gtctagaggc ccaggacacc gccttaagac 60

ggcggtaaca ggggttcgaa tcccctaggg gacgc 95

<210> 3
<211> 95
<212> DNA
<213> Artificial sequence

<220>
<223> an artificially synthesized sequence

<400> 3
ggcgtaatac gactcactat agtccccttc gtctagaggc ccaggacacc gccttaagaa 60

ggcggtaaca ggggttcgaa tcccctaggg gacgc 95

<210> 4
<211> 95
<212> DNA

<213> Artificial sequence

<220>
<223> an artificially synthesized sequence

<400> 4
ggcgtaatac gactcactat agtccccttc gtctagaggc ccaggacacc gccctaagac 60

ggcggtaaca ggggttcgaa tcccctaggg gacgc 95

<210> 5
<211> 95
<212> DNA
<213> Artificial sequence

<220>
<223> an artificially synthesized sequence

<400> 5
ggcgtaatac gactcactat agtccccttc gtctagaggc ccaggacacc gccctaagga 60

ggcggtaaca ggggttcgaa tcccctaggg gacgc 95

<210> 6
<211> 95
<212> DNA
<213> Artificial sequence

<220>
<223> an artificially synthesized sequence

<400> 6
ggcgtaatac gactcactat agtccccttc gtctagaggc ccaggacacc gccttaagat 60

ggcggtaaca ggggttcgaa tcccctaggg gacgc 95

<210> 7
<211> 95
<212> DNA
<213> Artificial sequence

<220>
<223> an artificially synthesized sequence

<400> 7
ggcgtaatac gactcactat agtccccttc gtctagaggc ccaggacacc gccttaaggt 60

ggcggtaaca ggggttcgaa tcccctaggg gacgc 95

<210> 8
<211> 95
<212> DNA
<213> Artificial sequence

<220>
<223> an artificially synthesized sequence

<400> 8
ggcgtaatac gactcactat agtccccttc gtctagaggc ccaggacacc gccttaagga 60

ggcggtaaca ggggttcgaa tcccctaggg gacgc 95

<210> 9
<211> 95
<212> DNA
<213> Artificial sequence

<220>
<223> an artificially synthesized sequence

<400> 9
ggcgtaatac gactcactat agtccccttc gtctagaggc ccaggacacc gccataaggt 60

ggcggtaaca ggggttcgaa tcccctaggg gacgc 95

<210> 10
<211> 95
<212> DNA
<213> Artificial sequence

<220>
<223> an artificially synthesized sequence

<400> 10
ggcgtaatac gactcactat agtccccttc gtctagaggc ccaggacacc gccataagat 60

ggcggtaaca ggggttcgaa tcccctaggg gacgc 95

<210> 11
<211> 95
<212> DNA
<213> Artificial sequence

<220>
<223> an artificially synthesized sequence

<400> 11
ggcgtaatac gactcactat agtccccttc gtctagaggc ccaggacacc gccctcagaa 60

ggcggtaaca ggggttcgaa tcccctaggg gacgc 95

<210> 12
<211> 95
<212> DNA
<213> Artificial sequence

<220>
<223> an artificially synthesized sequence

<400> 12
ggcgtaatac gactcactat agtccccttc gtctagaggc ccaggacacc gccttcagac 60

ggcggtaaca ggggttcgaa tcccctaggg gacgc 95

<210> 13
<211> 95
<212> DNA

<213> Artificial sequence

<220>
<223> an artificially synthesized sequence

<400> 13
ggcgtaatac gactcactat agtccccttc gtctagaggc ccaggacacc gccttcagaa 60

ggcggtaaca ggggttcgaa tcccctaggg gacgc 95

<210> 14
<211> 95
<212> DNA
<213> Artificial sequence

<220>
<223> an artificially synthesized sequence

<400> 14
ggcgtaatac gactcactat agtccccttc gtctagaggc ccaggacacc gccctcagac 60

ggcggtaaca ggggttcgaa tcccctaggg gacgc 95

<210> 15
<211> 95
<212> DNA
<213> Artificial sequence

<220>
<223> an artificially synthesized sequence

<400> 15
ggcgtaatac gactcactat agtccccttc gtctagaggc ccaggacacc gccctcagga 60

ggcggtaaca ggggttcgaa tcccctaggg gacgc 95

<210> 16
<211> 95
<212> DNA
<213> Artificial sequence

<220>
<223> an artificially synthesized sequence

<400> 16
ggcgtaatac gactcactat agtccccttc gtctagaggc ccaggacacc gccttcagat 60

ggcggtaaca ggggttcgaa tcccctaggg gacgc 95

<210> 17
<211> 95
<212> DNA
<213> Artificial sequence

<220>
<223> an artificially synthesized sequence

<400> 17
ggcgtaatac gactcactat agtccccttc gtctagaggc ccaggacacc gccttcaggt 60

ggcggtaaca ggggttcgaa tcccctaggg gacgc 95

<210> 18
<211> 95
<212> DNA
<213> Artificial sequence

<220>
<223> an artificially synthesized sequence

<400> 18
ggcgtaatac gactcactat agtccccttc gtctagaggc ccaggacacc gccttcagga 60

ggcggtaaca ggggttcgaa tcccctaggg gacgc 95

<210> 19
<211> 95
<212> DNA
<213> Artificial sequence

<220>
<223> an artificially synthesized sequence

<400> 19
ggcgtaatac gactcactat agtccccttc gtctagaggc ccaggacacc gccatcaggt 60

ggcggtaaca ggggttcgaa tcccctaggg gacgc 95

<210> 20
<211> 95
<212> DNA
<213> Artificial sequence

<220>
<223> an artificially synthesized sequence

<400> 20
ggcgtaatac gactcactat agtccccttc gtctagaggc ccaggacacc gccatcagat 60

ggcggtaaca ggggttcgaa tcccctaggg gacgc 95

<210> 21
<211> 95
<212> DNA
<213> Artificial sequence

<220>
<223> an artificially synthesized sequence

<400> 21
ggcgtaatac gactcactat agtccccttc gtctagaggc ccaggacacc gccctaggaa 60

ggcggtaaca ggggttcgaa tcccctaggg gacgc 95

<210> 22
<211> 95
<212> DNA

<213> Artificial sequence

<220>
<223> an artificially synthesized sequence

<400> 22
ggcgtaatac gactcactat agtccccttc gtctagaggc ccaggacacc gccttaggac 60

ggcggtaaca ggggttcgaa tcccctaggg gacgc 95

<210> 23
<211> 95
<212> DNA
<213> Artificial sequence

<220>
<223> an artificially synthesized sequence

<400> 23
ggcgtaatac gactcactat agtccccttc gtctagaggc ccaggacacc gccttaggaa 60

ggcggtaaca ggggttcgaa tcccctaggg gacgc 95

<210> 24
<211> 95
<212> DNA
<213> Artificial sequence

<220>
<223> an artificially synthesized sequence

<400> 24
ggcgtaatac gactcactat agtccccttc gtctagaggc ccaggacacc gccctaggac 60

ggcggtaaca ggggttcgaa tcccctaggg gacgc 95

<210> 25
<211> 95
<212> DNA
<213> Artificial sequence

<220>
<223> an artificially synthesized sequence

<400> 25
ggcgtaatac gactcactat agtccccttc gtctagaggc ccaggacacc gccctaggga 60

ggcggtaaca ggggttcgaa tcccctaggg gacgc 95

<210> 26
<211> 95
<212> DNA
<213> Artificial sequence

<220>
<223> an artificially synthesized sequence

<400> 26
ggcgtaatac gactcactat agtccccttc gtctagaggc ccaggacacc gccttaggat 60

ggcggtaaca ggggttcgaa tcccctaggg gacgc 95

<210> 27
<211> 95
<212> DNA
<213> Artificial sequence

<220>
<223> an artificially synthesized sequence

<400> 27
ggcgtaatac gactcactat agtccccttc gtctagaggc ccaggacacc gccttagggt 60

ggcggtaaca ggggttcgaa tcccctaggg gacgc 95

<210> 28
<211> 95
<212> DNA
<213> Artificial sequence

<220>
<223> an artificially synthesized sequence

<400> 28
ggcgtaatac gactcactat agtccccttc gtctagaggc ccaggacacc gccttaggga 60

ggcggtaaca ggggttcgaa tcccctaggg gacgc 95

<210> 29
<211> 95
<212> DNA
<213> Artificial sequence

<220>
<223> an artificially synthesized sequence

<400> 29
ggcgtaatac gactcactat agtccccttc gtctagaggc ccaggacacc gccatagggt 60

ggcggtaaca ggggttcgaa tcccctaggg gacgc 95

<210> 30
<211> 95
<212> DNA
<213> Artificial sequence

<220>
<223> an artificially synthesized sequence

<400> 30
ggcgtaatac gactcactat agtccccttc gtctagaggc ccaggacacc gccataggat 60

ggcggtaaca ggggttcgaa tcccctaggg gacgc 95

<210> 31
<211> 95
<212> DNA

<213> Artificial sequence

<220>
<223> an artificially synthesized sequence

<400> 31
ggcgtaatac gactcactat agtccccttc gtctagaggc ccaggacacc gccctcggaa 60

ggcggtaaca ggggttcgaa tcccctaggg gacgc 95

<210> 32
<211> 95
<212> DNA
<213> Artificial sequence

<220>
<223> an artificially synthesized sequence

<400> 32
ggcgtaatac gactcactat agtccccttc gtctagaggc ccaggacacc gccttcggac 60

ggcggtaaca ggggttcgaa tcccctaggg gacgc 95

<210> 33
<211> 95
<212> DNA
<213> Artificial sequence

<220>
<223> an artificially synthesized sequence

<400> 33
ggcgtaatac gactcactat agtccccttc gtctagaggc ccaggacacc gccttcggaa 60

ggcggtaaca ggggttcgaa tcccctaggg gacgc 95

<210> 34
<211> 95
<212> DNA
<213> Artificial sequence

<220>
<223> an artificially synthesized sequence

<400> 34
ggcgtaatac gactcactat agtccccttc gtctagaggc ccaggacacc gccctcggac 60

ggcggtaaca ggggttcgaa tcccctaggg gacgc 95

<210> 35
<211> 95
<212> DNA
<213> Artificial sequence

<220>
<223> an artificially synthesized sequence

<400> 35
ggcgtaatac gactcactat agtccccttc gtctagaggc ccaggacacc gccctcggga 60

ggcggtaaca ggggttcgaa tcccctaggg gacgc 95

<210> 36
<211> 95
<212> DNA
<213> Artificial sequence

<220>
<223> an artificially synthesized sequence

<400> 36
ggcgtaatac gactcactat agtccccttc gtctagaggc ccaggacacc gccttcggat 60

ggcggtaaca ggggttcgaa tcccctaggg gacgc 95

<210> 37
<211> 95
<212> DNA
<213> Artificial sequence

<220>
<223> an artificially synthesized sequence

<400> 37
ggcgtaatac gactcactat agtccccttc gtctagaggc ccaggacacc gccttcgggt 60

ggcggtaaca ggggttcgaa tcccctaggg gacgc 95

<210> 38
<211> 95
<212> DNA
<213> Artificial sequence

<220>
<223> an artificially synthesized sequence

<400> 38
ggcgtaatac gactcactat agtccccttc gtctagaggc ccaggacacc gccttcggga 60

ggcggtaaca ggggttcgaa tcccctaggg gacgc 95

<210> 39
<211> 95
<212> DNA
<213> Artificial sequence

<220>
<223> an artificially synthesized sequence

<400> 39
ggcgtaatac gactcactat agtccccttc gtctagaggc ccaggacacc gccatcgggt 60

ggcggtaaca ggggttcgaa tcccctaggg gacgc 95

<210> 40
<211> 95
<212> DNA

<213> Artificial sequence

<220>
<223> an artificially synthesized sequence

<400> 40
ggcgtaatac gactcactat agtccccttc gtctagaggc ccaggacacc gccatcggat 60

ggcggtaaca ggggttcgaa tcccctaggg gacgc 95

<210> 41
<211> 95
<212> DNA
<213> Artificial sequence

<220>
<223> an artificially synthesized sequence

<400> 41
ggcgtaatac gactcactat agtccccttc gtctagaggc ccaggacacc gccctaccaa 60

ggcggtaaca ggggttcgaa tcccctaggg gacgc 95

<210> 42
<211> 95
<212> DNA
<213> Artificial sequence

<220>
<223> an artificially synthesized sequence

<400> 42
ggcgtaatac gactcactat agtccccttc gtctagaggc ccaggacacc gccttaccac 60

ggcggtaaca ggggttcgaa tcccctaggg gacgc 95

<210> 43
<211> 95
<212> DNA
<213> Artificial sequence

<220>
<223> an artificially synthesized sequence

<400> 43
ggcgtaatac gactcactat agtccccttc gtctagaggc ccaggacacc gccttaccaa 60

ggcggtaaca ggggttcgaa tcccctaggg gacgc 95

<210> 44
<211> 95
<212> DNA
<213> Artificial sequence

<220>
<223> an artificially synthesized sequence

<400> 44
ggcgtaatac gactcactat agtccccttc gtctagaggc ccaggacacc gccctaccac 60

ggcggtaaca ggggttcgaa tcccctaggg gacgc 95

<210> 45
<211> 95
<212> DNA
<213> Artificial sequence

<220>
<223> an artificially synthesized sequence

<400> 45
ggcgtaatac gactcactat agtccccttc gtctagaggc ccaggacacc gccctaccga 60

ggcggtaaca ggggttcgaa tcccctaggg gacgc 95

<210> 46
<211> 95
<212> DNA
<213> Artificial sequence

<220>
<223> an artificially synthesized sequence

<400> 46
ggcgtaatac gactcactat agtccccttc gtctagaggc ccaggacacc gccttaccat 60

ggcggtaaca ggggttcgaa tcccctaggg gacgc 95

<210> 47
<211> 95
<212> DNA
<213> Artificial sequence

<220>
<223> an artificially synthesized sequence

<400> 47
ggcgtaatac gactcactat agtccccttc gtctagaggc ccaggacacc gccttaccgt 60

ggcggtaaca ggggttcgaa tcccctaggg gacgc 95

<210> 48
<211> 95
<212> DNA
<213> Artificial sequence

<220>
<223> an artificially synthesized sequence

<400> 48
ggcgtaatac gactcactat agtccccttc gtctagaggc ccaggacacc gccttaccga 60

ggcggtaaca ggggttcgaa tcccctaggg gacgc 95

<210> 49
<211> 95
<212> DNA

<213>  Artificial sequence

<220>
<223>  an artificially synthesized sequence

<400>  49
ggcgtaatac gactcactat agtccccttc gtctagaggc ccaggacacc gccataccgt       60

ggcggtaaca ggggttcgaa tcccctaggg gacgc                                  95

<210>  50
<211>  95
<212>  DNA
<213>  Artificial sequence

<220>
<223>  an artificially synthesized sequence

<400>  50
ggcgtaatac gactcactat agtccccttc gtctagaggc ccaggacacc gccataccat       60

ggcggtaaca ggggttcgaa tcccctaggg gacgc                                  95

<210>  51
<211>  95
<212>  DNA
<213>  Artificial sequence

<220>
<223>  an artificially synthesized sequence

<400>  51
ggcgtaatac gactcactat agtccccttc gtctagaggc ccaggacacc gccctcccaa       60

ggcggtaaca ggggttcgaa tcccctaggg gacgc                                  95

<210>  52
<211>  95
<212>  DNA
<213>  Artificial sequence

<220>
<223>  an artificially synthesized sequence

<400>  52
ggcgtaatac gactcactat agtccccttc gtctagaggc ccaggacacc gccttcccac       60

ggcggtaaca ggggttcgaa tcccctaggg gacgc                                  95

<210>  53
<211>  95
<212>  DNA
<213>  Artificial sequence

<220>
<223>  an artificially synthesized sequence

<400>  53
ggcgtaatac gactcactat agtccccttc gtctagaggc ccaggacacc gccttcccaa       60

ggcggtaaca ggggttcgaa tcccctaggg gacgc 95

<210> 54
<211> 95
<212> DNA
<213> Artificial sequence

<220>
<223> an artificially synthesized sequence

<400> 54
ggcgtaatac gactcactat agtccccttc gtctagaggc ccaggacacc gccctcccac 60

ggcggtaaca ggggttcgaa tcccctaggg gacgc 95

<210> 55
<211> 95
<212> DNA
<213> Artificial sequence

<220>
<223> an artificially synthesized sequence

<400> 55
ggcgtaatac gactcactat agtccccttc gtctagaggc ccaggacacc gccctcccga 60

ggcggtaaca ggggttcgaa tcccctaggg gacgc 95

<210> 56
<211> 95
<212> DNA
<213> Artificial sequence

<220>
<223> an artificially synthesized sequence

<400> 56
ggcgtaatac gactcactat agtccccttc gtctagaggc ccaggacacc gccttcccat 60

ggcggtaaca ggggttcgaa tcccctaggg gacgc 95

<210> 57
<211> 95
<212> DNA
<213> Artificial sequence

<220>
<223> an artificially synthesized sequence

<400> 57
ggcgtaatac gactcactat agtccccttc gtctagaggc ccaggacacc gccttcccgt 60

ggcggtaaca ggggttcgaa tcccctaggg gacgc 95

<210> 58
<211> 95
<212> DNA

<213> Artificial sequence

<220>
<223> an artificially synthesized sequence

<400> 58
ggcgtaatac gactcactat agtccccttc gtctagaggc ccaggacacc gccttcccga 60

ggcggtaaca ggggttcgaa tcccctaggg gacgc 95

<210> 59
<211> 95
<212> DNA
<213> Artificial sequence

<220>
<223> an artificially synthesized sequence

<400> 59
ggcgtaatac gactcactat agtccccttc gtctagaggc ccaggacacc gccatcccgt 60

ggcggtaaca ggggttcgaa tcccctaggg gacgc 95

<210> 60
<211> 95
<212> DNA
<213> Artificial sequence

<220>
<223> an artificially synthesized sequence

<400> 60
ggcgtaatac gactcactat agtccccttc gtctagaggc ccaggacacc gccatcccat 60

ggcggtaaca ggggttcgaa tcccctaggg gacgc 95

<210> 61
<211> 96
<212> DNA
<213> Artificial sequence

<220>
<223> an artificially synthesized sequence

<400> 61
ggcgtaatac gactcactat aggagcggta gttcagtcgg ttagaatacc tgcctaagaa 60

gcagggggtc gcgggttcga gtcccgtccg ttccgc 96

<210> 62
<211> 96
<212> DNA
<213> Artificial sequence

<220>
<223> an artificially synthesized sequence

<400> 62
ggcgtaatac gactcactat aggagcggta gttcagtcgg ttagaatacc tgcttaagac 60

gcagggggtc gcgggttcga gtcccgtccg ttccgc 96

<210> 63
<211> 96
<212> DNA
<213> Artificial sequence

<220>
<223> an artificially synthesized sequence

<400> 63
ggcgtaatac gactcactat aggagcggta gttcagtcgg ttagaatacc tgcttaagaa 60

gcagggggtc gcgggttcga gtcccgtccg ttccgc 96

<210> 64
<211> 96
<212> DNA
<213> Artificial sequence

<220>
<223> an artificially synthesized sequence

<400> 64
ggcgtaatac gactcactat aggagcggta gttcagtcgg ttagaatacc tgcctaagac 60

gcagggggtc gcgggttcga gtcccgtccg ttccgc 96

<210> 65
<211> 96
<212> DNA
<213> Artificial sequence

<220>
<223> an artificially synthesized sequence

<400> 65
ggcgtaatac gactcactat aggagcggta gttcagtcgg ttagaatacc tgcctaagga 60

gcagggggtc gcgggttcga gtcccgtccg ttccgc 96

<210> 66
<211> 96
<212> DNA
<213> Artificial sequence

<220>
<223> an artificially synthesized sequence

<400> 66
ggcgtaatac gactcactat aggagcggta gttcagtcgg ttagaatacc tgcttaagat 60

gcagggggtc gcgggttcga gtcccgtccg ttccgc 96

<210> 67
<211> 96
<212> DNA

<213> Artificial sequence

<220>
<223> an artificially synthesized sequence

<400> 67
ggcgtaatac gactcactat aggagcggta gttcagtcgg ttagaatacc tgcttaaggt 60

gcagggggtc gcgggttcga gtcccgtccg ttccgc 96

<210> 68
<211> 96
<212> DNA
<213> Artificial sequence

<220>
<223> an artificially synthesized sequence

<400> 68
ggcgtaatac gactcactat aggagcggta gttcagtcgg ttagaatacc tgcttaagga 60

gcagggggtc gcgggttcga gtcccgtccg ttccgc 96

<210> 69
<211> 96
<212> DNA
<213> Artificial sequence

<220>
<223> an artificially synthesized sequence

<400> 69
ggcgtaatac gactcactat aggagcggta gttcagtcgg ttagaatacc tgcataaggt 60

gcagggggtc gcgggttcga gtcccgtccg ttccgc 96

<210> 70
<211> 96
<212> DNA
<213> Artificial sequence

<220>
<223> an artificially synthesized sequence

<400> 70
ggcgtaatac gactcactat aggagcggta gttcagtcgg ttagaatacc tgcataagat 60

gcagggggtc gcgggttcga gtcccgtccg ttccgc 96

<210> 71
<211> 96
<212> DNA
<213> Artificial sequence

<220>
<223> an artificially synthesized sequence

<400> 71
ggcgtaatac gactcactat aggagcggta gttcagtcgg ttagaatacc tgcctcagaa 60

gcagggggtc gcgggttcga gtcccgtccg ttccgc 96

<210> 72
<211> 96
<212> DNA
<213> Artificial sequence

<220>
<223> an artificially synthesized sequence

<400> 72
ggcgtaatac gactcactat aggagcggta gttcagtcgg ttagaatacc tgcttcagac 60

gcagggggtc gcgggttcga gtcccgtccg ttccgc 96

<210> 73
<211> 96
<212> DNA
<213> Artificial sequence

<220>
<223> an artificially synthesized sequence

<400> 73
ggcgtaatac gactcactat aggagcggta gttcagtcgg ttagaatacc tgcttcagaa 60

gcagggggtc gcgggttcga gtcccgtccg ttccgc 96

<210> 74
<211> 96
<212> DNA
<213> Artificial sequence

<220>
<223> an artificially synthesized sequence

<400> 74
ggcgtaatac gactcactat aggagcggta gttcagtcgg ttagaatacc tgcctcagac 60

gcagggggtc gcgggttcga gtcccgtccg ttccgc 96

<210> 75
<211> 96
<212> DNA
<213> Artificial sequence

<220>
<223> an artificially synthesized sequence

<400> 75
ggcgtaatac gactcactat aggagcggta gttcagtcgg ttagaatacc tgcctcagga 60

gcagggggtc gcgggttcga gtcccgtccg ttccgc 96

<210> 76
<211> 96
<212> DNA

<213> Artificial sequence

<220>
<223> an artificially synthesized sequence

<400> 76
ggcgtaatac gactcactat aggagcggta gttcagtcgg ttagaatacc tgcttcagat 60

gcagggggtc gcgggttcga gtcccgtccg ttccgc 96

<210> 77
<211> 96
<212> DNA
<213> Artificial sequence

<220>
<223> an artificially synthesized sequence

<400> 77
ggcgtaatac gactcactat aggagcggta gttcagtcgg ttagaatacc tgcttcaggt 60

gcagggggtc gcgggttcga gtcccgtccg ttccgc 96

<210> 78
<211> 96
<212> DNA
<213> Artificial sequence

<220>
<223> an artificially synthesized sequence

<400> 78
ggcgtaatac gactcactat aggagcggta gttcagtcgg ttagaatacc tgcttcagga 60

gcagggggtc gcgggttcga gtcccgtccg ttccgc 96

<210> 79
<211> 96
<212> DNA
<213> Artificial sequence

<220>
<223> an artificially synthesized sequence

<400> 79
ggcgtaatac gactcactat aggagcggta gttcagtcgg ttagaatacc tgcatcaggt 60

gcagggggtc gcgggttcga gtcccgtccg ttccgc 96

<210> 80
<211> 96
<212> DNA
<213> Artificial sequence

<220>
<223> an artificially synthesized sequence

<400> 80
ggcgtaatac gactcactat aggagcggta gttcagtcgg ttagaatacc tgcatcagat 60

gcagggggtc gcgggttcga gtcccgtccg ttccgc 96

<210> 81
<211> 95
<212> DNA
<213> Artificial sequence

<220>
<223> an artificially synthesized sequence

<400> 81
ggcgtaatac gactcactat aggctctgta gttcagtcgg tagaacggcg gactaagaat 60

ccgtatgtca ctggttcgag tccagtcaga gccgc 95

<210> 82
<211> 95
<212> DNA
<213> Artificial sequence

<220>
<223> an artificially synthesized sequence

<400> 82
ggcgtaatac gactcactat aggctctgta gttcagtcgg tagaacggcg gattaagact 60

ccgtatgtca ctggttcgag tccagtcaga gccgc 95

<210> 83
<211> 95
<212> DNA
<213> Artificial sequence

<220>
<223> an artificially synthesized sequence

<400> 83
ggcgtaatac gactcactat aggctctgta gttcagtcgg tagaacggcg gattaagaat 60

ccgtatgtca ctggttcgag tccagtcaga gccgc 95

<210> 84
<211> 95
<212> DNA
<213> Artificial sequence

<220>
<223> an artificially synthesized sequence

<400> 84
ggcgtaatac gactcactat aggctctgta gttcagtcgg tagaacggcg gactaagact 60

ccgtatgtca ctggttcgag tccagtcaga gccgc 95

<210> 85
<211> 95
<212> DNA

<213> Artificial sequence

<220>
<223> an artificially synthesized sequence

<400> 85
ggcgtaatac gactcactat aggctctgta gttcagtcgg tagaacggcg gactaaggat 60

ccgtatgtca ctggttcgag tccagtcaga gccgc 95

<210> 86
<211> 95
<212> DNA
<213> Artificial sequence

<220>
<223> an artificially synthesized sequence

<400> 86
ggcgtaatac gactcactat aggctctgta gttcagtcgg tagaacggcg gattaagatt 60

ccgtatgtca ctggttcgag tccagtcaga gccgc 95

<210> 87
<211> 95
<212> DNA
<213> Artificial sequence

<220>
<223> an artificially synthesized sequence

<400> 87
ggcgtaatac gactcactat aggctctgta gttcagtcgg tagaacggcg gattaaggtt 60

ccgtatgtca ctggttcgag tccagtcaga gccgc 95

<210> 88
<211> 95
<212> DNA
<213> Artificial sequence

<220>
<223> an artificially synthesized sequence

<400> 88
ggcgtaatac gactcactat aggctctgta gttcagtcgg tagaacggcg gattaaggat 60

ccgtatgtca ctggttcgag tccagtcaga gccgc 95

<210> 89
<211> 95
<212> DNA
<213> Artificial sequence

<220>
<223> an artificially synthesized sequence

<400> 89
ggcgtaatac gactcactat aggctctgta gttcagtcgg tagaacggcg gaataaggtt 60

ccgtatgtca ctggttcgag tccagtcaga gccgc 95

<210> 90
<211> 95
<212> DNA
<213> Artificial sequence

<220>
<223> an artificially synthesized sequence

<400> 90
ggcgtaatac gactcactat aggctctgta gttcagtcgg tagaacggcg gaataagatt 60

ccgtatgtca ctggttcgag tccagtcaga gccgc 95

<210> 91
<211> 95
<212> DNA
<213> Artificial sequence

<220>
<223> an artificially synthesized sequence

<400> 91
ggcgtaatac gactcactat aggctctgta gttcagtcgg tagaacggcg gactcagaat 60

ccgtatgtca ctggttcgag tccagtcaga gccgc 95

<210> 92
<211> 95
<212> DNA
<213> Artificial sequence

<220>
<223> an artificially synthesized sequence

<400> 92
ggcgtaatac gactcactat aggctctgta gttcagtcgg tagaacggcg gattcagact 60

ccgtatgtca ctggttcgag tccagtcaga gccgc 95

<210> 93
<211> 95
<212> DNA
<213> Artificial sequence

<220>
<223> an artificially synthesized sequence

<400> 93
ggcgtaatac gactcactat aggctctgta gttcagtcgg tagaacggcg gattcagaat 60

ccgtatgtca ctggttcgag tccagtcaga gccgc 95

<210> 94
<211> 95
<212> DNA

<213> Artificial sequence

<220>
<223> an artificially synthesized sequence

<400> 94
ggcgtaatac gactcactat aggctctgta gttcagtcgg tagaacggcg gactcagact 60

ccgtatgtca ctggttcgag tccagtcaga gccgc 95

<210> 95
<211> 95
<212> DNA
<213> Artificial sequence

<220>
<223> an artificially synthesized sequence

<400> 95
ggcgtaatac gactcactat aggctctgta gttcagtcgg tagaacggcg gactcaggat 60

ccgtatgtca ctggttcgag tccagtcaga gccgc 95

<210> 96
<211> 95
<212> DNA
<213> Artificial sequence

<220>
<223> an artificially synthesized sequence

<400> 96
ggcgtaatac gactcactat aggctctgta gttcagtcgg tagaacggcg gattcagatt 60

ccgtatgtca ctggttcgag tccagtcaga gccgc 95

<210> 97
<211> 95
<212> DNA
<213> Artificial sequence

<220>
<223> an artificially synthesized sequence

<400> 97
ggcgtaatac gactcactat aggctctgta gttcagtcgg tagaacggcg gattcaggtt 60

ccgtatgtca ctggttcgag tccagtcaga gccgc 95

<210> 98
<211> 95
<212> DNA
<213> Artificial sequence

<220>
<223> an artificially synthesized sequence

<400> 98
ggcgtaatac gactcactat aggctctgta gttcagtcgg tagaacggcg gattcaggat 60

ccgtatgtca ctggttcgag tccagtcaga gccgc 95

<210> 99
<211> 95
<212> DNA
<213> Artificial sequence

<220>
<223> an artificially synthesized sequence

<400> 99
ggcgtaatac gactcactat aggctctgta gttcagtcgg tagaacggcg gaatcaggtt 60

ccgtatgtca ctggttcgag tccagtcaga gccgc 95

<210> 100
<211> 95
<212> DNA
<213> Artificial sequence

<220>
<223> an artificially synthesized sequence

<400> 100
ggcgtaatac gactcactat aggctctgta gttcagtcgg tagaacggcg gaatcagatt 60

ccgtatgtca ctggttcgag tccagtcaga gccgc 95

<210> 101
<211> 95
<212> DNA
<213> Artificial sequence

<220>
<223> an artificially synthesized sequence

<400> 101
ggcgtaatac gactcactat agtccccttc gtctagaggc ccaggacacc gccctgccac 60

ggcggtaaca ggggttcgaa tcccctaggg gacgc 95

<210> 102
<211> 95
<212> DNA
<213> Artificial sequence

<220>
<223> an artificially synthesized sequence

<400> 102
ggcgtaatac gactcactat agtccccttc gtctagaggc ccaggacacc gccttgccgt 60

ggcggtaaca ggggttcgaa tcccctaggg gacgc 95

<210> 103
<211> 95
<212> DNA

<213> Artificial sequence

<220>
<223> an artificially synthesized sequence

<400> 103
ggcgtaatac gactcactat agtccccttc gtctagaggc ccaggacacc gccttgccga 60

ggcggtaaca ggggttcgaa tcccctaggg gacgc 95

<210> 104
<211> 95
<212> DNA
<213> Artificial sequence

<220>
<223> an artificially synthesized sequence

<400> 104
ggcgtaatac gactcactat agtccccttc gtctagaggc ccaggacacc gcccttccac 60

ggcggtaaca ggggttcgaa tcccctaggg gacgc 95

<210> 105
<211> 96
<212> DNA
<213> Artificial sequence

<220>
<223> an artificially synthesized sequence

<400> 105
ggcgtaatac gactcactat aggcggggtg gagcagcctg gtagctcgtc gggctcataa 60

cccgaagatc gtcggttcaa atccggcccc cgcaac 96

<210> 106
<211> 96
<212> DNA
<213> Artificial sequence

<220>
<223> an artificially synthesized sequence

<400> 106
ggcgtaatac gactcactat aggagcggta gttcagtcgg ttagaatacc tgctttaggt 60

gcagggggtc gcgggttcga gtcccgtccg ttccgc 96

<210> 107
<211> 95
<212> DNA
<213> Artificial sequence

<220>
<223> an artificially synthesized sequence

<400> 107
ggcgtaatac gactcactat aggctctgta gttcagtcgg tagaacggcg gatttaggtt 60

ccgtatgtca ctggttcgag tccagtcaga gccgc 95

<210> 108
<211> 74
<212> RNA
<213> Artificial sequence

<220>
<223> an artificially synthesized sequence

<400> 108
guccccuucg ucuagaggcc caggacaccg cccuaagaag gcgguaacag ggguucgaau 60

ccccuagggg acgc 74

<210> 109
<211> 74
<212> RNA
<213> Artificial sequence

<220>
<223> an artificially synthesized sequence

<400> 109
guccccuucg ucuagaggcc caggacaccg ccuuaagacg gcgguaacag ggguucgaau 60

ccccuagggg acgc 74

<210> 110
<211> 74
<212> RNA
<213> Artificial sequence

<220>
<223> an artificially synthesized sequence

<400> 110
guccccuucg ucuagaggcc caggacaccg ccuuaagaag gcgguaacag ggguucgaau 60

ccccuagggg acgc 74

<210> 111
<211> 74
<212> RNA
<213> Artificial sequence

<220>
<223> an artificially synthesized sequence

<400> 111
guccccuucg ucuagaggcc caggacaccg cccuaagacg gcgguaacag ggguucgaau 60

ccccuagggg acgc 74

<210> 112
<211> 74
<212> RNA

<213> Artificial sequence

<220>
<223> an artificially synthesized sequence

<400> 112
guccccuucg ucuagaggcc caggacaccg cccuaaggag gcgguaacag ggguucgaau 60

ccccuagggg acgc 74

<210> 113
<211> 74
<212> RNA
<213> Artificial sequence

<220>
<223> an artificially synthesized sequence

<400> 113
guccccuucg ucuagaggcc caggacaccg ccuuaagaug gcgguaacag ggguucgaau 60

ccccuagggg acgc 74

<210> 114
<211> 74
<212> RNA
<213> Artificial sequence

<220>
<223> an artificially synthesized sequence

<400> 114
guccccuucg ucuagaggcc caggacaccg ccuuaaggug gcgguaacag ggguucgaau 60

ccccuagggg acgc 74

<210> 115
<211> 74
<212> RNA
<213> Artificial sequence

<220>
<223> an artificially synthesized sequence

<400> 115
guccccuucg ucuagaggcc caggacaccg ccuuaaggag gcgguaacag ggguucgaau 60

ccccuagggg acgc 74

<210> 116
<211> 74
<212> RNA
<213> Artificial sequence

<220>
<223> an artificially synthesized sequence

<400> 116
guccccuucg ucuagaggcc caggacaccg ccauaaggug gcgguaacag ggguucgaau 60

ccccuagggg acgc 74

<210> 117
<211> 74
<212> RNA
<213> Artificial sequence

<220>
<223> an artificially synthesized sequence

<400> 117
guccccuucg ucuagaggcc caggacaccg ccauaagaug gcgguaacag gguucgaau 60

ccccuagggg acgc 74

<210> 118
<211> 74
<212> RNA
<213> Artificial sequence

<220>
<223> an artificially synthesized sequence

<400> 118
guccccuucg ucuagaggcc caggacaccg cccucagaag gcgguaacag gguucgaau 60

ccccuagggg acgc 74

<210> 119
<211> 74
<212> RNA
<213> Artificial sequence

<220>
<223> an artificially synthesized sequence

<400> 119
guccccuucg ucuagaggcc caggacaccg ccuucagacg gcgguaacag gguucgaau 60

ccccuagggg acgc 74

<210> 120
<211> 74
<212> RNA
<213> Artificial sequence

<220>
<223> an artificially synthesized sequence

<400> 120
guccccuucg ucuagaggcc caggacaccg ccuucagaag gcgguaacag gguucgaau 60

ccccuagggg acgc 74

<210> 121
<211> 74
<212> RNA

<213> Artificial sequence

<220>
<223> an artificially synthesized sequence

<400> 121
guccccuucg ucuagaggcc caggacaccg cccucagacg gcgguaacag ggguucgaau 60

ccccuagggg acgc 74

<210> 122
<211> 74
<212> RNA
<213> Artificial sequence

<220>
<223> an artificially synthesized sequence

<400> 122
guccccuucg ucuagaggcc caggacaccg cccucaggag gcgguaacag ggguucgaau 60

ccccuagggg acgc 74

<210> 123
<211> 74
<212> RNA
<213> Artificial sequence

<220>
<223> an artificially synthesized sequence

<400> 123
guccccuucg ucuagaggcc caggacaccg ccuucagaug gcgguaacag ggguucgaau 60

ccccuagggg acgc 74

<210> 124
<211> 74
<212> RNA
<213> Artificial sequence

<220>
<223> an artificially synthesized sequence

<400> 124
guccccuucg ucuagaggcc caggacaccg ccuucaggug gcgguaacag ggguucgaau 60

ccccuagggg acgc 74

<210> 125
<211> 74
<212> RNA
<213> Artificial sequence

<220>
<223> an artificially synthesized sequence

<400> 125
guccccuucg ucuagaggcc caggacaccg ccuucaggag gcgguaacag ggguucgaau 60

ccccuagggg acgc 74

<210> 126
<211> 74
<212> RNA
<213> Artificial sequence

<220>
<223> an artificially synthesized sequence

<400> 126
guccccuucg ucuagaggcc caggacaccg ccaucaggug gcgguaacag gguucgaau 60

ccccuagggg acgc 74

<210> 127
<211> 74
<212> RNA
<213> Artificial sequence

<220>
<223> an artificially synthesized sequence

<400> 127
guccccuucg ucuagaggcc caggacaccg ccaucagaug gcgguaacag gguucgaau 60

ccccuagggg acgc 74

<210> 128
<211> 74
<212> RNA
<213> Artificial sequence

<220>
<223> an artificially synthesized sequence

<400> 128
guccccuucg ucuagaggcc caggacaccg cccuaggaag gcgguaacag gguucgaau 60

ccccuagggg acgc 74

<210> 129
<211> 74
<212> RNA
<213> Artificial sequence

<220>
<223> an artificially synthesized sequence

<400> 129
guccccuucg ucuagaggcc caggacaccg ccuuaggacg gcgguaacag gguucgaau 60

ccccuagggg acgc 74

<210> 130
<211> 74
<212> RNA

<213> Artificial sequence

<220>
<223> an artificially synthesized sequence

<400> 130
guccccuucg ucuagaggcc caggacaccg ccuuaggaag gcgguaacag ggguucgaau 60

ccccuagggg acgc 74

<210> 131
<211> 74
<212> RNA
<213> Artificial sequence

<220>
<223> an artificially synthesized sequence

<400> 131
guccccuucg ucuagaggcc caggacaccg cccuaggacg gcgguaacag ggguucgaau 60

ccccuagggg acgc 74

<210> 132
<211> 74
<212> RNA
<213> Artificial sequence

<220>
<223> an artificially synthesized sequence

<400> 132
guccccuucg ucuagaggcc caggacaccg cccuagggag gcgguaacag ggguucgaau 60

ccccuagggg acgc 74

<210> 133
<211> 74
<212> RNA
<213> Artificial sequence

<220>
<223> an artificially synthesized sequence

<400> 133
guccccuucg ucuagaggcc caggacaccg ccuuaggaug gcgguaacag ggguucgaau 60

ccccuagggg acgc 74

<210> 134
<211> 74
<212> RNA
<213> Artificial sequence

<220>
<223> an artificially synthesized sequence

<400> 134
guccccuucg ucuagaggcc caggacaccg ccuuagggug gcgguaacag ggguucgaau 60

ccccuagggg acgc 74

<210> 135
<211> 74
<212> RNA
<213> Artificial sequence

<220>
<223> an artificially synthesized sequence

<400> 135
guccccuucg ucuagaggcc caggacaccg ccuuagggag gcgguaacag gguucgaau 60

ccccuagggg acgc 74

<210> 136
<211> 74
<212> RNA
<213> Artificial sequence

<220>
<223> an artificially synthesized sequence

<400> 136
guccccuucg ucuagaggcc caggacaccg ccauagggug gcgguaacag gguucgaau 60

ccccuagggg acgc 74

<210> 137
<211> 74
<212> RNA
<213> Artificial sequence

<220>
<223> an artificially synthesized sequence

<400> 137
guccccuucg ucuagaggcc caggacaccg ccauaggaug gcgguaacag gguucgaau 60

ccccuagggg acgc 74

<210> 138
<211> 74
<212> RNA
<213> Artificial sequence

<220>
<223> an artificially synthesized sequence

<400> 138
guccccuucg ucuagaggcc caggacaccg cccucggaag gcgguaacag gguucgaau 60

ccccuagggg acgc 74

<210> 139
<211> 74
<212> RNA

<213>  Artificial sequence

<220>
<223>  an artificially synthesized sequence

<400>  139
guccccuucg ucuagaggcc caggacaccg ccuucggacg gcgguaacag ggguucgaau  60

ccccuagggg acgc  74

<210>  140
<211>  74
<212>  RNA
<213>  Artificial sequence

<220>
<223>  an artificially synthesized sequence

<400>  140
guccccuucg ucuagaggcc caggacaccg ccuucggaag gcgguaacag ggguucgaau  60

ccccuagggg acgc  74

<210>  141
<211>  74
<212>  RNA
<213>  Artificial sequence

<220>
<223>  an artificially synthesized sequence

<400>  141
guccccuucg ucuagaggcc caggacaccg cccucggacg gcgguaacag ggguucgaau  60

ccccuagggg acgc  74

<210>  142
<211>  74
<212>  RNA
<213>  Artificial sequence

<220>
<223>  an artificially synthesized sequence

<400>  142
guccccuucg ucuagaggcc caggacaccg cccucgggag gcgguaacag ggguucgaau  60

ccccuagggg acgc  74

<210>  143
<211>  74
<212>  RNA
<213>  Artificial sequence

<220>
<223>  an artificially synthesized sequence

<400>  143
guccccuucg ucuagaggcc caggacaccg ccuucggaug gcgguaacag ggguucgaau  60

ccccuagggg acgc 74

<210> 144
<211> 74
<212> RNA
<213> Artificial sequence

<220>
<223> an artificially synthesized sequence

<400> 144
guccccuucg ucuagaggcc caggacaccg ccuucgggug gcgguaacag ggguucgaau 60

ccccuagggg acgc 74

<210> 145
<211> 74
<212> RNA
<213> Artificial sequence

<220>
<223> an artificially synthesized sequence

<400> 145
guccccuucg ucuagaggcc caggacaccg ccuucgggag gcgguaacag ggguucgaau 60

ccccuagggg acgc 74

<210> 146
<211> 74
<212> RNA
<213> Artificial sequence

<220>
<223> an artificially synthesized sequence

<400> 146
guccccuucg ucuagaggcc caggacaccg ccaucgggug gcgguaacag ggguucgaau 60

ccccuagggg acgc 74

<210> 147
<211> 74
<212> RNA
<213> Artificial sequence

<220>
<223> an artificially synthesized sequence

<400> 147
guccccuucg ucuagaggcc caggacaccg ccaucggaug gcgguaacag ggguucgaau 60

ccccuagggg acgc 74

<210> 148
<211> 74
<212> RNA

<213> Artificial sequence

<220>
<223> an artificially synthesized sequence

<400> 148
guccccuucg ucuagaggcc caggacaccg cccuaccaag gcgguaacag ggguucgaau 60

ccccuagggg acgc 74

<210> 149
<211> 74
<212> RNA
<213> Artificial sequence

<220>
<223> an artificially synthesized sequence

<400> 149
guccccuucg ucuagaggcc caggacaccg ccuuaccacg gcgguaacag ggguucgaau 60

ccccuagggg acgc 74

<210> 150
<211> 74
<212> RNA
<213> Artificial sequence

<220>
<223> an artificially synthesized sequence

<400> 150
guccccuucg ucuagaggcc caggacaccg ccuuaccaag gcgguaacag ggguucgaau 60

ccccuagggg acgc 74

<210> 151
<211> 74
<212> RNA
<213> Artificial sequence

<220>
<223> an artificially synthesized sequence

<400> 151
guccccuucg ucuagaggcc caggacaccg cccuaccacg gcgguaacag ggguucgaau 60

ccccuagggg acgc 74

<210> 152
<211> 74
<212> RNA
<213> Artificial sequence

<220>
<223> an artificially synthesized sequence

<400> 152
guccccuucg ucuagaggcc caggacaccg cccuaccgag gcgguaacag ggguucgaau 60

ccccuagggg acgc 74

<210> 153
<211> 74
<212> RNA
<213> Artificial sequence

<220>
<223> an artificially synthesized sequence

<400> 153
guccccuucg ucuagaggcc caggacaccg ccuuaccaug gcgguaacag ggguucgaau 60

ccccuagggg acgc 74

<210> 154
<211> 74
<212> RNA
<213> Artificial sequence

<220>
<223> an artificially synthesized sequence

<400> 154
guccccuucg ucuagaggcc caggacaccg ccuuaccgug gcgguaacag ggguucgaau 60

ccccuagggg acgc 74

<210> 155
<211> 74
<212> RNA
<213> Artificial sequence

<220>
<223> an artificially synthesized sequence

<400> 155
guccccuucg ucuagaggcc caggacaccg ccuuaccgag gcgguaacag ggguucgaau 60

ccccuagggg acgc 74

<210> 156
<211> 74
<212> RNA
<213> Artificial sequence

<220>
<223> an artificially synthesized sequence

<400> 156
guccccuucg ucuagaggcc caggacaccg ccauaccgug gcgguaacag ggguucgaau 60

ccccuagggg acgc 74

<210> 157
<211> 74
<212> RNA

<213> Artificial sequence

<220>
<223> an artificially synthesized sequence

<400> 157
guccccuucg ucuagaggcc caggacaccg ccauaccaug gcgguaacag ggguucgaau 60

ccccuagggg acgc 74

<210> 158
<211> 74
<212> RNA
<213> Artificial sequence

<220>
<223> an artificially synthesized sequence

<400> 158
guccccuucg ucuagaggcc caggacaccg cccucccaag gcgguaacag ggguucgaau 60

ccccuagggg acgc 74

<210> 159
<211> 74
<212> RNA
<213> Artificial sequence

<220>
<223> an artificially synthesized sequence

<400> 159
guccccuucg ucuagaggcc caggacaccg ccuucccacg gcgguaacag ggguucgaau 60

ccccuagggg acgc 74

<210> 160
<211> 74
<212> RNA
<213> Artificial sequence

<220>
<223> an artificially synthesized sequence

<400> 160
guccccuucg ucuagaggcc caggacaccg ccuucccaag gcgguaacag ggguucgaau 60

ccccuagggg acgc 74

<210> 161
<211> 74
<212> RNA
<213> Artificial sequence

<220>
<223> an artificially synthesized sequence

<400> 161
guccccuucg ucuagaggcc caggacaccg cccucccacg gcgguaacag ggguucgaau 60

ccccuagggg acgc 74

<210> 162
<211> 74
<212> RNA
<213> Artificial sequence

<220>
<223> an artificially synthesized sequence

<400> 162
guccccuucg ucuagaggcc caggacaccg cccucccgag gcgguaacag ggguucgaau 60

ccccuagggg acgc 74

<210> 163
<211> 74
<212> RNA
<213> Artificial sequence

<220>
<223> an artificially synthesized sequence

<400> 163
guccccuucg ucuagaggcc caggacaccg ccuucccaug gcgguaacag ggguucgaau 60

ccccuagggg acgc 74

<210> 164
<211> 74
<212> RNA
<213> Artificial sequence

<220>
<223> an artificially synthesized sequence

<400> 164
guccccuucg ucuagaggcc caggacaccg ccuucccgug gcgguaacag ggguucgaau 60

ccccuagggg acgc 74

<210> 165
<211> 74
<212> RNA
<213> Artificial sequence

<220>
<223> an artificially synthesized sequence

<400> 165
guccccuucg ucuagaggcc caggacaccg ccuucccgag gcgguaacag ggguucgaau 60

ccccuagggg acgc 74

<210> 166
<211> 74
<212> RNA

<213> Artificial sequence

<220>
<223> an artificially synthesized sequence

<400> 166
gucccuucg ucuagaggcc caggacaccg ccaucccgug gcgguaacag ggguucgaau 60

ccccuagggg acgc 74

<210> 167
<211> 74
<212> RNA
<213> Artificial sequence

<220>
<223> an artificially synthesized sequence

<400> 167
gucccuucg ucuagaggcc caggacaccg ccaucccaug gcgguaacag ggguucgaau 60

ccccuagggg acgc 74

<210> 168
<211> 75
<212> RNA
<213> Artificial sequence

<220>
<223> an artificially synthesized sequence

<400> 168
ggagcgguag uucagucggu uagaauaccu gccuaagaag caggggggucg cggguucgag 60

ucccguccgu uccgc 75

<210> 169
<211> 75
<212> RNA
<213> Artificial sequence

<220>
<223> an artificially synthesized sequence

<400> 169
ggagcgguag uucagucggu uagaauaccu gcuuaagacg cagggggucg cggguucgag 60

ucccguccgu uccgc 75

<210> 170
<211> 75
<212> RNA
<213> Artificial sequence

<220>
<223> an artificially synthesized sequence

<400> 170
ggagcgguag uucagucggu uagaauaccu gcuuaagaag cagggggucg cggguucgag 60

ucccguccgu uccgc 75

<210> 171
<211> 75
<212> RNA
<213> Artificial sequence

<220>
<223> an artificially synthesized sequence

<400> 171
ggagcgguag uucagucggu uagaauaccu gccuaagacg caggggucg cggguucgag 60

ucccguccgu uccgc 75

<210> 172
<211> 75
<212> RNA
<213> Artificial sequence

<220>
<223> an artificially synthesized sequence

<400> 172
ggagcgguag uucagucggu uagaauaccu gccuaaggag caggggucg cggguucgag 60

ucccguccgu uccgc 75

<210> 173
<211> 75
<212> RNA
<213> Artificial sequence

<220>
<223> an artificially synthesized sequence

<400> 173
ggagcgguag uucagucggu uagaauaccu gcuuaagaug caggggucg cggguucgag 60

ucccguccgu uccgc 75

<210> 174
<211> 75
<212> RNA
<213> Artificial sequence

<220>
<223> an artificially synthesized sequence

<400> 174
ggagcgguag uucagucggu uagaauaccu gcuuaaggug caggggucg cggguucgag 60

ucccguccgu uccgc 75

<210> 175
<211> 75
<212> RNA

<213> Artificial sequence

<220>
<223> an artificially synthesized sequence

<400> 175
ggagcgguag uucagucggu uagaauaccu gcuuaaggag caggggggucg cggguucgag 60

ucccguccgu uccgc 75

<210> 176
<211> 75
<212> RNA
<213> Artificial sequence

<220>
<223> an artificially synthesized sequence

<400> 176
ggagcgguag uucagucggu uagaauaccu gcauaaggug caggggggucg cggguucgag 60

ucccguccgu uccgc 75

<210> 177
<211> 75
<212> RNA
<213> Artificial sequence

<220>
<223> an artificially synthesized sequence

<400> 177
ggagcgguag uucagucggu uagaauaccu gcauaagaug caggggggucg cggguucgag 60

ucccguccgu uccgc 75

<210> 178
<211> 75
<212> RNA
<213> Artificial sequence

<220>
<223> an artificially synthesized sequence

<400> 178
ggagcgguag uucagucggu uagaauaccu gccucagaag caggggggucg cggguucgag 60

ucccguccgu uccgc 75

<210> 179
<211> 75
<212> RNA
<213> Artificial sequence

<220>
<223> an artificially synthesized sequence

<400> 179
ggagcgguag uucagucggu uagaauaccu gcuucagacg caggggggucg cggguucgag 60

ucccguccgu uccgc 75

<210> 180
<211> 75
<212> RNA
<213> Artificial sequence

<220>
<223> an artificially synthesized sequence

<400> 180
ggagcgguag uucagucggu uagaauaccu gcuucagaag caggggggucg cggguucgag 60

ucccguccgu uccgc 75

<210> 181
<211> 75
<212> RNA
<213> Artificial sequence

<220>
<223> an artificially synthesized sequence

<400> 181
ggagcgguag uucagucggu uagaauaccu gccucagacg caggggggucg cggguucgag 60

ucccguccgu uccgc 75

<210> 182
<211> 75
<212> RNA
<213> Artificial sequence

<220>
<223> an artificially synthesized sequence

<400> 182
ggagcgguag uucagucggu uagaauaccu gccucaggag caggggggucg cggguucgag 60

ucccguccgu uccgc 75

<210> 183
<211> 75
<212> RNA
<213> Artificial sequence

<220>
<223> an artificially synthesized sequence

<400> 183
ggagcgguag uucagucggu uagaauaccu gcuucagaug caggggggucg cggguucgag 60

ucccguccgu uccgc 75

<210> 184
<211> 75
<212> RNA

<213> Artificial sequence

<220>
<223> an artificially synthesized sequence

<400> 184
ggagcgguag uucagucggu uagaauaccu gcuucaggug caggggggucg cggguucgag 60

ucccguccgu uccgc 75

<210> 185
<211> 75
<212> RNA
<213> Artificial sequence

<220>
<223> an artificially synthesized sequence

<400> 185
ggagcgguag uucagucggu uagaauaccu gcuucaggag caggggggucg cggguucgag 60

ucccguccgu uccgc 75

<210> 186
<211> 75
<212> RNA
<213> Artificial sequence

<220>
<223> an artificially synthesized sequence

<400> 186
ggagcgguag uucagucggu uagaauaccu gcaucaggug caggggggucg cggguucgag 60

ucccguccgu uccgc 75

<210> 187
<211> 75
<212> RNA
<213> Artificial sequence

<220>
<223> an artificially synthesized sequence

<400> 187
ggagcgguag uucagucggu uagaauaccu gcaucagaug caggggggucg cggguucgag 60

ucccguccgu uccgc 75

<210> 188
<211> 74
<212> RNA
<213> Artificial sequence

<220>
<223> an artificially synthesized sequence

<400> 188
ggcucuguag uucagucggu agaacggcgg acuaagaauc cguaugucac ugguucgagu 60

ccagucagag ccgc 74

<210> 189
<211> 74
<212> RNA
<213> Artificial sequence

<220>
<223> an artificially synthesized sequence

<400> 189
ggcucuguag uucagucggu agaacggcgg auuaagacuc cguaugucac ugguucgagu 60

ccagucagag ccgc 74

<210> 190
<211> 74
<212> RNA
<213> Artificial sequence

<220>
<223> an artificially synthesized sequence

<400> 190
ggcucuguag uucagucggu agaacggcgg auuaagaauc cguaugucac ugguucgagu 60

ccagucagag ccgc 74

<210> 191
<211> 74
<212> RNA
<213> Artificial sequence

<220>
<223> an artificially synthesized sequence

<400> 191
ggcucuguag uucagucggu agaacggcgg acuaagacuc cguaugucac ugguucgagu 60

ccagucagag ccgc 74

<210> 192
<211> 74
<212> RNA
<213> Artificial sequence

<220>
<223> an artificially synthesized sequence

<400> 192
ggcucuguag uucagucggu agaacggcgg acuaaggauc cguaugucac ugguucgagu 60

ccagucagag ccgc 74

<210> 193
<211> 74
<212> RNA

<213> Artificial sequence

<220>
<223> an artificially synthesized sequence

<400> 193
ggcucuguag uucagucggu agaacggcgg auuaagauuc cguaugucac ugguucgagu 60

ccagucagag ccgc 74

<210> 194
<211> 74
<212> RNA
<213> Artificial sequence

<220>
<223> an artificially synthesized sequence

<400> 194
ggcucuguag uucagucggu agaacggcgg auuaagguuc cguaugucac ugguucgagu 60

ccagucagag ccgc 74

<210> 195
<211> 74
<212> RNA
<213> Artificial sequence

<220>
<223> an artificially synthesized sequence

<400> 195
ggcucuguag uucagucggu agaacggcgg auuaaggauc cguaugucac ugguucgagu 60

ccagucagag ccgc 74

<210> 196
<211> 74
<212> RNA
<213> Artificial sequence

<220>
<223> an artificially synthesized sequence

<400> 196
ggcucuguag uucagucggu agaacggcgg aauaagguuc cguaugucac ugguucgagu 60

ccagucagag ccgc 74

<210> 197
<211> 74
<212> RNA
<213> Artificial sequence

<220>
<223> an artificially synthesized sequence

<400> 197
ggcucuguag uucagucggu agaacggcgg aauaagauuc cguaugucac ugguucgagu 60

ccagucagag ccgc 74

<210> 198
<211> 74
<212> RNA
<213> Artificial sequence

<220>
<223> an artificially synthesized sequence

<400> 198
ggcucuguag uucagucggu agaacggcgg acucagaauc cguaugucac ugguucgagu 60

ccagucagag ccgc 74

<210> 199
<211> 74
<212> RNA
<213> Artificial sequence

<220>
<223> an artificially synthesized sequence

<400> 199
ggcucuguag uucagucggu agaacggcgg auucagacuc cguaugucac ugguucgagu 60

ccagucagag ccgc 74

<210> 200
<211> 74
<212> RNA
<213> Artificial sequence

<220>
<223> an artificially synthesized sequence

<400> 200
ggcucuguag uucagucggu agaacggcgg auucagaauc cguaugucac ugguucgagu 60

ccagucagag ccgc 74

<210> 201
<211> 74
<212> RNA
<213> Artificial sequence

<220>
<223> an artificially synthesized sequence

<400> 201
ggcucuguag uucagucggu agaacggcgg acucagacuc cguaugucac ugguucgagu 60

ccagucagag ccgc 74

<210> 202
<211> 74
<212> RNA

<213> Artificial sequence

<220>
<223> an artificially synthesized sequence

<400> 202
ggcucuguag uucagucggu agaacggcgg acucaggauc cguaugucac ugguucgagu 60

ccagucagag ccgc 74

<210> 203
<211> 74
<212> RNA
<213> Artificial sequence

<220>
<223> an artificially synthesized sequence

<400> 203
ggcucuguag uucagucggu agaacggcgg auucagauuc cguaugucac ugguucgagu 60

ccagucagag ccgc 74

<210> 204
<211> 74
<212> RNA
<213> Artificial sequence

<220>
<223> an artificially synthesized sequence

<400> 204
ggcucuguag uucagucggu agaacggcgg auucagguuc cguaugucac ugguucgagu 60

ccagucagag ccgc 74

<210> 205
<211> 74
<212> RNA
<213> Artificial sequence

<220>
<223> an artificially synthesized sequence

<400> 205
ggcucuguag uucagucggu agaacggcgg auucaggauc cguaugucac ugguucgagu 60

ccagucagag ccgc 74

<210> 206
<211> 74
<212> RNA
<213> Artificial sequence

<220>
<223> an artificially synthesized sequence

<400> 206
ggcucuguag uucagucggu agaacggcgg aaucagguuc cguaugucac ugguucgagu 60

ccagucagag ccgc 74

<210> 207
<211> 74
<212> RNA
<213> Artificial sequence

<220>
<223> an artificially synthesized sequence

<400> 207
ggcucuguag uucagucggu agaacggcgg aaucagauuc cguaugucac ugguucgagu 60

ccagucagag ccgc 74

<210> 208
<211> 74
<212> RNA
<213> Artificial sequence

<220>
<223> an artificially synthesized sequence

<400> 208
guccccuucg ucuagaggcc caggacaccg cccugccacg gcgguaacag ggguucgaau 60

ccccuagggg acgc 74

<210> 209
<211> 74
<212> RNA
<213> Artificial sequence

<220>
<223> an artificially synthesized sequence

<400> 209
guccccuucg ucuagaggcc caggacaccg ccuugccgug gcgguaacag ggguucgaau 60

ccccuagggg acgc 74

<210> 210
<211> 74
<212> RNA
<213> Artificial sequence

<220>
<223> an artificially synthesized sequence

<400> 210
guccccuucg ucuagaggcc caggacaccg ccuugccgag gcgguaacag ggguucgaau 60

ccccuagggg acgc 74

<210> 211
<211> 74
<212> RNA

<213> Artificial sequence

<220>
<223> an artificially synthesized sequence

<400> 211
guccccuucg ucuagaggcc caggacaccg cccuuccacg gcgguaacag ggguucgaau 60

ccccuagggg acgc 74

<210> 212
<211> 75
<212> RNA
<213> Artificial sequence

<220>
<223> an artificially synthesized sequence

<400> 212
ggcggggugg agcagccugg uagcucgucg ggcucauaac ccgaagaucg ucgguucaaa 60

uccggccccc gcaac 75

<210> 213
<211> 75
<212> RNA
<213> Artificial sequence

<220>
<223> an artificially synthesized sequence

<400> 213
ggagcgguag uucagucggu uagaauaccu gcuuuaggug cagggggucg cggguucgag 60

ucccguccgu uccgc 75

<210> 214
<211> 74
<212> RNA
<213> Artificial sequence

<220>
<223> an artificially synthesized sequence

<400> 214
ggcucuguag uucagucggu agaacggcgg auuuagguuc cguaugucac ugguucgagu 60

ccagucagag ccgc 74

<210> 215
<211> 75
<212> RNA
<213> Artificial sequence

<220>
<223> an artificially synthesized sequence

<220>

<221> misc_feature
<222> (35)..(35)
<223> n = lysidine (2-lysylcytidine)

<400> 215
ggagcgguag uucagucggu uagaauaccu gcuunaggug cagggggucg cggguucgag 60

ucccguccgu uccgc 75

<210> 216
<211> 74
<212> RNA
<213> Artificial sequence

<220>
<223> an artificially synthesized sequence

<220>
<221> misc_feature
<222> (34)..(34)
<223> n = lysidine (2-lysylcytidine)

<400> 216
ggcucuguag uucagucggu agaacggcgg auunagguuc cguaugucac ugguucgagu 60

ccagucagag ccgc 74

<210> 217
<211> 34
<212> RNA
<213> Artificial sequence

<220>
<223> an artificially synthesized sequence

<400> 217
ggagcgguag uucagucggu uagaauaccu gcuu 34

<210> 218
<211> 40
<212> RNA
<213> Artificial sequence

<220>
<223> an artificially synthesized sequence

<400> 218
aggugcaggg ggucgcgggu ucgagucccg uccguuccgc 40

<210> 219
<211> 33
<212> RNA
<213> Artificial sequence

<220>
<223> an artificially synthesized sequence

<400> 219

ggcucuguag uucagucggu agaacggcgg auu 33

<210> 220
<211> 40
<212> RNA
<213> Artificial sequence

<220>
<223> an artificially synthesized sequence

<400> 220
agguuccgua ugucacuggu ucgaguccag ucagagccgc 40

<210> 221
<211> 97
<212> DNA
<213> Artificial sequence

<220>
<223> an artificially synthesized sequence

<400> 221
ggcgtaatac gactcactat agggttaact ttaagaagga gatatacata tgacttttat 60

tattggtttt cttattattc cgattggtta agcttcg 97

<210> 222
<211> 97
<212> DNA
<213> Artificial sequence

<220>
<223> an artificially synthesized sequence

<400> 222
ggcgtaatac gactcactat agggttaact ttaagaagga gatatacata tgacttttat 60

tattggtttt ctgattattc cgattggtta agcttcg 97

<210> 223
<211> 97
<212> DNA
<213> Artificial sequence

<220>
<223> an artificially synthesized sequence

<400> 223
ggcgtaatac gactcactat agggttaact ttaagaagga gatatacata tgacttttat 60

tattggtttt cctattattg ctattggtta agcttcg 97

<210> 224
<211> 97
<212> DNA
<213> Artificial sequence

<220>

<223> an artificially synthesized sequence

<400> 224
ggcgtaatac gactcactat agggttaact ttaagaagga gatatacata tgacttttat 60

tattggtttt ccgattattg ctattggtta agcttcg 97

<210> 225
<211> 97
<212> DNA
<213> Artificial sequence

<220>
<223> an artificially synthesized sequence

<400> 225
ggcgtaatac gactcactat agggttaact ttaagaagga gatatacata tgacttttat 60

tattctattt ggtattattc cgattctata agcttcg 97

<210> 226
<211> 97
<212> DNA
<213> Artificial sequence

<220>
<223> an artificially synthesized sequence

<400> 226
ggcgtaatac gactcactat agggttaact ttaagaagga gatatacata tgacttttat 60

tattctattt gggattattc cgattctata agcttcg 97

<210> 227
<211> 97
<212> DNA
<213> Artificial sequence

<220>
<223> an artificially synthesized sequence

<400> 227
ggcgtaatac gactcactat agggttaact ttaagaagga gatatacata tgacttttat 60

tattctattt ggaattattc cgattctata agcttcg 97

<210> 228
<211> 97
<212> DNA
<213> Artificial sequence

<220>
<223> an artificially synthesized sequence

<400> 228
ggcgtaatac gactcactat agggttaact ttaagaagga gatatacata tgacttttat 60

tattggtttt ctaattattc cgattggtta agcttcg 97

<210> 229
<211> 76
<212> RNA
<213> Artificial sequence

<220>
<223> an artificially synthesized sequence

<400> 229
ggguuaacuu uaagaaggag auauacauau gacuuuuauu auugguuuuc uuauuauucc 60

gauugguuaa gcuucg 76

<210> 230
<211> 76
<212> RNA
<213> Artificial sequence

<220>
<223> an artificially synthesized sequence

<400> 230
ggguuaacuu uaagaaggag auauacauau gacuuuuauu auugguuuuc ugauuauucc 60

gauugguuaa gcuucg 76

<210> 231
<211> 76
<212> RNA
<213> Artificial sequence

<220>
<223> an artificially synthesized sequence

<400> 231
ggguuaacuu uaagaaggag auauacauau gacuuuuauu auugguuuuc cuauuauugc 60

uauugguuaa gcuucg 76

<210> 232
<211> 76
<212> RNA
<213> Artificial sequence

<220>
<223> an artificially synthesized sequence

<400> 232
ggguuaacuu uaagaaggag auauacauau gacuuuuauu auugguuuuc cgauuauugc 60

uauugguuaa gcuucg 76

<210> 233
<211> 76
<212> RNA
<213> Artificial sequence

<220>

<223> an artificially synthesized sequence

<400> 233
gggguuaacuu uaagaaggag auauacauau gacuuuuauu auucuauuug guauuauucc 60

gauucuauaa gcuucg 76

<210> 234
<211> 76
<212> RNA
<213> Artificial sequence

<220>
<223> an artificially synthesized sequence

<400> 234
gggguuaacuu uaagaaggag auauacauau gacuuuuauu auucuauuug ggauuauucc 60

gauucuauaa gcuucg 76

<210> 235
<211> 76
<212> RNA
<213> Artificial sequence

<220>
<223> an artificially synthesized sequence

<400> 235
gggguuaacuu uaagaaggag auauacauau gacuuuuauu auucuauuug gaauuauucc 60

gauucuauaa gcuucg 76

<210> 236
<211> 76
<212> RNA
<213> Artificial sequence

<220>
<223> an artificially synthesized sequence

<400> 236
gggguuaacuu uaagaaggag auauacauau gacuuuuauu auugguuuuc uaauuauucc 60

gauugguuaa gcuucg 76

<210> 237
<211> 13
<212> PRT
<213> Artificial sequence

<220>
<223> an artificially synthesized sequence

<220>
<221> MISC_FEATURE
<222> (1)..(1)
<223> Xaa = BdpF

<400> 237

Xaa Thr Phe Ile Ile Gly Phe Ile Ile Ile Pro Ile Gly
1 5 10

<210> 238
<211> 19
<212> PRT
<213> Artificial sequence

<220>
<223> an artificially synthesized sequence

<220>
<221> MISC_FEATURE
<222> (1)..(1)
<223> Xaa = BdpF

<400> 238

Xaa Thr Ile Phe Pro Gly Phe Ile Ile Thr Thr Gly Thr Gly Thr Gly
1 5 10 15

Thr Gly Ala

<210> 239
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = MeHph

<400> 239

Xaa Thr Phe Ile Ile Gly Phe Xaa Ile Ile Pro Ile Gly
1 5 10

<210> 240
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = SPh2Cl

<400> 240

Xaa Thr Phe Ile Ile Gly Phe Xaa Ile Ile Pro Ile Gly
1               5                   10

<210> 241
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = MeHph

<400> 241

Xaa Thr Phe Ile Ile Gly Phe Xaa Ile Ile Ala Ile Gly
1               5                   10

<210> 242
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = SPh2Cl

<400> 242

Xaa Thr Phe Ile Ile Gly Phe Xaa Ile Ile Ala Ile Gly
1 5 10

<210> 243
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = MeHPh

<400> 243

Xaa Thr Phe Ile Ile Leu Phe Xaa Ile Ile Pro Ile Leu
1 5 10

<210> 244
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = SPh2Cl

<400> 244

Xaa Thr Phe Ile Ile Leu Phe Xaa Ile Ile Pro Ile Leu
1 5 10

<210> 245
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = Pic2

<400> 245

Xaa Thr Phe Ile Ile Leu Phe Xaa Ile Ile Pro Ile Leu
1 5 10

<210> 246
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = MeG

<400> 246

Xaa Thr Phe Ile Ile Leu Phe Xaa Ile Ile Pro Ile Leu
1 5 10

<210> 247
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = nBuG

<400> 247

Xaa Thr Phe Ile Ile Leu Phe Xaa Ile Ile Pro Ile Leu
1 5 10

<210> 248
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = dA

<400> 248

Xaa Thr Phe Ile Ile Leu Phe Xaa Ile Ile Pro Ile Leu
1 5 10

<210> 249
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = MeG

<400> 249

Xaa Thr Phe Ile Ile Gly Phe Xaa Ile Ile Ala Ile Gly
1 5 10

<210> 250
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = nBuG

<400> 250

Xaa Thr Phe Ile Ile Gly Phe Xaa Ile Ile Pro Ile Gly
1 5 10

<210> 251
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = SPh2Cl

<400> 251

Xaa Thr Phe Ile Ile Gly Phe Xaa Ile Ile Pro Ile Gly
1 5 10

<210> 252
<211> 13
<212> PRT
<213> Artificial sequence

<220>
<223> an artificially synthesized sequence

<220>
<221> MISC_FEATURE
<222> (1)..(1)
<223> Xaa = BdpF

<220>
<221> MISC_FEATURE
<222> (8)..(8)
<223> Xaa = dA

<400> 252

Xaa Thr Phe Ile Ile Gly Phe Xaa Ile Ile Pro Ile Gly

1 5 10

<210> 253
<211> 74
<212> RNA
<213> Artificial sequence

<220>
<223> tRNA Glu2 body (-CA) (32-38 = n)

<220>
<221> misc_feature
<222> (33)..(39)
<223> n is a, c, g, or u

<400> 253
guccccuucg ucuagaggcc caggacaccg ccnnnnnnng gcgguaacag ggguucgaau 60

ccccuagggg acgc 74

<210> 254
<211> 75
<212> RNA
<213> Artificial sequence

<220>
<223> tRNA Asp1 body (-CA) (32-38 = n)

<220>
<221> misc_feature
<222> (33)..(39)
<223> n is a, c, g, or u

<400> 254
ggagcgguag uucagucggu uagaauaccu gcnnnnnnng caggggggucg cggguucgag 60

ucccguccgu uccgc 75

<210> 255
<211> 74
<212> RNA
<213> Artificial sequence

<220>
<223> tRNA AsnE2 body (-CA) (32-38 = n)

<220>
<221> misc_feature
<222> (32)..(38)
<223> n is a, c, g, or u

<400> 255
ggcucuguag uucagucggu agaacggcgg annnnnnnuc cguaugucac ugguucgagu 60

ccagucagag ccgc 74

<210> 256
<211> 74
<212> RNA
<213> Artificial sequence

<220>
<223> tRNA(Glu2+Leu2) body -CA

<220>
<221> misc_feature
<222> (35)..(37)
<223> n is a, c, g, or u

<400> 256
guccccuucg ucuagaggcc caggacaccg ccuunnngug gcgguaacag ggguucgaau 60

ccccuagggg acgc 74

<210> 257
<211> 74
<212> RNA
<213> Artificial sequence

<220>
<223> tRNA(Glu2+Pro3) body -CA

<220>
<221> misc_feature
<222> (35)..(37)
<223> n is a, c, g, or u

<400> 257
guccccuucg ucuagaggcc caggacaccg ccuunnngag gcgguaacag ggguucgaau 60

ccccuagggg acgc 74

<210> 258
<211> 74
<212> RNA
<213> Artificial sequence

<220>
<223> tRNA(Glu2+Pro2) body -CA

<220>
<221> misc_feature
<222> (35)..(37)
<223> n is a, c, g, or u

<400> 258
guccccuucg ucuagaggcc caggacaccg ccaunnngug gcgguaacag ggguucgaau 60

ccccuagggg acgc 74

<210> 259
<211> 74
<212> RNA

<213> Artificial sequence

<220>
<223> tRNA(Glu2+Ala2) body -CA

<220>
<221> misc_feature
<222> (35)..(37)
<223> n is a, c, g, or u

<400> 259
guccccuucg ucuagaggcc caggacaccg ccaunnnaug gcgguaacag ggguucgaau 60

ccccuagggg acgc 74

<210> 260
<211> 74
<212> RNA
<213> Artificial sequence

<220>
<223> tRNA(Glu2+Val2) body -CA

<220>
<221> misc_feature
<222> (35)..(37)
<223> n is a, c, g, or u

<400> 260
guccccuucg ucuagaggcc caggacaccg ccuunnnaug gcgguaacag ggguucgaau 60

ccccuagggg acgc 74

<210> 261
<211> 74
<212> RNA
<213> Artificial sequence

<220>
<223> tRNA(Glu2+Arg3) body -CA

<220>
<221> misc_feature
<222> (35)..(37)
<223> n is a, c, g, or u

<400> 261
guccccuucg ucuagaggcc caggacaccg cccunnngag gcgguaacag ggguucgaau 60

ccccuagggg acgc 74

<210> 262
<211> 75
<212> RNA
<213> Artificial sequence

<220>

<223>  tRNA(Asp1+Leu2) body -CA

<220>
<221>  misc_feature
<222>  (35)..(37)
<223>  n is a, c, g, or u

<400>  262
ggagcgguag uucagucggu uagaauaccu gcuunnngug cagggggucg cggguucgag    60

ucccguccgu uccgc    75

<210>  263
<211>  75
<212>  RNA
<213>  Artificial sequence

<220>
<223>  tRNA(Asp1+Pro3) body -CA

<220>
<221>  misc_feature
<222>  (35)..(37)
<223>  n is a, c, g, or u

<400>  263
ggagcgguag uucagucggu uagaauaccu gcuunnngag cagggggucg cggguucgag    60

ucccguccgu uccgc    75

<210>  264
<211>  75
<212>  RNA
<213>  Artificial sequence

<220>
<223>  tRNA(Asp1+Pro2) body -CA

<220>
<221>  misc_feature
<222>  (35)..(37)
<223>  n is a, c, g, or u

<400>  264
ggagcgguag uucagucggu uagaauaccu gcaunnngug cagggggucg cggguucgag    60

ucccguccgu uccgc    75

<210>  265
<211>  75
<212>  RNA
<213>  Artificial sequence

<220>
<223>  tRNA(Asp1+Ala2) body -CA

<220>
<221> misc_feature
<222> (35)..(37)
<223> n is a, c, g, or u

<400> 265
ggagcgguag uucagucggu uagaauaccu gcaunnnaug caggggggucg cggguucgag 60

ucccguccgu uccgc 75

<210> 266
<211> 75
<212> RNA
<213> Artificial sequence

<220>
<223> tRNA(Asp1+Val2) body -CA

<220>
<221> misc_feature
<222> (35)..(37)
<223> n is a, c, g, or u

<400> 266
ggagcgguag uucagucggu uagaauaccu gcuunnnaug caggggggucg cggguucgag 60

ucccguccgu uccgc 75

<210> 267
<211> 75
<212> RNA
<213> Artificial sequence

<220>
<223> tRNA(Asp1+Arg3) body -CA

<220>
<221> misc_feature
<222> (35)..(37)
<223> n is a, c, g, or u

<400> 267
ggagcgguag uucagucggu uagaauaccu gccunnngag caggggggucg cggguucgag 60

ucccguccgu uccgc 75

<210> 268
<211> 74
<212> RNA
<213> Artificial sequence

<220>
<223> tRNA(AsnE2+Leu2) body -CA

<220>
<221> misc_feature
<222> (34)..(36)

<223> n is a, c, g, or u

<400> 268
ggcucuguag uucagucggu agaacggcgg auunnnguuc cguaugucac ugguucgagu 60

ccagucagag ccgc 74

<210> 269
<211> 74
<212> RNA
<213> Artificial sequence

<220>
<223> tRNA(AsnE2+Pro3) body -CA

<220>
<221> misc_feature
<222> (34)..(36)
<223> n is a, c, g, or u

<400> 269
ggcucuguag uucagucggu agaacggcgg auunnngauc cguaugucac ugguucgagu 60

ccagucagag ccgc 74

<210> 270
<211> 74
<212> RNA
<213> Artificial sequence

<220>
<223> tRNA(AsnE2+Pro2) body -CA

<220>
<221> misc_feature
<222> (34)..(36)
<223> n is a, c, g, or u

<400> 270
ggcucuguag uucagucggu agaacggcgg aaunnnguuc cguaugucac ugguucgagu 60

ccagucagag ccgc 74

<210> 271
<211> 74
<212> RNA
<213> Artificial sequence

<220>
<223> tRNA(AsnE2+Ala2) body -CA

<220>
<221> misc_feature
<222> (34)..(36)
<223> n is a, c, g, or u

<400> 271

ggcucuguag uucagucggu agaacggcgg aaunnnauuc cguaugucac ugguucgagu 60

ccagucagag ccgc 74

<210> 272
<211> 74
<212> RNA
<213> Artificial sequence

<220>
<223> tRNA(AsnE2+Val2) body -CA

<220>
<221> misc_feature
<222> (34)..(36)
<223> n is a, c, g, or u

<400> 272
ggcucuguag uucagucggu agaacggcgg auunnnauuc cguaugucac ugguucgagu 60

ccagucagag ccgc 74

<210> 273
<211> 74
<212> RNA
<213> Artificial sequence

<220>
<223> tRNA(AsnE2+Arg3) body -CA

<220>
<221> misc_feature
<222> (34)..(36)
<223> n is a, c, g, or u

<400> 273
ggcucuguag uucagucggu agaacggcgg acunnngauc cguaugucac ugguucgagu 60

ccagucagag ccgc 74

<210> 274
<211> 76
<212> RNA
<213> Artificial sequence

<220>
<223> tRNA Ala1B

<400> 274
ggggcuauag cucagcuggg agagcgccug cuuugcacgc aggaggucug cgguucgauc 60

ccgcauagcu ccacca 76

<210> 275
<211> 76
<212> RNA
<213> Artificial sequence

<220>
<223> tRNA Ala2

<400> 275
ggggcuauag cucagcuggg agagcgcuug cauggcaugc aagaggucag cgguucgauc 60

ccgcuuagcu ccacca 76

<210> 276
<211> 77
<212> RNA
<213> Artificial sequence

<220>
<223> tRNA Arg2

<400> 276
gcauccguag cucagcugga uagaguacuc ggcuacgaac cgagcggucg gagguucgaa 60

uccucccgga ugcacca 77

<210> 277
<211> 77
<212> RNA
<213> Artificial sequence

<220>
<223> tRNA Arg3

<400> 277
gcgcccguag cucagcugga uagagcgcug cccuccggag gcagaggucu cagguucgaa 60

uccugucggg cgcgcca 77

<210> 278
<211> 77
<212> RNA
<213> Artificial sequence

<220>
<223> tRNA Arg4

<400> 278
gcgcccuuag cucaguugga uagagcaacg accuucuaag ucgugggccg cagguucgaa 60

uccugcaggg cgcgcca 77

<210> 279
<211> 75
<212> RNA
<213> Artificial sequence

<220>
<223> tRNA Arg5

<400> 279
guccucuuag uuaaauggau auaacgagcc ccuccuaagg gcuaauugca gguucgauuc 60

cugcagggga cacca 75

<210> 280
<211> 76
<212> RNA
<213> Artificial sequence

<220>
<223> tRNA Asn

<400> 280
uccucuguag uucagucggu agaacggcgg acuguuaauc cguaugucac ugguucgagu 60

ccagucagag gagcca 76

<210> 281
<211> 77
<212> RNA
<213> Artificial sequence

<220>
<223> tRNA Asp1

<400> 281
ggagcgguag uucagucggu uagaauaccu gccugucacg caggggcucg cggguucgag 60

ucccguccgu uccgcca 77

<210> 282
<211> 74
<212> RNA
<213> Artificial sequence

<220>
<223> tRNA Cys

<400> 282
ggcgcguuaa caaagcgguu auguagcgga uugcaaaucc gucuaguccg guucgacucc 60

ggaacgcgcc ucca 74

<210> 283
<211> 77
<212> RNA
<213> Artificial sequence

<220>
<223> tRNA fMet1

<400> 283
cgcggggugg agcagccugg uagcucgucg ggcucauaac ccgaaggucg ucgguucaaa 60

uccggccccc gcaacca 77

<210> 284
<211> 77
<212> RNA
<213> Artificial sequence

<220>
<223> tRNA fMet2

<400> 284
cgcggggugg agcagccugg uagcucgucg ggcucauaac ccgaagaucg ucgguucaaa 60

uccggccccc gcaacca 77

<210> 285
<211> 75
<212> RNA
<213> Artificial sequence

<220>
<223> tRNA Gln1

<400> 285
ugggguaucg ccaagcggua aggcaccggu uuuugauacc ggcauucccu gguucgaauc 60

cagguacccc agcca 75

<210> 286
<211> 75
<212> RNA
<213> Artificial sequence

<220>
<223> tRNA Gln2

<400> 286
uggguaucg ccaagcggua aggcaccgga uucugauucc ggcauuccga gguucgaauc 60

cucguacccc agcca 75

<210> 287
<211> 76
<212> RNA
<213> Artificial sequence

<220>
<223> tRNA Glu2

<400> 287
guccccuucg ucuagaggcc caggacaccg cccuuucacg gcgguaacag ggguucgaau 60

ccccuagggg acgcca 76

<210> 288
<211> 74
<212> RNA
<213> Artificial sequence

<220>
<223> tRNA Gly1

<400> 288
gcgggcguag uucaauggua gaacgagagc uucccaagcu cuauacgagg guucgauucc 60

cuucgcccgc ucca 74

<210> 289
<211> 75
<212> RNA
<213> Artificial sequence

<220>
<223> tRNA Gly2

<400> 289
gcgggcaucg uauaauggcu auuaccucag ccuuccaagc ugaugaugcg gguucgauuc 60

ccgcugcccg cucca 75

<210> 290
<211> 76
<212> RNA
<213> Artificial sequence

<220>
<223> tRNA Gly3

<400> 290
gcgggaauag cucaguuggu agagcacgac cuugccaagg ucggggucgc gaguucgagu 60

cucguuuccc gcucca 76

<210> 291
<211> 76
<212> RNA
<213> Artificial sequence

<220>
<223> tRNA His

<400> 291
guggcuauag cucaguuggu agagcccugg auugugauuc caguugucgu ggguucgaau 60

cccauuagcc acccca 76

<210> 292
<211> 77
<212> RNA
<213> Artificial sequence

<220>
<223> tRNA Ile1

<400> 292
aggcuuguag cucaggtggu uagagcgcac cccugauaag ggugaggucg gugguucaag 60

uccacucagg ccuacca 77

<210> 293
<211> 76
<212> RNA
<213> Artificial sequence

<220>
<223> tRNA Ile2

<400> 293
ggccccuuag cucagugguu agagcaggcg acucauaauc gcuuggucgc ugguucaagu 60

ccagcagggg ccacca 76

<210> 294
<211> 87
<212> RNA
<213> Artificial sequence

<220>
<223> tRNA Leu1

<400> 294
gcgaaggugg cggaauuggu agacgcgcua gcuucaggug uuaguguucu uacggacgug 60

gggguucaag ucccccccu cgcacca 87

<210> 295
<211> 87
<212> RNA
<213> Artificial sequence

<220>
<223> tRNA Leu2

<400> 295
gccgaggugg uggaauuggu agacacgcua ccuugaggug guagugccca auagggcuua 60

cggguucaag ucccguccuc gguacca 87

<210> 296
<211> 85
<212> RNA
<213> Artificial sequence

<220>
<223> tRNA Leu3

<400> 296
gcgggagugg cgaaauuggu agacgcacca gauuuagguu cuggcgccgc aaggugugcg 60

aguucaaguc ucgccucccg cacca 85

<210> 297
<211> 87
<212> RNA
<213> Artificial sequence

<220>
<223> tRNA Leu4

<400> 297
gcccggaugg uggaaucggu agacacaagg gauuuaaaau cccucggcgu ucgcgcugug 60

cggguucaag ucccgcuccg gguacca 87

<210> 298
<211> 85
<212> RNA
<213> Artificial sequence

<220>
<223> tRNA Leu5

<400> 298
gccgaagugg cgaaaucggu agacgcaguu gauucaaaau caaccguaga aauacgugcc 60

gguucgaguc cggccuucgg cacca 85

<210> 299
<211> 76
<212> RNA
<213> Artificial sequence

<220>
<223> tRNA Lys

<400> 299
gggucguuag cucaguuggu agagcaguug acuuuuaauc aauuggucgc agguucgaau 60

ccugcacgac ccacca 76

<210> 300
<211> 77
<212> RNA
<213> Artificial sequence

<220>
<223> tRNA Met

<400> 300
ggcuacguag cucaguuggu uagagcacau cacucauaau gaugggguca cagguucgaa 60

ucccgucgua gccacca 77

<210> 301
<211> 76
<212> RNA
<213> Artificial sequence

<220>
<223> tRNA Phe

<400> 301
gcccggauag cucagucggu agagcagggg auugaaaauc cccguguccu ugguucgauu 60

ccgaguccgg gcacca 76

<210> 302
<211> 77
<212> RNA
<213> Artificial sequence

<220>
<223> tRNA Pro1

<400> 302
cggugauugg cgcagccugg uagcgcacuu cguucgggac gaaggggucg gagguucgaa 60

uccucuauca ccgacca 77

<210> 303
<211> 77
<212> RNA
<213> Artificial sequence

<220>
<223> tRNA Pro2

<400> 303
cggcacguag cgcagccugg uagcgcaccg ucauggggug ucgggggucg gagguucaaa 60

uccucucgug ccgacca 77

<210> 304
<211> 77
<212> RNA
<213> Artificial sequence

<220>
<223> tRNA Pro3

<400> 304
cggcgaguag cgcagcuugg uagcgcaacu gguuugggac caguggucg gagguucgaa 60

uccucucucg ccgacca 77

<210> 305
<211> 95
<212> RNA
<213> Artificial sequence

<220>
<223> tRNA Sec

<400> 305
ggaagaucgu cgucuccggu gaggcggcug gacuucaaau ccaguugggg ccgccagcgg 60

ucccgggcag guucgacucc ugugaucuuc cgcca 95

<210> 306
<211> 88
<212> RNA
<213> Artificial sequence

<220>
<223> tRNA Ser1

<400> 306
ggaagugugg ccgagcgguu gaaggcaccg gucuugaaaa ccggcgaccc gaaaggguuc 60

cagaguucga aucucugcgc uuccgcca 88

<210> 307
<211> 90
<212> RNA
<213> Artificial sequence

<220>
<223> tRNA Ser2

<400> 307
ggagagaugc cggagcggcu gaacggaccg gucucgaaaa ccggaguagg ggcaacucua 60

ccggggguuc aaaucccccu cucuccgcca 90

<210> 308
<211> 93
<212> RNA
<213> Artificial sequence

<220>
<223> tRNA Ser3

<400> 308
ggugaggugg ccgagaggcu gaaggcgcuc cccugcuaag ggaguaugcg gucaaaagcu 60

gcauccgggg uucgaauccc cgccucaccg cca 93

<210> 309
<211> 88
<212> RNA
<213> Artificial sequence

<220>
<223> tRNA Ser5

<400> 309
ggugaggugu ccgaguggcu gaaggagcac gccuggaaag uguguauacg gcaacguauc 60

gggguucga auccccccu caccgcca 88

<210> 310
<211> 76
<212> RNA
<213> Artificial sequence

<220>
<223> tRNA Thr1

<400> 310
gcugauaugg cucaguuggu agagcgcacc cuugguaagg gugaggaccc caguucgacu 60

cuggguauca gcacca 76

<210> 311
<211> 76
<212> RNA
<213> Artificial sequence

<220>
<223> tRNA Thr2

<400> 311
gccgauauag cucaguuggu agagcagcgc auucguaaug cgaaggucgu agguucgacu 60

ccuauuaucg gcacca 76

<210> 312
<211> 76
<212> RNA
<213> Artificial sequence

<220>
<223> tRNA Thr3

<400> 312
gcugauauag cucaguuggu agagcgcacc cuugguaagg gugaggucgg caguucgaau 60

cugccuauca gcacca 76

<210> 313
<211> 76
<212> RNA
<213> Artificial sequence

<220>
<223> tRNA Thr4

<400> 313
gccgacuuag cucaguaggu agagcaacug acuuguaauc aguaggucac caguucgauu 60

ccgguagucg gcacca 76

<210> 314
<211> 76
<212> RNA
<213> Artificial sequence

<220>
<223> tRNA Trp

<400> 314
aggggcguag uucaauuggu agagcaccgg ucuccaaaac cggguguugg gaguucgagu 60

cucuccgccc cugcca 76

<210> 315
<211> 85
<212> RNA
<213> Artificial sequence

<220>
<223> tRNA Tyr1

<400> 315
gguggggguuc ccgagcggcc aaagggagca gacuguaaau cugccgucau cgacuucgaa 60

gguucgaauc cuucccccac cacca 85

<210> 316
<211> 85
<212> RNA
<213> Artificial sequence

<220>
<223> tRNA Tyr2

<400> 316
gguggguuc ccgagcggcc aaagggagca gacuguaaau cugccgucac agacuucgaa 60

gguucgaauc cuucccccac cacca 85

<210> 317
<211> 76
<212> RNA
<213> Artificial sequence

<220>
<223> tRNA Val1

<400> 317
gggugauuag cucagcuggg agagcaccuc ccuuacaagg agggggucgg cgguucgauc 60

ccgucaucac ccacca 76

<210> 318
<211> 77
<212> RNA
<213> Artificial sequence

<220>
<223> tRNA Val2A

<400> 318
gcguccguag cucaguuggu uagagcacca ccuugacaug gugggggucg gugguucgag 60

uccacucgga cgcacca 77

<210> 319
<211> 77
<212> RNA
<213> Artificial sequence

<220>
<223> tRNA Val2B

<400> 319
gcguucauag cucaguuggu uagagcacca ccuugacaug gugggggucg uugguucgag 60

uccaauugaa cgcacca 77

<210> 320
<211> 75
<212> RNA
<213> Artificial sequence

<220>
<223> tRNAPro1E2 body (-CA) (32-38 = n)

<220>
<221> misc_feature
<222> (33)..(39)
<223> n is a, c, g, or u

<400> 320
gggugauugg cgcagccugg uagcgcacuu cgnnnnnnnc gaagggguca gggguucgaa 60

uccccuauca cccgc 75

<210> 321
<211> 75
<212> RNA
<213> Artificial sequence

<220>
<223> tRNAPro1E1 body (-CA) (32-38 = n)

<220>
<221> misc_feature
<222> (33)..(39)
<223> n is a, c, g, or u

<400> 321
cggugauugg cgcagccugg uagcgcacuu cgnnnnnnnc gaagggguca gggguucgaa 60

uccccuauca ccgac 75

<210> 322
<211> 76
<212> RNA
<213> Artificial sequence

<220>
<223> tRNA Glu2 body

<220>
<221> misc_feature
<222> (35)..(37)
<223> n is a, c, g, or u

<400> 322
guccccuucg ucuagaggcc caggacaccg cccunnnacg gcgguaacag ggguucgaau 60

ccccuagggg acgcca 76

<210> 323
<211> 76
<212> RNA
<213> Artificial sequence

<220>
<223> tRNA AsnE2 body

<220>
<221> misc_feature
<222> (34)..(36)
<223> n is a, c, g, or u

<400> 323
ggcucuguag uucagucggu agaacggcgg acunnnaauc cguaugucac ugguucgagu 60

ccagucagag ccgcca 76

<210> 324
<211> 77
<212> RNA
<213> Artificial sequence

<220>
<223> tRNA Asp1 body

<220>
<221> misc_feature
<222> (35)..(37)
<223> n is a, c, g, or u

<400> 324
ggagcgguag uucagucggu uagaauaccu gccunnnacg cagggggucg cggguucgag 60

ucccguccgu uccgcca 77